# DEMANDE DE BREVET EUROPEEN

(11) **EP 1 950 221 A2**
(43) Date de publication de la demande: **30.07.2008**
(21) Numéro de dépôt: 08154762.2
(22) Date de dépôt: 14.08.1998
(51) Int. Cl.: C07K 14/35, A61K 39/04, C12Q 1/68, C07K 16/12, G01N 33/53, A61P 31/06

(54) **Séquences génomiques de polypeptides exportés de mycobactéries, vecteurs les comprenant et applications au diagnostic et à la prévention de la tuberculose**

(30) Priorité: 14.08.1997 FR 9710404; 11.09.1997 FR 9711325
(62) Demande divisionnaire de: 98942748.9
(71) Demandeur: INSTITUT PASTEUR, 75015 Paris (FR)
(72) Inventeur: Gicquel, Brigitte, 75014 Paris (FR); Portnoi, Denis, 75004 Paris (FR); Lim, Eng-Mong, 75015 Paris (FR); Pelicic, Vladimir, 75009 Paris (FR); Guigueno, Agnès, 62026 Arras (FR); Goguet de la Salmonière, Yves, 75015 Paris (FR)
(74) Mandataire: Warcoin, Jacques

(57) **Abrégé**

L'invention a pour objet de nouveaux vecteurs recombinants se réplicant chez les mycobactéries, un ensemble de séquences codant pour des polypeptides exportés détectés par des fusions avec la phosphatase alcaline, notamment un polypeptide, dénommé DP428, d'environ 12kD correspondant à une protéine exportée retrouvée dans les mycobactéries appartenant au complexe de *Mycobacterium tuberculosis.* L'invention concerne également des procédés et des kits de détection in vitro de la présence d'une mycobactérie et en particulier une mycobactérie appartenant au complexe de *Mycobacterium tuberculosis* dans un échantillon biologique utilisant lesdits polypeptides, leurs fragments ou des polynucléotides codant pour ces derniers. L'invention vise des compositions immunogènes ou vaccins pour la prévention et/ou le traitement d'infections provoquées par des mycobactéries et en particulier une mycobactérie appartenant audit complexe, en particulier la tuberculose.

## Description

L'invention a pour objet de nouveaux vecteurs recombinants de criblage, de clonage et/ou d'expression se répliquant chez les mycobactéries. Elle a également pour objet un ensemble de séquences codant pour des polypeptides exportés détectés par des fusions avec la phosphatase alcaline et dont l'expression est régulée (induite ou réprimée) ou constitutive lors de l'ingestion des mycobactéries par les macrophages. L'invention concerne également un polypeptide, dénommé DP428, d'environ 12kD correspondant à une protéine exportée retrouvée dans les mycobactéries appartenant au complexe de *Mycobacterium tuberculosis.* L'invention vise aussi un polynucléotide comprenant une séquence codant pour ce polypeptide. Elle concerne également l'utilisation du polypeptide ou de fragments de celui-ci et des polynucléotides codant pour ces derniers (ou encore les polynucléotides complémentaires à ces derniers) pour la réalisation de moyens de détection in vitro, ou in vivo de la présence d'une mycobactérie appartenant au complexe de *Mycobacterium tuberculosis* dans un échantillon biologique ou pour la détection de réactions de l'hôte infecté par ces espèces bactériennes. L'invention vise enfin l'utilisation du polypeptide ou de fragments de celui-ci ainsi que des polynucléotides codant pour ces derniers en tant que moyens destinés à la préparation d'une composition immunogène, susceptible d'induire une réponse immunitaire dirigée contre les mycobactéries appartenant au complexe de *Mycobacterium tuberculosis,* ou d'une composition vaccinale pour la prévention et/ou le traitement d'infections provoquées par des mycobactéries appartenant audit complexe, en particulier la tuberculose.

La présente invention a aussi pour but d'utiliser ces séquences (polypeptidiques et polynucléotidiques) comme cible pour la recherche de nouveaux inhibiteurs de la croissance et de la multiplication des mycobactéries et de leur maintien chez l'hôte, ses inhibiteurs pouvant servir d'antibiotiques.

Le genre Mycobacterium, qui comprend au moins 56 espèces différentes, inclut des pathogènes humains majeurs tels *que M. leprae* et *M. tuberculosis,* les agents responsables de la lèpre et de la tuberculose, qui restent des problèmes graves de santé publique dans le monde entier.

La tuberculose continue d'être un problème de santé publique dans le monde. Aujourd'hui, cette maladie est la cause de 2 à 3 millions de morts dans le monde et environ 8 millions de nouveaux cas sont observés chaque année (Bouvet, 1994). Dans les pays développés *M. tuberculosis* est la cause la plus commune des infections mycobactériennes. En France il apparaît environ 10 000 nouveaux cas par an et parmi les maladies à déclaration obligatoire c'est la tuberculose qui comprend le plus grand nombre de cas. La vaccination par le BCG (Bacille de Calmette et Guérin), une souche avirulente dérivée de *M. bovis* et qui est très utilisée comme vaccin contre la tuberculose, est loin d'être efficace au sein de toutes les populations. Cette efficacité varie environ de 80 % dans les pays occidentaux comme l'Angleterre, à 0 % en Inde (résultats du dernier essai de vaccination à Chingleput., publiés en 1972 dans Indian J. Med. Res.). De plus, l'apparition de souches de *M. tuberculosis* résistantes aux antituberculeux et le risque accru chez les patients immunodéprimés, patients atteints du SIDA, de développer une tuberculose, rendent nécessaire la mise au point de méthodes rapides, spécifiques et fiables pour le diagnostic de la tuberculose et la mise au point de nouveaux vaccins. Par exemple, une étude épidémiologique réalisée en Floride, et dont les résultats ont été publiés en 1993 dans AIDS thérapies, a montré que 10 % des malades atteints de SIDA sont atteints de tuberculose au moment du diagnostic du SIDA ou 18 mois avant celui-ci. Chez ces malades, la tuberculose apparaît dans 60 % des cas sous une forme disséminée donc non repérable par les critères de diagnostic classiques comme la radiographie pulmonaire ou l'analyse de crachats.

Actuellement, une certitude sur le diagnostic apporté par la mise en évidence de bacilles cultivables dans un prélèvement provenant du malade n'est obtenue que pour moins de la moitié des cas de tuberculose, même dans les cas de tuberculose pulmonaire. Le diagnostic de la tuberculose et des autres mycobactéries apparentées est donc difficile à réaliser, et cela pour différentes raisons : les mycobactéries sont souvent présentes en faible quantité, leur temps de génération est très long (24h pour *M*. *tuberculosis*) et leur culture est difficile (Bates et al., 1986).

D'autres techniques sont utilisables en clinique, pour identifier une infection mycobactérienne.
a) L'identification directe des microorganismes au microscope ; cette technique est rapide, mais ne permet pas l'identification de l'espèce mycobactérienne observée et manque de sensibilité (Bates, 1979).
   Les cultures, lorsqu'elles sont positives, ont une spécificité approchant 100 % et permettent l'identification de l'espèce mycobactérienne isolée ; néanmoins, comme précisé ci-dessus, la croissance des mycobactéries *in vitro* est longue (ne peut être réalisée qu'en 3 à 6 semaines de cultures répétées (Bates, 1979 ; Bates et al., 1986)) et coûteuse.
b) Les techniques sérologiques peuvent s'avérer utiles dans certaines conditions, mais leur utilisation est parfois limitée par leur sensibilité et/ou leur spécificité faibles (Daniel et al., 1987).
c) La présence de mycobactéries au sein d'un échantillon biologique peut aussi être déterminée par hybridation moléculaire avec de l'ADN ou de l'ARN en utilisant des sondes d'oligonucléotides spécifiques des séquences recherchées (Kiehn et al., 1987 ; Roberts et al., 1987 ; Drake et al., 1987). Plusieurs études ont montré l'intérêt de cette technique pour le diagnostic des infections à mycobactéries. Les sondes utilisées sont constituées d'ADN, d'ARN ribosomique ou de fragments d'ADN mycobactériens provenant de banque de gènes. Le principe de ces techniques repose sur le polymorphisme des séquences nucléotidiques des fragments utilisés ou sur le polymorphisme des régions avoisinantes. Dans tous les cas, elles nécessitent l'utilisation de cultures et ne sont pas applicables directement sur les échantillons biologiques.

La faible quantité de mycobactéries présentes au sein d'un échantillon biologique et en conséquence la quantité faible d'ADN cible à détecter dans cet échantillon peut nécessiter le recours à une amplification spécifique *in vitro* de l'ADN cible avant sa détection à l'aide de la sonde nucléotidique et en utilisant des techniques d'amplification *in vitro* telles que la PCR (amplification en chaîne à la polymérase. L'amplification spécifique de l'ADN par la technique PCR peut constituer la première étape d'un procédé de détection de la présence d'un ADN mycobactérien dans un échantillon biologique, la détection proprement dite de l'ADN amplifié étant effectuée dans un second temps à l'aide d'une sonde oligonucléotidique capable de s'hybrider spécifiquement à l'ADN amplifié.

Un test de détection de mycobactéries appartenant au complexe de *Mycobacterium tuberculosis,* par hybridation sandwich (test utilisant une sonde de capture et une sonde de détection) a été décrit par Chevrier et al. en 1993. Le complexe de *Mycobacterium tuberculosis* est un groupe de mycobactéries qui comprend *M. bovis-BCG, M. bovis, M. tuberculosis, M. africanum* et *M. microti.*

Un procédé de détection de faibles quantités de mycobactéries, appartenant au complexe tuberculosis, par amplification génique et hybridation directement sur des échantillons biologiques a été mis au point. Ledit procédé utilise la séquence d'insertion IS6*110* (Brevet européen EP 0 490 951 B1). Thierry et al. ont décrit en 1990 une séquence spécifique du complexe *Mycobacterium tuberculosis* et nommée IS 6110. Certains auteurs ont proposé d'amplifier spécifiquement l'ADN provenant de *Mycobacterium* en utilisant des amorces nucléiques dans une méthode d'amplification, telle que la réaction de polymérase en chaîne (PCR). Patel et al. ont décrit en 1990 l'utilisation de plusieurs amorces nucléiques choisies à partir d'une séquence connue en tant que sonde dans l'identification de *M. tuberculosis.* Cependant, la longueur des fragments obtenus en utilisant ces amorces était différente de la longueur théorique attendue et plusieurs fragments de taille variable étaient obtenus. De plus, les auteurs ont observé l'absence d'hybridation des produits amplifiés avec le plasmide ayant servi à déterminer les amorces. Ces résultats indiquent que ces amorces ne seraient pas appropriées dans la détection de la présence de *M. tuberculosis* dans un échantillon biologique et confirment la nature critique du choix des amorces. La même année, J.L. Guesdon et D. Thierry ont décrit une méthode de détection de *M. tuberculosis,* de grande sensibilité, par amplification d'un fragment d'ADN de *M. tuberculosis* localisé au sein de la séquence IS6110 (Brevet européen EP 0 461 045) à l'aide d'amorces générant des fragments d'ADN amplifié de longueur constante, même lorsque le choix des amorces conduisait à l'amplification de fragments longs (de l'ordre de 1000 à 1500 bases) où le risque d'interruption de la polymérisation est élevée en raison des effets de la structure secondaire de la séquence. D'autres amorces spécifiques de la séquence IS6110 sont décrites dans le brevet européen N° EP 0 490 951.

Les inventeurs ont montré (résultats non publiés) que certains isolats cliniques de *Mycobacterium tuberculosis* étaient exempts de la séquence d'insertion IS6110 et ne pouvaient donc être détectés à l'aide des oligonucléotides spécifiques de cette séquence pouvant conduire ainsi à des résultats de diagnostic faussement négatifs. Ces résultats confirment une observation similaire faite par Yuen et al. en 1993. L'impossibilité de détecter ces souches pathogènes potentiellement présentes dans un échantillon biologique prélevé sur un patient est ainsi susceptible de conduire à des difficultés voire des erreurs de diagnostic. La disponibilité de plusieurs séquences spécifiques du Bacille de la tuberculose, à l'intérieur desquelles des amorces appropriées pour l'amplification seront choisis, est importante. La séquence DP428 décrite ici pourra être utilisée.

*M. bovis et M. tuberculosis,* les agents causals de la tuberculose, sont des bactéries facultatives intra-cellulaires.

Ces agents ont développé des mécanismes pour assurer leur survie et leur réplication à l'intérieur du macrophage, un des types cellulaires qui est supposé éradiquer l'invasion par des microorganismes. Ces agents sont capables de moduler l'évolution normale de leur phagosome et de les empêcher de se différencier en un compartiment acide riche en hydrolase (Clemens, 1979 ; Clemens et al., 1996 ; Sturgill-Koszycki et al., 1994 et Xu et al., 1994). Cependant, cette modulation n'est possible que si la bactérie est vivante au sein du phagosome, suggérant que des composés synthétisés de manière active et/ou sécrétés à l'intérieur de la cellule font partie de ce mécanisme. Des protéines exportées sont probablement impliquées dans ce mécanisme. En dépit des problèmes majeurs de santé liés à ces organismes pathogènes, on sait peu de choses sur leurs protéines exportées et/ou sécrétées. Des analyses en SDS-PAGE de filtrat de culture de *M. tuberculosis* montrent au moins 30 protéines sécrétées (Altschul et al., 1990 ; Nagal et al., 1991 et Young et al., 1992). Certaines d'entre elles ont été caractérisées, leurs gènes clonés et séquencés (Borremans et al., 1989 ; Wiker et al., 1992 et Yamaguchi et al., 1989). D'autres, bien qu'il s'agisse d'antigènes immunodominants d'importance majeure pour induire une immunité protectrice (Anderson et al., 1991 et Orme et al., 1993), ne sont pas totalement identifiés. En outre, il est probable que de nombreuses protéines exportées restent fixées sur la membrane cellulaire et par conséquent ne soient pas présentes dans les surnageants de culture. Il a été montré que les protéines localisées à la surface externe de diverses bactéries pathogènes, telles que l'invasine de 103 kDa de *Yersina Pseudotuberculosis* (Isberg et al., 1987) ou l'internaline de 80 kDa de *Listeria monocytoqenes* (Gaillard et al., 1991 et Dramsi et al., 1997) jouent un rôle important dans les interactions avec les cellules hôtes et par conséquent, dans la pathogénicité comme dans l'induction de réponses protectrices. Ainsi, une protéine liée à la membrane pourrait être importante pour l'infection à *M. tuberculosis* comme pour l'induction de réponse protectrice contre cette infection. Ces protéines pourraient revêtir un intérêt certain pour la préparation de vaccins.

Récemment, il a été décrit l'adaptation aux mycobactéries d'une méthodologie génétique pour l'identification et la sélection phénotypique de protéines exportées (Lim et al., 1995). Cette méthode utilise la phosphatase alkaline (PhoA) périplasmique d'E. *coli.* Un vecteur plasmidique a été construit permettant la fusion de gènes entre un gène *PhoA* tronqué et des gènes codant pour des protéines exportées (Manoil et al., 1990).

Par cette méthode, il a pu être identifié un gène de *M. tuberculosis* (*erp* (Berthet et al., 1995)) présentant des homologies avec une protéine exportée de 28 kDa de *M. leprae,* qui est une cible fréquente des réponses humorales de la forme lépromateuse de la lèpre. Une protéine présentant des motifs aminoacides caractéristiques de la désaturase de plante (*des*) a aussi été caractérisée par la technique de fusion avec PhoA.

Cependant, cette méthode génétique d'identification de protéines exportées ne permet pas d'évaluer facilement l'expression intracellulaire des gènes correspondants. Une telle évaluation est d'une importance primordiale à la fois pour la sélection de bons candidats vaccins et pour la compréhension des interactions entre les bactéries et leurs cellules hôtes. L'induction de l'expression de facteur de virulence par contact de cellule cible pathogène a été décrite. C'est le cas par exemple pour les facteurs de virulence Yops (Petersson et al., 1996) de *Yersinia pseudotuberculosis. Shigella* par contact avec les cellules cibles relargue les protéines Ipa dans le milieu de culture, et *Salmonella* synthétise de nouvelles structures de surface.

Compte tenu de ce qui précède, il existe aujourd'hui un grand besoin de développer de nouveaux vaccins contre les mycobactéries pathogènes ainsi que de nouveaux tests de diagnostic spécifiques, fiables et rapides. Ces développements nécessitent la mise au point d'outils spécifiques encore plus performants permettant, d'une part, d'isoler ou d'obtenir des séquences de nouveaux polypeptides spécifiques, notamment immunogènes, et, d'autre part, de mieux comprendre le mécanisme des interactions entre les bactéries et leurs cellules hôtes comme notamment l'induction de l'expression de facteur de virulence. Ceci est précisément l'objet de la présente invention.

Les inventeurs ont défini et réalisé dans ce but de nouveaux vecteurs permettant le criblage, le clonage et/ou l'expression de séquences d'ADN de mycobactéries afin d'identifier parmi ces séquences, des acides nucléiques codant pour des protéines d'intérêt, de préférence des protéines exportées, pouvant être localisées sur la membrane bactérienne et/ou sécrétées, et d'identifier parmi ces séquences celles qui sont induites ou réprimées lors de l'infection (croissance intracellulaire).

### Description

La présente invention décrit l'utilisation du gène rapporteur *phoA* chez les mycobactéries. Il permet d'identifier des systèmes d'expression et d'exportation dans un contexte mycobactérien. Beaucoup de gènes ne sont exprimés que dans un tel contexte, ce qui montre l'avantage de la présente invention. Au cours du clonage de segments d'ADN de souches du complexe *M. Tuberculosis* en fusion avec *phoA* dans une autre mycobactérie comme *M. smegmatis,* le début du gène, ses régions régulatrices et son régulateur seront clonés ce qui permettra d'observer une régulation. Si cette régulation est positive, le clonage du régulateur constituera un avantage pour observer l'expression et l'exportation.

Dans le contexte de l'invention, on entend par mycobactérie toutes les mycobactéries appartenant aux diverses espèces énumérées par Wayne L. G. and Kubica G. P. (1980). Family Mycobacteriaceae in Bergey's manual of systematic bacteriology, J. P. Butler Ed. (Baltimore USA: Williams et Wilkins P. 1436-1457).

Dans certains cas les gènes clonés sont soumis dans leur hôte d'origine à une régulation négative rendant l'observation de l'expression et de l'exportation difficile chez l'hôte d'origine. Dans ce cas, le clonage du gène en absence de son régulateur négatif, dans un hôte ne le contenant pas, constituera un avantage.

L'invention vise aussi de nouveaux polypeptides et de nouveaux polynucléotides de mycobactéries ayant pu être isolés au moyen des vecteurs précédents et susceptibles d'entrer dans la réalisation de compositions pour la détection d'une infection par des mycobactéries, ou pour la protection contre une infection due à des mycobatéries ou pour la recherche d'inhibiteurs comme cela est décrit précédemment pour DP428.

L'invention a donc pour objet un vecteur recombinant de criblage, de clonage et/ou d'expression, caractérisé en ce qu'il se réplique chez des mycobactéries et en ce qu'il contient :
1) un réplicon fonctionnel chez les mycobactéries ;
2) un marqueur de sélection ;
3) une cassette rapporteur comprenant :
   a) un site de clonage multiple (polylinker),
   b) éventuellement un terminateur de transcription actif chez les mycobactéries, en amont du polylinker,
   c) une séquence nucléotidique codante issue d'un gène codant pour un marqueur d'expression, d'exportation et/ou de sécrétion de protéine, ladite séquence nucléotidique étant dépourvue de son codon d'initiation et de ses séquences de régulation, et
   d) une séquence nucléotidique codante issue d'un gène codant pour un marqueur d'activité de promoteurs contenus dans le même fragment, ladite séquence nucléotidique étant pourvue de son codon d'initiation. Eventuellement, le vecteur recombinant contient également un réplicon fonctionnel chez E. coli.

De manière préférée, le marqueur d'exportation et/ou de sécrétion est placé dans la même orientation que le marqueur d'activité de promoteurs.

Préférentiellement, le vecteur recombinant de criblage selon l'invention comprendra, en outre, un terminateur de transcription placé en aval du marqueur d'activité de promoteurs, ce qui est de nature à permettre l'obtention de transcrits courts qui se révèlent plus stables et qui, par conséquent, permettent un plus haut niveau d'expression des produits de traduction.

Le marqueur d'exportation et/ou de sécrétion est une séquence de nucléotides dont l'expression suivie de l'exportation et/ou de la sécrétion dépend des éléments de régulation qui contrôlent son expression.

Par « séquences ou éléments de régulation de l'expression de la production de polypeptides et de sa localisation », on entend une séquence promotrice de la transcription, une séquence comprenant le site de liaison au ribosome (RBS), les séquences responsables de l'exportation et/ou la sécrétion telles que la séquence dite séquence signal.

Un premier marqueur intéressant d'exportation et/ou d'expression est une séquence codante issue du gène *phoA.* Le cas échéant, elle est tronquée de telle façon que l'activité phosphatase alcaline est cependant susceptible d'être restaurée lorsque la séquence codante tronquée est placée sous le contrôle d'un promoteur et d'éléments de régulation appropriés.

D'autres marqueurs d'exposition, d'exportation et/ou de sécrétion peuvent être utilisés. On citera à titre d'exemples une séquence du gène β-agarase, de la nucléase d'un staphylocoque ou d'une β-lactamase.

Parmi les marqueurs intéressants d'activité de promoteurs contenus dans le même fragment, on préfère une séquence codante issue du gène *luc* de luciférase de luciole pourvue de son codon d'initiation.

D'autres marqueurs d'activité de promoteurs contenus dans le même fragment peuvent être utilisés. On citera à titre d'exemples une séquence du gène de la GFP (Green Fluorescent Protein).

Le terminateur de transcription doit être fonctionnel chez les mycobactéries. Un terminateur avantageux est à cet égard le terminateur du coliphage T4 (tT4). D'autres terminateurs appropriés pour la réalisation de l'invention peuvent être isolés en utilisant la technique présentée dans les exemples, par exemple au moyen d'une cassette « omega » (Prentki et al., 1984).

Un vecteur particulièrement préféré pour la réalisation de l'invention est un plasmide choisi parmi les plasmides suivants déposés à la CNCM (Collection Nationale de Cultures de Microorganismes, 25 rue de Docteur Roux, 75724 Paris cedex 15, France) :
a) pJVEDa déposé à la CNCM sous le N° I-1797, le 12 décembre 1996,
b) pJVEDb déposé à la CNCM sous le N° I-1906, le 25 juillet 1997,
c) pJVEDc déposé à la CNCM sous le N° I-1799, le 12 décembre 1996.

Pour la sélection, ou l'identification de séquences d'acides nucléiques de mycobactéries codant pour des polypeptides susceptibles d'être incorporés dans des compositions immunogènes, ou antigéniques pour la détection d'une infection, ou susceptibles d'induire ou de réprimer un facteur de virulence de mycobactéries, le vecteur de l'invention comprendra, en l'un des sites de clonage multiple du polylinker, une séquence de nucléotides d'une mycobactérie chez laquelle on détecte la présence de séquences correspondant à des polypeptides exportés et/ou sécrétés pouvant être induits ou réprimés lors de l'infection, ou encore exprimés ou produits de façon constitutive, leurs séquences promotrices et/ou régulatrices associées susceptibles de permettre ou de favoriser l'exportation et/ou la sécrétion desdits polypeptides d'intérêt, ou tout ou partie de gènes d'intérêt codant pour lesdits polypeptides.

De préférence, cette séquence est obtenue par fragmentation physique ou par digestion enzymatique de l'ADN génomique ou de l'ADN complémentaire d'un ARN d'une mycobactérie, de préférence *M. tuberculosis* ou choisie parmi *M. africanum, M. bovis, M. avium* ou *M. leprae.*

Les vecteurs de l'invention peuvent en effet également être utilisés pour déterminer la présence de séquences d'intérêt, de préférence correspondant à des protéines exportées et/ou sécrétées, et/ou capables d'être induites ou réprimées ou produites de façon constitutive lors de l'infection, notamment lors de la phagocytose par les macrophages, et selon ce qui a été exposé précédemment, chez des mycobactéries telles que *M. africanum, M. bovis, M. avium* ou *M. leprae* dont on aura traité l'ADN ou l'ADNc par fragmentation physique ou avec des enzymes déterminées.

Selon un premier mode de réalisation de l'invention la digestion enzymatique de l'ADN génomique ou de l'ADN complémentaire est effectuée à partir de *M*. *tuberculosis.*

De préférence cet ADN est digéré avec une enzyme telle que sau3A, BclI, BglII.

D'autres enzymes de digestion telles que ScaI, ApaI, SacII, KpnI ou encore des nucléases ou des polymérases, peuvent naturellement être mises en oeuvre, dès lors qu'elles permettent l'obtention de fragments dont les extrémités peuvent être insérées dans l'un des sites de clonage du polylinker du vecteur de l'invention.

Le cas échéant, des digestions avec différentes enzymes seront effectuées simultanément.

Des vecteurs recombinants préférés pour la réalisation de l'invention sont choisis parmi les vecteurs recombinants suivants déposés à la CNCM :
a) p6D7 déposé le 28 janvier 1997 à la CNCM sous le N° I-1814,
b) p5A3 déposé le 28 janvier 1997 à la CNCM sous le N° I-1815,
c) p5F6 déposé le 28 janvier 1997 à la CNCM sous le N° I-1816,
d) p2A29 déposé le 28 janvier 1997 à la CNCM sous le N° I-1817,
e) pDP428 déposé le 28 janvier 1997 à la CNCM sous le N° I-1818,
f) p5B5 déposé le 28 janvier 1997 à la CNCM sous le N° I-1819,
g) p1C7 déposé le 28 janvier 1997 à la CNCM sous le N° I-1820,
h) p2D7 déposé le 28 janvier 1997 à la CNCM sous le N° I-1821,
i) p1B7 déposé le 31 janvier 1997 à la CNCM sous le N° I-1843,
j) pJVED/*M*. *tuberculosis* déposé le 25 juillet 1997 à la CNCM sous le N° I-1907,
k) pM1C25 déposé le 4 août 1998 à la CNCM sous le N° I-2062.

Parmi les plus préférés, on préfère le vecteur recombinant pDP428 déposé le 28 janvier 1997 à la CNCM sous le N° I-1818, et le vecteur pM1C25 déposé le 4 août 1998 à la CNCM sous le N° I-2062.

L'invention a également pour objet un procédé de criblage de séquences de nucléotides issues de mycobactéries pour déterminer la présence de séquences correspondant à des polypeptides exportés et/ou sécrétés pouvant être induits ou réprimés lors de l'infection, leurs séquences promotrices et/ou régulatrices associées susceptibles notamment de permettre ou de favoriser l'exportation et/ou la sécrétion desdits polypeptides d'intérêt, ou tout ou partie de gènes d'intérêt codant pour lesdits polypeptides, caractérisé en ce qu'il met en oeuvre un vecteur recombinant selon l'invention.

L'invention concerne aussi un procédé de criblage, selon l'invention, caractérisé en ce qu'il comprend les étapes suivantes :
a) la fragmentation physique des séquences d'ADN de mycobactéries ou leur digestion par au moins une enzyme déterminée et la récupération des fragments obtenus ;
b) l'insertion des fragments obtenus à l'étape a) dans un site de clonage, compatible le cas échéant avec l'enzyme de l'étape a), du polylinker d'un vecteur selon l'invention ;
c) si besoin, l'amplification desdits fragments contenus dans le vecteur, par exemple par réplication de ce dernier après insertion du vecteur ainsi modifié dans une cellule déterminée, de préférence *E coli ;*
d) la transformation des cellules hôtes par le vecteur amplifié à l'étape c), ou en l'absence d'amplification, par le vecteur de l'étape b) ;
e) la culture des cellules hôtes transformées dans un milieu permettant la mise en évidence du marqueur d'exportation et/ou de sécrétion, et/ou du marqueur d'activité de promoteurs contenu dans le vecteur ;
f) la détection des cellules hôtes positives (colonies positives) pour l'expression du marqueur d'exportation et/ou de sécrétion, et/ou du marqueur d'activité de promoteurs ;
g) l'isolement de l'ADN des colonies positives et l'insertion de cet ADN dans une cellule identique à celle de l'étape c) ;
h) la sélection des insertions contenues dans le vecteur, permettant l'obtention de clones positifs pour le marqueur d'exportation et/ou de sécrétion, et/ou pour le marqueur d'activité de promoteurs ;
i) l'isolement et la caractérisation des fragments d'ADN de mycobactéries contenues dans ces insérats.

Dans l'un des modes de réalisation préférés du procédé de criblage selon l'invention, les cellules hôtes positives, détectées à l'étape f), pour le marqueur d'exportation et/ou de sécrétion sont, éventuellement dans un second temps, testées pour la capacité de l'insert nucléotidique sélectionné à stimuler l'expression du marqueur d'activité de promoteurs lorsque lesdites cellules hôtes sont phagocytées par des cellules du type macrophagique.

De manière plus spécifique, on compare la stimulation de l'expression du marqueur d'activité de promoteurs chez des cellules hôtes placées en culture axénique (cellules hôtes seules en culture) à la stimulation de l'expression du marqueur d'activité de promoteurs chez des cellules hôtes cultivées en présence de macrophages et ainsi phagocytées par ces derniers.

La sélection de cellules hôtes positives pour le marqueur d'activité de promoteurs peut être réalisée dès l'étape e) du procédé de criblage décrit ci-dessus, ou encore après l'une quelconque des étapes f), g), h) ou i), c'est-à-dire une fois que les cellules hôtes ont été sélectionnées positivement pour le marqueur d'exportation et/ou de sélection.

La mise en oeuvre de ce procédé permet la construction de banques d'ADN comportant des séquences correspondant à des polypeptides susceptibles d'être exportés et/ou sécrétés, et/ou susceptibles d'être induits ou réprimés lors de l'infection lorsqu'ils sont produits au sein de mycobactéries recombinantes. L'étape i) du procédé peut comprendre une étape de séquençage des insertions sélectionnées.

De préférence, dans le procédé selon l'invention, le vecteur utilisé est choisi parmi les plasmides pJVEDa (CNCM, N° I-1797), pJVEDb (CNCM, N° I-1906), pJVEDc (CNCM, N° I-1799) ou pJVED/M. *tuberculosis* (CNCM, N° I-1907), et la digestion des séquences d'ADN de mycobactéries est effectuée au moyen de l'enzyme Sau3A.

Selon un mode de réalisation préféré de l'invention, le procédé de criblage est caractérisé en ce que les séquences de mycobactéries sont issues d'une mycobactérie pathogène, par exemple de *M. tuberculosis, M. bovis, M. avium, M. africanum* ou *M*. *leprae.*

L'invention comprend également une banque d'ADN génomique ou d'ADNc complémentaire d'ARNm de mycobactérie, caractérisée en ce qu'elle est obtenue par un procédé comprenant les étapes a) et b), ou a), b) et c) du procédé précédent selon l'invention, de préférence une banque d'ADN génomique ou d'ADNc complémentaire d'ARNm de mycobactéries pathogènes, de préférence de mycobactéries appartenant au groupe du complexe *Mycobacterium tuberculosis,* de préférence de *Mycobacterium tuberculosis.*

Dans la présente invention, on entend désigner par « séquences nucléiques » ou « séquences d'acides aminés » SEQ ID N° X à SEQ ID N° Y, où X et Y peuvent représenter indépendamment un nombre ou un caractère alphanumérique, respectivement l'ensemble des séquences nucléiques ou l'ensemble des séquences d'acides aminés représentées par les figures X à Y, extrémités comprises.

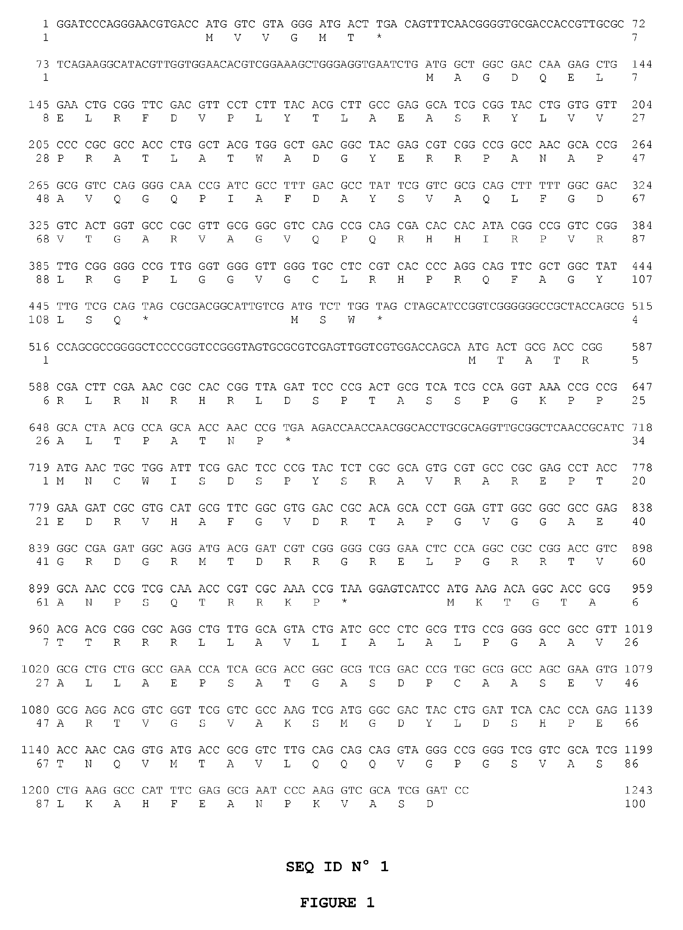

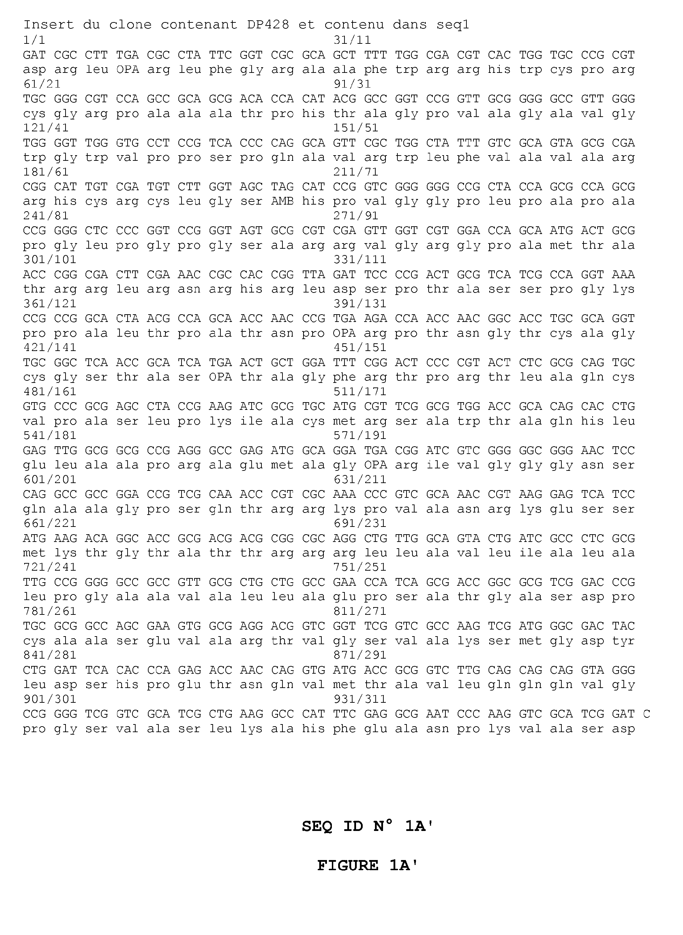

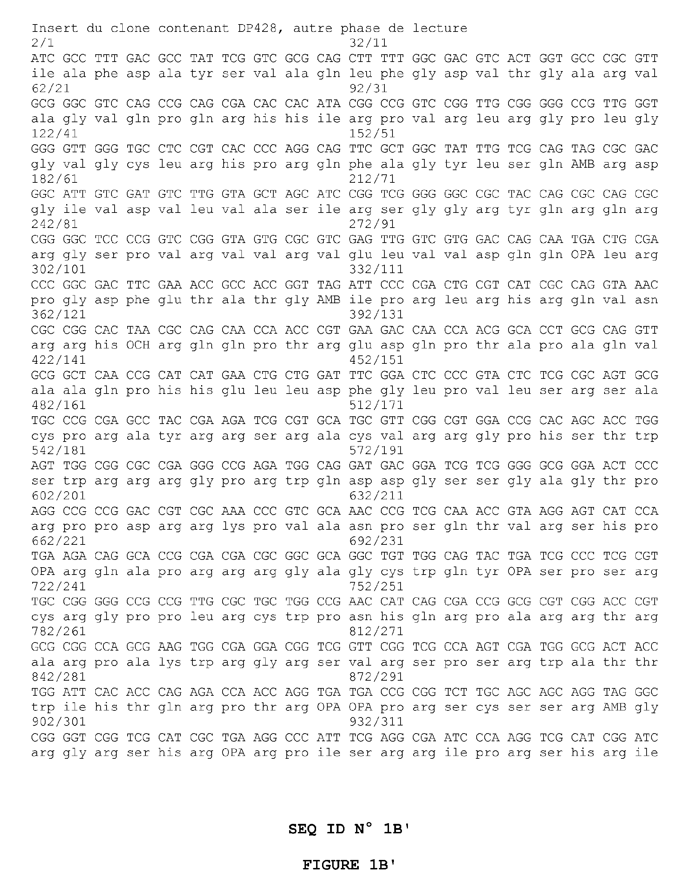

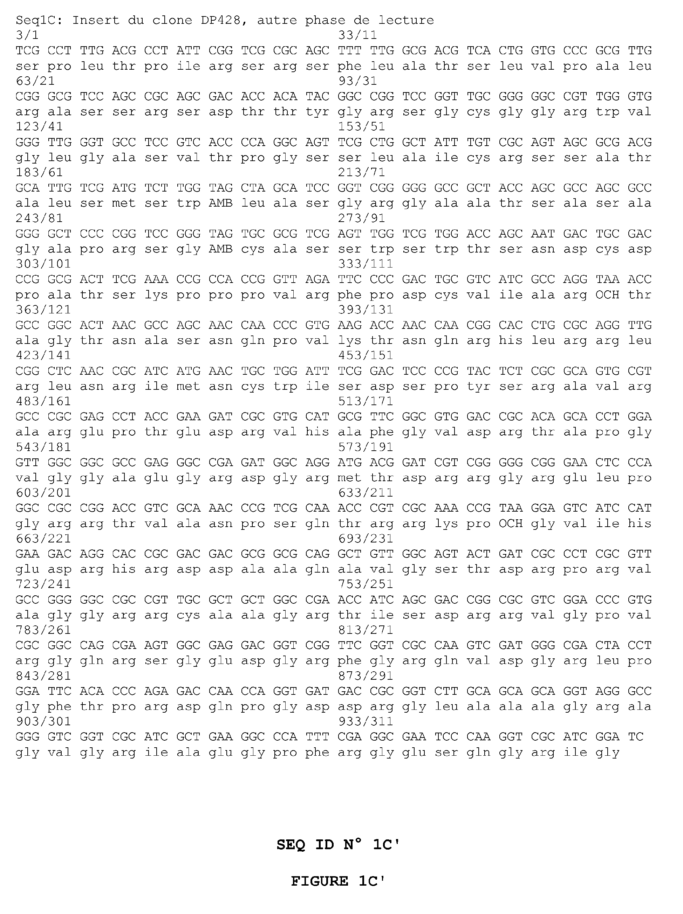

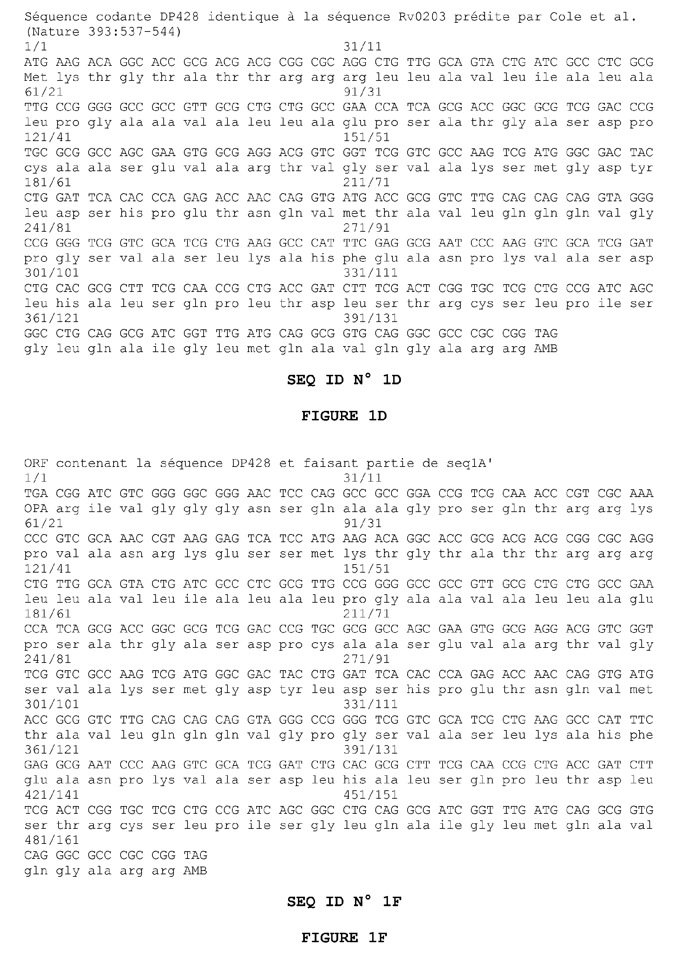

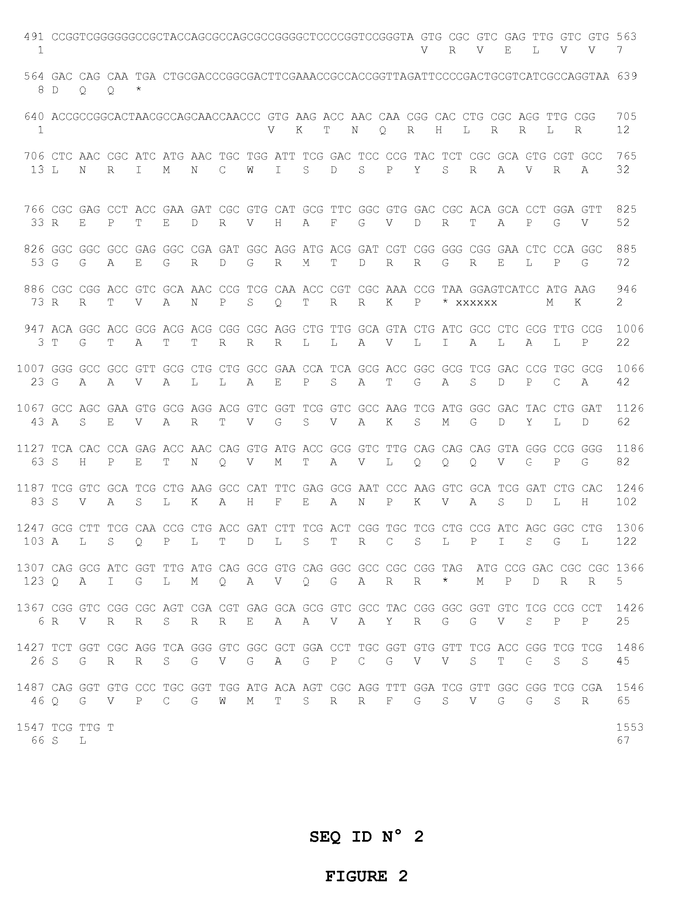

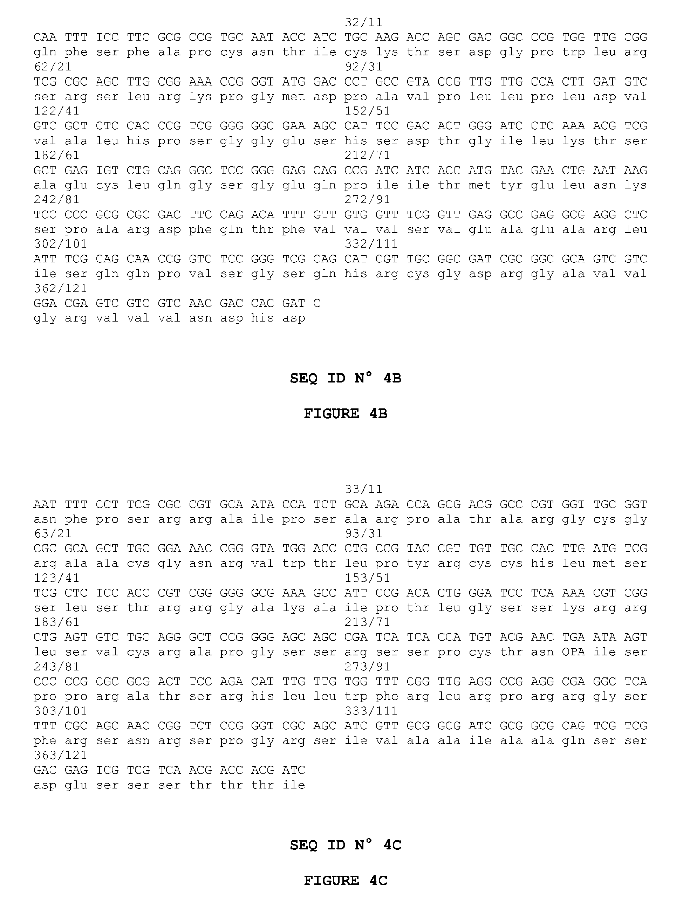

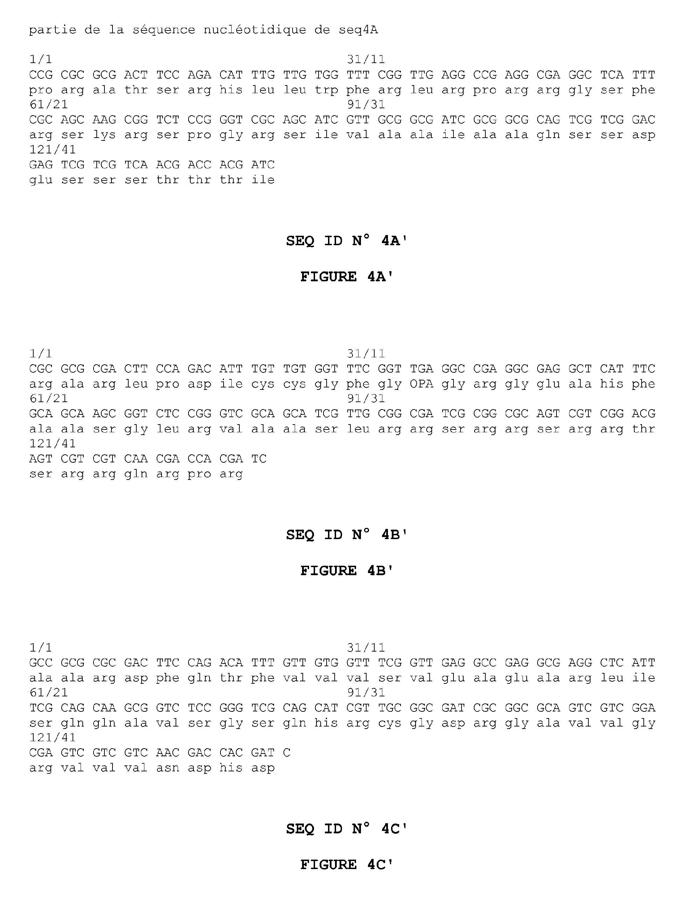

Par exemple, les séquences nucléiques ou les séquences d'acides aminés SEQ ID N° 1 à SEQ ID N° 4N sont respectivement les séquences nucléiques ou les séquences d'acides aminés représentées par les figures 1 à 4N, c'est-à-dire respectivement les séquences nucléiques ou les séquences d'acides aminés SEQ ID N° 1, SEQ ID N° 1A', SEQ ID N° 1B', SEQ ID N° 1C', SEQ ID N° 1D, SEQ ID N° 1F, SEQ ID N° 2, SEQ ID N° 3A, SEQ ID N° 3B, SEQ ID N° 3C, SEQ ID N° 4A, SEQ ID N° 4B, SEQ ID N° 4C, SEQ ID N° 4A', SEQ ID N° 4B', SEQ ID N° 4C', SEQ ID N° 4F, SEQ ID N° 4J, SEQ ID N° 4K, SEQ ID N° 4L, SEQ ID N° 4M et SEQ ID N° 4N.

L'invention a également pour objet les séquences nucléotidiques de mycobactéries ou comprenant des séquences nucléotidiques de mycobactéries sélectionnées après la réalisation du procédé selon l'invention ci-dessus décrit.

De préférence, ladite mycobactérie est choisie parmi *M. tuberculosis, M. bovis, M. africanum, M. avium, M. leprae, M. paratuberculosis, M. kansassi* ou *M. xénopi.*

On préfère les séquences nucléotidiques de mycobactéries ou comprenant une séquence nucléotidique de mycobactérie, ladite séquence nucléotidique de mycobactérie étant choisie parmi les séquences de fragments d'ADN de mycobactérie de séquence nucléique SEQ ID N° 1 à SEQ ID N° 24C, SEQ ID N° 27A à SEQ ID N° 27C, SEQ ID N° 29 et SEQ ID N° 31A à SEQ ID N° 50F, respectivement représentées par les figures 1 à 24C (planches 1 à 150), par les figures 27A à 27C (planches 152 à 154), par la figure 29 (planche 156) et par les figures 31A à 50F (planches 158 à 275).

Selon un mode de réalisation particulier de l'invention, des séquences préférées sont par exemple les fragments d'ADN de mycobactéries de séquence SEQ ID N° 1, SEQ ID N° 3A, SEQ ID N° 5A, SEQ ID N° 6A, SEQ ID N° 7A, SEQ ID N° 8A, SEQ ID N° 9A, SEQ ID N° 10A, SEQ ID N° 27A ou SEQ ID N° 29 contenus respectivement dans les vecteurs pDP428 (CNCM, N° 1-1818), p6D7 (CNCM, N° I-1814), p5F6 (CNCM, N° I-1816), p2A29 (CNCM, N° I-1817), p5B5 (CNCM, N° I-1819), p1C7 (CNCM, N° I-1820), p2D7 (CNCM,N° I-1821), p1B7(CNCM, N° I-1843), p5A3 (CNCM, N° I-1815) et pM1C25 (CNCM, N° I-2062).

L'invention concerne également un acide nucléique comprenant la totalité de la phase de lecture ouverte d'une des séquences nucléotidiques selon l'invention, notamment une des séquences SEQ ID N° 1 à SEQ ID N° 24C, SEQ ID N° 27A à SEQ ID N° 27C, SEQ ID N° 29 et SEQ ID N° 31A à SEQ ID N° 50F selon l'invention. Ledit acide nucléique peut être isolé par exemple de la façon suivante :
a) préparation d'une banque de cosmides à partir de l'ADN de *M. tuberculosis,* par exemple selon la technique décrite par Jacobs et al., 1991 ;
b) hybridation de tout ou partie d'un acide nucléique sonde de séquence choisie par exemple parmi SEQ ID N° 1 à SEQ ID N° 24C, SEQ ID N° 27A à SEQ ID N° 27C, SEQ ID N° 29 et SEQ ID N° 31A à SEQ ID N° 50F avec les cosmides de la banque préalablement préparée à l'étape a) ;
c) sélection des cosmides hybridant avec l'acide nucléique sonde de l'étape b) ;
d) séquençage des inserts d'ADN des clones sélectionnés à l'étape c) et identification du cadre de lecture ouvert complet ;
e) le cas échéant, clonage des inserts séquencés à l'étape d) dans un vecteur d'expression et/ou de clonage approprié.

Les acides nucléiques comprenant la totalité du cadre de lecture ouvert des séquences SEQ ID N° 1 à SEQ ID N° 24C, SEQ ID N° 27A à SEQ ID N° 27C, SEQ ID N° 29 et SEQ ID N° 31A à SEQ ID N° 50F sont parmi les acides nucléiques préférés.

La présente invention permet de déterminer un fragment de gène codant pour un polypeptide exporté. La comparaison avec la séquence du génome publiée par Cole et al. (Cole et al., 1998, Nature, 393, 537-544) permet de déterminer le gène en entier portant la séquence identifiée selon la présente invention.

Par séquence nucléotidique comprenant la totalité du cadre ouvert de lecture d'une séquence selon l'invention, on entend la séquence nucléotidique (génomique, ADNc, semi-synthétique ou synthétique) comprenant l'une des séquences selon l'invention et s'étendant d'une part en 5' de ces séquences jusqu'au premier codon d'initiation de la traduction (ATG ou GTG) ou même jusqu'au premier codon stop, et d'autre part en 3' de ces séquences jusqu'au codon stop suivant, et ceci dans l'une quelconque des trois phases de lecture possibles.

Les séquences nucléotidiques complémentaires des séquences ci-dessus selon 14invention font également partie de l'invention.

Par polynucléotide de séquence complémentaire d'une séquence nucléotidique selon l'invention, on entend toute séquence d'ADN ou d'ARN dont les nucléotides sont complémentaires de ceux de ladite séquence selon l'invention et dont l'orientation est inversée.

Les fragments nucléotidiques des séquences ci-dessus selon 14invention notamment utiles en tant que sondes ou amorces font également partie de l'invention.

L'invention concerne aussi les polynucléotides caractérisés en ce qu'ils comprennent un polynucléotide choisi parmi :
a) un polynucléotide dont la séquence est complémentaire de la séquence d'un polynucléotide selon l'invention,
b) un polynucléotide dont la séquence comporte au moins 50 % d'identité avec un polynucléotide selon l'invention,
c) un polynucléotide hybridant dans des conditions de forte stringence avec une séquence de polynucléotide selon l'invention,
d) un fragment d'au moins 8 nucléotides consécutifs d'un polynucléotide défini selon l'invention.

Les conditions de forte stringence ainsi que le pourcentage d'identité seront définis ci-après dans la présente description.

Lorsque la séquence codante issue du gène marqueur d'exportation et/ou de sécrétion est une séquence issue du gène *phoA,* l'exportation et/ou la sécrétion du produit du gène *phoA,* le cas échéant tronqué, n'est obtenue que lorsque cette séquence est insérée en phase avec la séquence ou élément de régulation de l'expression de la production de polynucléotides et sa localisation placée en amont, qui contient les éléments contrôlant l'expression, l'exportation et/ou la sécrétion issus de séquence de mycobactéries.

Les vecteurs recombinants de l'invention peuvent bien entendu comprendre des sites de clonage multiples décalés de un ou deux nucléotides par rapport à un vecteur selon l'invention, permettant ainsi d'exprimer le polypeptide correspondant au fragment d'ADN de mycobactérie inséré et susceptible d'être traduit selon l'un des trois cadres de lecture possibles.

Par exemple les vecteurs préférés pJVEDb et pJVEDc de l'invention se distinguent du vecteur préféré pJVEDa par un décalage respectif de un et de deux nucléotides au niveau du site de clonage multiple.

Ainsi, les vecteurs de l'invention sont capables d'exprimer chacun des polypeptides susceptibles d'être codés par un fragment d'ADN de mycobactérie inséré. Cesdits polypeptides, caractérisés en ce qu'ils sont donc susceptibles d'être exportés et/ou sécrétés, et/ou induits ou réprimés, ou exprimés de façon constitutive lors de l'infection, font partie de l'invention.

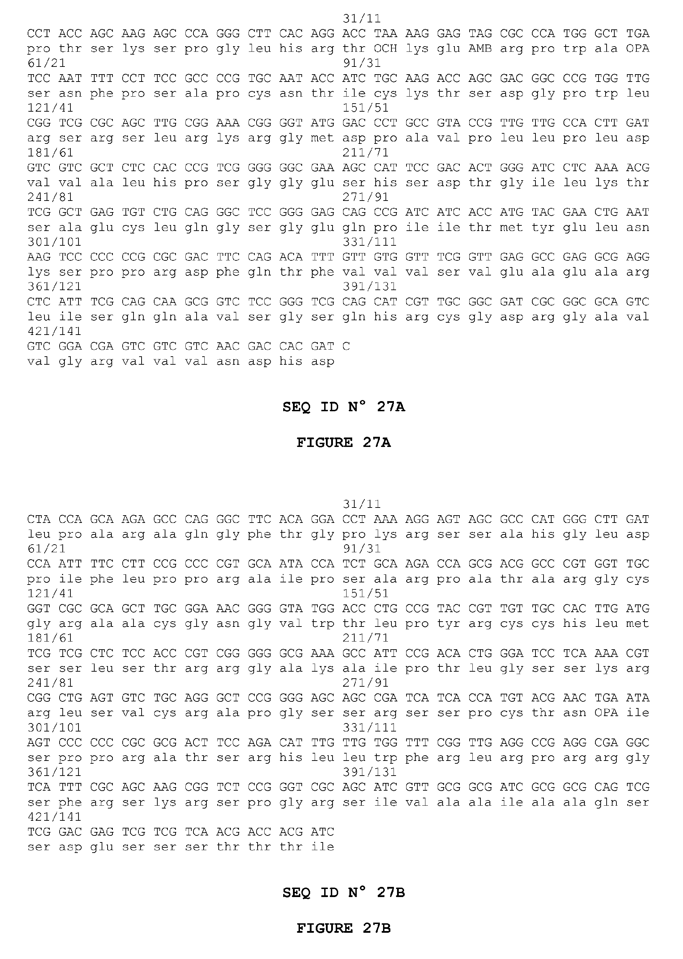

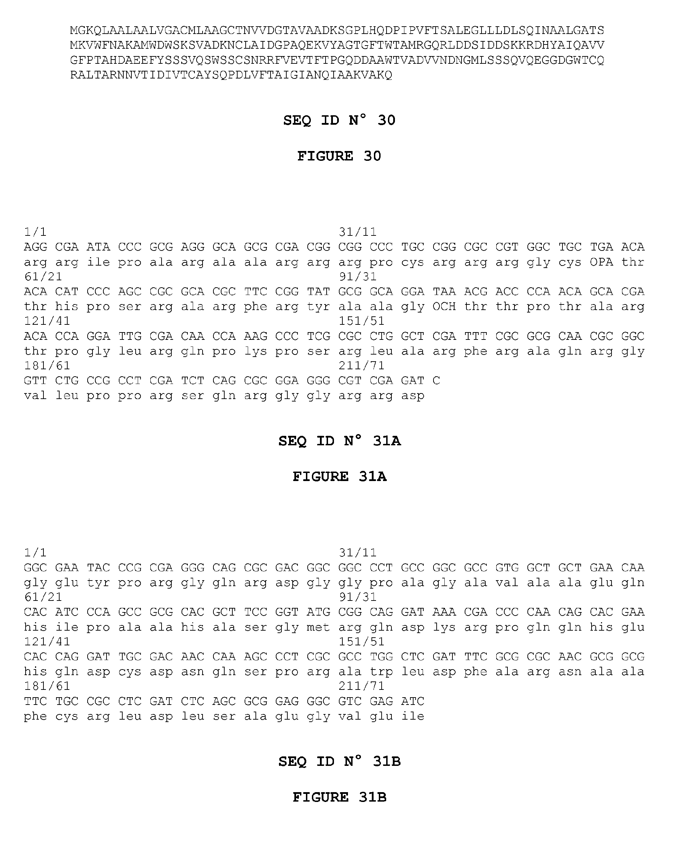

On préfère notamment les polypeptides de l'invention dont les séquences d'acides aminés sont choisies parmi les séquences d'acides aminés SEQ ID N° 1 à SEQ ID N° 24C, SEQ ID N° 27A à SEQ ID N° 28 et SEQ ID N° 30 à SEQ ID N° 50F et représentées respectivement par les figures 1 à 24C (planches 1 à 150), les figures 27A à 28 (planches 152 à 155) et les figures 30 à 50F (planches 157 à 275).

Font également partie de l'invention, les fragments ou fragments biologiquement actifs ainsi que les polypeptides homologues desdits polypeptides. Fragment, fragment biologiquement actif et polypeptides homologues de polypeptide, étant tels que définis ci-après dans la description.

L'invention concerne également les polypeptides comprenant un polypeptide ou un de leurs fragments selon l'invention.

L'invention a aussi pour objet des mycobactéries recombinantes contenant un vecteur recombinant selon l'invention décrit précédemment. Une mycobactérie préférée est une mycobactérie du type *M. smeqmatis.*

*M. smeqmatis* permet avantageusement de tester l'efficacité de séquences de mycobactéries, pour le contrôle de l'expression, de l'exportation et/ou de la sécrétion, et/ou de l'activité de promoteurs d'une séquence donnée, par exemple d'une séquence codant pour un marqueur tel que la phosphatase alcaline et/ou la luciférase.

Une autre mycobactérie préférée est une mycobactérie du type *M. bovis,* par exemple la souche BCG utilisée actuellement pour la vaccination contre la tuberculose.

Une autre mycobactérie préférée est une souche de *M. tuberculosis, M. bovis* ou *M. africanum* possédant potentiellement tous les systèmes de régulation appropriés.

Les inventeurs ont ainsi caractérisé en particulier un polynucléotide constitué par une séquence de nucléotides présente chez toutes les souches testées de mycobactéries appartenant au complexe de *Mycobacterium tuberculosis.* Ce polynucléotide, dénommé *DP428* contient un cadre ouvert de lecture (ORF) codant pour un polypeptide d'environ 12 kD. Le cadre de lecture ouvert (ORF) codant pour le polypeptide DP428 s'étend du nucléotide en position nt 941 au nucléotide en position nt 1351 de la séquence SEQ ID N° 2, le polypeptide DP428 ayant la séquence en acides aminés SEQ ID N° 28 suivante :

MKTGTATTRRRLLAVLIALALPGAAVALLAEPSATGASDPCAASEVARTVGSV
AKSMGDYLDSHPETNQVMTAVLQQQVGPGSVASLKAHFEANPKVASDLHALS
QPLTDLSTRCSLPISGLQAIGLMQAVQGARR.

Ce poids moléculaire (PM) correspond au PM théorique de la protéine mature obtenue après clivage de la séquence signale, le PM de la protéine ou polypeptide DP428 étant d'environ 10 kD après ancrage potentiel au peptidoglycane et coupure potentielle entre S et G du motif LPISG.

Ce polynucléotide inclut, d'une part, un cadre ouvert de lecture correspondant à un gène de structure et, d'autre part, les signaux de régulation de l'expression de la séquence codante en amont et en aval de cette dernière. Le polypeptide DP428 est composé d'un peptide signal, d'une région centrale hydrophile et d'une région C-terminale hydrophobe. Cette dernière se termine par deux résidus arginines (R), signal de rétention, et est précédé par un motif LPISG qui rappelle le motif LPXTG d'ancrage au peptidoglycane (Schneewind et al., 1995).

Par gène de structure aux fins de la présente invention, on entend un polynucléotide codant pour une protéine, un polypeptide ou encore un fragment de ces derniers, ledit polynucléotide ne comprenant que la séquence correspondant au cadre ouvert de lecture (ORF), ce qui exclut les séquences du côté 5' du cadre ouvert de lecture (ORF) qui dirigent l'initiation de la transcription.

Ainsi, l'invention concerne en particulier un polynucléotide dont la séquence est choisie parmi les séquences nucléotidiques SEQ ID N° 1 à SEQ ID N° 2.

Plus particulièrement, l'invention concerne un polynucléotide caractérisé en ce qu'il comprend un polynucléotide choisi parmi :
a) un polynucléotide dont la séquence est choisie parmi les séquences nucléotidiques SEQ ID N° 1 à SEQ ID N° 2,
b) un polynucléotide dont la séquence nucléique est la séquence comprise entre le nucléotide en position nt 964 et le nucléotide en position nt 1234, extrémités incluses, de la séquence SEQ ID N° 1,
c) un polynucléotide dont la séquence est complémentaire de la séquence d'un polynucléotide défini en a) ou b),
d) un polynucléotide dont la séquence comporte au moins 50 % d'identité avec un polynucléotide défini en a), b) ou c),
e) un polynucléotide hybridant dans des conditions de forte stringence avec une séquence de polynucléotide défini en a), b), c) ou d),
f) un fragment d'au moins 8 nucléotides consécutifs d'un polynucléotide défini en a), b), c), d) ou e).

On entend par séquence nucléotidique, polynucléotide ou acide nucléique, selon la présente invention, aussi bien un ADN double brin, un ADN simple brin que des produits de transcription desdits ADN.

Par pourcentage d'identité au sens de la présente invention, on entend un pourcentage d'identité entre les bases de deux polynucléotides, ce pourcentage étant purement statistique et les différences entre les deux polynucléotides étant réparties au hasard et sur toute leur longueur.

Une hybridation dans des conditions de forte stringence signifie que les conditions de température et de force ionique sont choisies de telle manière qu'elles permettent le maintien de l'hybridation entre deux fragments d'ADN complémentaires.

A titre illustratif, des conditions de forte stringence de l'étape d'hybridation aux fins de définir les fragments polynucléotidiques décrits ci-dessus, sont avantageusement les suivantes.

L'hybridation est réalisée à une température préférentielle de 65°C, en présence de tampon commercialisé sous le nom de rapid-hyb buffer par Amersham (RPN 1636) et 100 µg/ml d'ADN de E.coli.

Les étapes de lavage peuvent, par exemple, être les suivantes :
- deux lavages de 10 min, préférentiellement à 65°C, dans un tampon 2 x SSC et 0,1% SDS ;
- deux lavages de 10 min, préférentiellement à 65°C, dans un tampon 1 x SSC et 0,1 % SDS ;
- un lavage de 10 min, préférentiellement à 65°C, dans un tampon de 0,1 x SSC et 0,1 % SDS.

1 x SSC correspond à 0,15 M NaCl et 0,05M citrate de Na et une solution de 1 x Denhardt correspond à 0,02% Ficoll, 0,02% de polyvinylpyrrolidone et 0,02% de sérum albumine bovine.

Avantageusement, un fragment nucléotidique répondant à la définition précédente aura au moins 8 nucléotides, de préférence au moins 12 nucléotides, et encore plus préférentiellement au moins 20 nucléotides consécutifs de la séquence dont il est issu. Les conditions d'hybridation de forte stringence décrites ci-avant pour un polynucléotide d'une taille d'environ 200 bases, seront adaptées par l'homme du métier pour des oligonucléotides de taille plus grande ou plus petite, selon l'enseignement de Sambrook et al., 1989.

Pour les conditions de mise en oeuvre des enzymes de restriction dans le but d'obtenir des fragments nucléotidiques des polynucléotides selon l'invention, on se référera avantageusement à l'ouvrage de Sambrook et al., 1989.

Avantageusement, un polynucléotide de l'invention contiendra au moins une séquence comprenant l'enchaînement de nucléotides allant du nucléotide en position nt 964 au nucléotide nt 1234 du polynucléotide de séquence SEQ ID N° 1.

La présente invention a pour objet un polynucléotide selon l'invention, caractérisé en ce que sa séquence nucléique hybride avec l'ADN de séquence de mycobactéries et préférentiellement avec l'ADN de séquence de mycobactéries appartenant au complexe de *Mycobacterium tuberculosis.*

Le polynucléotide est codé par une séquence polynucléotidique telle que décrite supra.

La présente invention a également pour objet un polypeptide issu d'une mycobactérie, caractérisé en ce qu'il est présent uniquement chez les mycobactéries appartenant au complexe de *Mycobacterium tuberculosis.*

L'invention concerne également un polypeptide caractérisé en ce qu'il comprend un polypeptide choisi parmi :
a) un polypeptide dont la séquence d'acides aminés est comprise dans une séquence d'acides aminés choisie parmi les séquences d'acides aminés SEQ ID N° 1 à SEQ ID N° 24C, SEQ ID N° 27A à SEQ ID N° 28 et SEQ ID N° 30 à SEQ ID N° 50F,
b) un polypeptide homologue au polypeptide défini en a),
c) un fragment d'au moins 5 acides aminés d'un polypeptide défini en a) ou b),
d) un fragment biologiquement actif d'un polypeptide défini en a), b), ou c).

La présente invention a aussi pour objet un polypeptide dont la séquence d'acides aminés est comprise dans la séquence d'acides aminés SEQ ID N° 1 ou SEQ ID N° 2, ou un polypeptide de séquence d'acides aminés SEQ ID N° 28.

Par polypeptide homologue, on entendra désigner les polypeptides présentant, par rapport au polypeptide naturel selon l'invention tel que le polypeptide DP428, certaines modifications comme en particulier une délétion, addition ou substitution d'au moins un acide aminé, une troncation, un allongement, une fusion chimérique, et/ou une mutation. Parmi les polypeptides homologues, on préfère ceux dont la séquence d'acides aminés présente au moins 30 %, de préférence 50 %, d'homologie avec les séquences d'acides aminés des polypeptides selon l'invention. Dans le cas d'une substitution, un ou plusieurs acides aminés consécutifs ou non consécutifs, sont remplacés par des acides aminés « équivalents ». L'expression acide aminé « équivalent » vise ici à désigner tout acide aminé susceptible d'être substitué à l'un des acides aminés de la structure de base sans cependant modifier essentiellement les propriétés immunogènes des peptides correspondants. En d'autres termes, les acides aminés équivalents seront ceux qui permettent l'obtention d'un polypeptide de séquence modifiée qui permet l'induction *in vivo* d'anticorps ou de cellules capables de reconnaître le polypeptide dont la séquence d'acides aminés est comprise dans la séquence d'acides aminés de polypeptide selon l'invention, telle que les séquences d'acides aminés SEQ ID N° 1 à SEQ ID N° 2, ou un polypeptide de séquence d'acides aminés SEQ ID N° 28 (polypeptide DP428) ou l'un de ses fragments ci-dessus définis.

Ces aminoacyles équivalents peuvent être déterminés soit en s'appuyant sur leur homologie de structure avec les aminoacyles auxquels ils se substituent, soit sur les résultats des essais d'immunogénicité croisée auxquels les différents peptides sont susceptibles de donner lieu.

A titre d'exemple, on mentionnera les possibilités de substitutions susceptibles d'être effectuées sans qu'il en résulte une modification approfondie de l'immunogénicité des peptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Par fragment biologiquement actif, on entendra désigner en particulier un fragment de séquence d'acides aminés de polypeptide présentant au moins une des caractéristiques des polypeptides selon l'invention, notamment en ce qu'il est :
- capable d'être exporté et/ou sécrété par une mycobactérie, et/ou d'être induit ou réprimé lors de l'infection par la mycobactérie ; et/ou
- capable d'induire, de réprimer ou de moduler, directement ou indirectement, un facteur de virulence de mycobactérie ; et/ou
- capable d'induire une réaction d'immunogénicité dirigée contre les mycobactéries ; et/ou
- capable d'être reconnu par un anticorps spécifique de mycobactérie.

Par fragment de polypeptide, on entend désigner un polypeptide comportant au minimun 5 acides aminés, de préférence 10 acides aminés et 15 acides aminés.

Un polypeptide de l'invention, ou un de ses fragments, tels que définis précédemment, est susceptible d'être reconnu spécifiquement par les anticorps présents dans le sérum de patients infectés par des mycobactéries et préférentiellement des bactéries appartenant au complexe de *Mycobacterium tuberculosis* ou par des cellules de l'hôte infecté.

Font ainsi partie de l'invention les fragments du polypeptide dont la séquence d'acides aminés est comprise dans la séquence d'acides aminés de polypeptide selon l'invention, telle que les séquences d'acides aminés SEQ ID N° 1 à SEQ ID N° 2, ou un polypeptide de séquence d'acides aminés SEQ ID N° 28, qui peuvent être obtenus par clivage dudit polypeptide par une enzyme protéolytique, telle que la trypsine ou la chymotrypsine ou la collagénase, ou par un réactif chimique, tel que le bromure de cyanogène (CNBr) ou encore en plaçant un polypeptide selon l'invention tel que le polypeptide DP428 dans un environnement très acide, par exemple à pH 2,5.

Des fragments peptidiques préférés selon l'invention, pour une utilisation en diagnostic ou en vaccination, sont les fragments contenus dans des régions de polypeptide selon l'invention tel que le polypeptide DP428 susceptibles d'être naturellement exposées au solvant et de présenter ainsi des propriétés d'immunogénicité importante. De tels fragments peptidiques peuvent être préparés indifféremment par synthèse chimique, à partir d'hôtes transformés par un vecteur d'expression selon l'invention contenant un acide nucléique permettant l'expression desdits fragments, placé sous le contrôle des éléments de régulation et/ou d'expression appropriés ou encore par clivage chimique ou enzymatique.

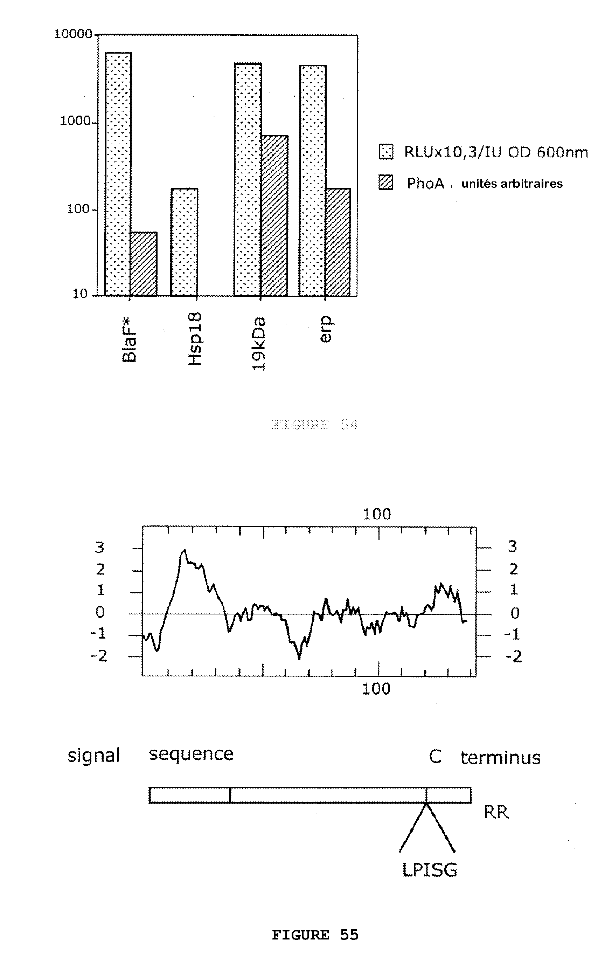

Une analyse de l'hydrophilicité du polypeptide DP428 a été réalisée à l'aide du logiciel DNA Strider^{™} (commercialisé par le CEA Saclay), sur la base d'un calcul du caractère hydrophile de la région codante pour le DP428 de la SEQ ID N° 28. Les résultats de cette analyse sont présentés à la figure 54, où sont détaillés, pour chacun des acides aminés (AA) de position définie dans la SEQ ID N° 28, l'indice d'hydrophilicité. Plus l'indice d'hydrophilicité est élevé, plus l'acide aminé considéré est susceptible d'être exposé au solvant dans la molécule native, et est en conséquence susceptible de présenter un degré d'antigénicité élevé. Ainsi, un enchaînement d'au moins sept acides aminés possédant un indice élevé d'hydrophilicité (>0,3) peut constituer la base de la structure d'un peptide candidat immunogène selon la présente invention.

Les réponses immunitaires cellulaires de l'hôte à un polypeptide selon l'invention, peuvent être mises en évidence selon les techniques décrites par Colignon et al., 1996.

D'après les données de la carte d'hydrophilicité présentée à la Figure 54, les inventeurs ont pu définir des régions du polypeptide DP428 préférentiellement exposées au solvant, plus particulièrement la région localisée entre les acides aminés 55 et 72 de la séquence SEQ ID N° 28 et la région localisée entre les acides aminés 99 et 107 de la SEQ ID N° 28.

Les régions peptidiques du polypeptide DP428 définies ci-dessus peuvent être avantageusement mises en oeuvre pour la réalisation des compositions immunogènes ou des compositions vaccinales selon l'invention.

Les polynucléotides caractérisés en ce qu'ils codent pour un polypeptide selon l'invention, font également partie de l'invention.

L'invention concerne également les séquences d'acide nucléique utilisables comme sonde ou amorce, caractérisées en ce que lesdites séquences sont choisies parmi les séquences d'acide nucléique de polynucléotides selon l'invention.

L'invention concerne en outre l'utilisation d'une séquence d'acide nucléique de polynucléotides selon l'invention comme sonde ou amorce, pour la détection et/ou l'amplification de séquence d'acide nucléique. Parmi ces séquences d'acide nucléique selon l'invention utilisables comme sonde ou amorce, on préfère les séquences d'acide nucléique de l'invention, caractérisées en ce que lesdites séquences sont des séquences, ou leur séquence complémentaire, comprises entre le nucléotide en position nt 964 et le nucléotide en position nt 1234, extrémités incluses, de la séquence SEQ ID N° 1.

Parmi les polynucléotides selon l'invention, utilisables comme amorces nucléotidiques, on préfère particulièrement les polynucléotides de séquence SEQ ID N° 25 et SEQ ID N° 26.

Les polynucléotides selon l'invention peuvent ainsi être utilisés pour sélectionner des amorces nucléotidiques, notamment pour la technique PCR (Erlich, 1989 ; Innis et al., 1990, et, Rolfs et al., 1991).

Cette technique nécessite le choix de paires d'oligonucléotides encadrant le fragment qui doit être amplifié. On peut, par exemple, se référer à la technique décrite dans le brevet américain U.S. N° 4,683,202. Ces amorces oligodésoxyribonucléotidiques ou oligoribo-nucléotidiques ont avantageusement une longueur d'au moins 8 nucléotides, de préférence d'au moins 12 nucléotides, et encore plus préférentiellement au moins 20 nucléotides. On préférera en particulier des amorces d'une longueur comprise entre 8 et 30 et de préférence 12 et 22 nucléotides. L'une des deux amorces est complémentaires du brin (+) [amorce aller] de la matrice et l'autre amorce est complémentaire du brin (-) [amorce retour]. Il est important que les amorces ne possèdent pas de structure secondaire ou de séquence complémentaire l'une de l'autre. D'autre part, la longueur et la séquence de chaque amorce doivent être choisies de manière à ce que les amorces ne s'hybrident pas avec d'autres acides nucléiques provenant de cellules procaryotes ou eucaryotes, en particulier avec les acides nucléiques provenant d'autres mycobactéries pathogènes, ni avec l'ADN ou l'ARN humain pouvant éventuellement contaminer l'échantillon biologique.

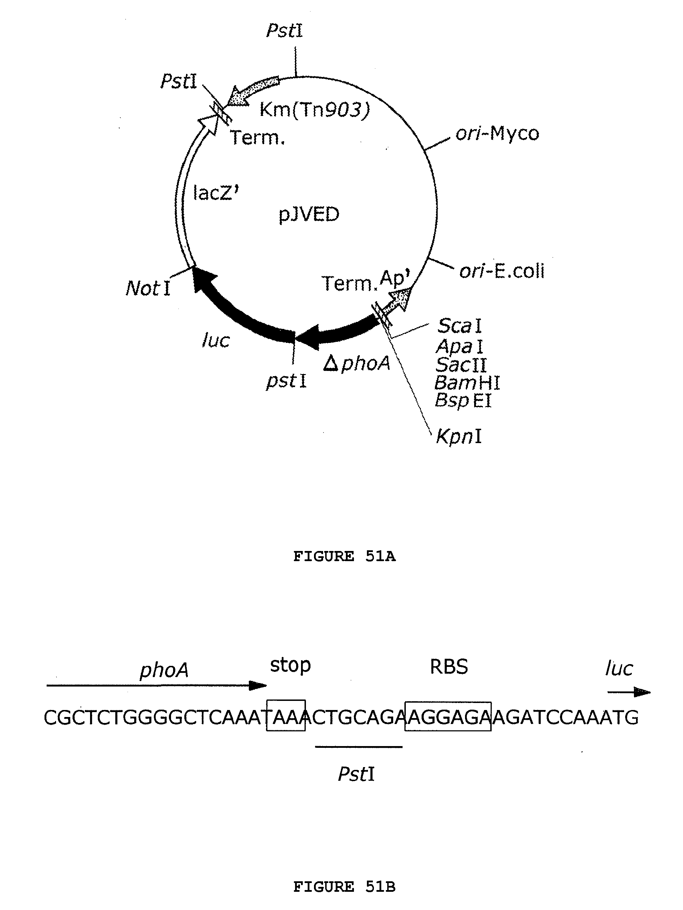

Les résultats présentés à la figure 51, montrent que la séquence codant pour le polypeptide DP428 (SEQ ID N° 28) n'est pas retrouvée dans les ADNs de M. *fortuitum, M. simiae, M. avium, M. chelonae, M. flavescens, M. gordonae, M. marinum* et M. *kansasii.*

Les fragments amplifiés peuvent être identifiés après une électrophorèse en gel d'agarose ou de polyacrylamide, ou après une électrophorèse capillaire, ou encore après une technique chromatographique (filtration sur gel, chromatographie hydrophobe ou chromatographie échangeuse d'ions). La spécificité de l'amplification peut être contrôlée par hybridation moléculaire en utilisant comme sondes les séquences nucléotidiques de polynucléotides de l'invention, des plasmides contenant ces séquences ou leurs produits d'amplification.

Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés.

Parmi les polynucléotides selon l'invention, utilisables comme sondes nucléotidiques, on préfère tout particulièrement le fragment polynucléotidique comprenant la séquence comprise entre le nucléotide en position nt 964 et le nucléotide en position nt 1234, extrémités incluses, de la séquence de SEQ ID N° 1.

Ces sondes et amplicons peuvent être marqués ou non par des éléments radioactifs ou par des molécules non radioactives, telles que des enzymes ou des éléments fluorescents.

L'invention vise également les fragments nucléotidiques susceptibles d'être obtenus par amplification à l'aide d'amorces selon l'invention.

D'autres techniques d'amplification de l'acide nucléique cible peuvent être avantageusement employées comme alternatives à la PCR.

La technique SDA (Strand Displacement Amplification) ou technique d'amplification à déplacement de brin (Walker et al., 1992) est une technique d'amplification isotherme dont le principe est fondé sur la capacité d'une enzyme de restriction de couper l'un des deux brins de son site de reconnaissance qui se trouve sous une forme hemiphosphorothioate et sur la propriété d'une ADN polymérase d'initier la synthèse d'un nouveau brin d'ADN à partir de l'extrémité 3'OH créée par l'enzyme de restriction et de déplacer le brin préalablement synthétisé qui se trouve en aval.

Les polynucléotides de l'invention, en particulier les amorces selon l'invention, peuvent également être mis en oeuvre dans d'autres procédés d'amplification d'un acide nucléique cible, tels que :
- la technique TAS (Transcription-based Amplification System), décrite par Kwoh et al. en 1989 ;
- la technique 3SR (Self-Sustained Sequence Replication), décrite par Guatelli et al. en 1990 ;
- la technique NASBA (Nucleic Acid Sequence Based Amplification), décrite par Kievitis et al. en 1991 ;
- la technique TMA (Transcription Mediated Amplification).

Les polynucléotides de l'invention peuvent aussi être employés dans des techniques d'amplification ou de modification de l'acide nucléique servant de sonde, telles que :
- la technique LCR (Ligase Chain Reaction), décrite par Landegren et al. en 1988 et perfectionnée par Barany et al. en 1991, qui emploie une ligase thermostable ;
- la technique de RCR (Repair Chain Reaction), décrite par Segev en 1992 ;
- la technique CPR (Cycling Probe Reaction), décrite par Duck et al. en 1990 ;
- la technique d'amplification à la Q-beta-réplicase, décrite par Miele et al. en 1983 et perfectionnée notamment par Chu et al. en 1986, Lizardi et al. en 1988, puis par Burg et al. ainsi que par Stone et al. en 1996.

Dans le cas où le polynucléotide cible à détecter est un ARN, par exemple un ARNm, on utilisera avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADNc à partir de l'ARN contenu dans l'échantillon biologique. L'ADNc obtenu servira alors de cible pour les amorces ou les sondes mises en oeuvre dans le procédé d'amplification ou de détection selon l'invention.

La sonde de détection sera choisie de telle manière à ce qu'elle hybride avec l'amplicon généré. Une telle sonde de détection aura avantageusement pour séquence une séquence d'au moins 12 nucléotides, en particulier d'au moins 15 nucléotides, et de préférence au moins de 200 nucléotides.

Les sondes nucléotidiques selon l'invention sont capables de détecter des mycobactéries et préférentiellement des bactéries appartenant au complexe de *Mycobacterium tuberculosis,* plus précisément du fait que ces mycobactéries possèdent dans leur génome au moins une copie de polynucléotides selon l'invention. Ces sondes selon l'invention, sont capables, par exemple, de s'hybrider avec la séquence nucléotidique d'un polypeptide selon l'invention, plus particulièrement tout oligonucléotide hybridant avec la séquence SEQ ID N° 1 codant pour le polypeptide DP428 de *M. tuberculosis,* et ne présentant pas de réaction d'hybridation croisée ou d'amplification (PCR) avec par exemple des séquences présentes chez des mycobactéries n'appartenant pas au complexe de *Mycobacterium tuberculosis.* Les sondes nucléotidiques selon l'invention hybrident spécifiquement avec une molécule d'ADN ou d'ARN de polynucléotide selon l'invention, dans des conditions d'hybridation de forte stringence telles que données sous forme d'exemple précédemment.

Les séquences non marquées peuvent être utilisées directement comme sondes, cependant les séquences sont généralement marquées par un élément radioactif (³²P, ³⁵S, ³H, ¹²⁵I) ou par une molécule non-radioactive (biotine, acétylaminofluorène, digoxigénine, 5-bromo-désoxyuridine, fluorescéine) pour obtenir des sondes utilisables pour de nombreuses applications.

Des exemples de marquages non radioactifs de sondes sont décrits, par exemple, dans le brevet français N° 78 10975 ou par Urdea et al. ou par Sanchez-Pescador et al. en 1988.

Dans ce dernier cas, on pourra aussi utiliser l'une des méthodes de marquage décrites dans les brevets FR 2 422 956 et FR 2 518 755. La technique d'hybridation peut être réalisée de manières diverses (Matthews et al., 1988). La méthode la plus générale consiste à immobiliser l'acide nucléique extrait des cellules de mycobactéries sur un support (tel que nitrocellulose, nylon, polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Avantageusement, les sondes nucléotidiques marquées selon l'invention peuvent avoir une structure telle qu'elles rendent possible une amplification du signal radioactif ou non-radioactif. Un système d'amplification répondant à la définition ci-dessus comprendra des sondes de détection sous la forme d'un ADN ramifié, branché («branched DNA») telles que celles décrites par Urdea et al. en 1991. Selon cette technique, on utilisera avantageusement plusieurs types de sondes notamment une sonde de capture, afin d'immobiliser l'ADN ou l'ARN cible sur un support, et une sonde de détection. La sonde de détection lie un ADN «branché» présentant une structure ramifiée. L'ADN branché, à son tour, est capable de fixer des sondes oligonucléotidiques qui sont elles-mêmes couplées à des molécules de phosphatase alcaline. Puis l'activité de cette enzyme est mise en évidence grâce à un substrat chimio-luminescent, par exemple un dérivé du dioxétane-phosphate.

Selon un autre mode avantageux de mise en oeuvre des sondes nucléiques selon l'invention, ces dernières peuvent être immobilisées sur un support, de manière covalente ou non covalente, et utilisées comme sondes de capture. Dans ce cas, une sonde, dite «sonde de capture», est immobilisée sur un support et sert à capturer par hybridation spécifique l'acide nucléique cible obtenu à partir de l'échantillon biologique à tester. Si nécessaire, le support solide est séparé de l'échantillon et le duplex formé entre la sonde de capture et l'acide nucléique cible est ensuite détecté grâce à une seconde sonde, dite «sonde de détection», marquée par un élément facilement détectable.

Les fragments oligonucléotidiques peuvent être obtenus à partir des séquences selon l'invention, par coupure avec des enzymes de restriction, ou par synthèse chimique selon les méthodes classiques, par exemple selon la méthode décrite dans le brevet européen N° EP-0305929 (Millipore Corporation) ou encore par d'autres procédés.

Un mode de préparation approprié des acides nucléiques de l'invention comportant au maximum 200 nucléotides (ou 200 pb s'il s'agit d'acides nucléiques bicaténaires) comprend les étapes suivantes :
- la synthèse d'ADN en utilisant la méthode automatisée des béta-cyanethylphosphoramidite décrite en 1986,
- le clonage des acides nucléiques ainsi obtenus dans un vecteur approprié et la récupération des acides nucléiques par hybridation avec une sonde appropriée.

Un mode de préparation, par voie chimique, d'acides nucléiques selon l'invention de longueur supérieure à 200 nucléotides (ou 200 pb lorsqu'il s'agit d'acides nucléiques bicaténaires) comprend les étapes suivantes :
- l'assemblage d'oligonucléotides synthétisés chimiquement, pourvus à leur extrémité de sites de restrictions différents, dont les séquences sont compatibles avec l'enchaînement en acides aminés du polypeptide naturel selon le principe décrit en 1983,
- le clonage des acides nucléiques ainsi obtenus dans un vecteur approprié et la récupération des acides nucléiques recherchés par hybridation avec une sonde appropriée.

Les sondes nucléotidiques utilisées pour la récupération des acides nucléiques recherchés dans les procédés sus-mentionnés, sont constituées généralement de 8 à 200 nucléotides de la séquence de polypeptide selon l'invention et sont susceptibles de s'hybrider avec l'acide nucléique recherché dans les conditions d'hybridation définies précédemment. La synthèse de ces sondes peut être effectuée selon la méthode automatisée des béta cyanethylphosphoramidites décrite en 1986.

Les sondes oligonucléotidiques selon l'invention peuvent être mises en oeuvre au sein d'un dispositif de détection comprenant une banque matricielle d'oligonucléotides. Un exemple de réalisation d'une telle banque matricielle peut consister en une matrice d' oligonucléotides sondes fixés sur un support, la séquence de chaque sonde d'une longueur donnée étant située en décalage d'une ou plusieurs bases par rapport à la sonde précédente, chacune des sondes de l'arrangement matriciel étant ainsi complémentaire d'une séquence distincte de l'ADN ou l'ARN cible à détecter et chaque sonde de séquence connue étant fixée en une position prédéterminée du support. La séquence cible à détecter peut être avantageusement marquée radioactivement ou non radioactivement. Lorsque la séquence cible marquée est mise en contact avec le dispositif matriciel, celle-ci forme des hybrides avec les sondes de séquences complémentaires. Un traitement à la nucléase, suivi d'un lavage, permet d'éliminer les hybrides sondes-séquence cible qui ne sont pas parfaitement complémentaires. Du fait de la connaissance précise de la séquence d'une sonde à une position déterminée de la matrice, il est alors possible de déduire la séquence nucléotidique de la séquence d'ADN ou d'ARN cible. Cette technique est particulièrement efficace lorsque sont utilisées des matrices de sondes oligonucléotidiques de grande taille.

Une alternative à l'utilisation d'une séquence cible marquée peut consister en l'utilisation d'un support permettant une détection « bioélectronique » de l'hybridation de la séquence cible sur les sondes du support matrice, lorsque que ledit support est constitué ou comprend un matériau capable d'agir, par exemple, en tant que donneur d'électrons aux positions de la matrice auxquelles un hybride a été formé. Un tel matériau donneur d'électron est par exemple de l'or. La détection de la séquence nucléotidique de l'ADN ou ARN cible est alors déterminée par un dispositif électronique.

Un exemple de réalisation d'un biocapteur, tel que défini ci-dessus, est décrit dans la demande de brevet européen N° EP-0721 016 au nom de Affymax technologies N.V. ou encore dans le brevet américain N° US 5,202,231 au nom de Drmanac.

L'invention a aussi pour objet les polynucléotides hybrides résultant :
- soit de la formation d'une molécule hybride entre un ARN ou un ADN (ADN génomique ou ADNc) provenant d'un échantillon biologique avec une sonde ou une amorce selon l'invention,
- soit de la formation d'une molécule hybride entre un ARN ou un ADN (ADN génomique ou ADNc) provenant d'un échantillon biologique avec un fragment nucléotidique amplifié à l'aide d'un couple d'amorces selon l'invention.

Par ADNc au sens de la présente invention, on entend une molécule d'ADN obtenue en faisant agir une enzyme de type transcriptase inverse sur une molécule d'ARN, en particulier une molécule d'ARN messager (ARNm), selon les techniques décites dans Sambrook et al. en 1989.

La présente invention a également pour objet une famille de plasmides recombinants, caractérisés en ce qu'ils contiennent au moins une séquence nucléotidique de polynucléotide selon l'invention. Selon un mode de réalisation avantageux dudit plasmide, il comprend la séquence nucléotidique SEQ ID N° 1 ou un fragment de celle-ci.

Un autre objet de la présente invention est un vecteur pour le clonage, l'expression et/ou l'insertion d'une séquence, caractérisé en ce qu'il comprend une séquence nucléotidique de polynucléotide selon l'invention en un site non essentiel pour sa réplication, le cas échéant sous le contrôle d'éléments de régulation susceptibles d'intervenir dans l'expression du polypeptide DP428, chez un hôte déterminé.

Des vecteurs particuliers sont par exemple des plasmides, des phages, des cosmides, des phagemides, des YAC.

Ces vecteurs sont utiles pour transformer des cellules hôtes afin de cloner ou d'exprimer les séquences nucléotidiques de l'invention.

L'invention comprend également les cellules hôtes transformées par un vecteur selon l'invention.

De préférence, les cellules hôtes sont transformées dans des conditions permettant l'expression d'un polypeptide recombinant selon l'invention.

Une cellule hôte préférée selon l'invention est la souche *E. coli* transformée par le plasmide pDP428 déposé le 28 janvier 1997 à la CNCM sous le N° I-1818 ou transformée par le plasmide pM1C25 déposé le 4 août 1998 à la CNCM sous le N° I-2062 ou une mycobactérie appartenant à une souche de *M. tuberculosis, M. bovis* ou *M*. *africanum* possédant potentiellement tous les systèmes de régulation appropriés.

Il est aujourd'hui facile de produire des protéines ou polypeptides en quantité relativement importante par génie génétique en utilisant comme vecteurs d'expression des plasmides, des phages, des phagemides. Tout ou partie du gène *DP428,* ou tout polynucléotide selon l'invention, peut être inséré dans un vecteur d'expression approprié pour produire *in vitro* un polypeptide selon l'invention, notamment le polypeptide DP428. Ledit polypeptide pourra être fixé sur une microplaque pour développer un test sérologique destiné à rechercher, dans un but de diagnostic, les anticorps spécifiques chez les patients atteints de tuberculose.

Ainsi, la présente invention concerne un procédé de préparation d'un polypeptide, caractérisé en ce qu'il met en oeuvre un vecteur selon l'invention. Plus particulièrement l'invention concerne un procédé de préparation d'un polypeptide de l'invention comprenant les étapes suivantes :
- le cas échéant, l'amplification préalable suivant la technique PCR de la quantité de séquences de nucléotides codant pour ledit polypeptide à l'aide de deux amorces d'ADN choisies de manière à ce que l'une de ces amorces soit identique aux 10 à 25 premiers nucléotides de la séquence nucléotidique codant pour ledit polypeptide, tandis que l'autre amorce est complémentaire des 10 à 25 derniers nucléotides (ou s'hybride avec ces 10 à 25 derniers nucléotides) de ladite séquence nucléotidique, ou inversement de manière à ce que l'une de ces amorces soit identique aux 10 à 25 derniers nucléotides de ladite séquence, tandis que l'autre amorce est complémentaire des 10 à 25 premiers nucléotides (ou s'hybride avec les 10 à 25 premiers nucléotides) de ladite séquence nucléotidique, suivie de l'introduction desdites séquences ainsi amplifiées dans un vecteur approprié,
- la mise en culture, dans un milieu de culture approprié, d'un hôte cellulaire préalablement transformé par un vecteur approprié contenant un acide nucléique selon l'invention comprenant la séquence nucléotidique codant pour ledit polypeptide, et
- la séparation, à partir du susdit milieu de culture, dudit polypeptide produit par ledit hôte cellulaire transformé.

L'invention a aussi pour objet un polypeptide susceptible d'être obtenu par un procédé de l'invention tel que décrit précédemment.

Les peptides selon l'invention peuvent également être préparés par les techniques classiques, dans le domaine de la synthèse des peptides. Cette synthèse peut être réalisée en solution homogène ou en phase solide.

Par exemple, on aura recours à la technique de synthèse en solution homogène décrite par Houbenweyl en 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal.

Selon une autre technique préférée de l'invention, on a recours à celle décrite par Merrifield.

Pour fabriquer une chaîne peptidique selon le procédé de Merrifield, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second aminoacyle de la séquence recherchée, à partir du résidu aminoacyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acides aminés, et dont la fonction amine terminale est protégée. Comme précédemment, on déprotège la fonction amine et on peut ensuite procéder à la fixation du troisième aminoacyle, dans des conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine, par exemple à l'aide d'acide fluorhydrique.

De manière préférentielle, lesdits polypeptides susceptibles d'être obtenus par un procédé de l'invention tel que décrit précédemment comprendront une région exposée au solvant et auront une longueur d'au moins 20 acides aminés.

Selon un autre mode de réalisation de l'invention, lesdits polypeptides sont spécifiques de mycobactéries du complexe *Mycobacterium tuberculosis* et ne sont donc pas reconnus par des anticorps spécifiques d'autres protéines de mycobactéries.

L'invention est en outre relative à des polypeptides hybrides présentant au moins un polypeptide selon l'invention et une séquence d'un polypeptide susceptible d'induire une réponse immunitaire chez l'homme ou l'animal.

Avantageusement, le déterminant antigénique est tel qu'il est susceptible d'induire une réponse humorale et/ou cellulaire.

Un tel déterminant pourra comprendre un polypeptide selon l'invention sous forme glycosylée utilisé en vue d'obtenir des compositions immunogènes susceptibles d'induire la synthèse d'anticorps dirigés contre des épitopes multiples. Lesdits polypeptides glycosylés font également partie de l'invention.

Ces molécules hybrides peuvent être constituées en partie d'une molécule porteuse de polypeptide selon l'invention associée à une partie, en particulier un épitope de la toxine diphtérique, la toxine tétanique, un antigène de surface du virus de l'hépatite B (brevet FR 79 21811), l'antigène VP1 du virus de la poliomyélite ou toute autre toxine ou antigène viral ou bactérien.

Avantageusement, ledit déterminant antigénique correspond à un déterminant antigénique de protéines immunogènes de 45/47 kD de *M. tuberculosis* (demande internationale PCT/FR 96/0166), ou encore sélectionnées par exemple parmi ESAT6 (Harboe et al., 1996 ; Andersen et al., 1995 et Sorensen et al., 1995) et DES (PCT/FR 97/00923, Gicquel et al.).

Un antigène viral, tel que défini ci-dessus, sera préférentiellement une protéine de surface ou d'enveloppe d'un virus de l'hépatite, par exemple la protéine de surface de l'hépatite B sous l'une de ses formes S, S-préS1, S-préS2 ou S-préS2-préS1 ou encore une protéine d'un virus de l'hépatite A, ou d'une hépatite non-A, non-B, tel qu'un virus de l'hépatite C, E ou delta.

Plus particulièrement, un antigène viral tel que défini ci-dessus sera tout ou partie de l'une des glycoprotéines codées par le génome du virus HIV-1 (brevets GB 8324800, EP 84401834 ou EP 85905513) ou du virus HIV-2 (EP 87400151), et en particulier tout ou partie d'une protéine sélectionnée parmi gag, pol, nef ou env de HIV-1 ou de HIV-2.

Les procédés de synthèse des molécules hybrides englobent les méthodes utilisées en génie génétique pour construire des polynucléotides hybrides codant pour les séquences polypeptidiques recherchées. On pourra, par exemple, se référer avantageusement à la technique d'obtention de gènes codant pour des protéines de fusion décrite par Minton en 1984.

Lesdits polynucléotides hybrides codant pour un polypeptide hybride ainsi que les polypeptides hybrides selon l'invention caractérisés en ce qu'il s'agit de protéines recombinantes obtenues par l'expression desdits polynucléotides hybrides, font également partie de l'invention.

Les polypeptides selon l'invention peuvent avantageusement être mis en oeuvre dans un procédé pour la détection *in vitro* d'anticorps dirigés contre lesdits polypeptides, notamment le polypeptide DP428, et ainsi d'anticorps dirigés contre une bactérie du complexe Mycobacterium *tuberculosis,* dans un échantillon biologique (tissu ou fluide biologique) susceptible de les contenir, ce procédé comprenant la mise en contact de cet échantillon biologique avec un polypeptide selon l'invention dans des conditions permettant une réaction immunologique *in vitro* entre ledit polypeptide et les anticorps éventuellement présents dans l'échantillon biologique, et la mise en évidence *in vitro* des complexes antigène-anticorps éventuellement formés.

Les polypeptides selon l'invention peuvent également et avantageusement être mis en oeuvre dans un procédé pour la détection d'une infection par une bactérie du complexe *Mycobacterium tuberculosis* dans un mammifère basé sur la détection *in vitro* d'une réaction cellulaire indiquant une sensibilisation préalable du mammifère audit polypeptide comme par exemple la prolifération cellulaire, la synthèse de protéines telles que l'interféron gamma. Ce procédé pour la détection d'une infection par une bactérie du complexe *Mycobacterium tuberculosis* dans un mammifère, est caractérisé en ce qu'il comprend les étapes suivantes :
a) préparation d'un échantillon biologique contenant des cellules dudit mammifère plus particulièrement des cellules du système immunitaire dudit mammifère et plus particulièrement encore des cellules T ;
b) incubation de l'échantillon biologique de l'étape a) avec un polypeptide selon l'invention ;
c) détection d'une réaction cellulaire indiquant une sensibilisation préalable du mammifère audit polypeptide comme par exemple la prolifération cellulaire et/ou la synthèse de protéines telles que l'interféron gamma.

La prolifération cellulaire pourra être mesurée, par exemple par incorporation de ³H-Thymidine.

Font également partie de l'invention, les procédés de détection d'une réaction d'hypersensibilité retardée (DTH), caractérisés en ce qu'ils mettent en oeuvre un polypeptide selon l'invention.

De préférence, l'échantillon biologique est constitué par un fluide, par exemple un sérum humain ou animal, du sang, des biopsies, le liquide broncho-alvéolaire ou le liquide pleural.

Toute procédure classique peut être mise en oeuvre pour réaliser une telle détection.

A titre d'exemple, une méthode préférée met en jeu des processus immunoenzymatiques selon la technique ELISA, par immunofluorescence, ou radio-immunologique (RIA) ou équivalent.

Ainsi, l'invention concerne également les polypeptides selon l'invention, marqués à l'aide d'un marqueur adéquat tel que du type enzymatique, fluorescent, radioactif.

De telles méthodes comprennent par exemple les étapes suivantes :
- dépôt de quantités déterminées d'une composition polypeptidique selon l'invention dans les puits d'une plaque de microtitration,
- introduction dans lesdits puits de dilutions croissantes de sérum, ou d'échantillon biologique autre tel que défini précédemment, devant être analysé,
- incubation de la microplaque,
- introduction dans les puits de la plaque de microtitration d'anticorps marqués dirigés contre des immunoglobulines humaines ou animales, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée, par exemple à 550 nm,
- détection, en comparaison avec un témoin de contrôle, de la quantité de substrat hydrolysé.

L'invention concerne également un nécessaire ou kit pour le diagnostic in vitro d'une infection par une mycobactérie appartenant au complexe *Mycobacterium tuberculosis,* comprenant :
- un polypeptide selon l'invention,
- le cas échéant les réactifs pour la constitution du milieu propice à la réaction immunologique ou spécifique,
- les réactifs permettant la détection des complexes antigène-anticorps produits par la réaction immunologique éventuellement présents dans l'échantillon biologique, et la mise en évidence *in vitro* des complexes antigène-anticorps éventuellement formés, ces réactifs pouvant également porter un marqueur, ou être susceptibles d'être reconnus à leur tour par un réactif marqué, plus particulièrement dans le cas où le polypeptide selon l'invention n'est pas marqué,
- le cas échéant, un échantillon biologique de référence (témoin négatif) dépourvu d'anticorps reconnus par un polypeptide selon l'invention,
- le cas échéant, un échantillon biologique de référence (témoin positif) contenant une quantité prédéterminée d'anticorps reconnus par un polypeptide selon l'invention.

Les polypeptides selon l'invention permettent de préparer des anticorps monoclonaux ou polyclonaux caractérisés en ce qu'ils reconnaissent spécifiquement les polypeptides selon l'invention. Les anticorps monoclonaux pourront avantageusement être préparés à partir d'hybridomes selon la technique décrite par Kohler et Milstein en 1975. Les anticorps polyclonaux pourront être préparés, par exemple par immunisation d'un animal, en particulier une souris, avec un polypeptide selon l'invention associé à un adjuvant de la réponse immunitaire, puis purification des anticorps spécifiques contenus dans le sérum des animaux immunisés sur une colonne d'affinité sur laquelle a préalablement été fixé le polypeptide ayant servi d'antigène. Les anticorps polyclonaux selon l'invention peuvent aussi être préparés par purification sur une colonne d'affinité, sur laquelle a préalablement été immobilisé un polypeptide selon l'invention, des anticorps contenus dans le sérum de patients infectés par une mycobactérie et préférentiellement une bactérie appartenant au complexe *Mycobacterium tuberculosis.*

L'invention a également pour objet des anticorps mono ou polyclonaux ou leurs fragments, ou anticorps chimériques, caractérisés en ce qu'ils sont capables de reconnaître spécifiquement un polypeptide selon l'invention.

Les anticorps de l'invention pourront également être marqués de la même manière que décrit précédemment pour les sondes nucléiques de l'invention telles qu'un marquage de type enzymatique, fluorescent ou radioactif.

L'invention vise en outre un procédé pour la détection spécifique de la présence d'un antigène d'une mycobactérie et préférentiellement une bactérie du complexe *Mycobacterium tuberculosis* dans un échantillon biologique, caractérisé en ce qu'il comprend les étapes suivantes :
a) mise en contact de l'échantillon biologique (tissu ou fluide biologique) prélevé chez un individu avec un anticorps mono ou polyclonal selon l'invention, dans des conditions permettant une réaction immunologique in vitro entre lesdits anticorps et les polypeptides spécifiques des mycobactéries et préférentiellement des bactéries du complexe de *Mycobacterium tuberculosis* éventuellement présents dans l'échantillon biologique, et
b) mise en évidence du complexe antigène-anticorps formé.

Entre également dans le cadre de l'invention, un nécessaire ou kit pour le diagnostic *in vitro* sur un échantillon biologique, de la présence de souches de mycobactéries des mycobactéries et préférentiellement des bactéries appartenant au complexe de *Mycobacterium tuberculosis,* de préférence *M. tuberculosis,* caractérisé en ce qu'il comprend :
- un anticorps polyclonal ou monoclonal selon l'invention, le cas échéant marqué ;
- le cas échéant, un réactif pour la constitution du milieu propice à la réalisation de la réaction immunologique ;
- un réactif permettant la détection des complexes antigène-anticorps produits par la réaction immunologique, ce réactif pouvant également porter un marqueur, ou être susceptible d'être reconnu à son tour par un réactif marqué, plus particulièrement dans le cas où ledit anticorps monoclonal ou polyclonal n'est pas marqué ;
- le cas échéant, des réactifs pour effectuer la lyse des cellules de l'échantillon testé.

La présente invention a également pour objet un procédé de détection et d'identification rapide des mycobactéries et préférentiellement des bactéries de *M*. *tuberculosis* dans un échantillon biologique, caractérisé en ce qu'il comporte les étapes suivantes :
a) isolement de l'ADN à partir de l'échantillon biologique à analyser, ou obtention d'un ADNc à partir de l'ARN de l'échantillon biologique ;
b) amplification spécifique de l'ADN des mycobactéries et préférentiellement des bactéries appartenant au complexe *Mycobacterium tuberculosis* à l'aide d'amorces selon l'invention ;
g) analyse des produits d'amplification.

Les produits d'amplification peuvent être analysés par différentes méthodes.

Deux méthodes d'analyse sont données à titre d'exemple ci-dessous :
- Analyse électrophorétique en gel d'agarose des produits d'amplification. La présence d'un fragment d'ADN migrant à l'endroit attendu suggère que l'échantillon analysé contenait de l'ADN de mycobactéries appartenant au complexe tuberculosis, ou
- Analyse par la technique d'hybridation moléculaire en utilisant une sonde nucléique selon l'invention. Cette sonde sera avantageusement marquée par un élément non radioactif (sonde froide) ou radioactif.

Aux fins de la présente invention, on entendra par « ADN de l'échantillon biologique » ou « ADN contenu dans l'échantillon biologique », soit l'ADN présent dans l'échantillon biologique considéré, soit l'ADNc obtenu après l'action d'une enzyme de type transcriptase inverse sur l'ARN présent dans ledit échantillon biologique.

Un autre procédé de la présente invention permet la détection d'une infection par une mycobactérie et préférentiellement une bactérie du complexe *Mycobacterium tuberculosis* dans un mammifère. Ce procédé comprend les étapes suivantes :
a) préparation d'un échantillon biologique contenant des cellules dudit mammifère plus particulièrement des cellules du système immunitaire dudit mammifère et plus particulièrement encore des cellules T ;
b) incubation de l'échantillon biologique de l'étape a) avec un polypeptide selon l'invention ;
c) détection d'une réaction cellulaire indiquant une sensibilisation préalable du mammifère audit polypeptide notamment la prolifération cellulaire et/ou la synthèse de protéines telles que l'interféron gamma ;
d) détection d'une réaction d'hypersensibilité retardée ou de sensibilisation du mammifère audit polypeptide.

Cette méthode de détection est une méthode intradermique, qui est décrite par exemple par M. J. Elhay et al. (1988) Infection and Immunity, 66(7):3454-3456.

Un autre but de la présente invention consiste en un procédé pour la détection des mycobactéries et préférentiellement des bactéries appartenant au complexe *Mycobacterium tuberculosis* dans un échantillon biologique, caractérisé en ce qu'il comprend les étapes suivantes :
a) mise en contact d'une sonde oligonucléotidique selon l'invention avec un échantillon biologique, l'ADN contenu dans l'échantillon biologique, ou l'ADNc obtenu par transcription inverse de l'ARN de l'échantillon biologique, ayant, le cas échéant, préalablement été rendu accessible à l'hybridation, dans des conditions permettant l'hybridation de la sonde à l'ADN ou l'ADNc des mycobactéries et préférentiellement des bactéries du complexe *Mycobacterium tuberculosis ;*
b) détection de l'hybride formé entre la sonde oligonucléotidique et l'ADN de l'échantillon biologique.

L'invention vise également un procédé pour la détection des mycobactéries et préférentiellement des bactéries appartenant au complexe *Mycobacterium tuberculosis* dans un échantillon biologique, caractérisé en ce qu'il comprend les étapes suivantes :
a) mise en contact d'une sonde oligonucléotidique selon l'invention immobilisée sur un support, avec un échantillon biologique, l'ADN de l'échantillon biologique ayant, le cas échéant, été préalablement rendu accessible à l'hybridation, dans des conditions permettant l'hybridation de ladite sonde à l'ADN des mycobactéries et préférentiellement des bactéries du complexe *Mycobacterium tuberculosis ;*
b) mise en contact de l'hybride formé entre ladite sonde oligonucléotidique immobilisée sur un support et l'ADN contenu dans l'échantillon biologique, le cas échéant après élimination de l'ADN de l'échantillon biologique n'ayant pas hybridé avec la sonde, avec une sonde oligonucléotidique marquée selon l'invention.

Selon un mode de réalisation avantageux du procédé de détection défini précédemment, celui-ci est caractérisé en ce que, préalablement à l'étape a), l'ADN de l'échantillon biologique est préalablement amplifié à l'aide d'un couple d'amorces selon l'invention.

Une autre forme de mise en oeuvre du procédé de détection selon l'invention consiste en un procédé pour la détection de la présence des mycobactéries et préférentiellement des bactéries appartenant au complexe de *Mycobacterium tuberculosis* dans un échantillon biologique, caractérisé en ce qu'il comprend les étapes suivantes :
a) mise en contact de l'échantillon biologique avec un couple d'amorces selon l'invention, l'ADN contenu dans l'échantillon ayant été, le cas échéant, préalablement rendu accessible à l'hybridation, dans des conditions permettant une hybridation desdites amorces à l'ADN des mycobactéries et préférentiellement des bactéries du complexe *Mycobacterium tuberculosis ;*
b) amplification de l'ADN d'une mycobactérie et préférentiellement d'une bactérie du complexe *Mycobacterium tuberculosis ;*
c) mise en évidence de l'amplification de fragments d'ADN correspondant au fragment encadré par les amorces, par exemple par électrophorèse sur gel ou au moyen d'une sonde oligonucléotidique selon invention.

L'invention a aussi pour objet un procédé pour la détection de la présence des mycobactéries et préférentiellement des bactéries appartenant au complexe de *Mycobacterium tuberculosis* dans un échantillon biologique par déplacement de brin, caractérisé en ce qu'il comprend les étapes suivantes :
a) mise en contact de l'échantillon biologique avec deux couples d'amorces selon l'invention spécifiquement destinées à l'amplification de type SDA décrites ci-dessus, l'ADN contenu dans l'échantillon ayant été, le cas échéant, préalablement rendu accessible à l'hybridation, dans des conditions permettant une hybridation des amorces à l'ADN des mycobactéries et préférentiellement des bactéries du complexe *Mycobacterium tuberculosis ;*
b) amplification de l'ADN des mycobactéries et préférentiellement des bactéries du complexe *Mycobacterium tuberculosis ;*
c) mise en évidence de l'amplification de fragments d'ADN correspondant au fragment encadré par les amorces, par exemple par électrophorèse sur gel ou au moyen d'une sonde oligonucléotidique selon l'invention.

L'invention concerne aussi un nécessaire ou kit pour la mise en oeuvre du procédé décrit ci-dessus, destiné à la détection de la présence des mycobactéries et préférentiellement des bactéries du complexe *Mycobacterium tuberculosis* dans un échantillon biologique, caractérisé en ce qu'il comprend les éléments suivants :
a) une sonde oligonucléotidique selon l'invention ;
b) les réactifs nécessaires à la mise en oeuvre d'une réaction d'hybridation ;
c) le cas échéant, un couple d'amorces selon l'invention ainsi que les réactifs nécessaires à une réaction d'amplification de l'ADN (ADN génomique, ADN plasmidique ou ADNc) des mycobactéries et préférentiellement des bactéries du complexe *Mycobacterium tuberculosis.*

L'invention a aussi pour objet un kit ou nécessaire pour la détection de la présence des mycobactéries et préférentiellement des bactéries du complexe *Mycobacterium tuberculosis* dans un échantillon biologique, caractérisé en ce qu'il comprend les éléments suivants :
a) une sonde oligonucléotidique, dite sonde de capture, selon l'invention ;
b) une sonde oligonucléotidique, dite sonde de révélation, selon l'invention ;
c) le cas échéant, un couple d'amorces selon l'invention ainsi que les réactifs nécessaires à une réaction d'amplification de l'ADN des mycobactéries et préférentiellement des bactéries du complexe *Mycobacterium tuberculosis.*

L'invention concerne encore un kit ou nécessaire pour l'amplification de l'ADN des mycobactéries et préférentiellement des bactéries du complexe *Mycobacterium tuberculosis* présent dans un échantillon biologique, caractérisé en ce qu'il comprend les éléments suivants :
a) un couple d'amorces selon l'invention ;
b) les réactifs nécessaires pour effectuer une réaction d'amplification d'ADN ;
c) éventuellement un composant permettant de vérifier la séquence du fragment amplifié, plus particulièrement une sonde oligonucléotidique selon l'invention.

Un autre objet de la présente invention concerne une composition immunogène, caractérisée en ce qu'elle comprend un polypeptide selon l'invention.

Une autre composition immunogène selon l'invention est caractérisée en ce qu'elle comprend un ou plusieurs polypeptides selon l'invention et/ou un ou plusieurs polypeptides hybrides selon l'invention.

Selon un mode de réalisation avantageux, la composition immunogène ci-dessus définie est constitutive d'un vaccin, lorsqu'elle est présentée en association avec un véhicule pharmaceutiquement acceptable et éventuellement un ou plusieurs adjuvants de l'immunité tels que l'alun ou un représentant de la famille des muramyl peptides ou encore l'adjuvant incomplet de Freund.

Aujourd'hui, divers types de vaccins sont disponibles pour protéger l'homme contre des maladies infectieuses : micro-organismes vivants atténués (*M*. *bovis -* BCG pour la tuberculose), micro-organismes inactivés (virus de la grippe), des extraits acellulaires (*Bordetella pertussis* pour la coqueluche), protéines recombinées (antigène de surface du virus de l'hépatite B), des polyosides (pneumocoques). Des vaccins préparés à partir de peptides de synthèse ou de micro-organismes génétiquement modifiés exprimant des antigènes hétérologues sont en cours d'expérimentation. Plus récemment encore, des ADN plasmidiques recombinés portant des gènes codant pour des antigènes protecteurs ont été proposés comme stratégie vaccinale alternative. Ce type de vaccination est réalisé avec un plasmide particulier dérivant d'un plasmide de *E*. *coli* qui ne se réplique pas *in vivo* et qui code uniquement pour la protéine vaccinante. Les principaux composants fonctionnels de ce plasmide sont : un promoteur fort permettant l'expression dans les cellules eucaryotes (par exemple celui du CMV), un site de clonage approprié pour insérer le gène d'intérêt, une séquence de terminaison-polyadénylation, une origine de réplication procaryote pour produire le plasmide recombiné *in vitro* et un marqueur de sélection (par exemple le gène de résistance à l'ampicilline) pour faciliter la sélection des bactéries qui contiennent le plasmide. Des animaux ont été immunisés en injectant simplement l'ADN plasmidique nu dans le muscle. Cette technique conduit à l'expression de la protéine vaccinale *in situ* et à une réponse immunitaire en particulier de type cellulaire (CTL) et de type humoral (anticorps). Cette double induction de la réponse immunitaire est l'un des principaux avantages de la technique de vaccination avec de l'ADN nu. Huygen et al. (1996) et Tascon et al. (1996) ont réussi à obtenir une certaine protection contre *M. tuberculosis* en injectant des plasmides recombinés contenant des gènes de *M. leprae (hsp65, 36kDa pra)* comme inserts. *M. leprae* est l'agent responsable de la lèpre. L'utilisation d'un insert spécifique de *M. tuberculosis* comme par exemple tout ou partie du gène *DP428,* objet de la présente invention conduirait probablement à une meilleure protection contre la tuberculose. Tout ou partie du gène *DP428,* ou tout polynucléotide selon l'invention, peut être facilement inséré dans les plasmides vecteurs V1J (Montgomery et al., 1993), pcDNA3 (Invitrogen, R & D Systems) ou pcDNA1/Neo (Invitrogen) qui possèdent les caractéristiques nécessaires pour une utilisation vaccinale.

L'invention vise ainsi un vaccin, caractérisé en ce qu'il comprend un ou plusieurs polypeptides selon l'invention et/ou un ou plusieurs polypeptides hybrides selon l'invention tels que précédemment définis en association avec un véhicule pharmaceutiquement compatible et, le cas échéant, un ou plusieurs adjuvants de l'immunité appropriés.

L'invention vise aussi une composition vaccinale destinée à l'immunisation de l'homme ou l'animal à l'encontre d'une infection bactérienne ou virale, telle que la tuberculose ou l'hépatite, caractérisée en ce qu'elle comprend un ou plusieurs polypeptides hybrides tels que précédemment définis en association avec un véhicule pharmaceutiquement compatible et, le cas échéant, un ou plusieurs adjuvants de l'immunité.

Avantageusement, dans le cas d'une protéine hybride entre un polypeptide selon l'invention et l'antigène de surface de l'hépatite B, la composition vaccinale sera administrée, chez l'homme, à raison de 0,1 à 1 µg de protéine hybride purifiée par kilogramme du poids du patient, de préférence 0,2 à 0,5 µg/kg de poids du patient, pour une dose destinée à une administration donnée. Dans le cas de patients atteints de troubles du système immunitaire, en particulier les patients immunodéprimés, chaque dose injectée contiendra préférentiellement la moitié de la quantité pondérale de la protéine hybride contenue dans une dose destinée à un patient n'étant pas affecté de troubles du système immunitaire.

De préférence, la composition vaccinale sera administrée à plusieurs reprises, de manière étalée dans le temps, par voie intradermique ou sous-cutanée. A titre d'exemple, trois doses telles que définies ci-dessus seront respectivement administrées au patient au temps t0, au temps t0 + 1 mois et au temps t0 + 1 an.

Alternativement, trois doses seront respectivement administrées au patient au temps t0, au temps t0 + 1 mois et au temps t0 + 6 mois.

Chez la souris, chez laquelle une dose pondérale de la composition vaccinale comparable à la dose utilisée chez l'homme est administrée, la réaction anticorps est testée par prélèvement du sérum suivi d'une étude de la formation d'un complexe entre les anticorps présents dans le sérum et l'antigène de la composition vaccinale, selon les techniques usuelles.

L'invention concerne également une composition immunogène caractérisée en ce qu'elle comprend un polynucléotide ou un vecteur d'expression selon l'invention, en association avec un véhicule permettant son administration à l'homme ou l'animal.

L'invention a encore pour objet un vaccin destiné à l'immunisation à l'encontre d'une infection bactérienne ou virale, telle que la tuberculose ou l'hépatite, caractérisé en ce qu'il comprend un polynucléotide ou un vecteur d'expression selon l'invention, en association avec un véhicule pharmaceutiquement acceptable.

De telles compositions immunogènes ou vaccinales sont notamment décrites dans la demande internationale N° WO 90/11092 (Vical Inc.) et également dans la demande internationale N° WO 95/11307 (Institut Pasteur).

Le polynucléotide constitutif de la composition immunogène ou de la composition vaccinale selon l'invention peut être injecté à l'hôte après avoir été couplé à des composés qui favorisent la pénétration de ce polynucléotide à l'intérieur de la cellule ou son transport jusqu'au noyau cellulaire. Les conjugués résultants peuvent être encapsulés dans des microparticules polymères, comme décrit dans la demande internationale N° WO 94/27238 (medisorb Technologies International).

Selon un autre mode de réalisation de la composition immunogène et/ou vaccinale selon l'invention, le polynucléotide, de préférence un ADN, est complexé avec du DEAE-dextran (Pagano et al., 1967) ou avec des protéines nucléaires (Kaneda et al., 1989), avec des lipides (Felgner et al., 1987) ou encore encapsulés dans des liposomes (Fraley et al., 1980).

Selon encore un autre mode de réalisation avantageux de la composition immunogène et/ou vaccinale selon l'invention, le polynucléotide selon l'invention peut être introduit sous la forme d'un gel facilitant sa transfection dans les cellules. Une telle composition sous forme de gel peut être un complexe de poly-L-lysine et de lactose, comme décrit par Midoux en 1993, ou encore le Poloxamer 407^{™}, comme décrit par Pastore en 1994. Le polynucléotide ou le vecteur selon l'invention peuvent aussi être en suspension dans une solution tampon ou être associés à des liposomes.

Avantageusement, un tel vaccin sera préparé conformément à la technique décrite par Tacson et al. ou Huygen et al. en 1996 ou encore conformément à la technique décrite par Davis et al. dans la demande internationale N° WO 95/11307 (Whalen et al.).

Un tel vaccin sera avantageusement préparé sous la forme d'une composition contenant un vecteur selon l'invention, placée sous le contrôle d'éléments de régulation permettant son expression chez l'homme ou l'animal.

Pour réaliser un tel vaccin, le polynucléotide selon l'invention est tout d'abord sous-cloné dans un vecteur d'expression approprié, plus particulièrement un vecteur d'expression contenant des signaux de régulation et d'expression reconnus par les enzymes des cellules eucaryotes et contenant également une origine de réplication active chez les procaryotes, par exemple chez *E*. *coli,* qui permet son amplification préalable. Puis le plasmide recombinant purifié obtenu est injecté à l'hôte, par exemple par voie intramusculaire.

On pourra par exemple utiliser, en tant que vecteur d'expression in vivo de l'antigène d'intérêt, le plasmide pcDNA3 ou le plasmide pcDNA1/neo, tous les deux commercialisés par Invitrogen (R&D Systems, Abingdon, Royaume-Uni). On peut aussi utiliser le plasmide V1Jns.tPA, décrit par Shiver et al. en 1995.

Un tel vaccin comprendra avantageusement, outre le vecteur recombinant, une solution saline, par exemple une solution de chlorure de sodium.

Une composition vaccinale telle que définie ci-dessus sera par exemple administrée par voie parentérale ou par voie intramusculaire.

La présente invention concerne également un vaccin caractérisé en ce qu'il contient une ou plusieurs séquences nucléotidiques selon l'invention et/ou un ou plusieurs polynucléotides tels que mentionnés ci-dessus en association avec un véhicule pharmaceutiquement compatible et, le cas échéant, un ou plusieurs adjuvants de l'immunité appropriés.

Un autre aspect porte sur une méthode de criblage de molécules capables d'inhiber la croissance de mycobactéries ou le maintien de mycobactéries dans un hôte, caractérisée en ce que lesdites molécules bloquent la synthèse ou la fonction des polypeptides codés par une séquence nucléotidique selon l'invention ou par un polynucléotide tel que décrit supra.

Dans ladite méthode de criblage, les molécules peuvent être des anti-messagers ou peuvent induire la synthèse d'anti-messagers.

La présente invention vise également des molécules capables d'inhiber la croissance de mycobactéries ou le maintien de mycobactéries dans un hôte, caractérisées en ce que lesdites molécules sont synthétisées d'après la structure des polypeptides codés par une séquence nucléotidique selon l'invention ou par un polynucléotide tel que décrit supra.

D'autres caractéristiques et avantages de l'invention apparaissent dans les exemples et les figures suivants :

### FIGURES

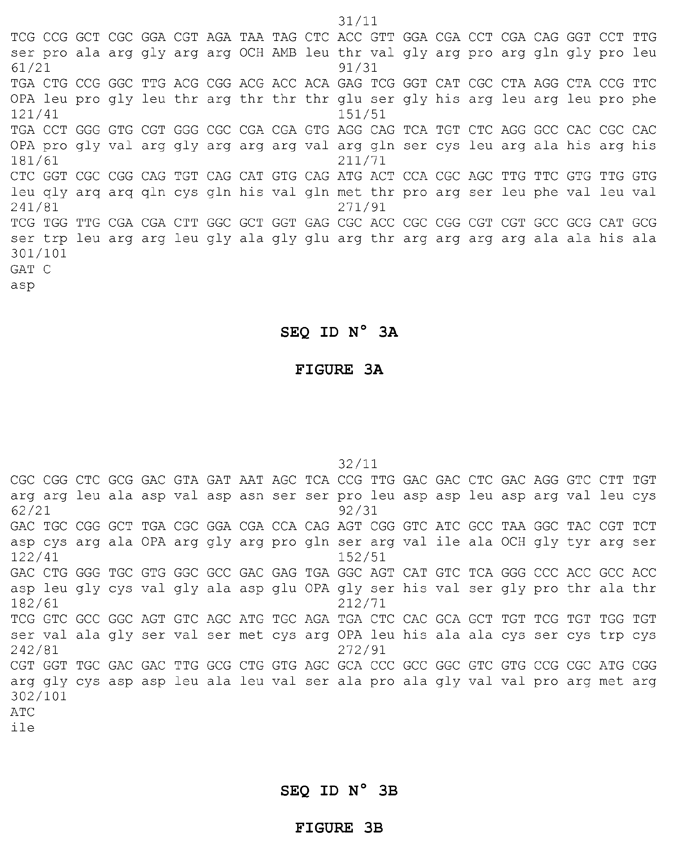

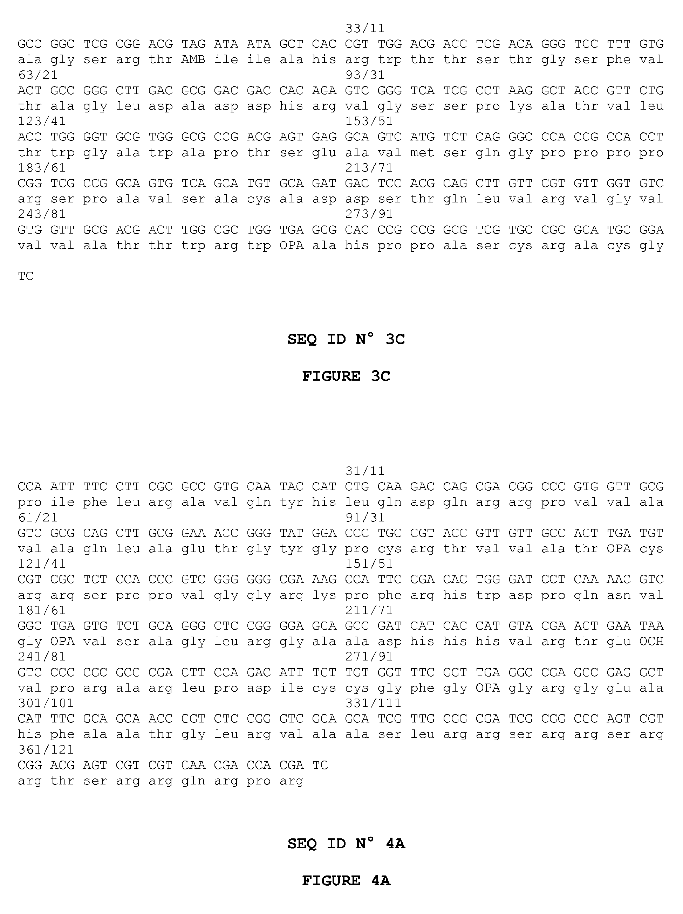

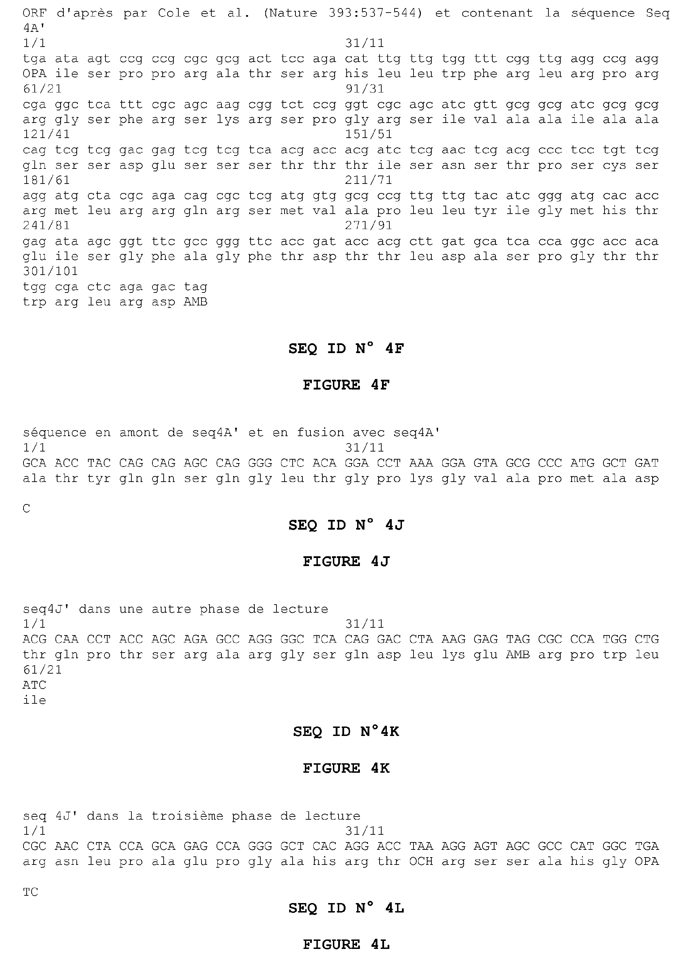

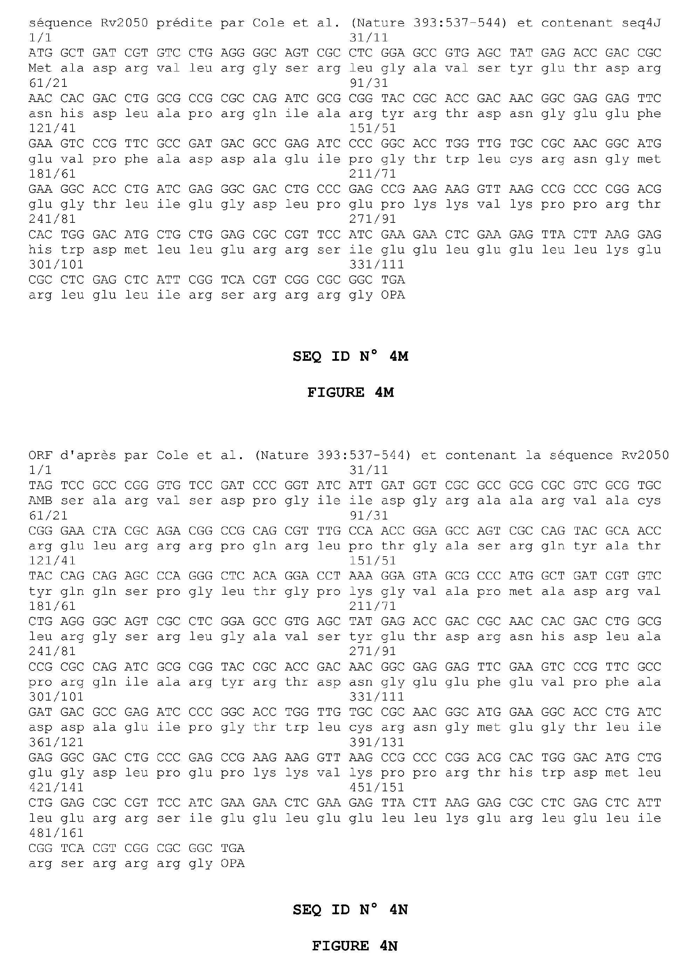

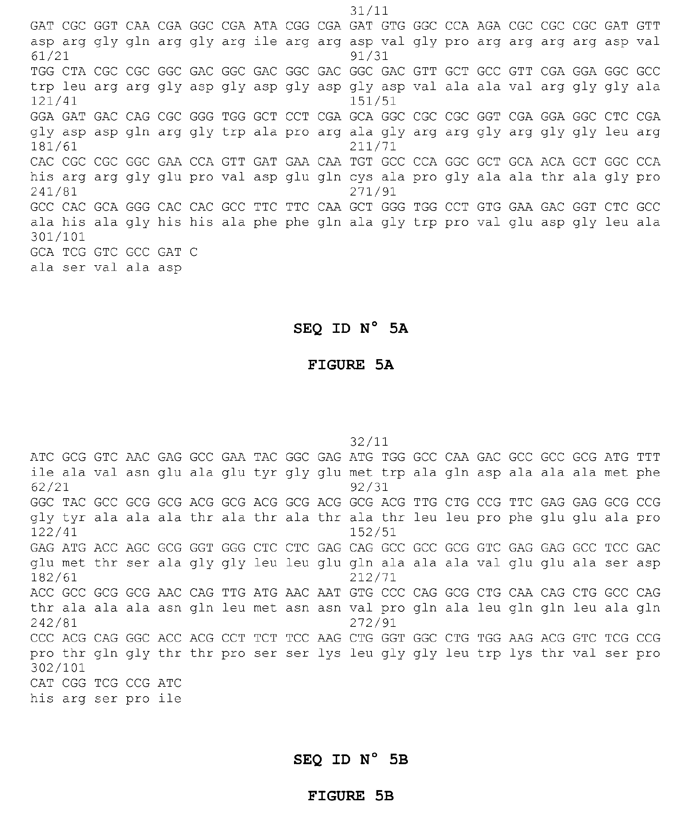

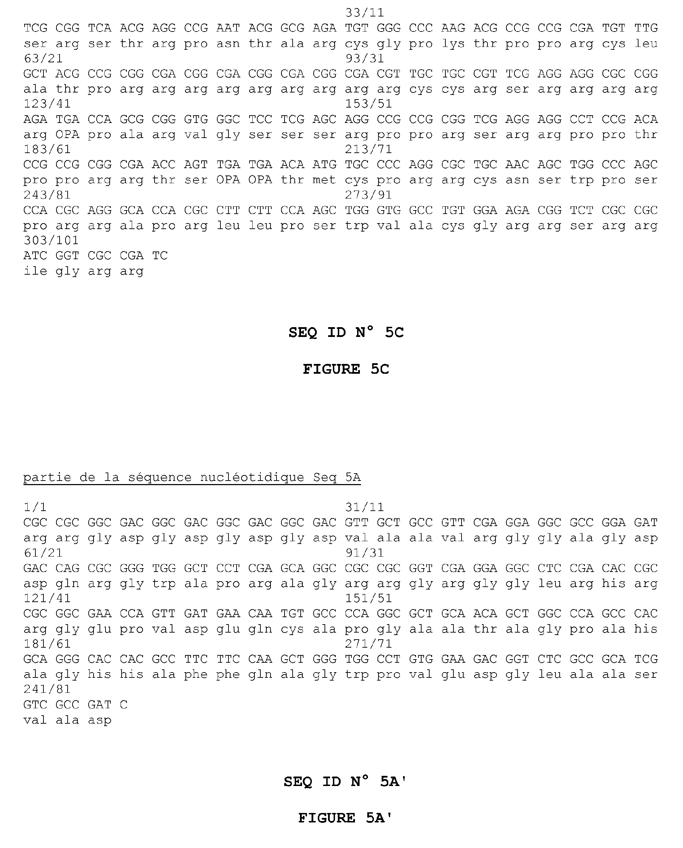

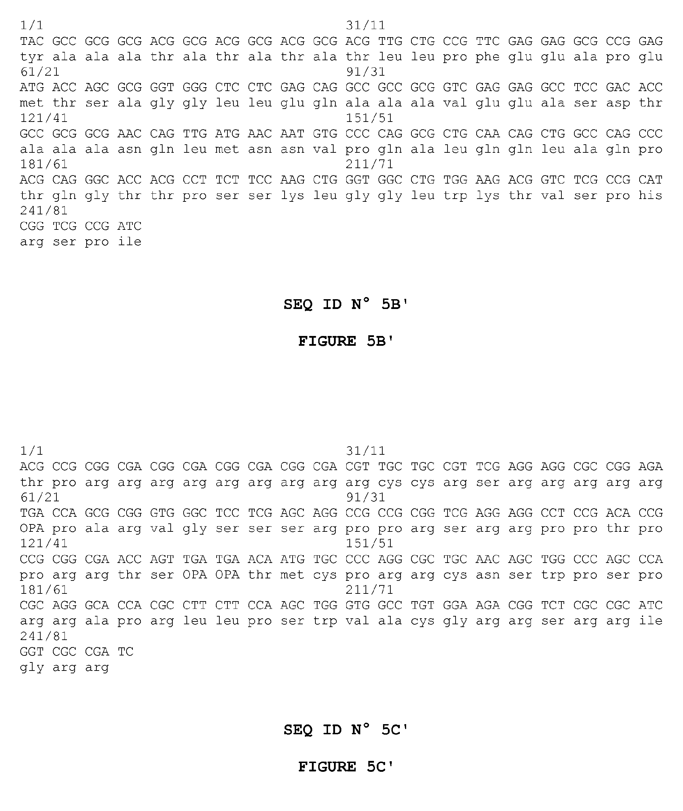

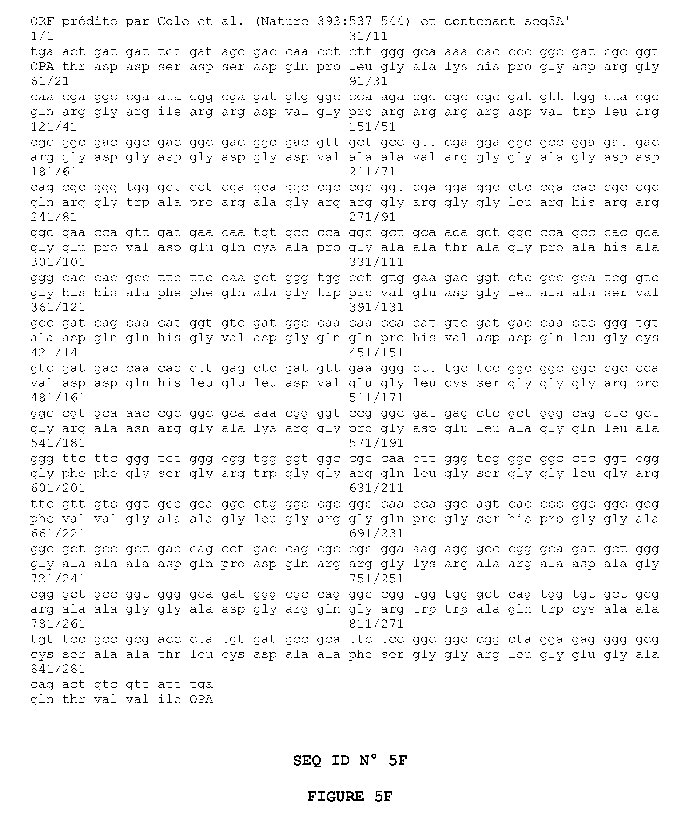

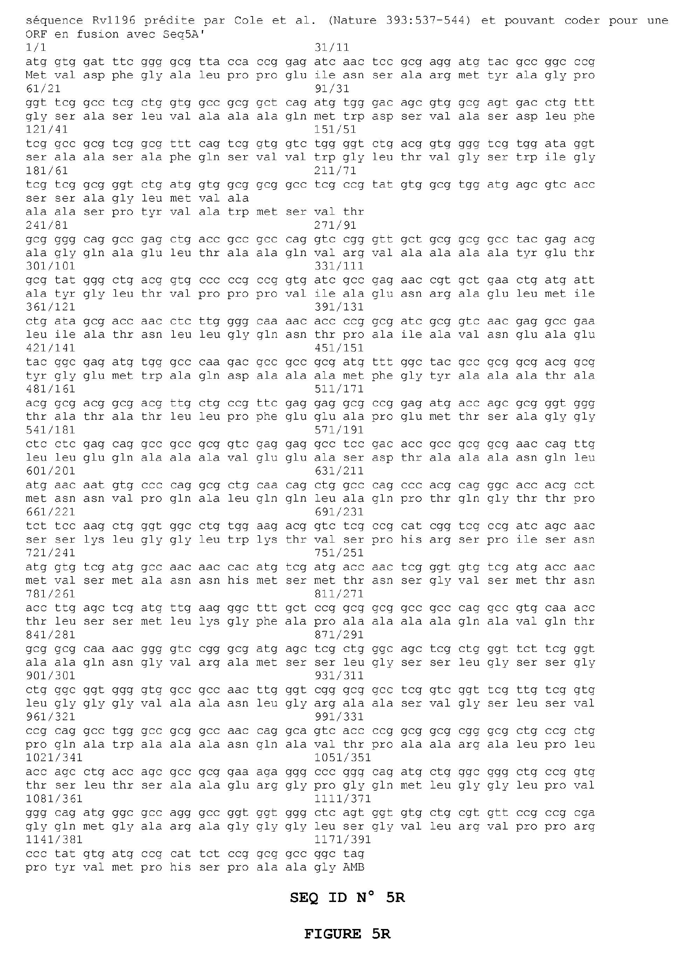

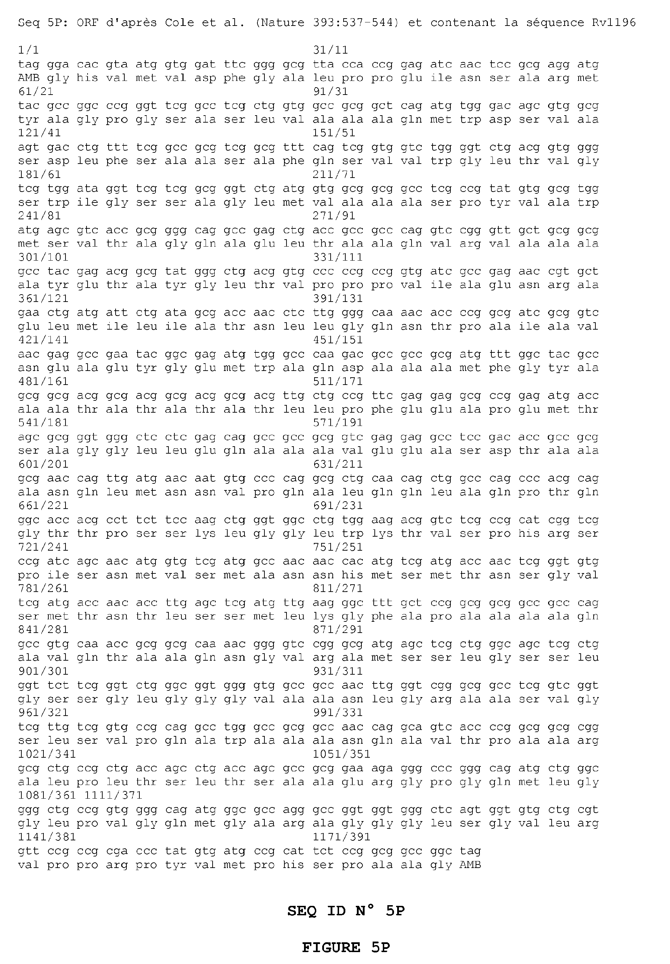

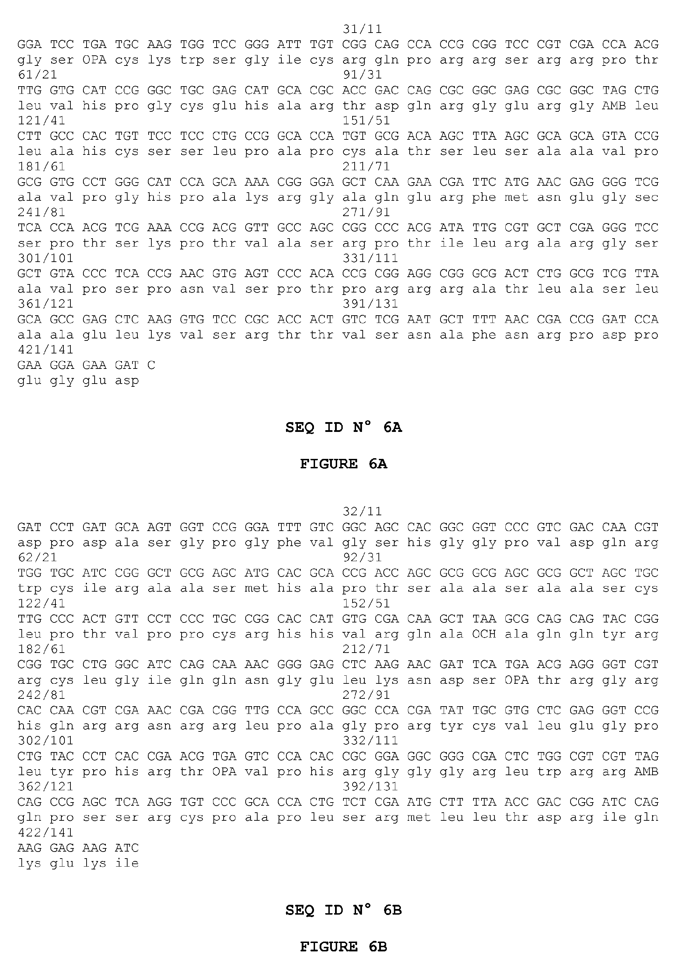

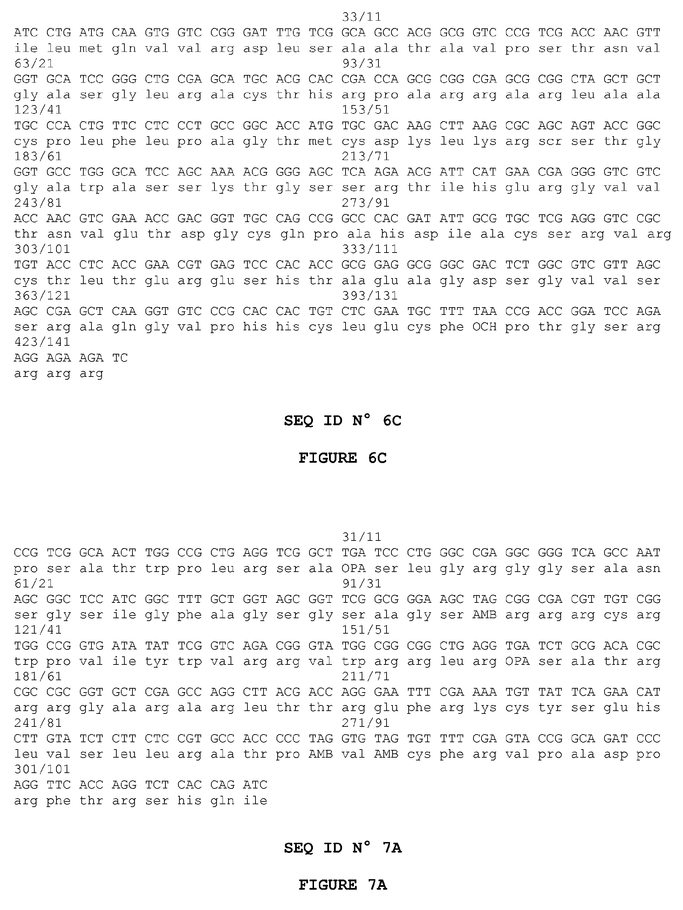

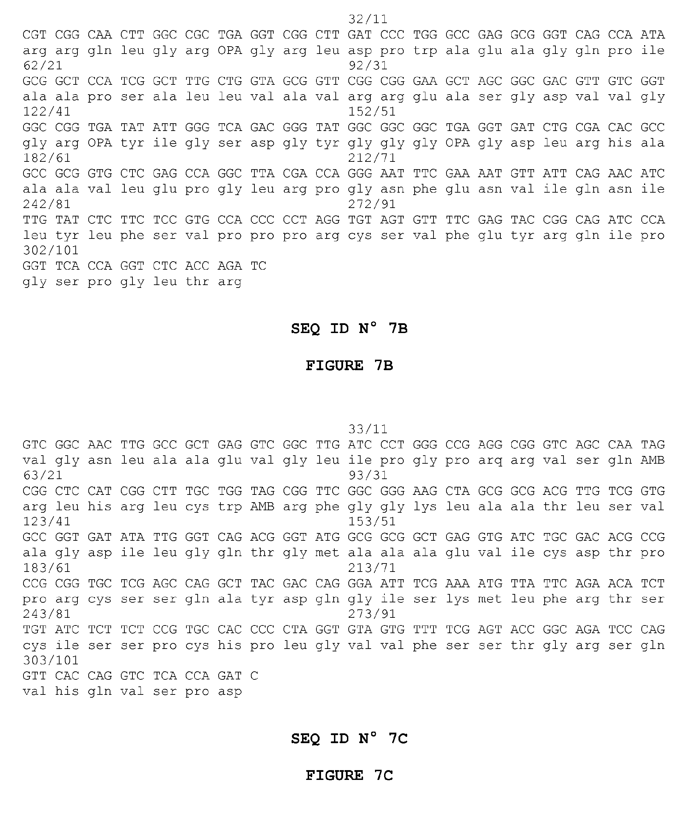

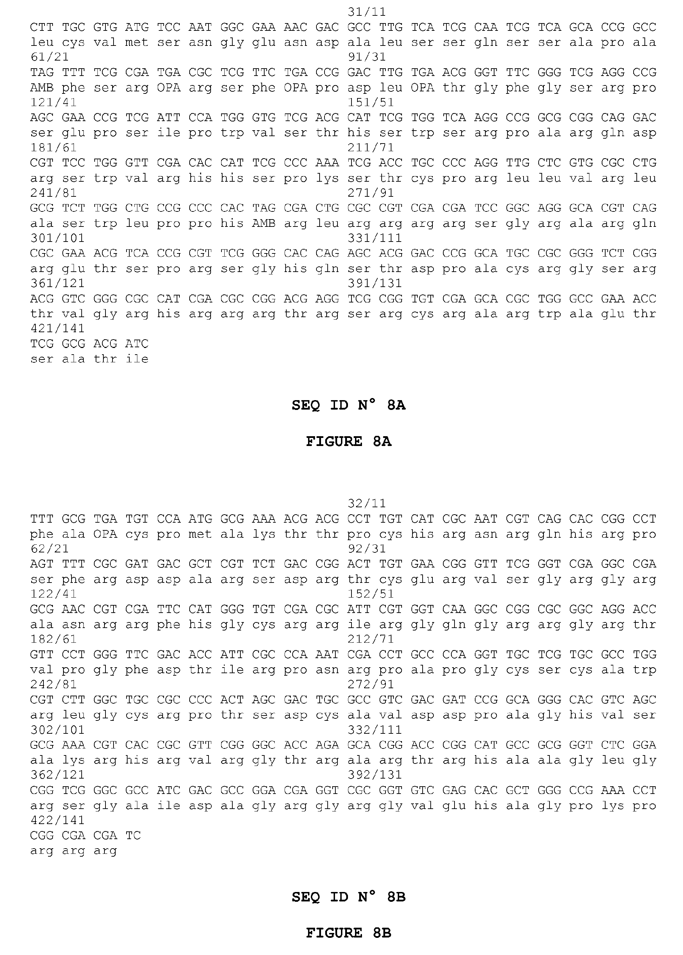

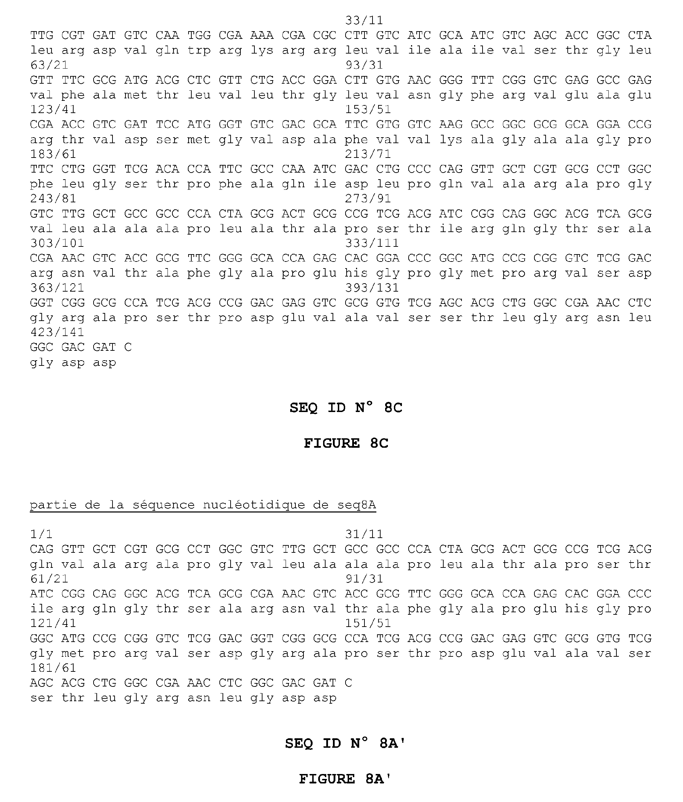

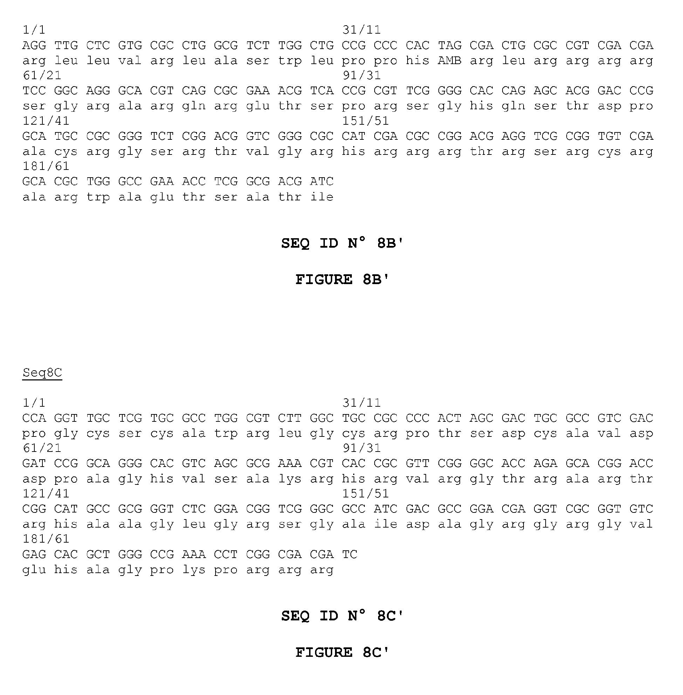

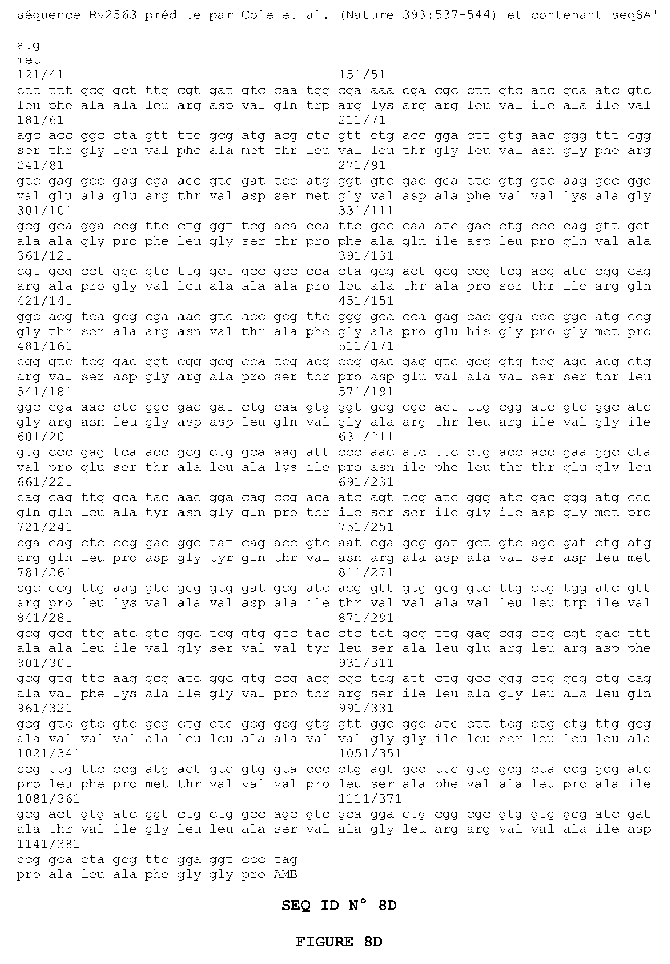

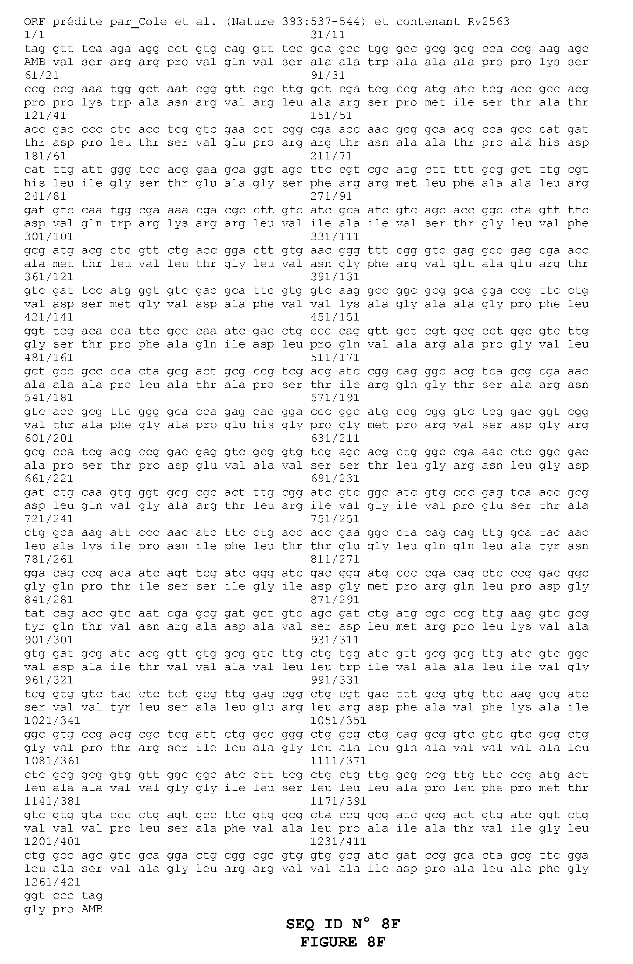

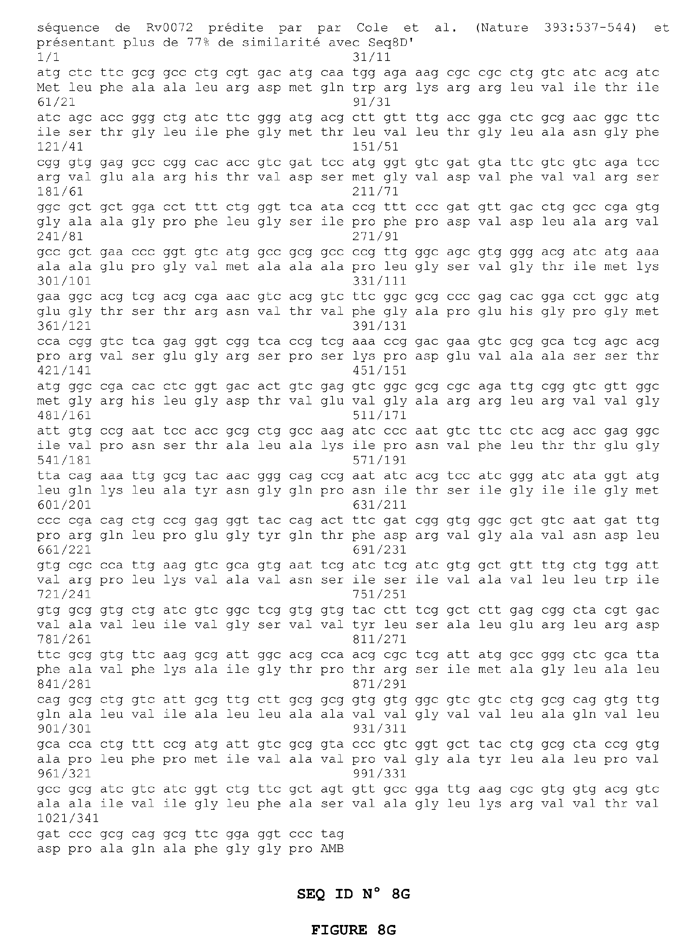

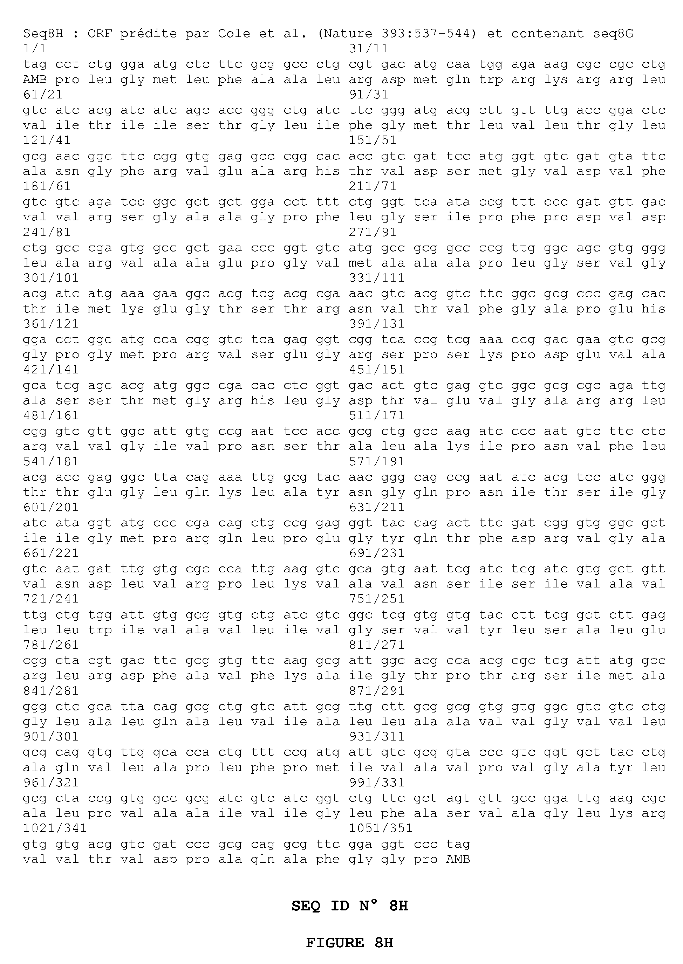

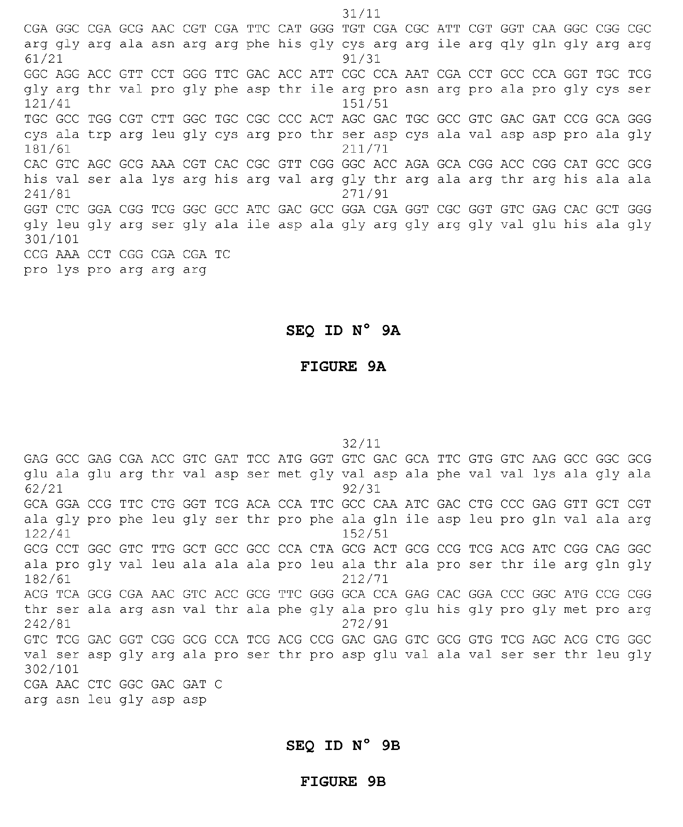

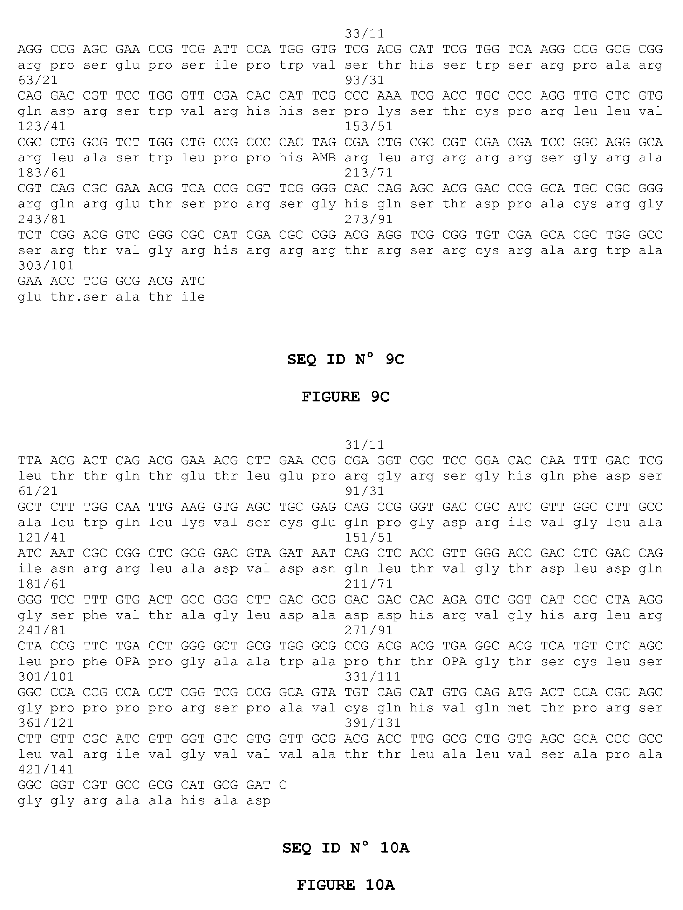

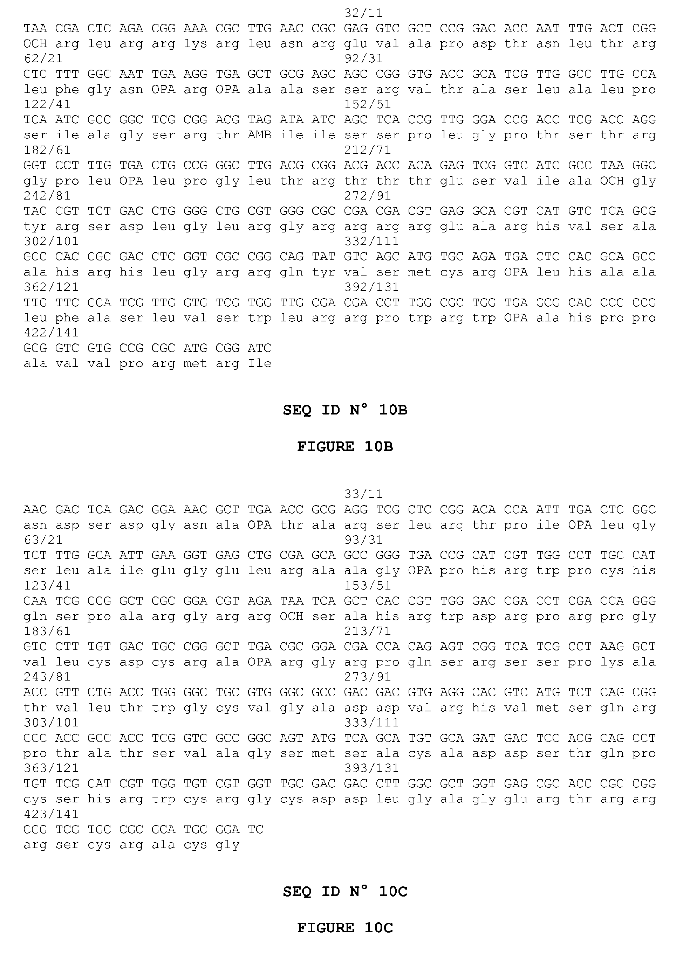

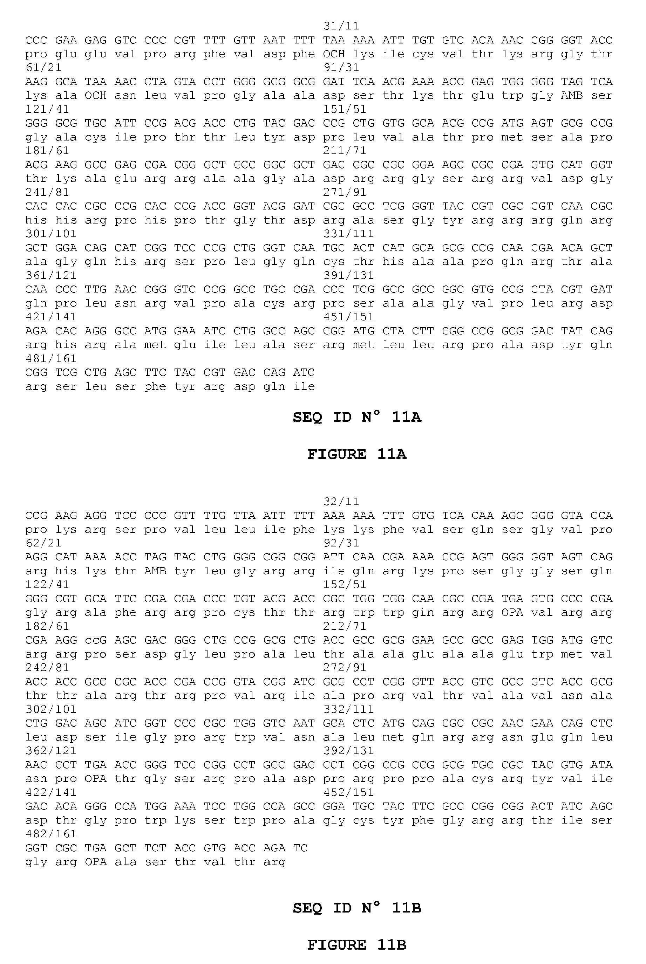

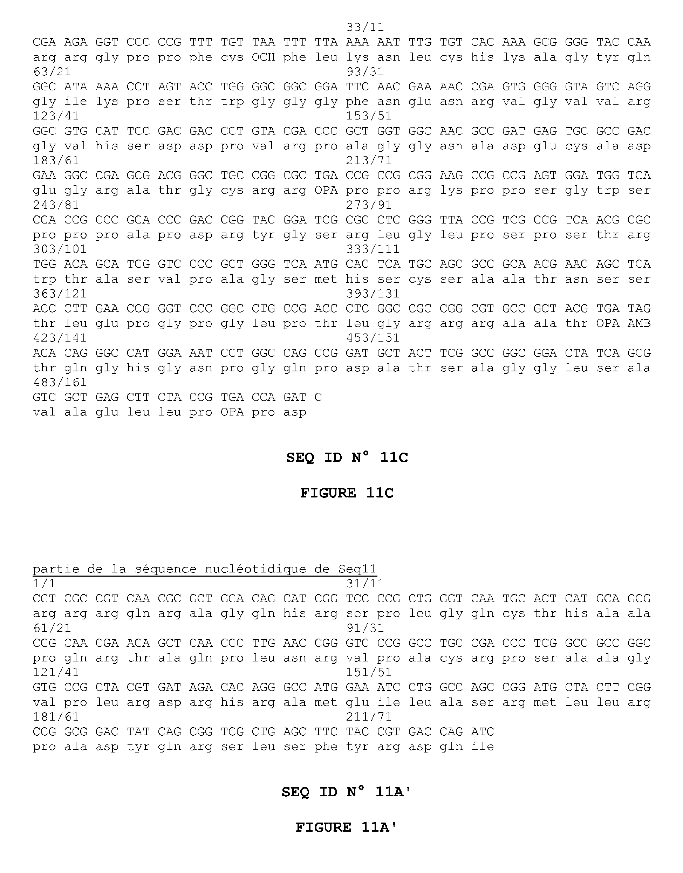

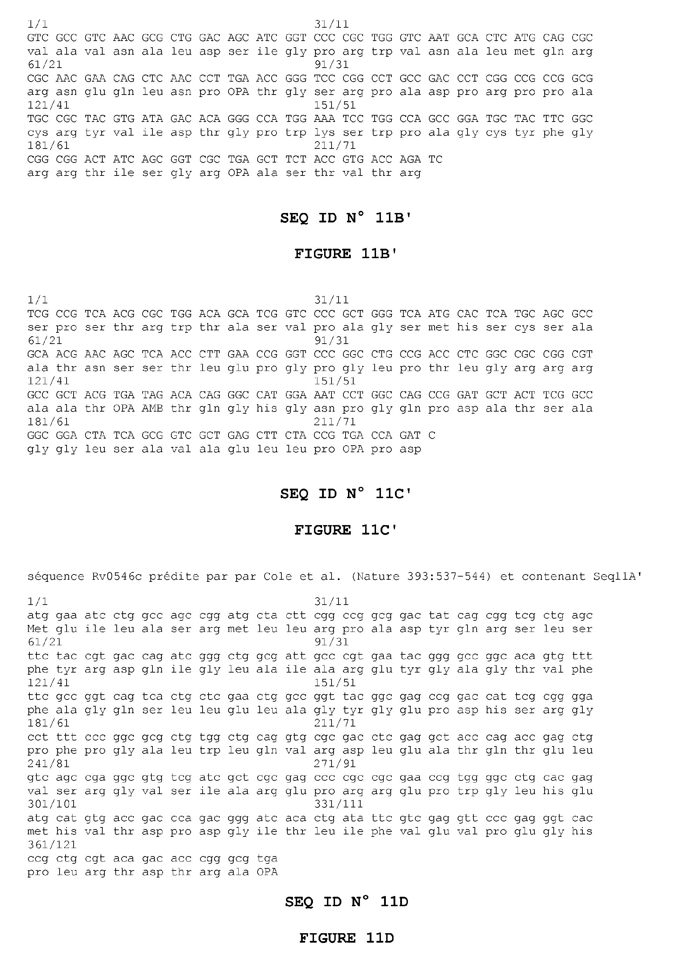

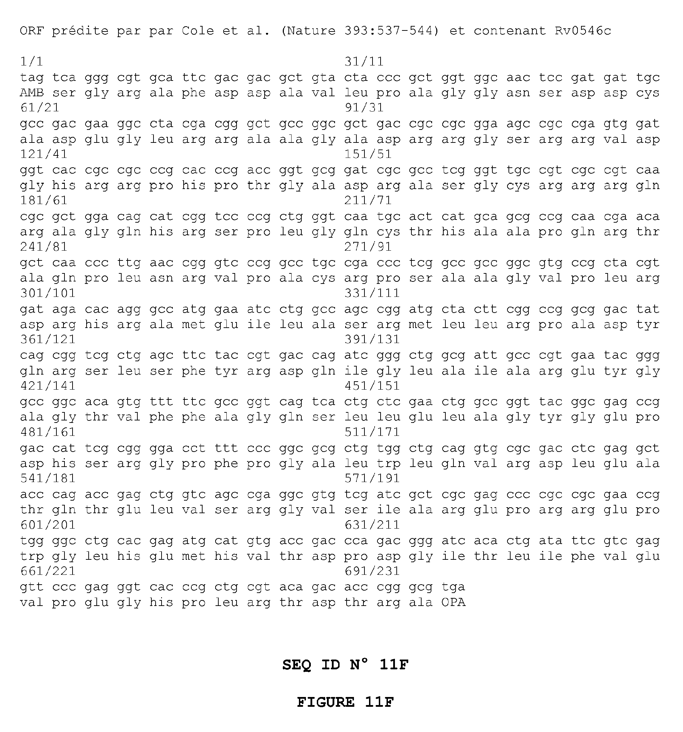

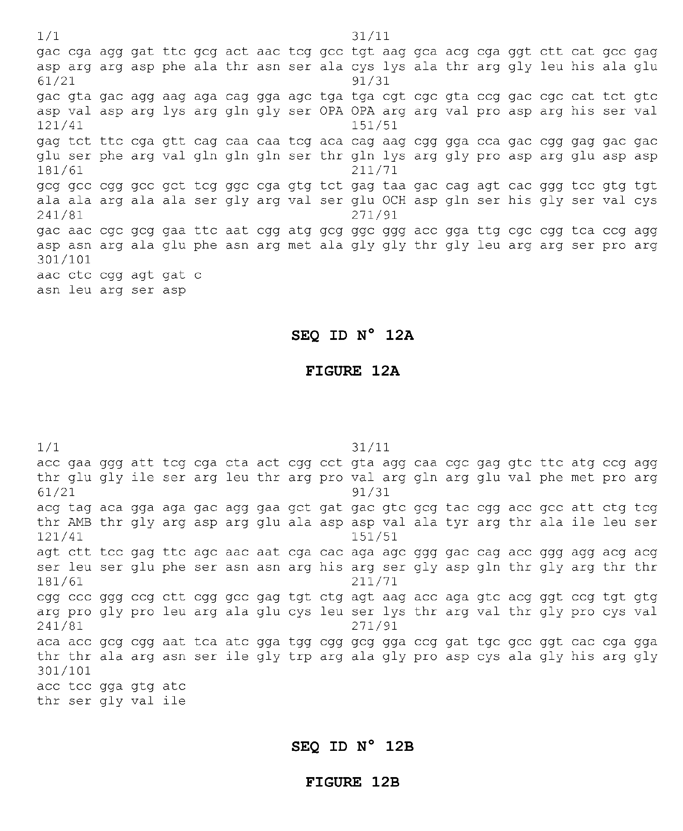

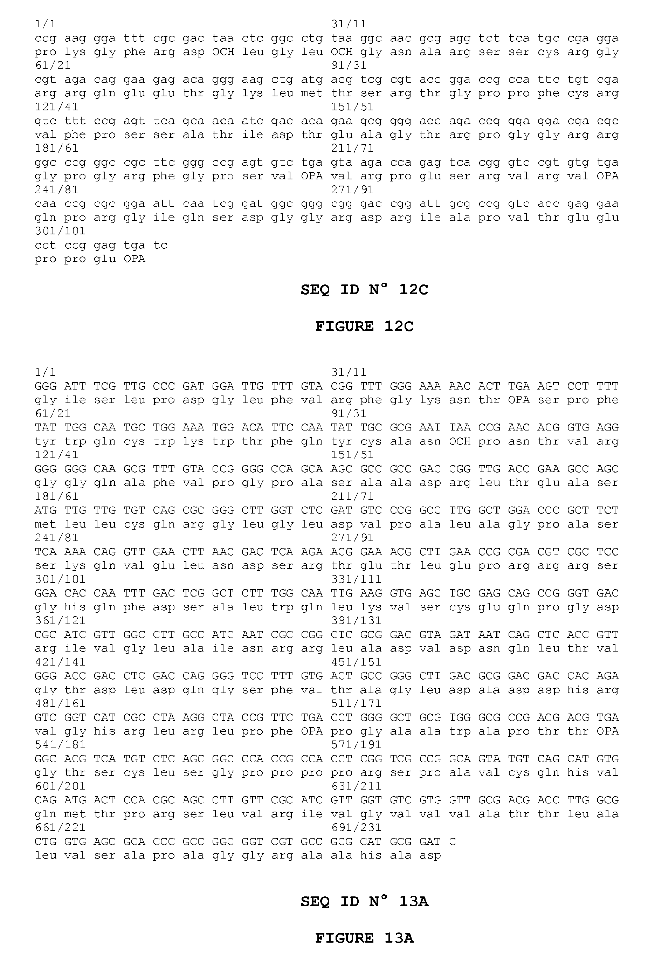

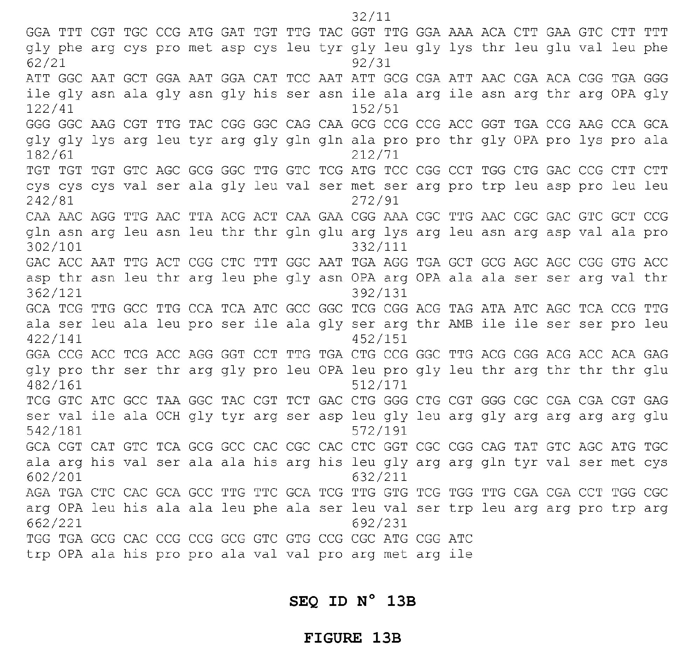

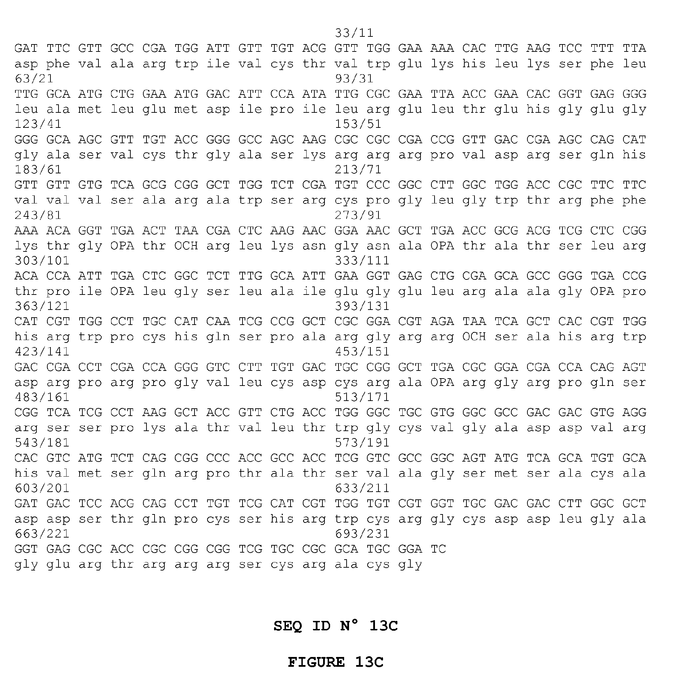

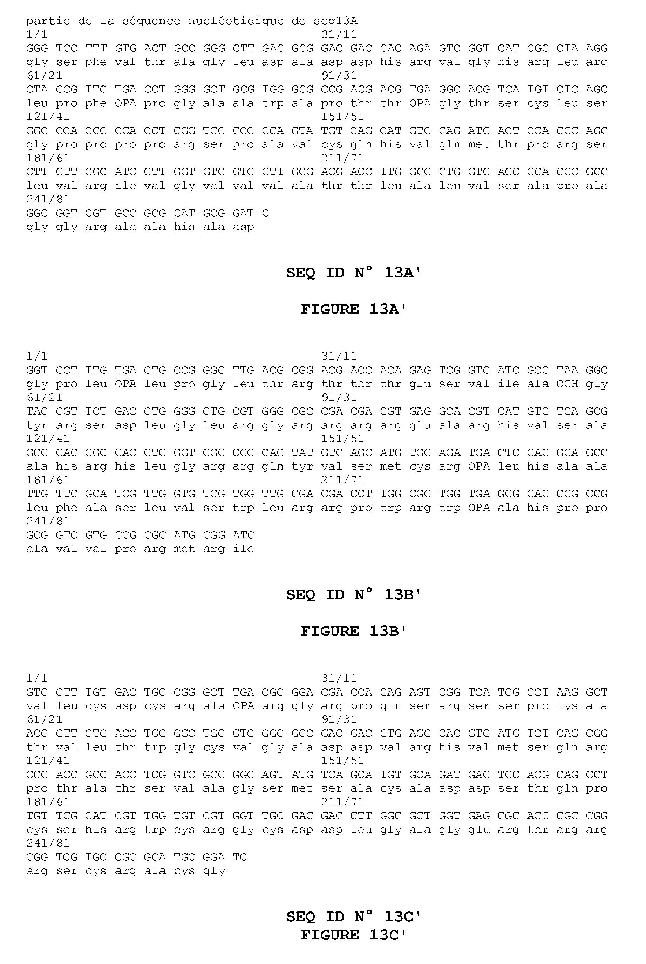

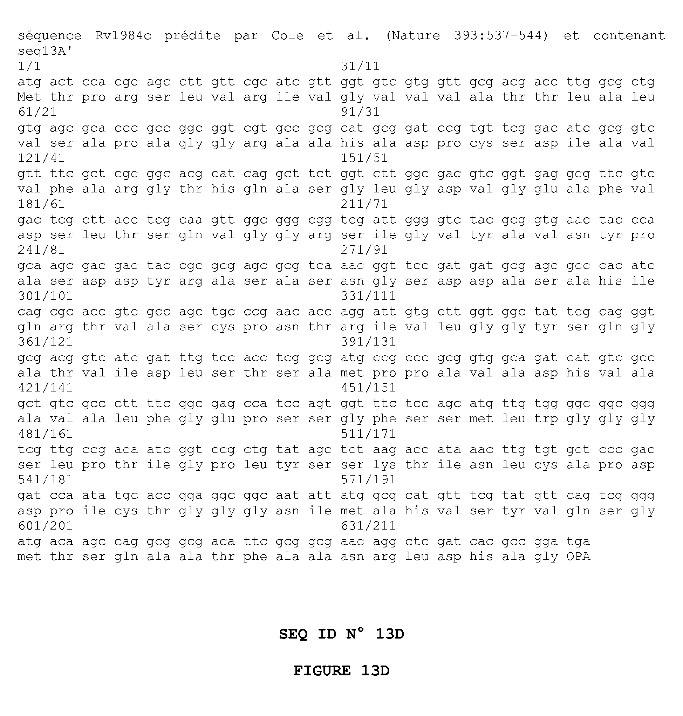

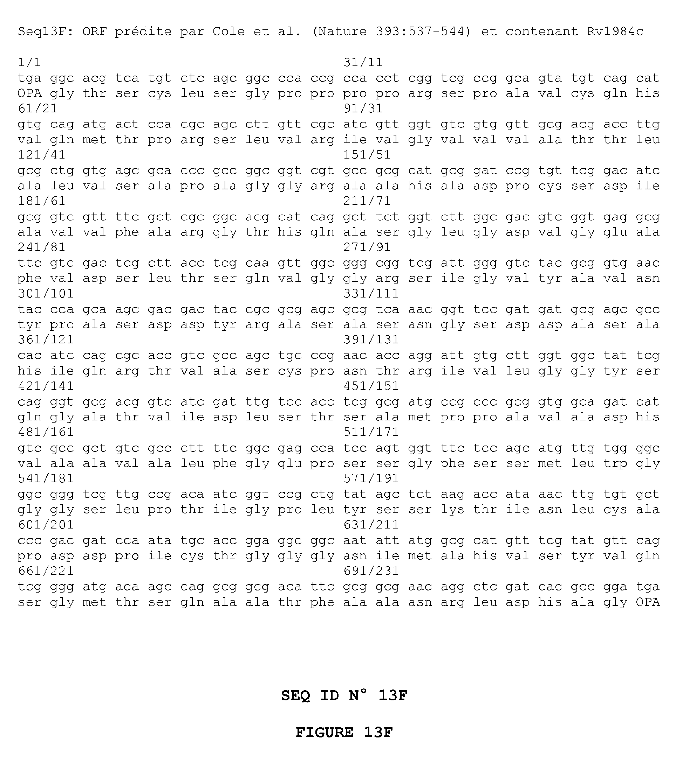

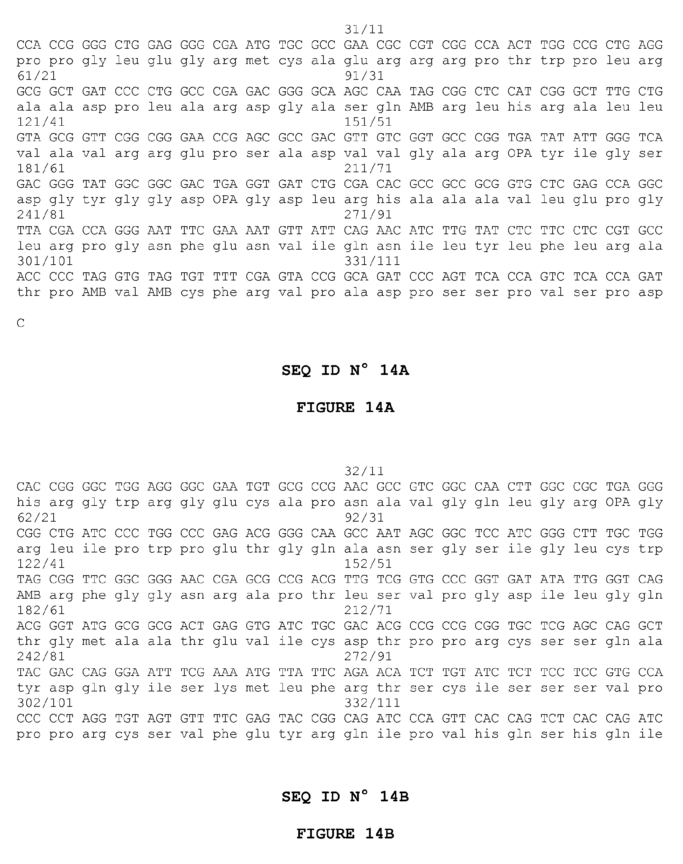

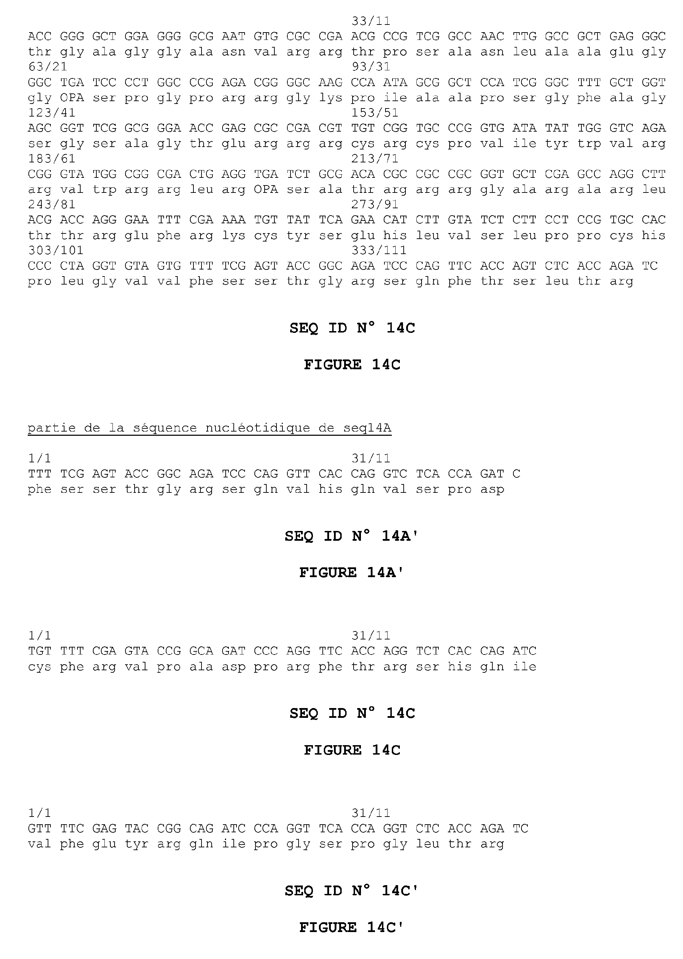

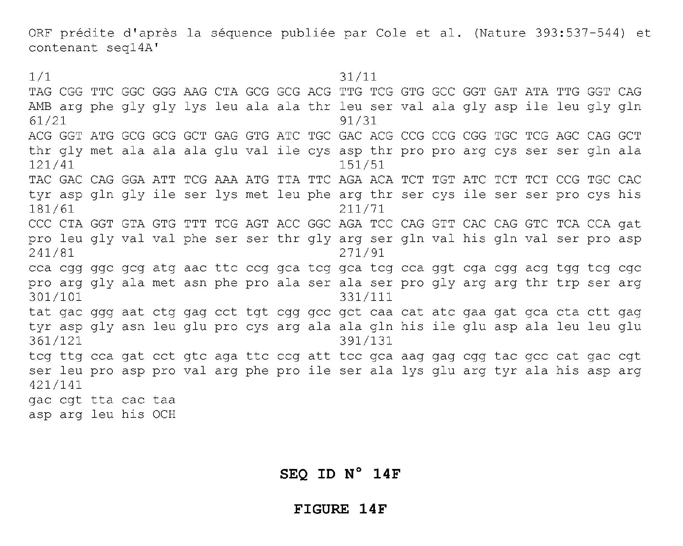

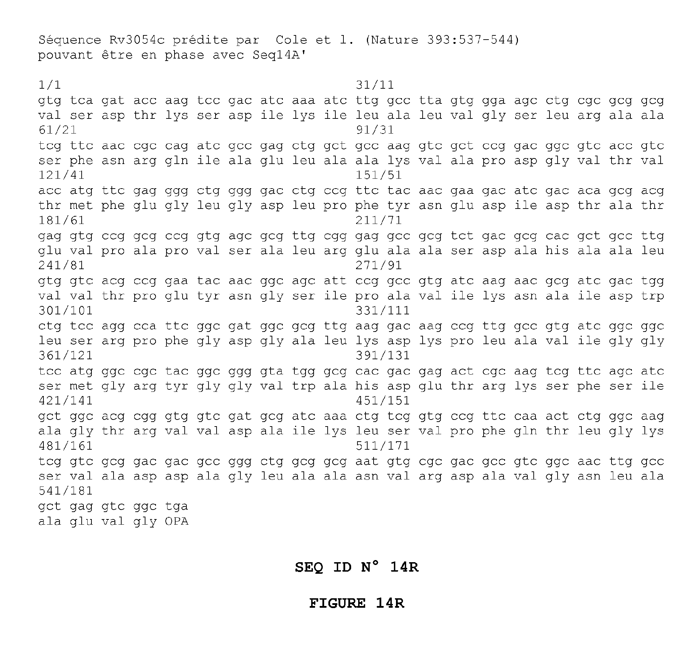

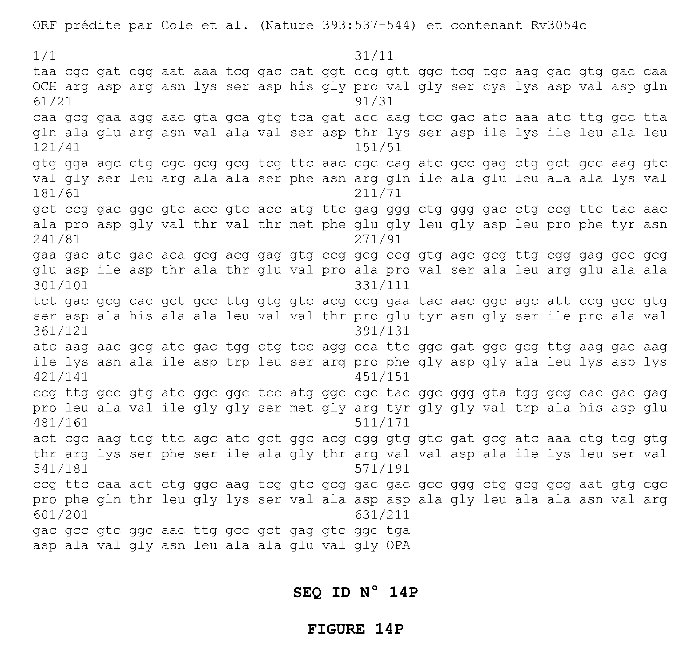

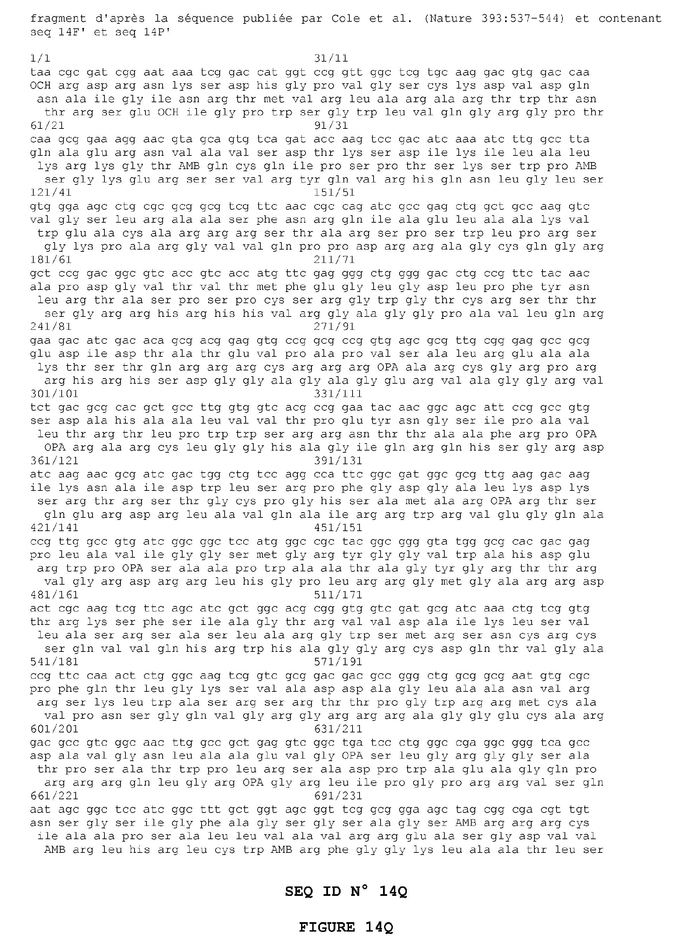

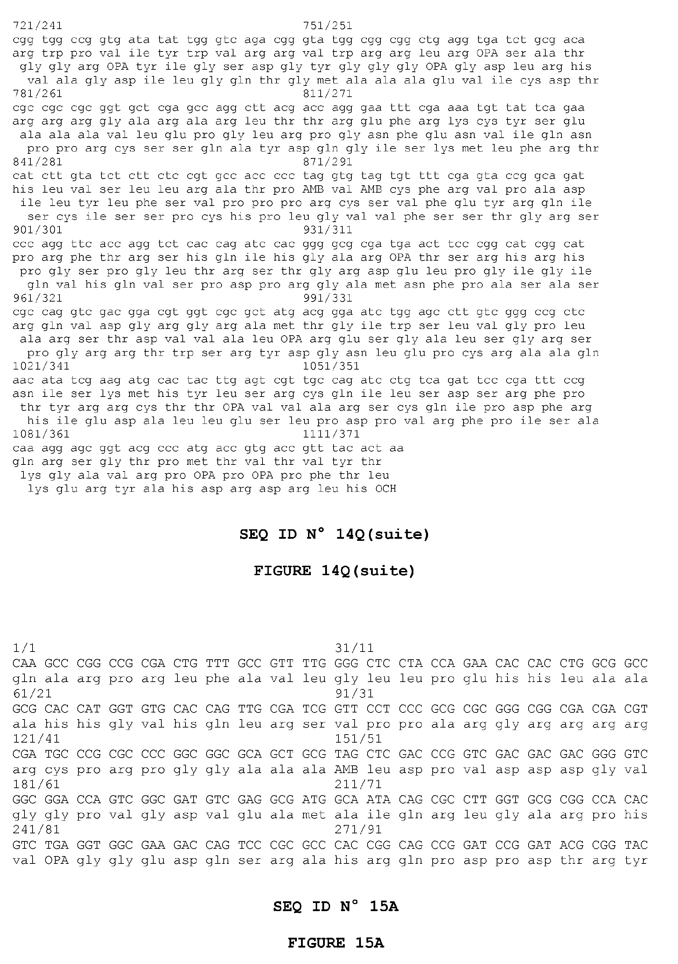

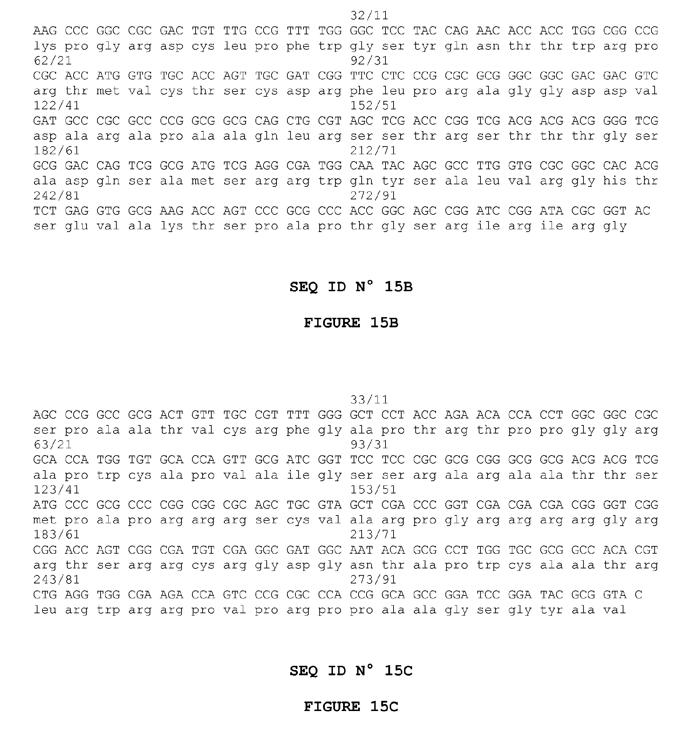

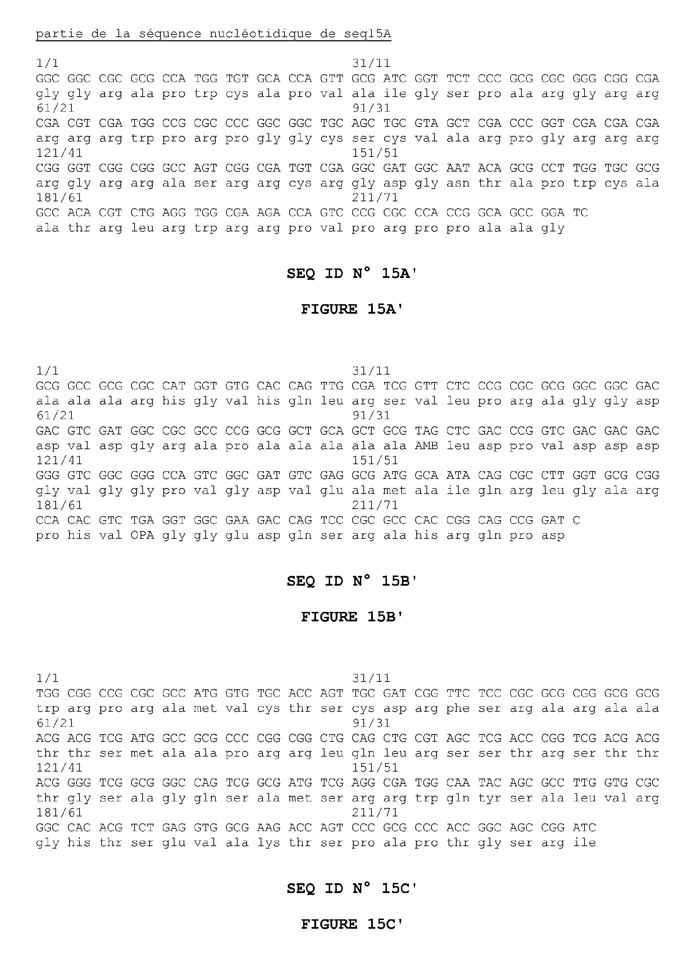

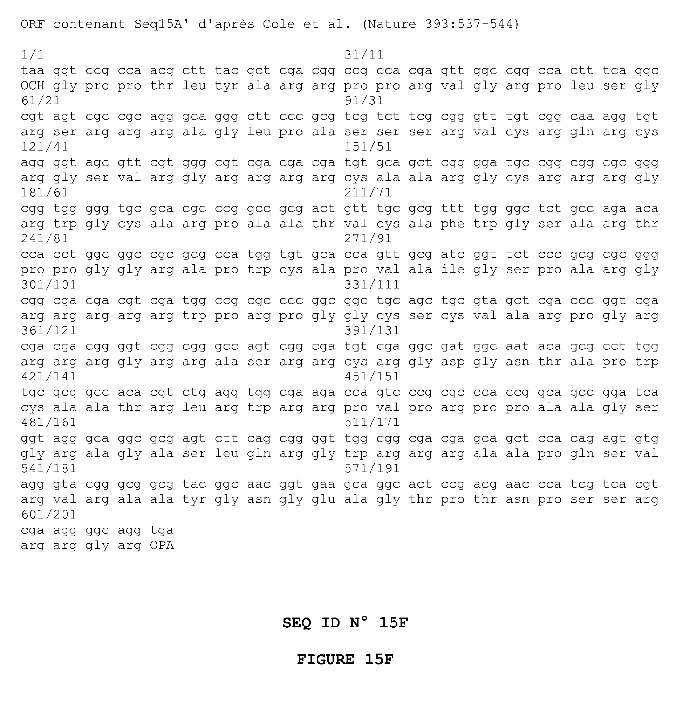

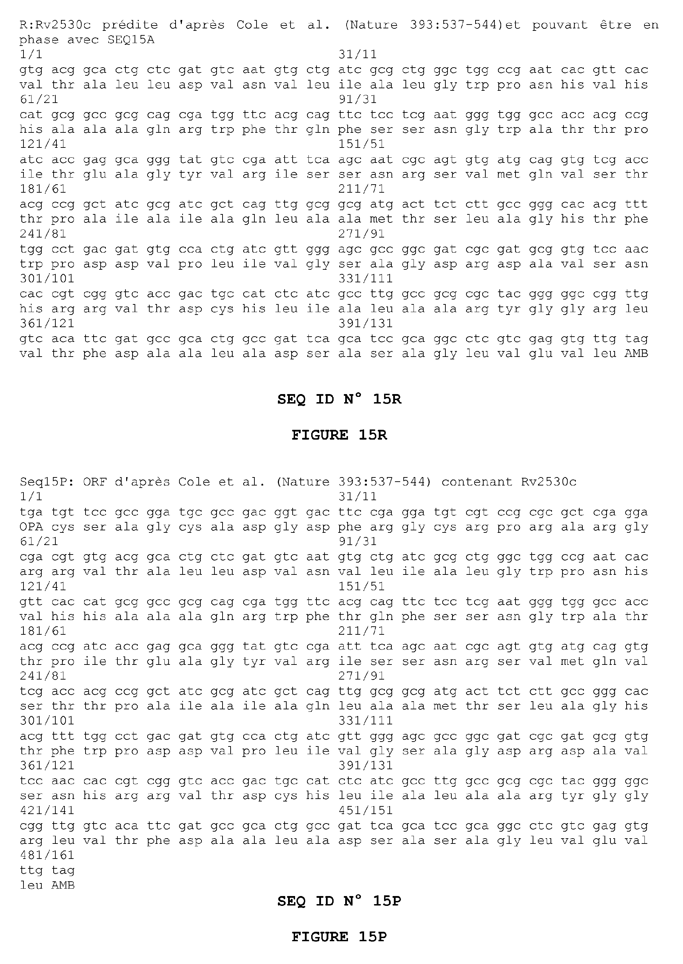

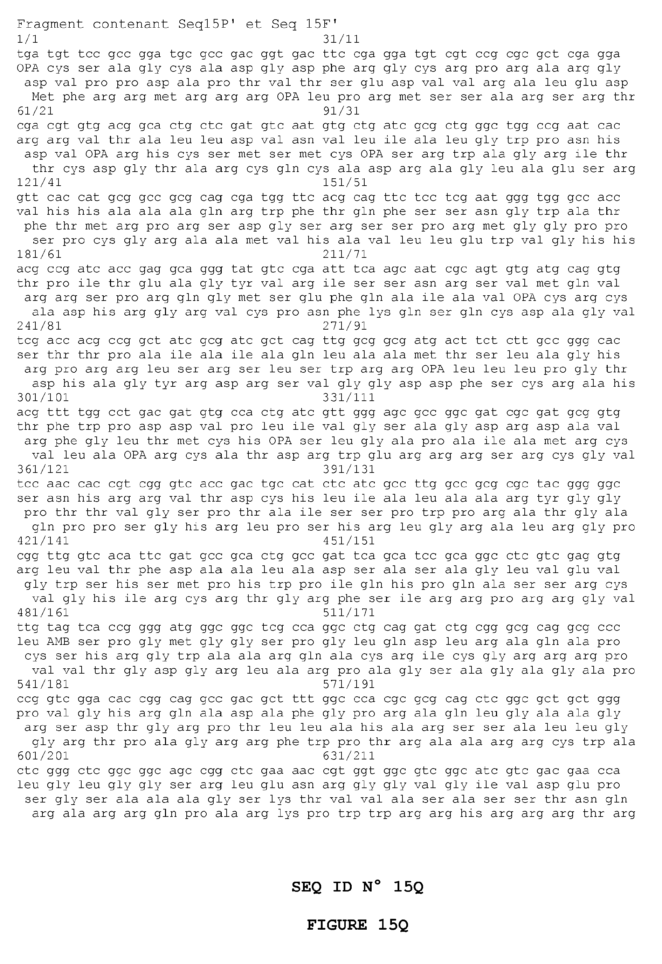

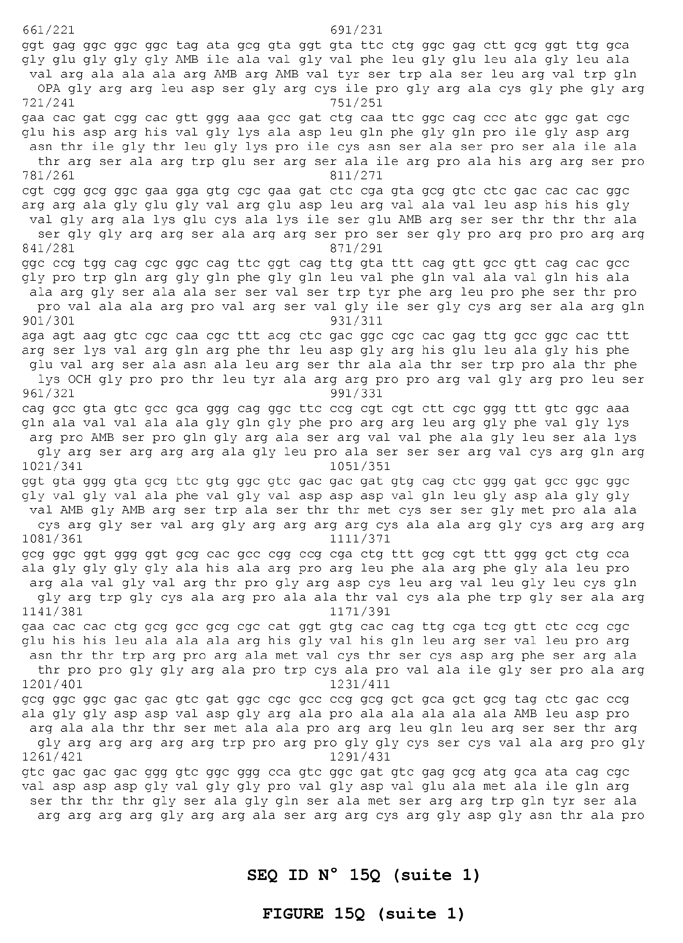

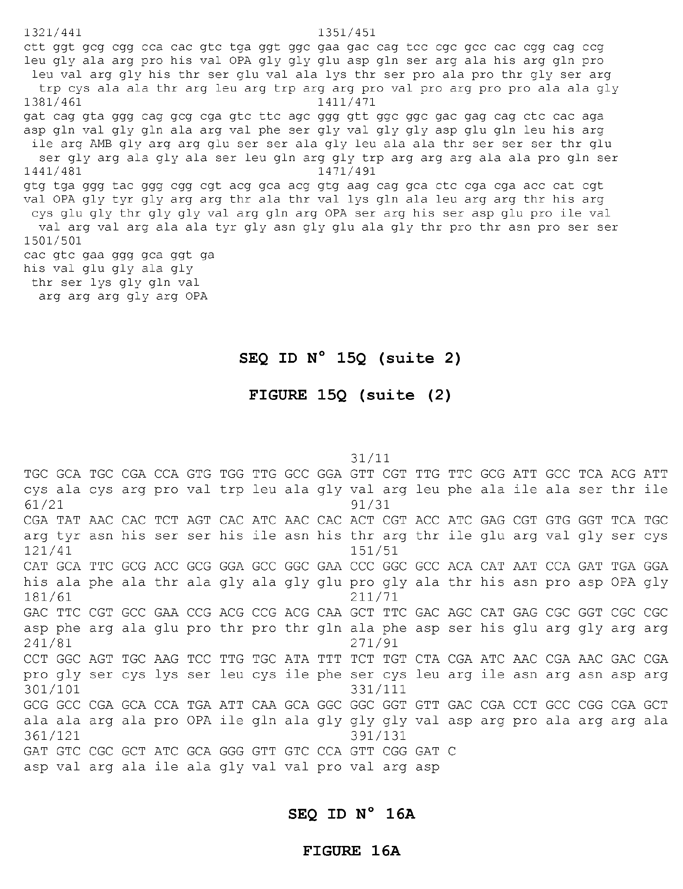

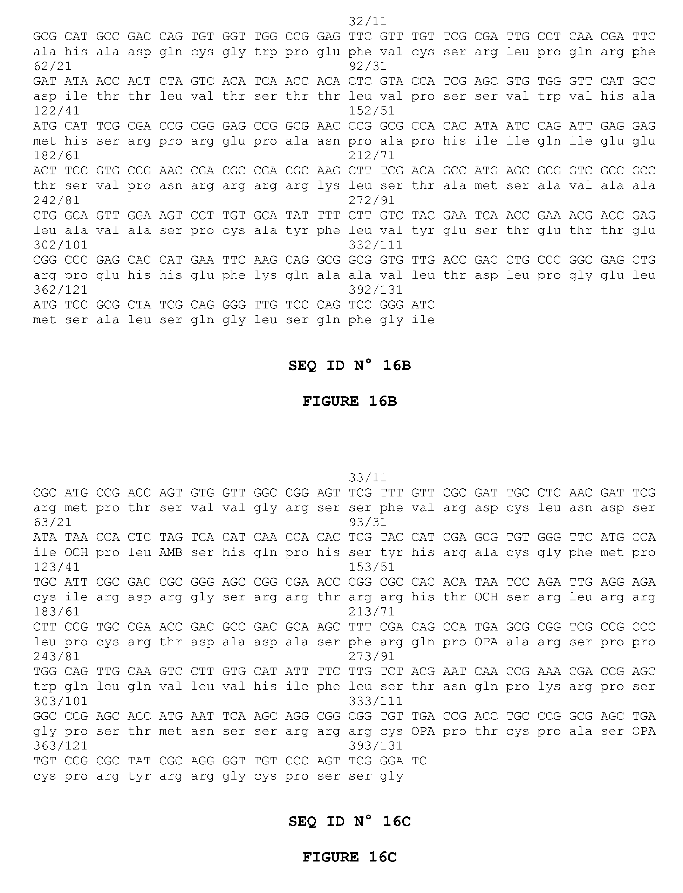

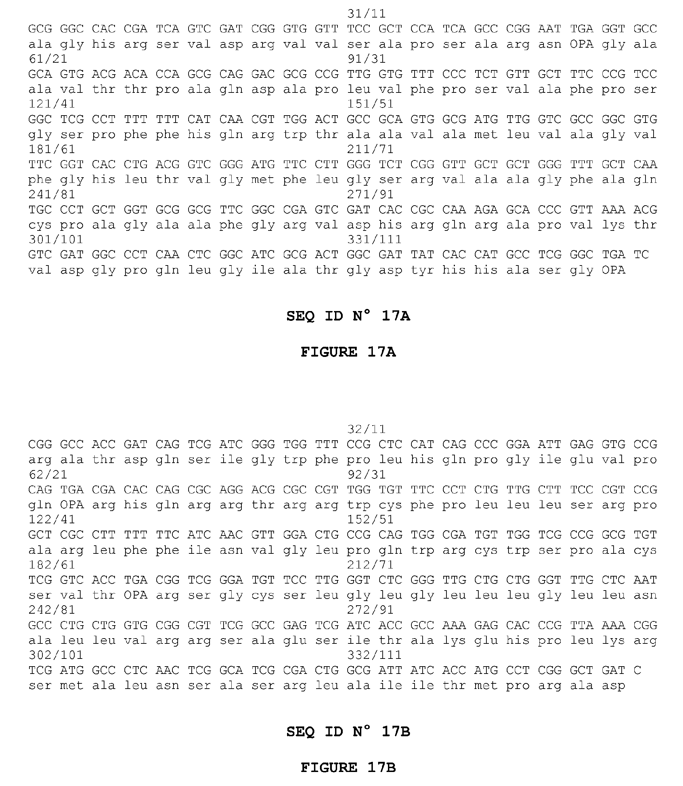

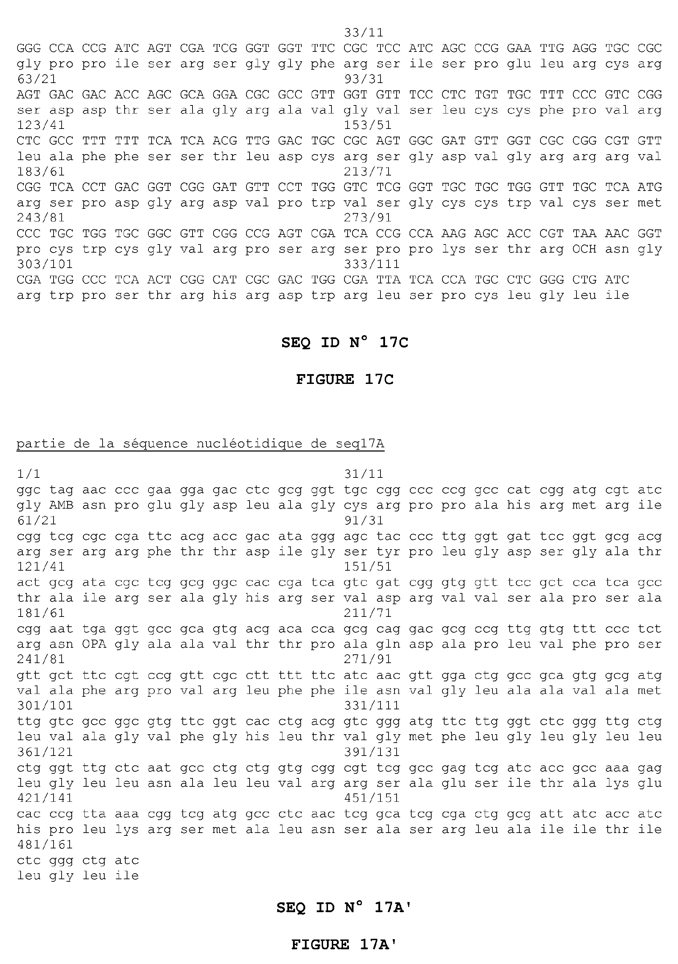

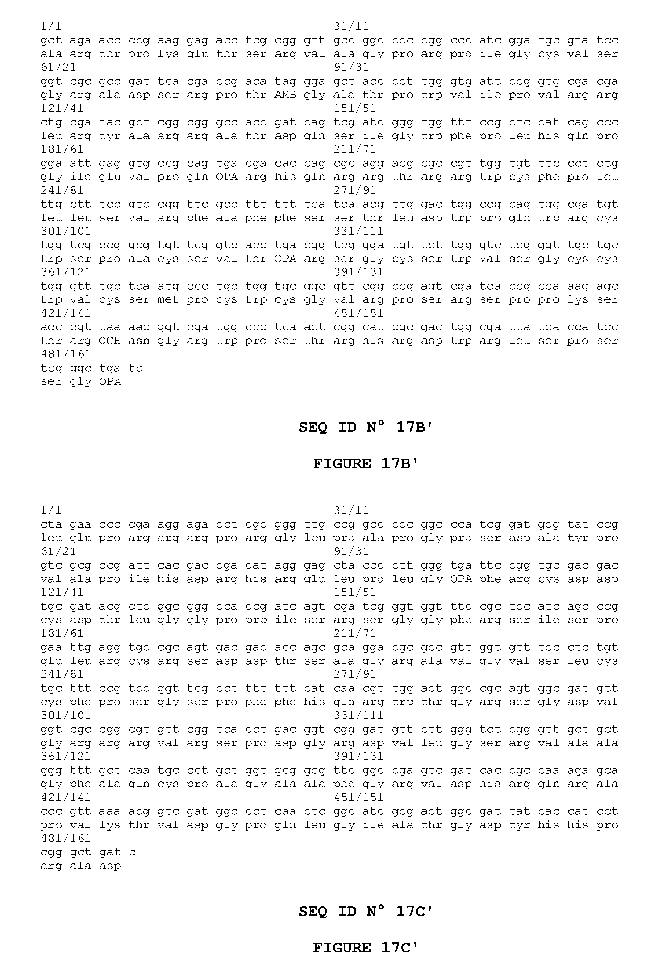

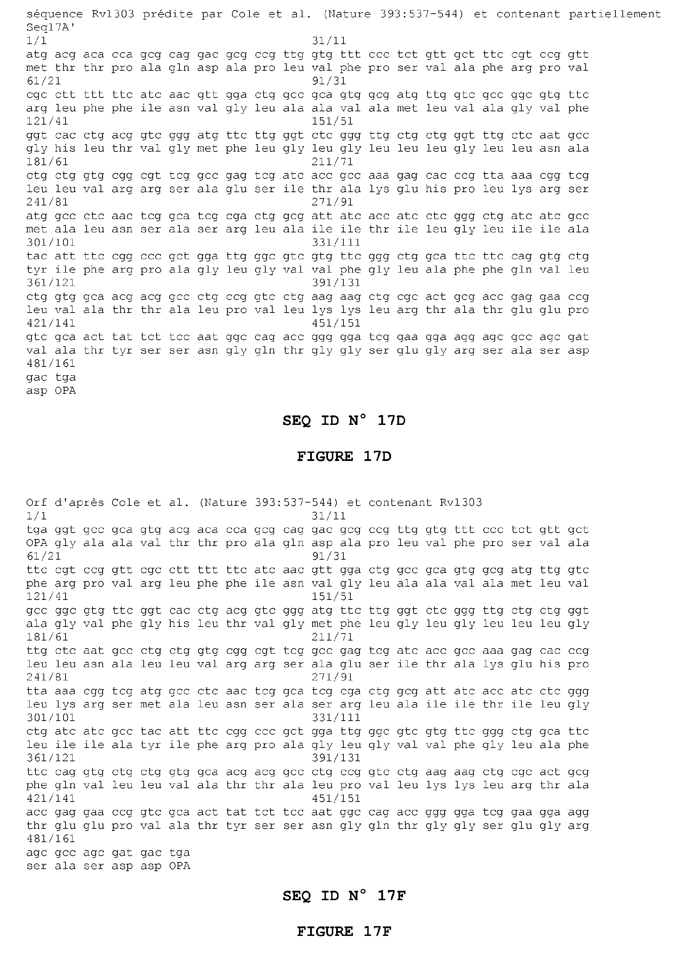

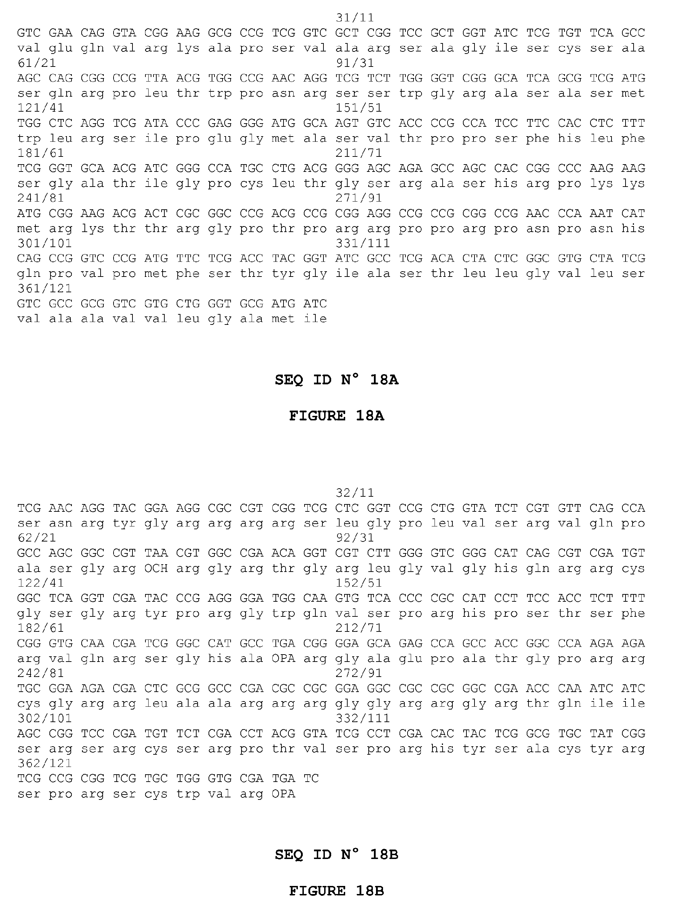

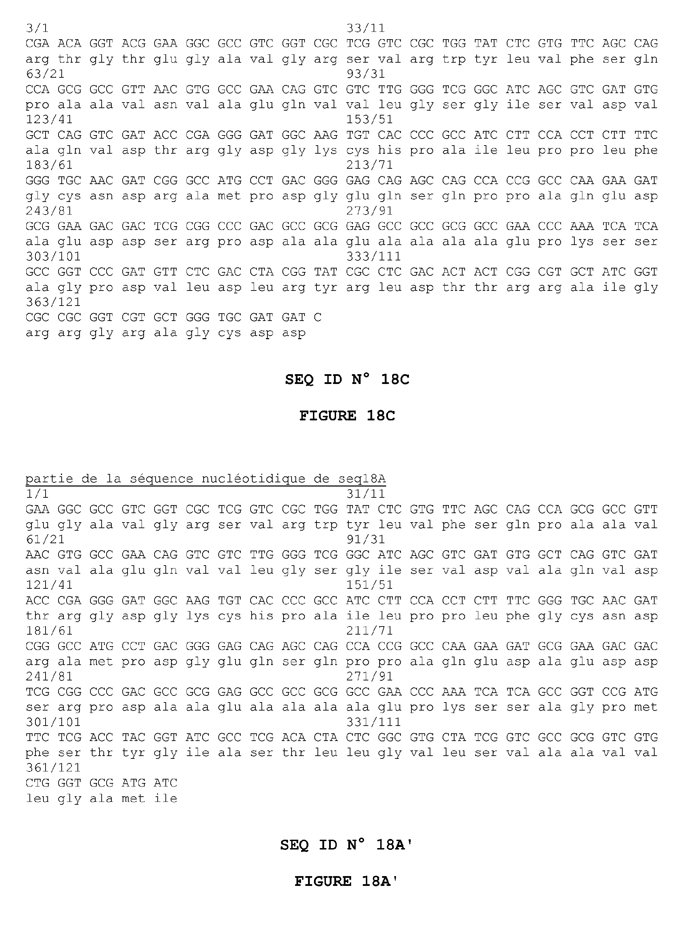

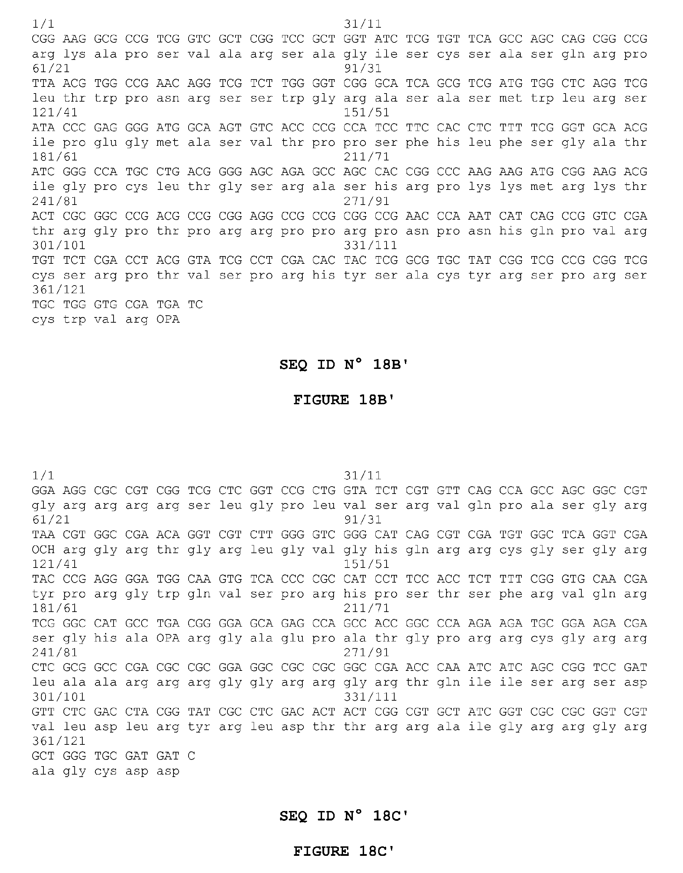

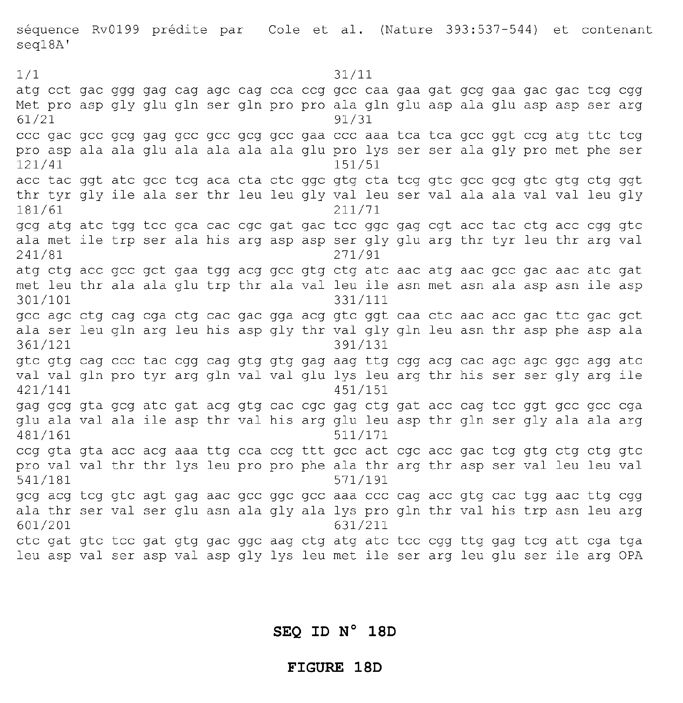

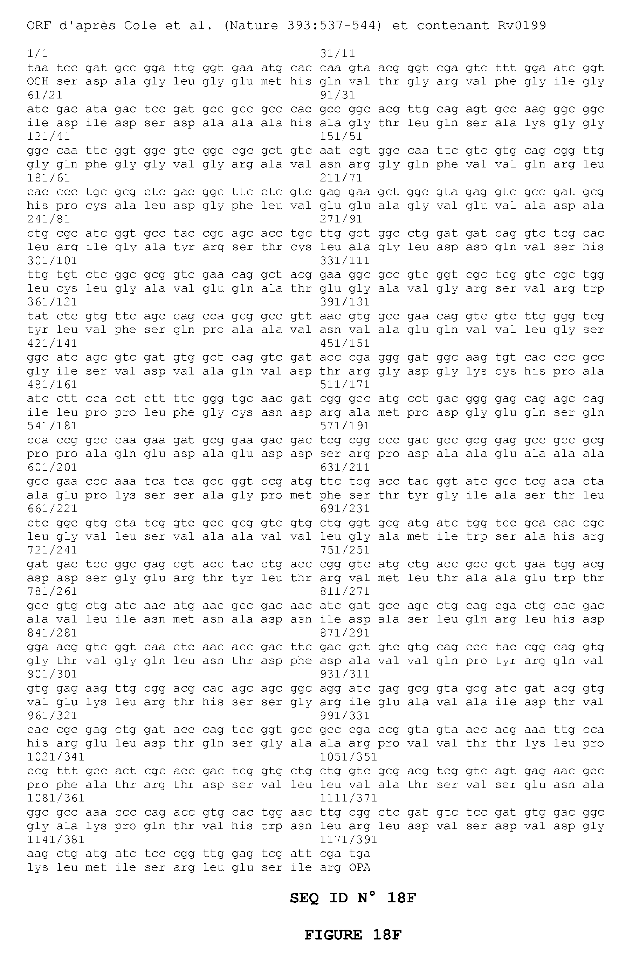

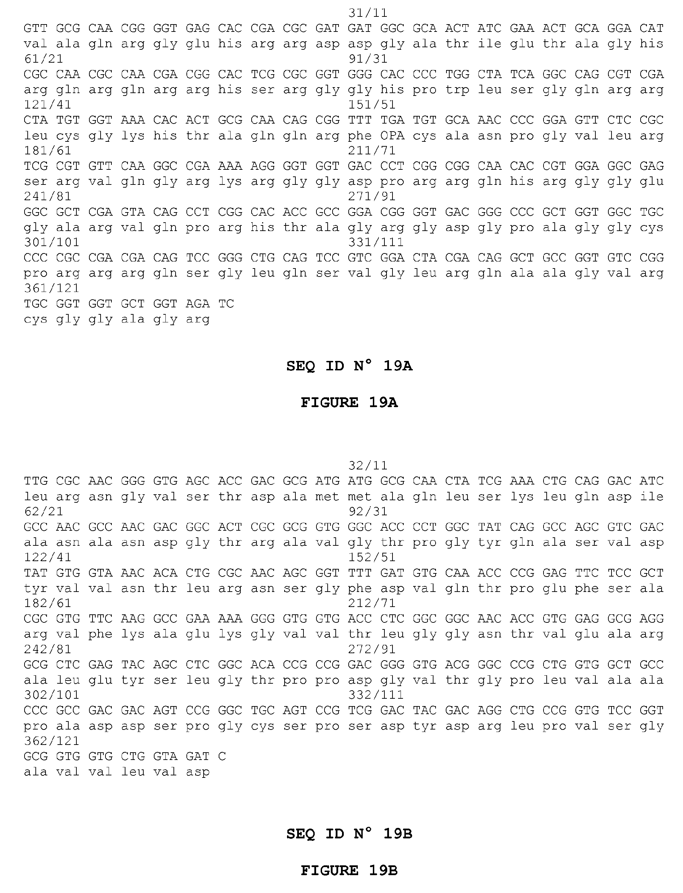

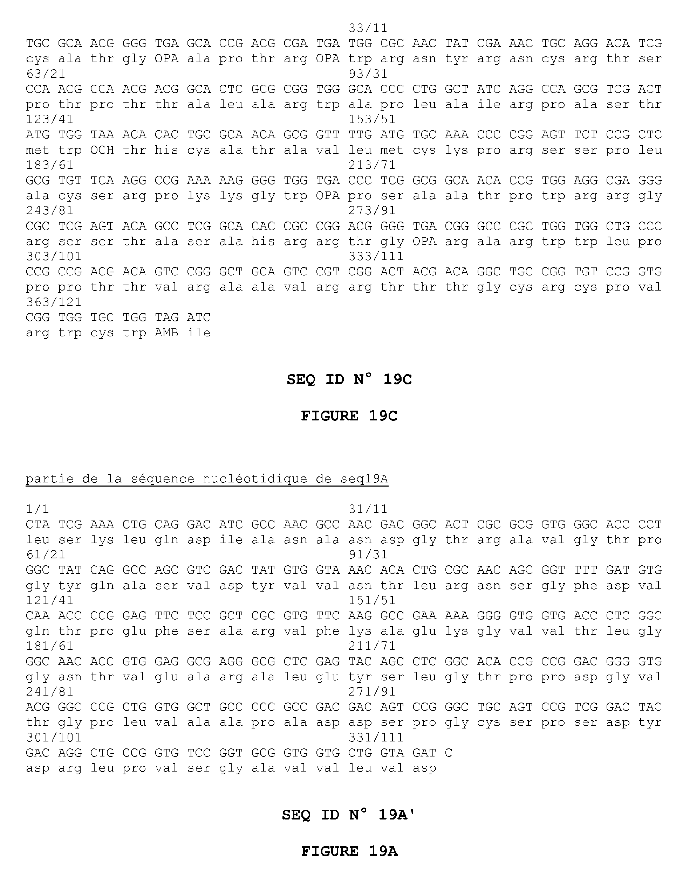

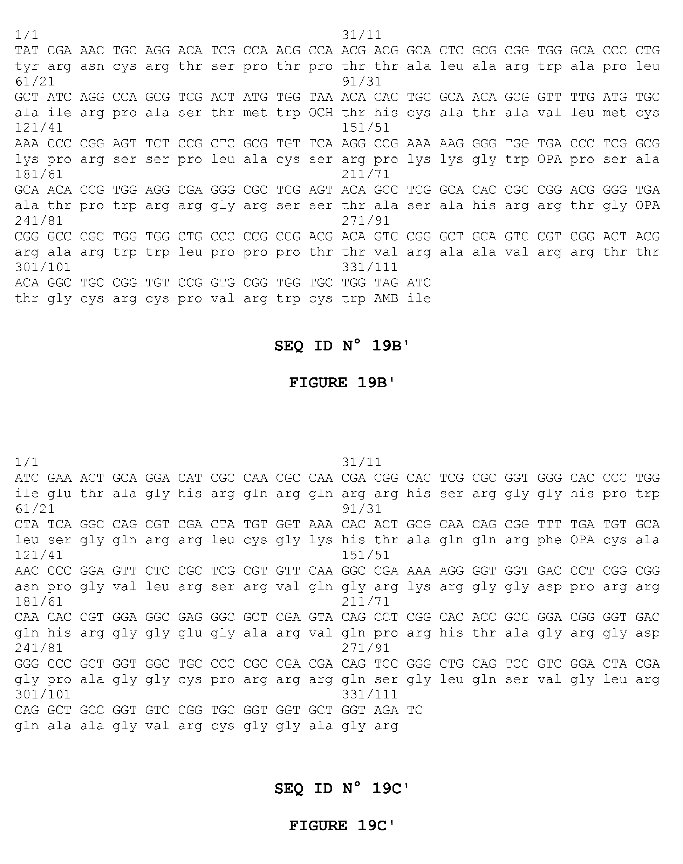

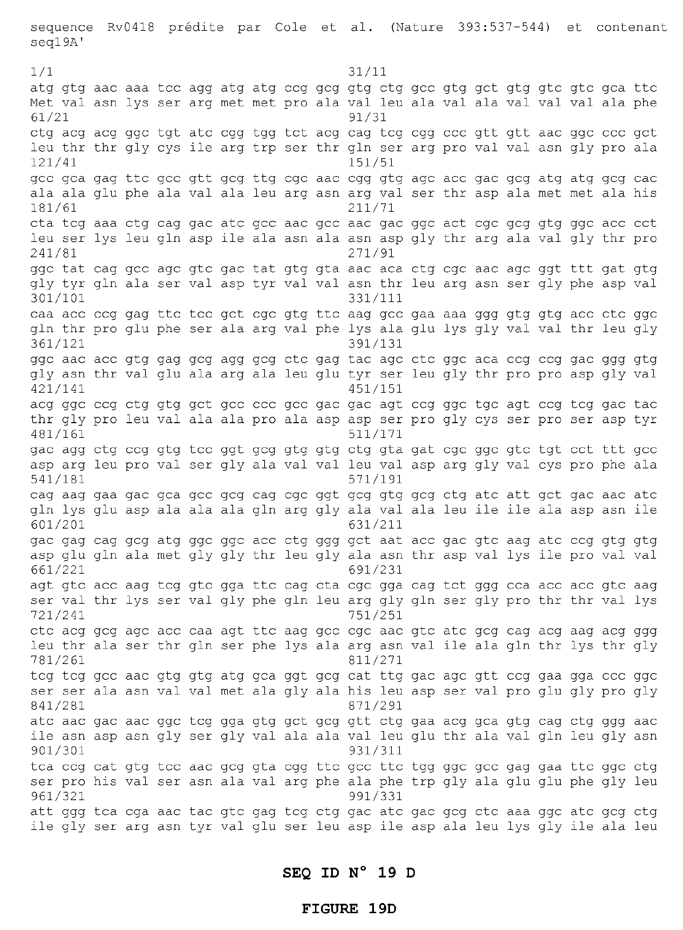

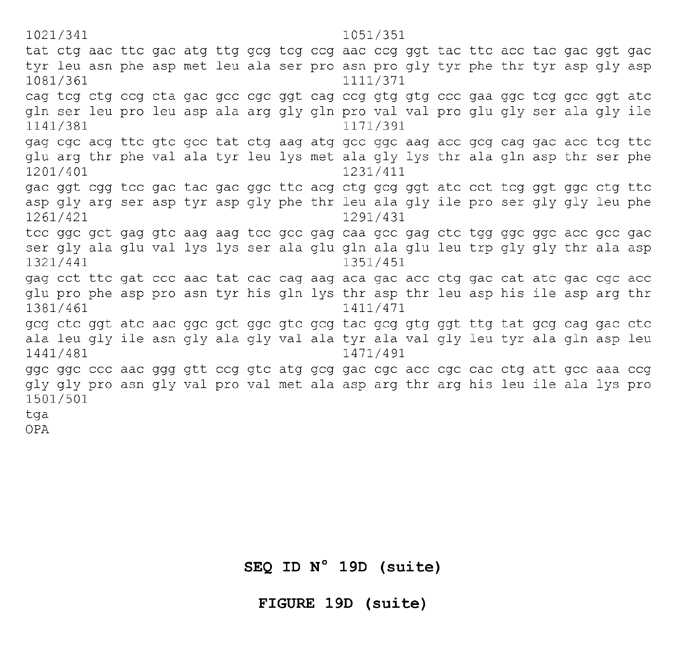

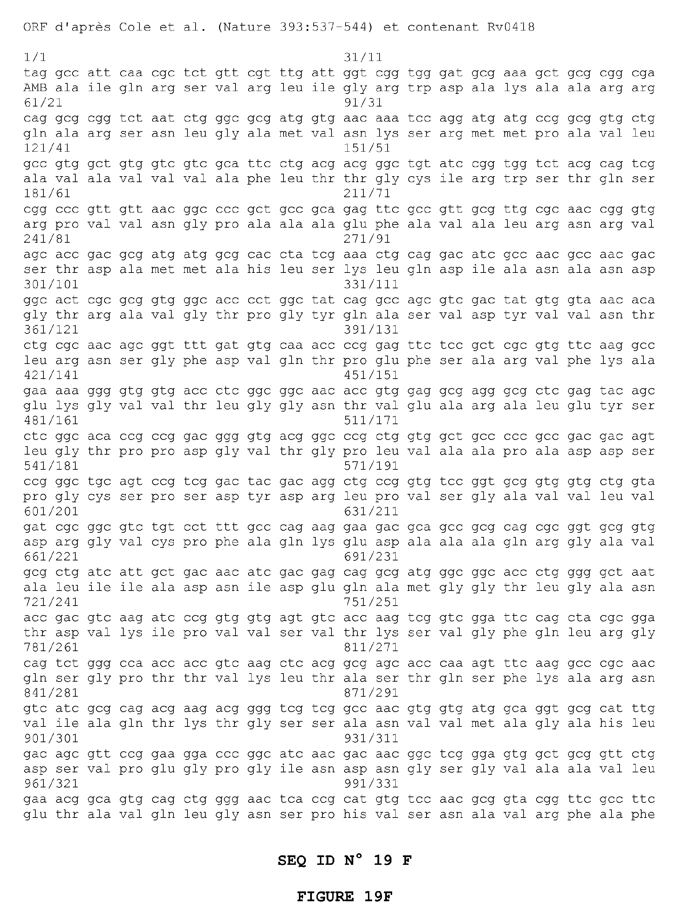

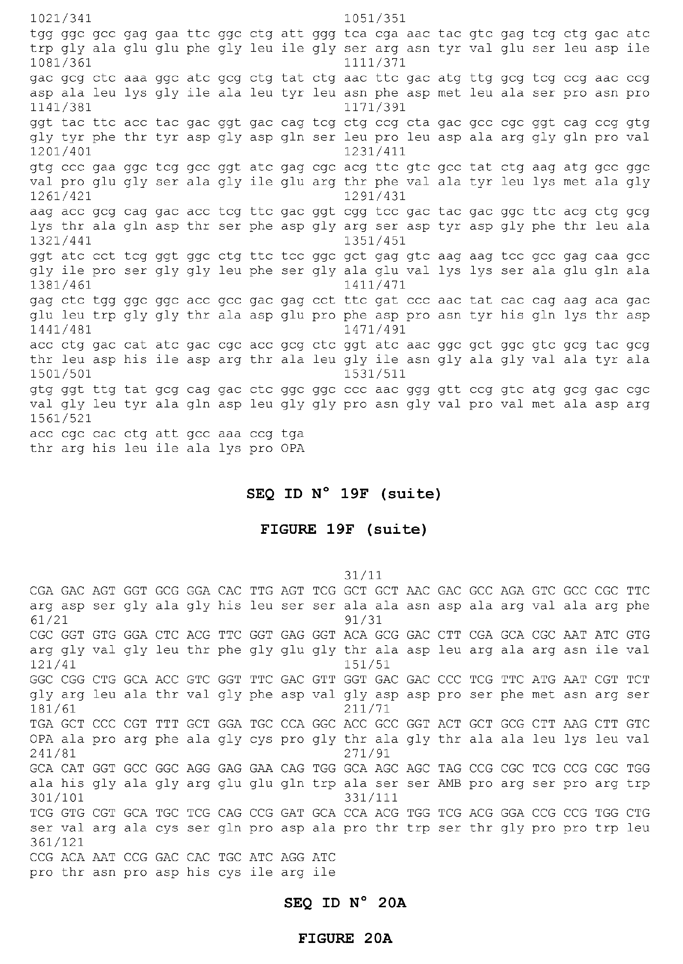

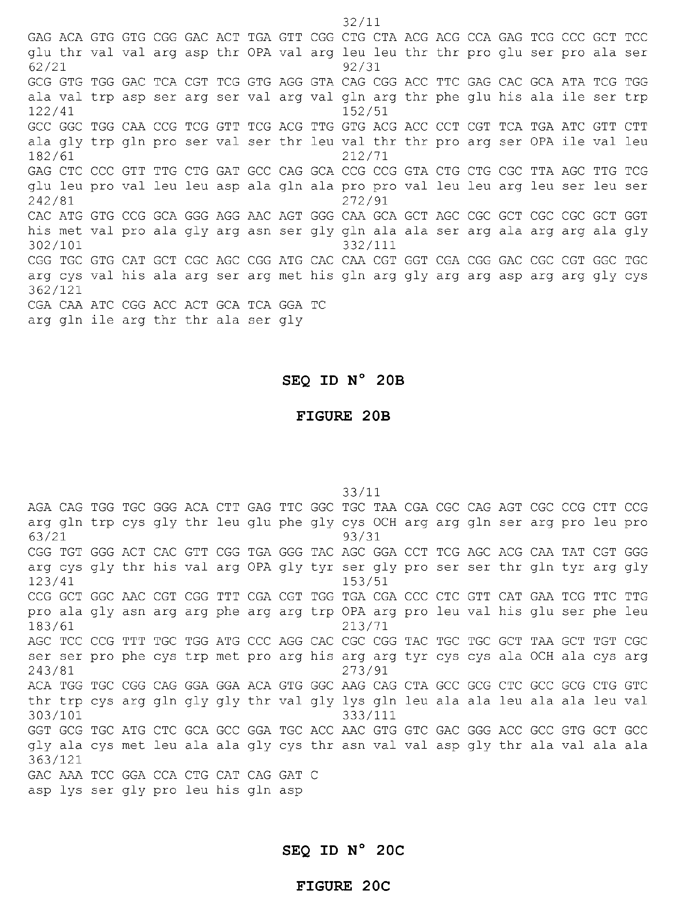

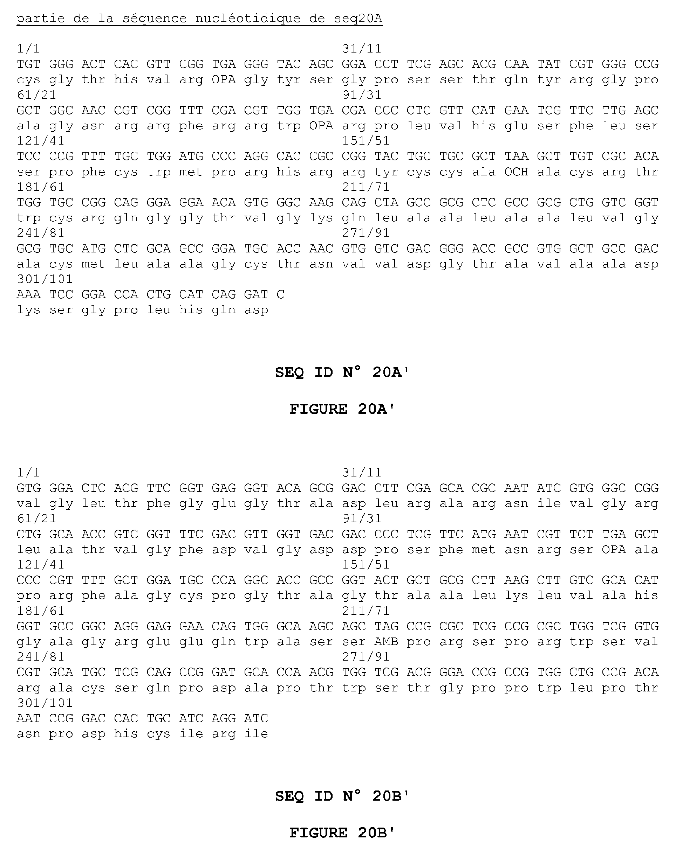

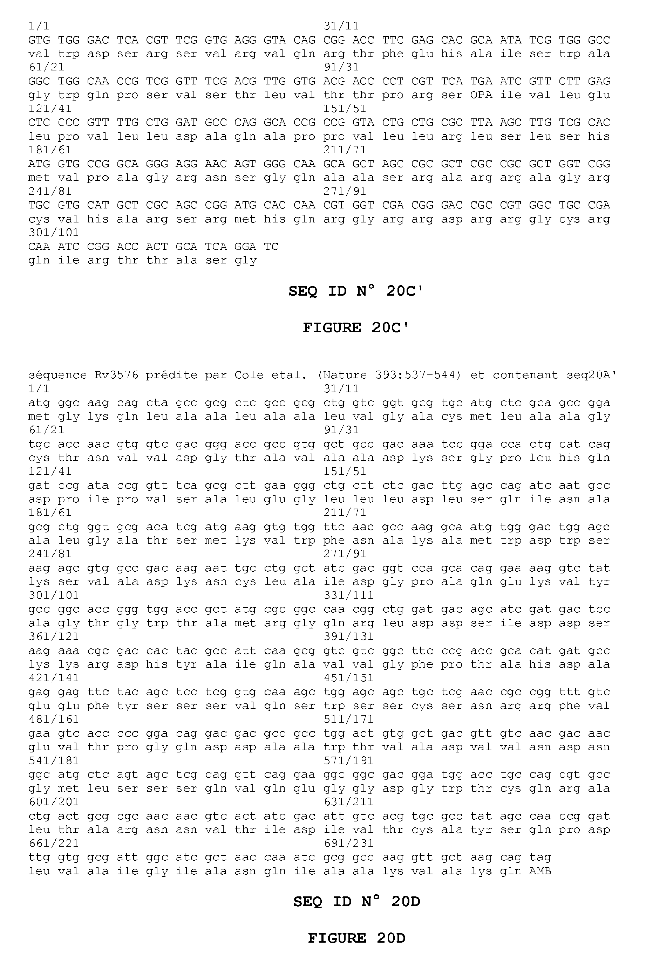

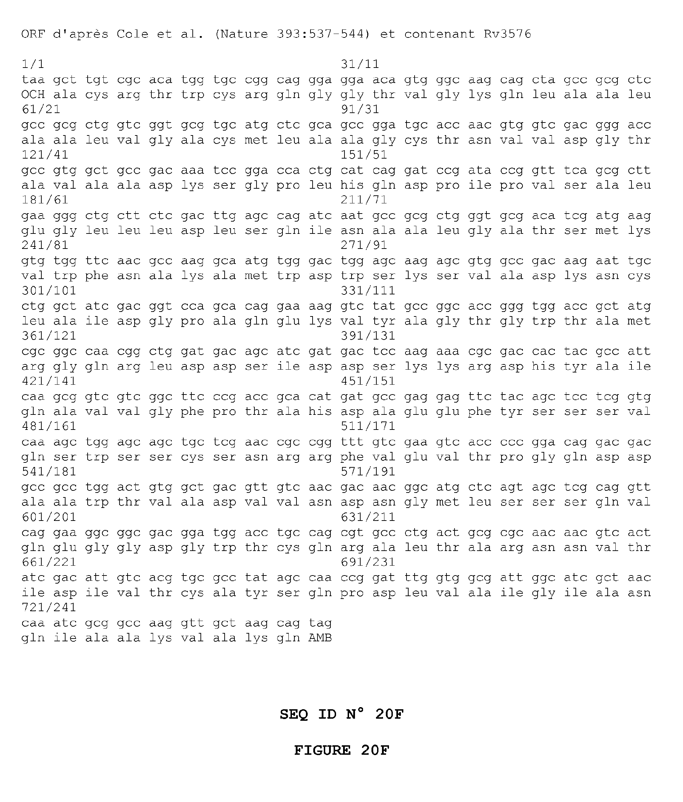

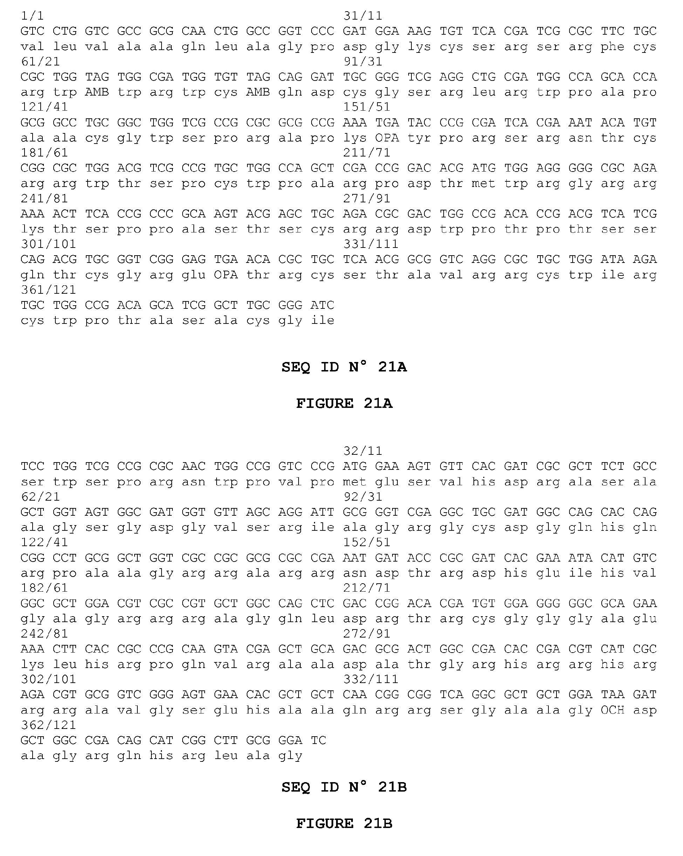

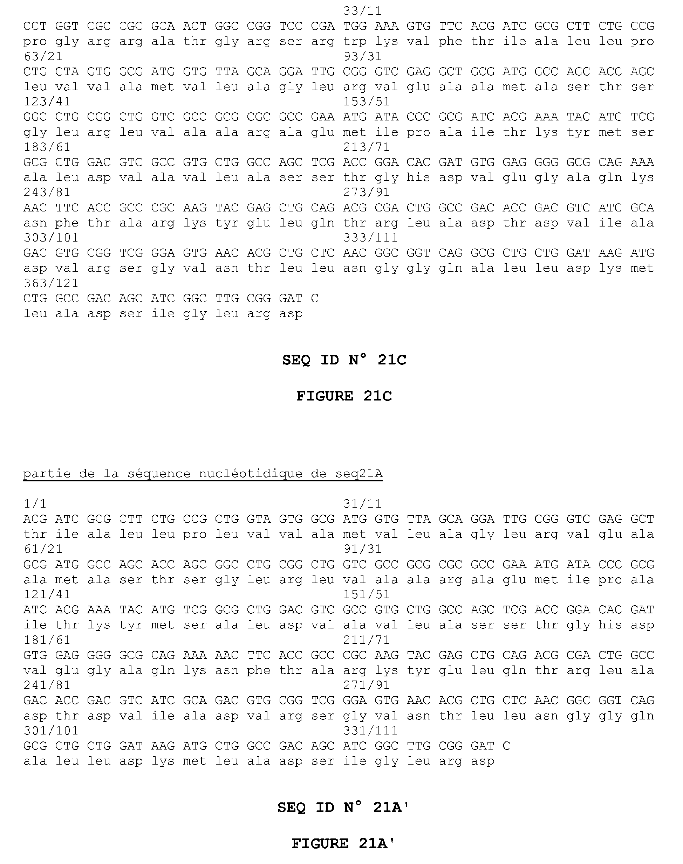

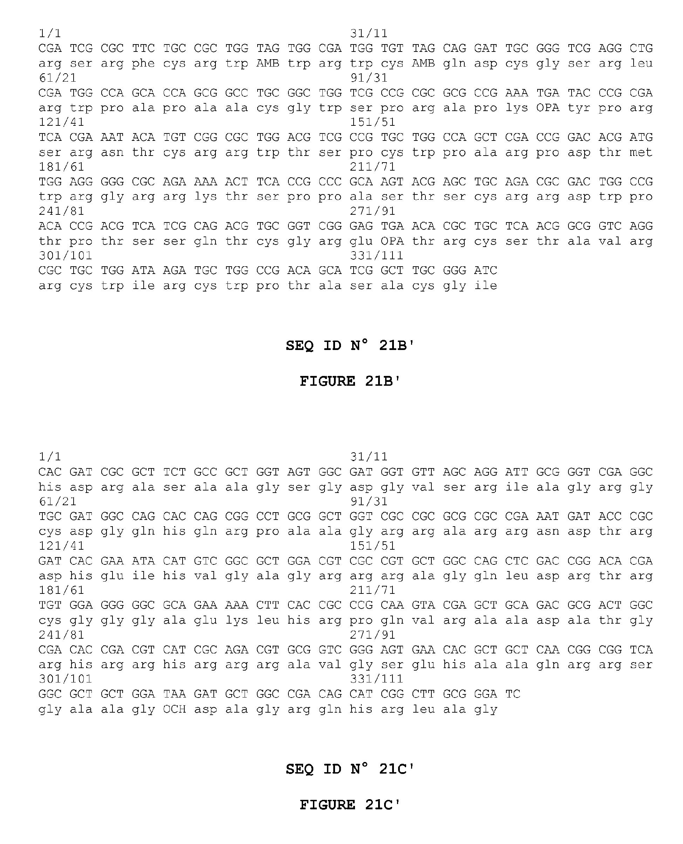

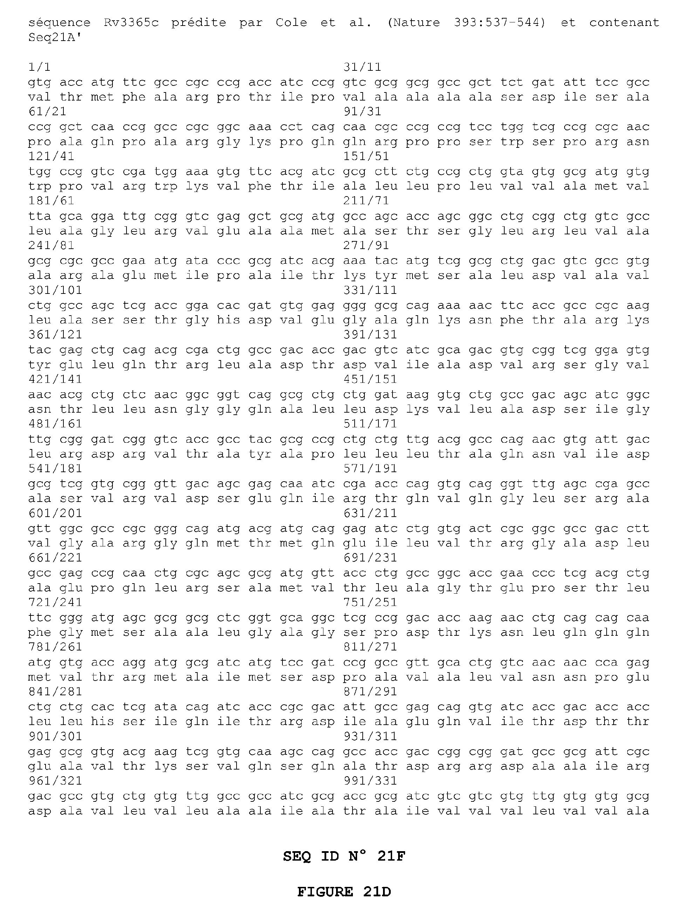

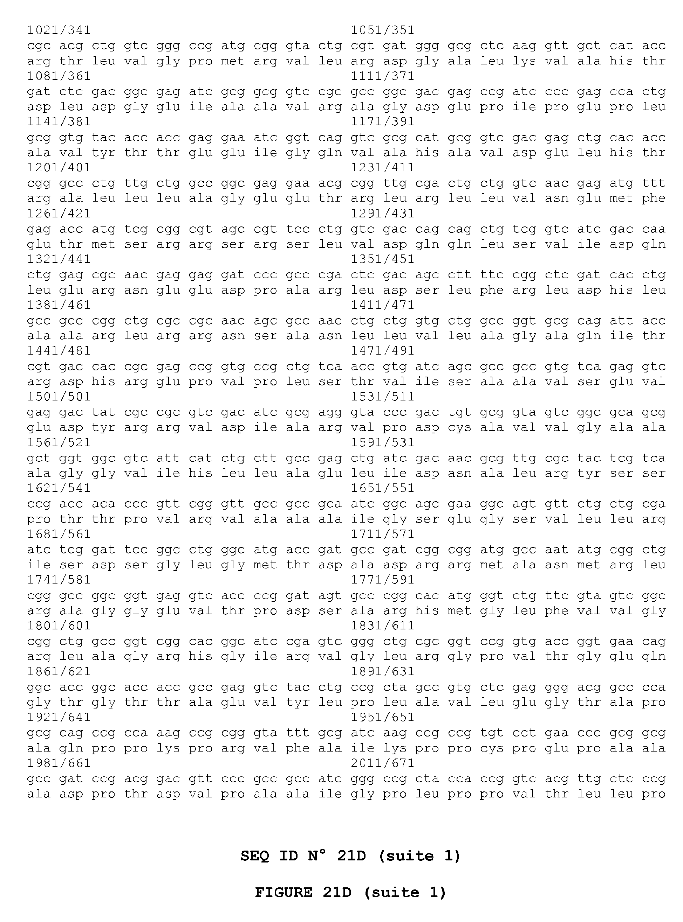

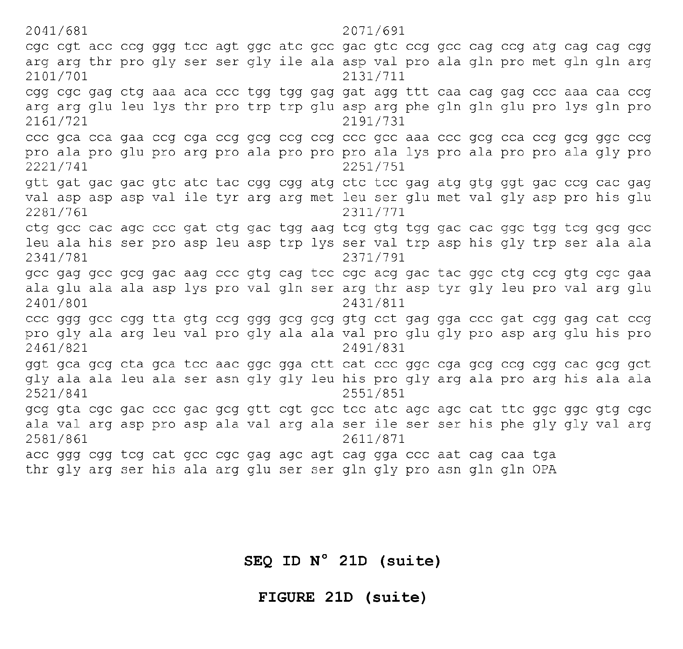

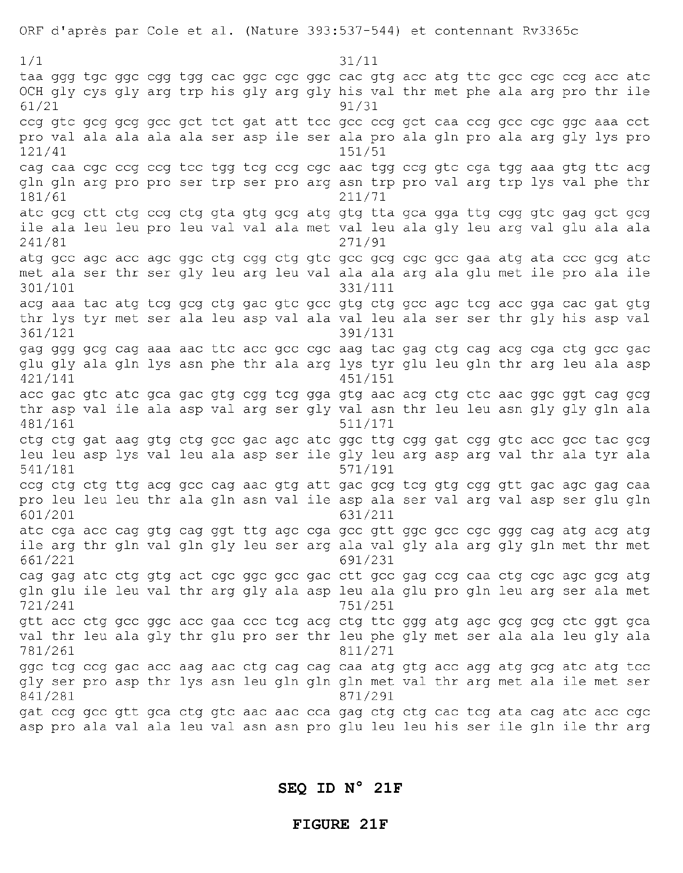

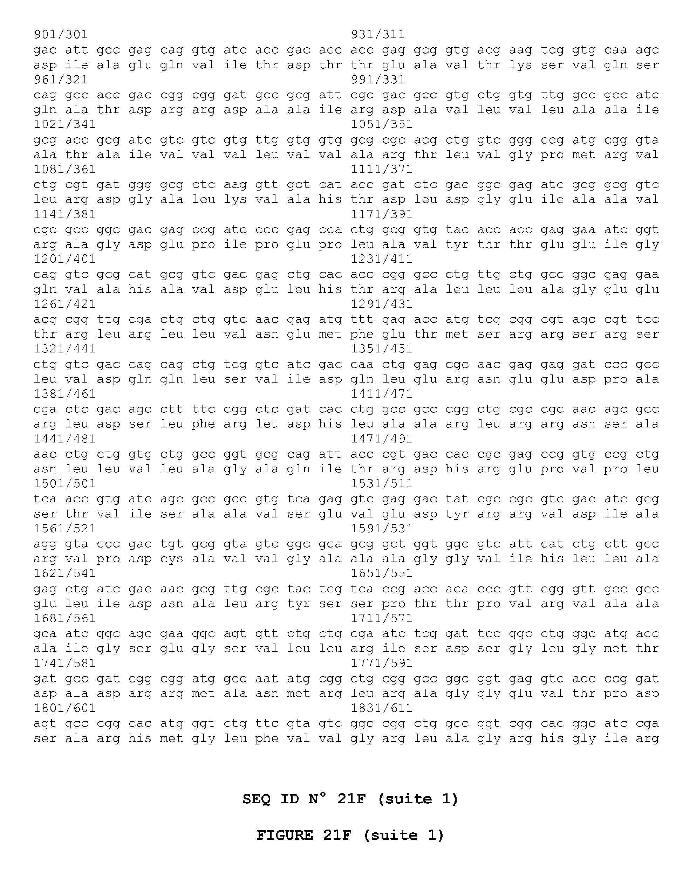

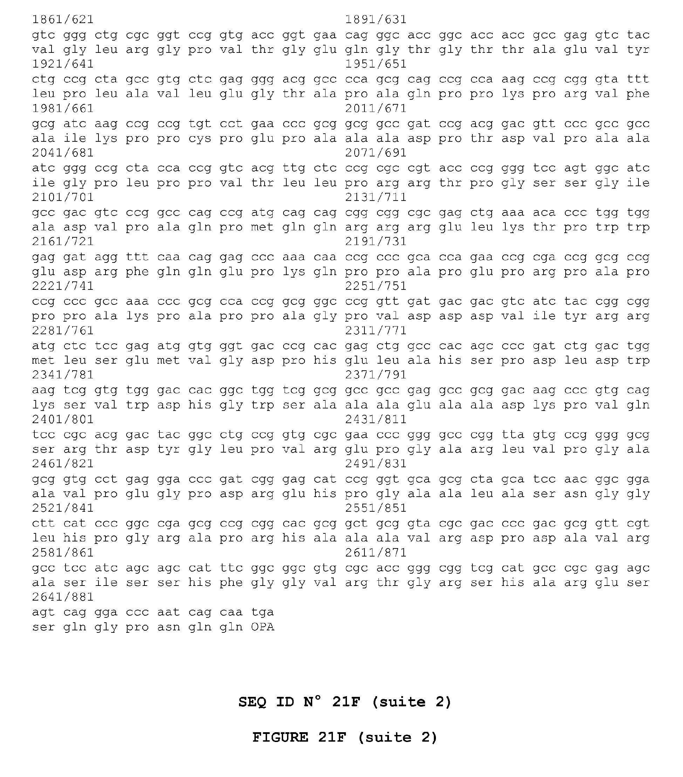

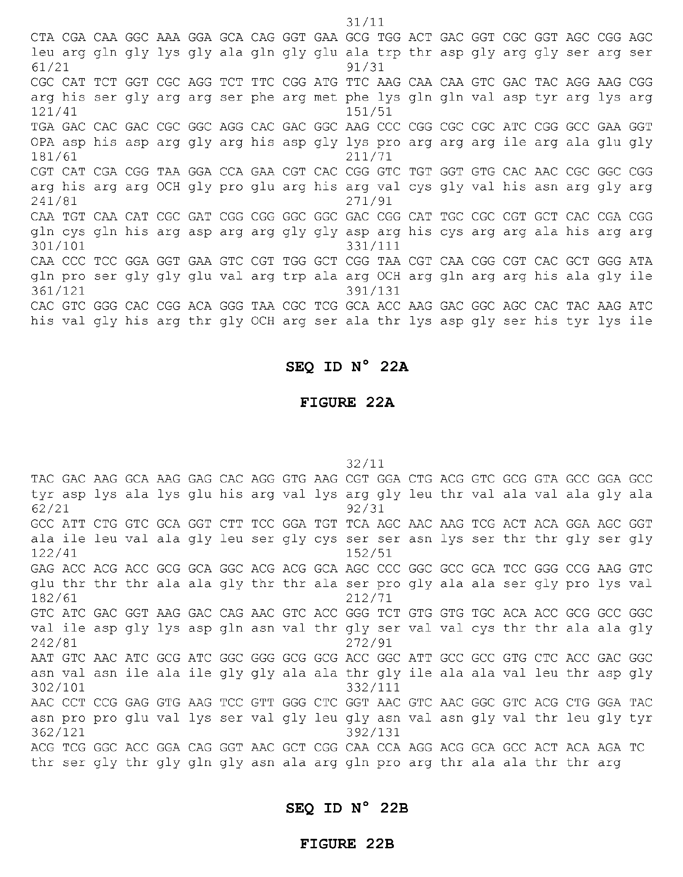

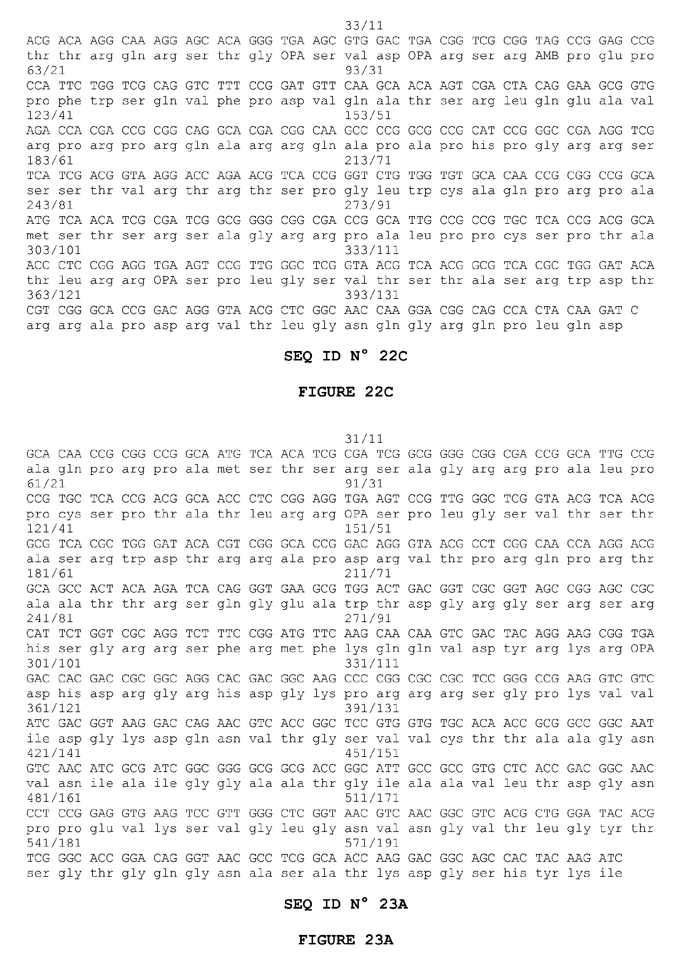

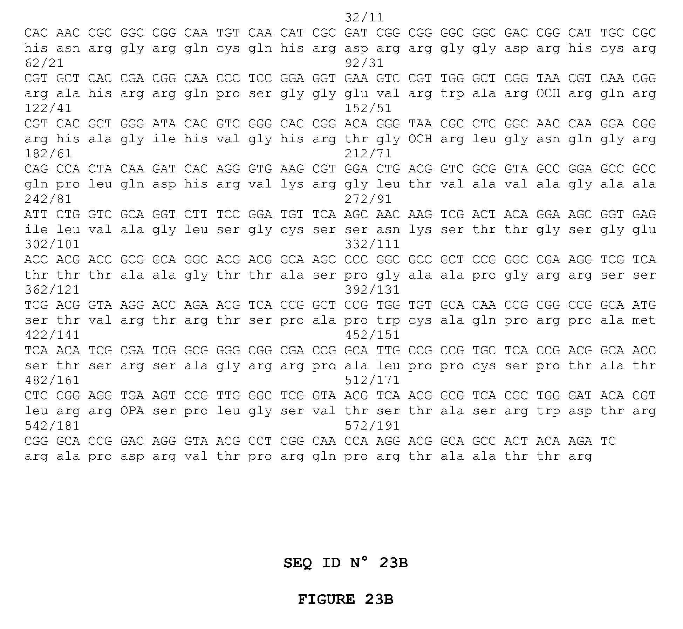

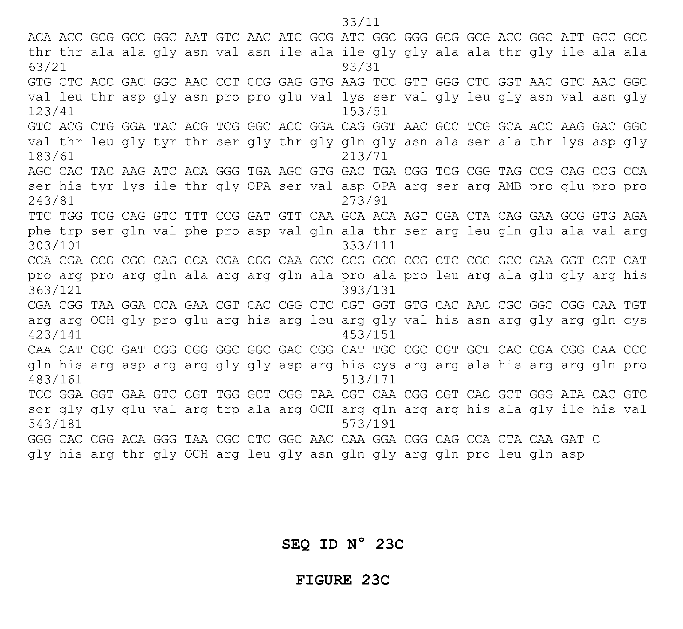

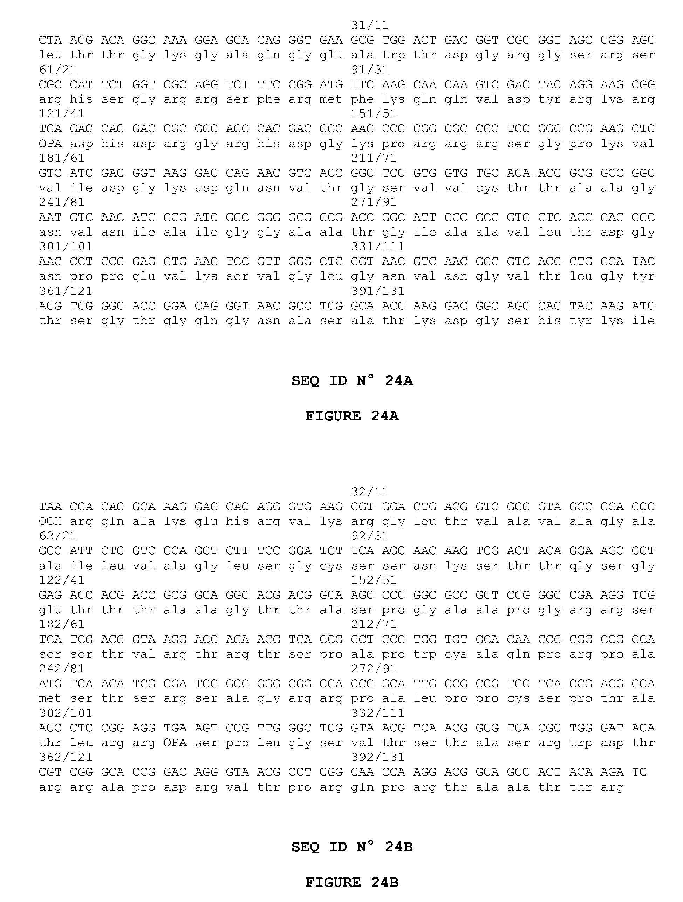

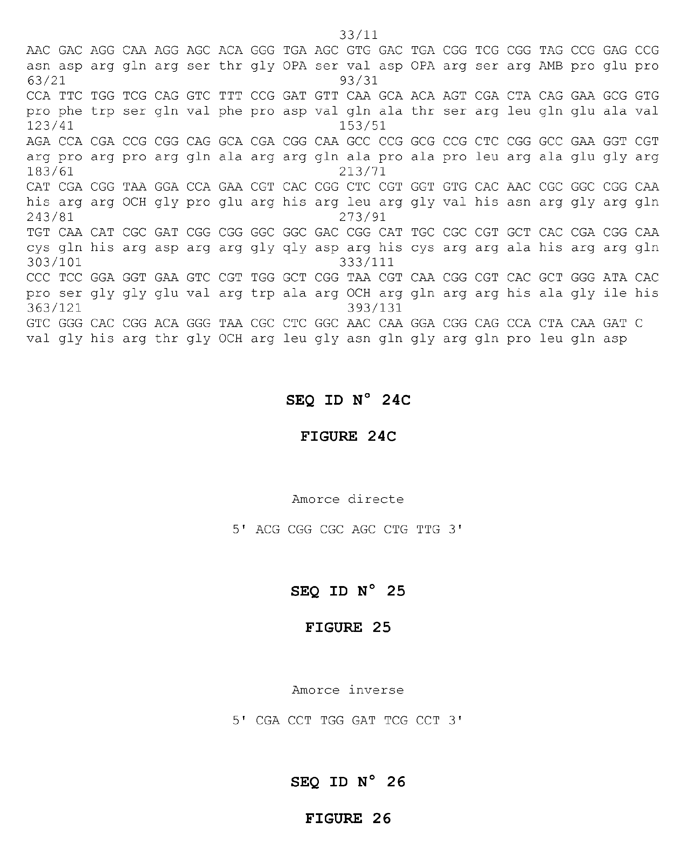

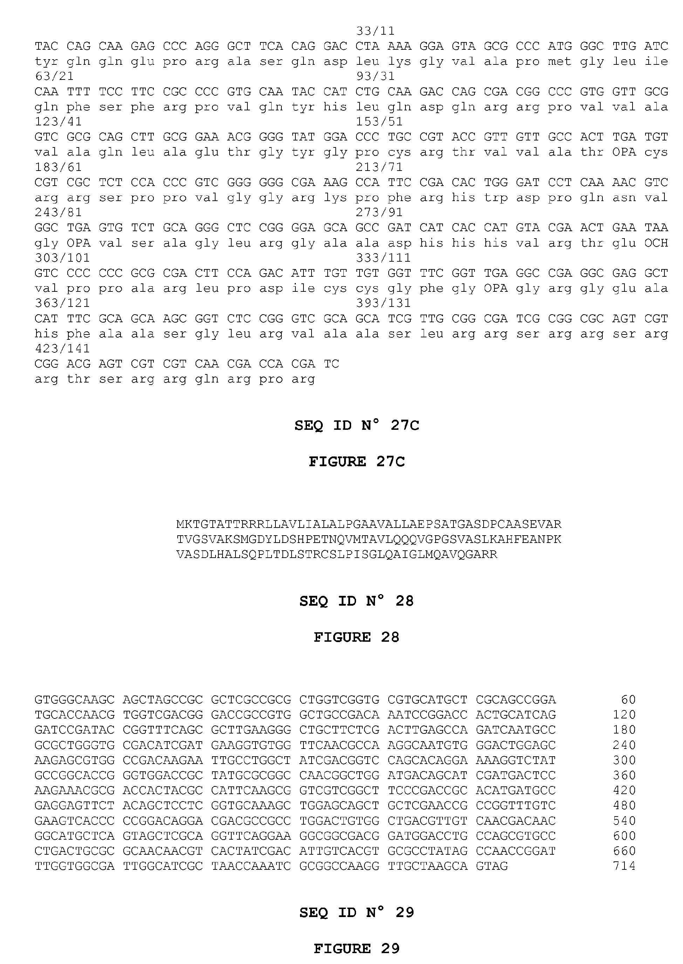

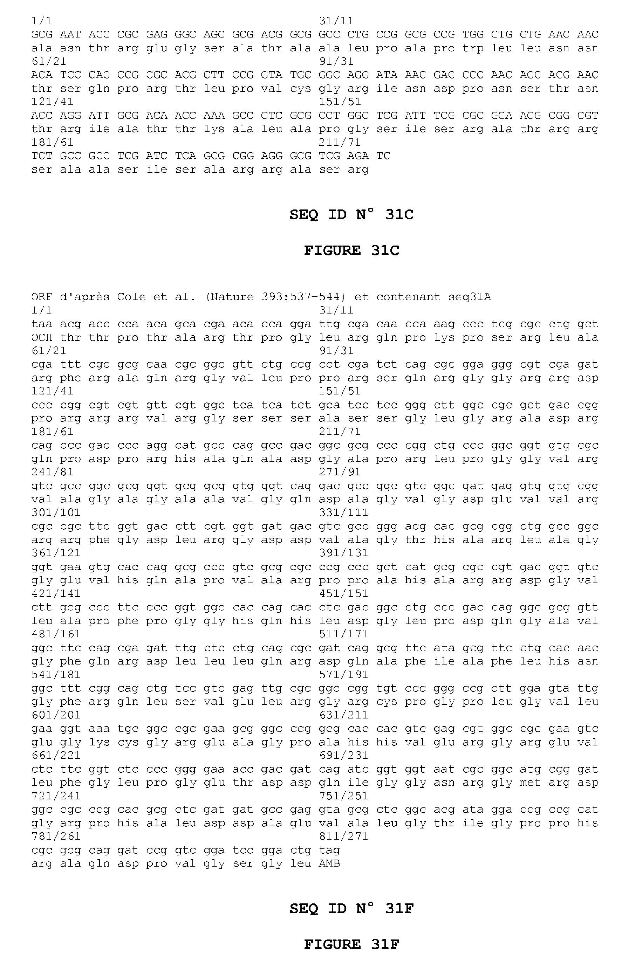

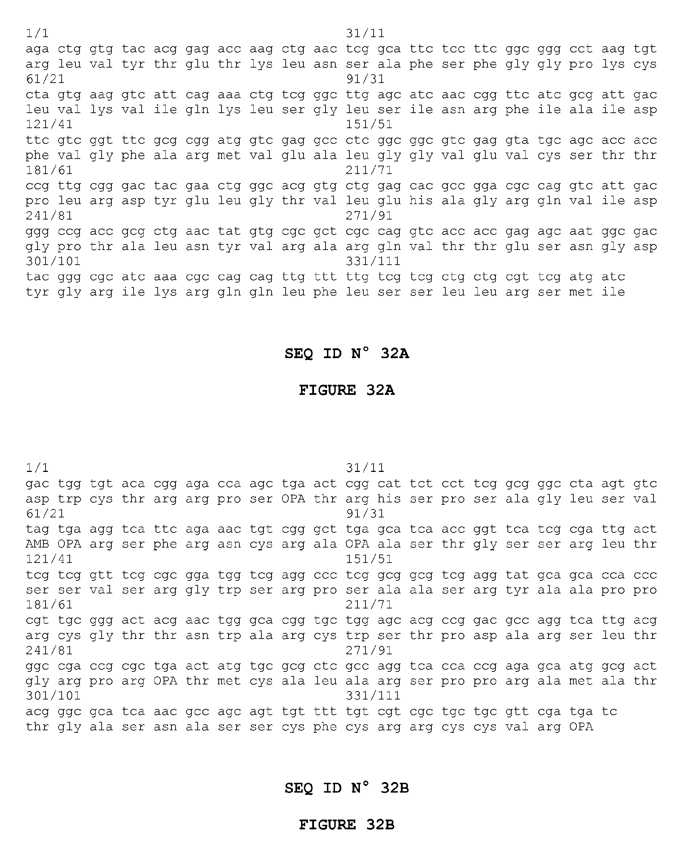

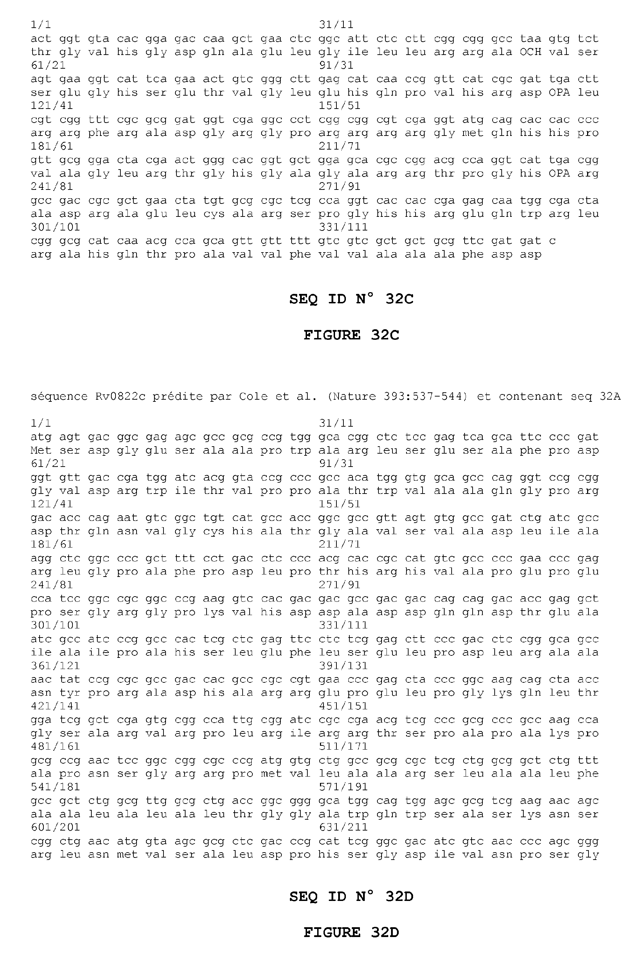

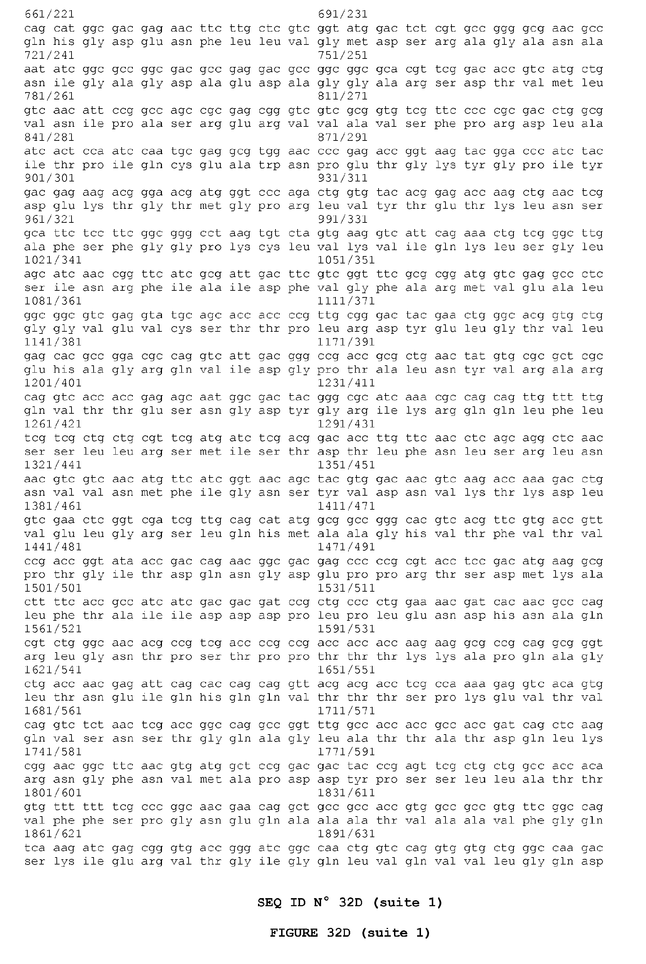

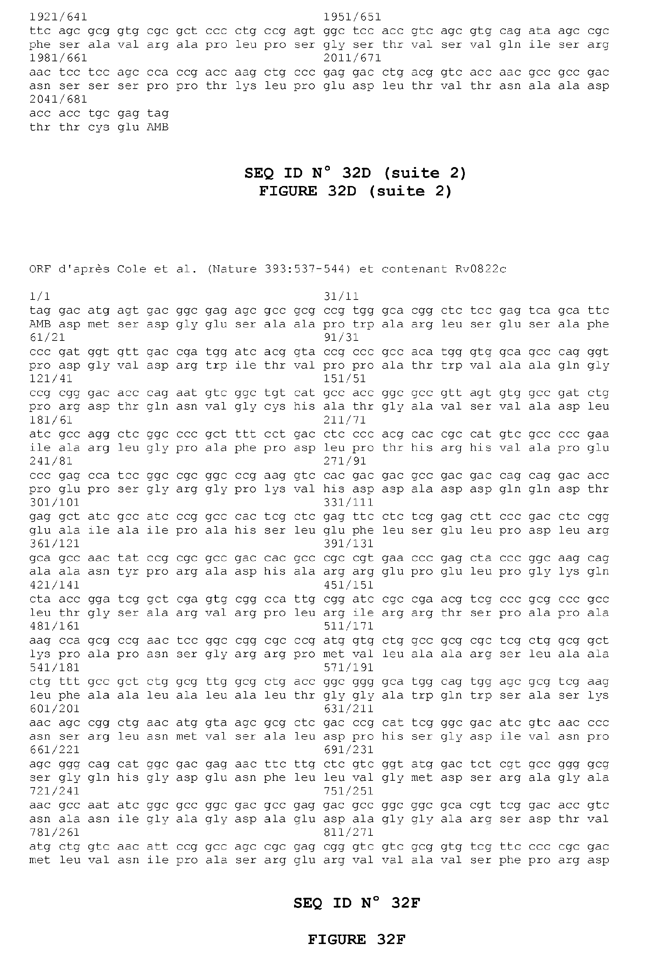

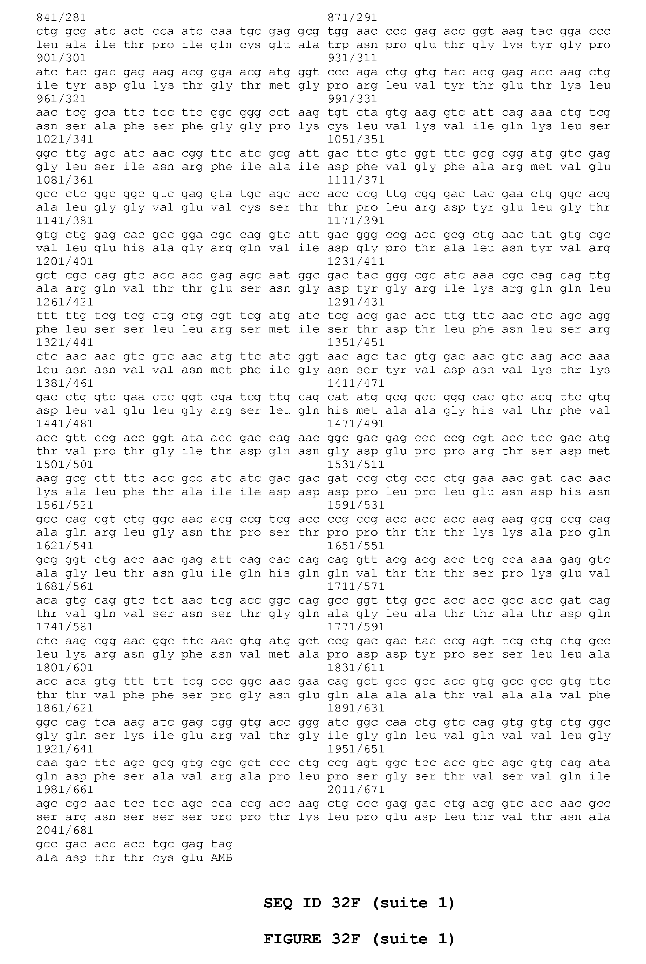

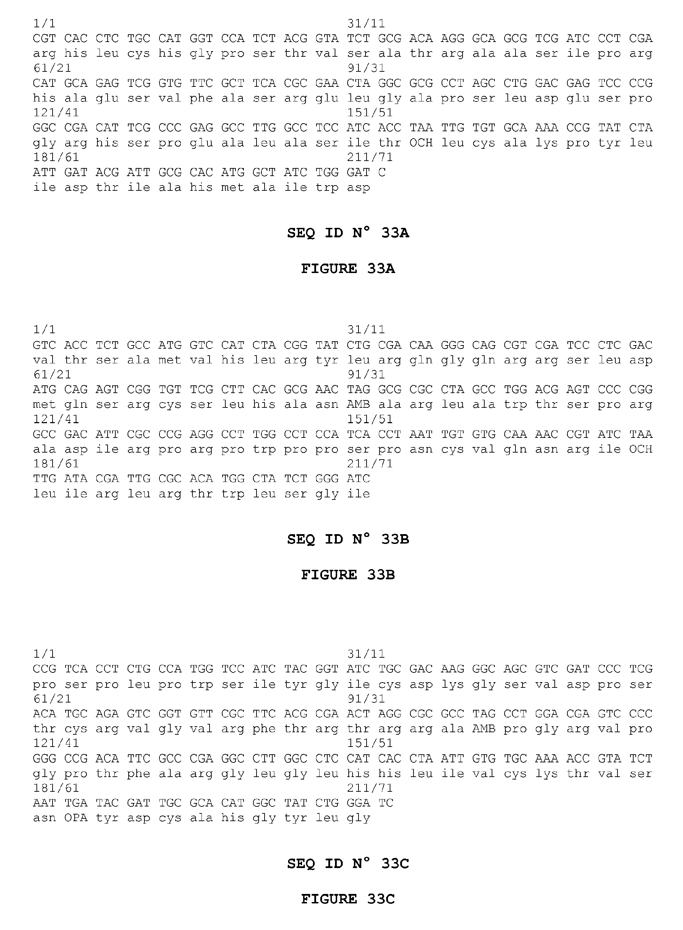

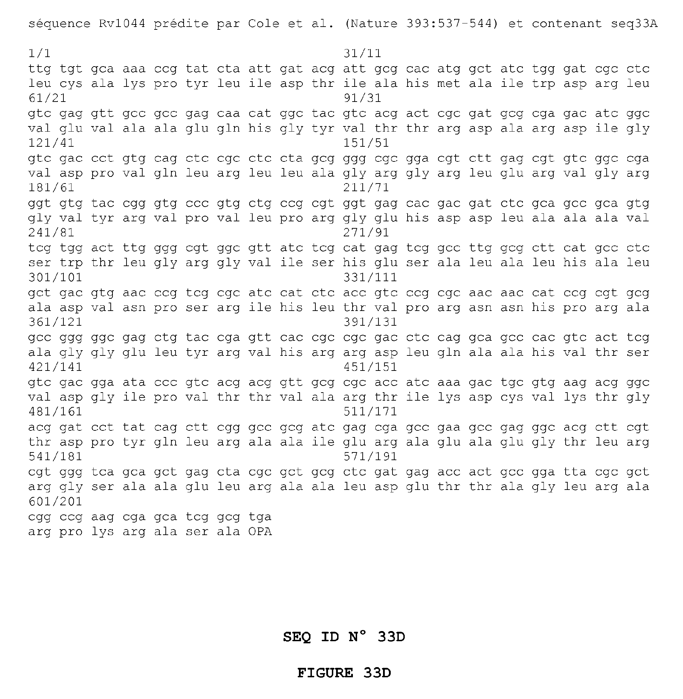

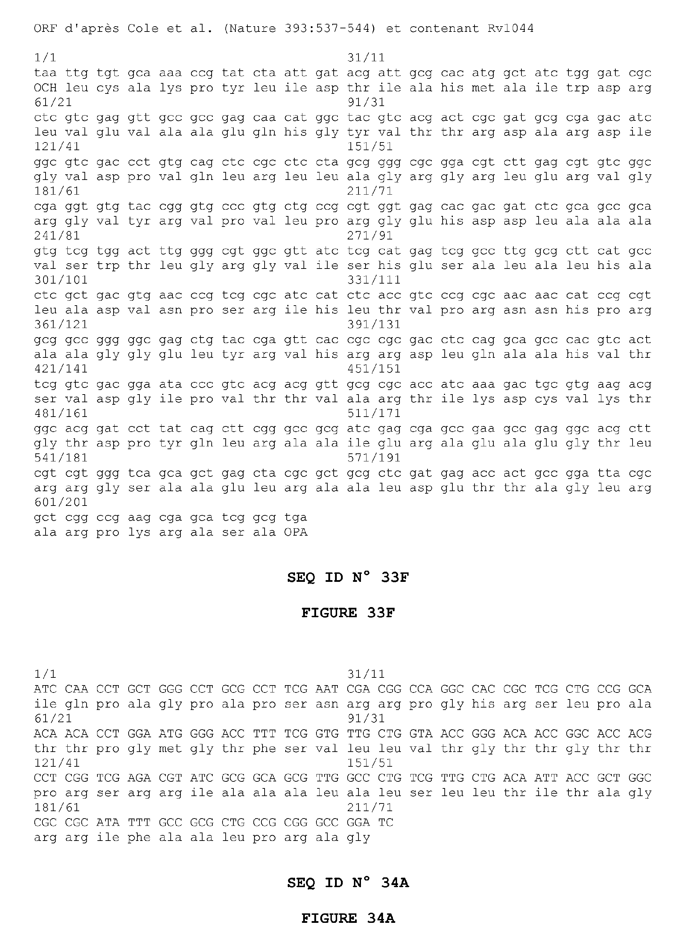

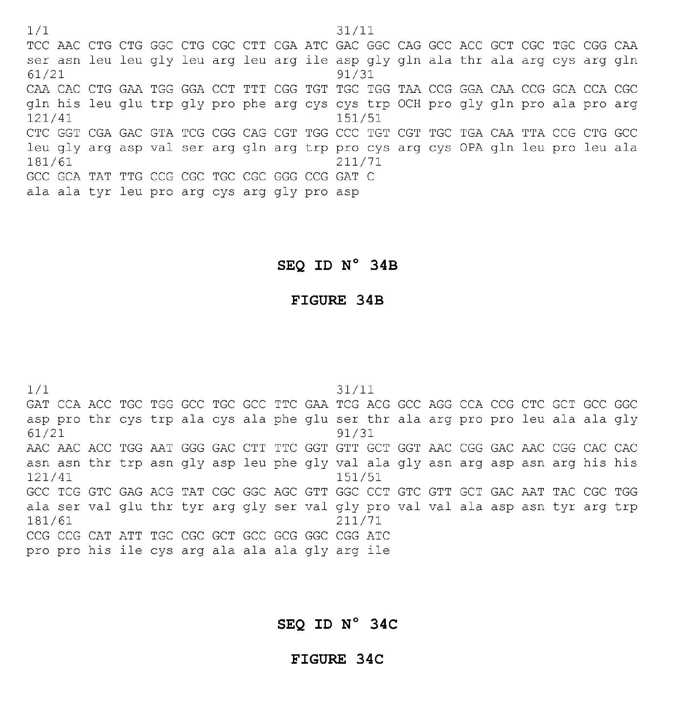

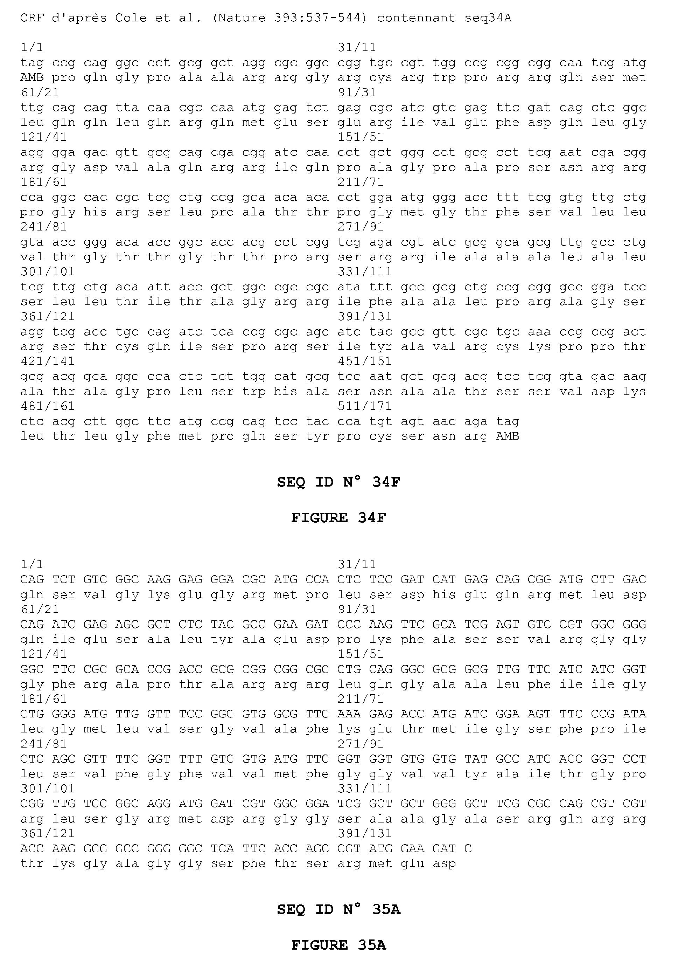

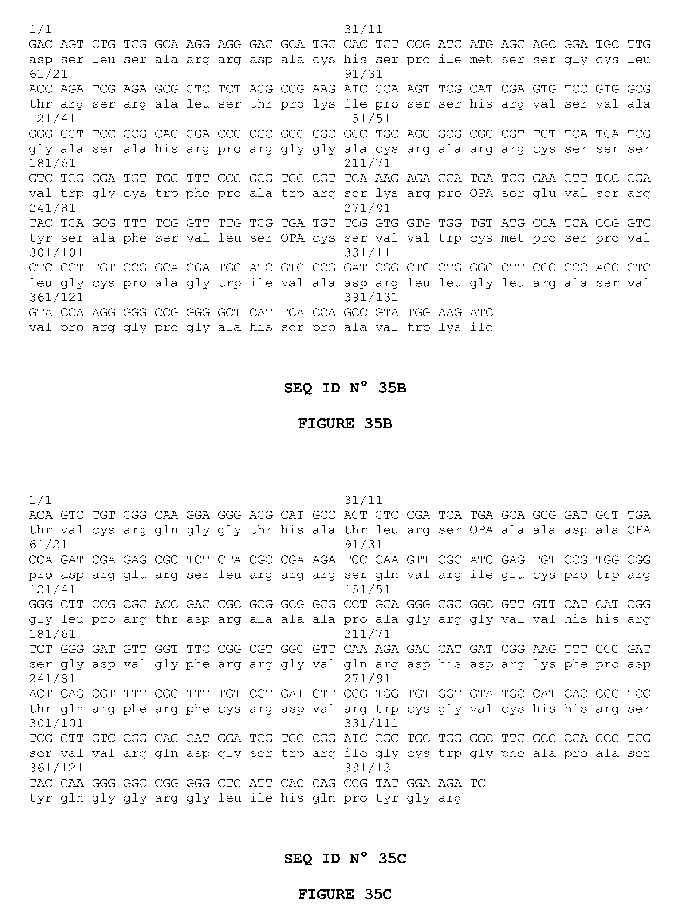

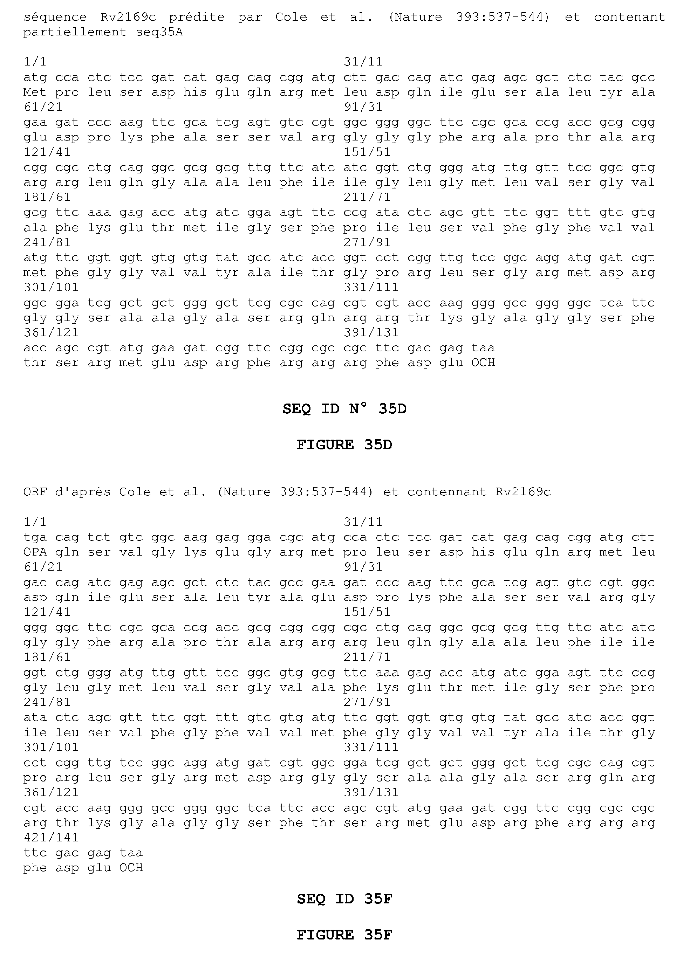

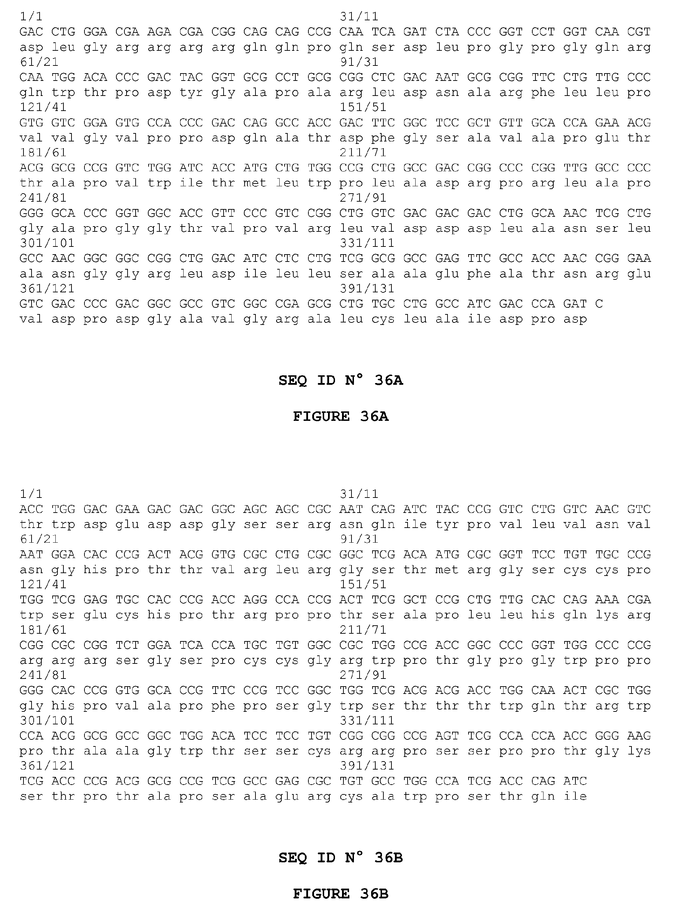

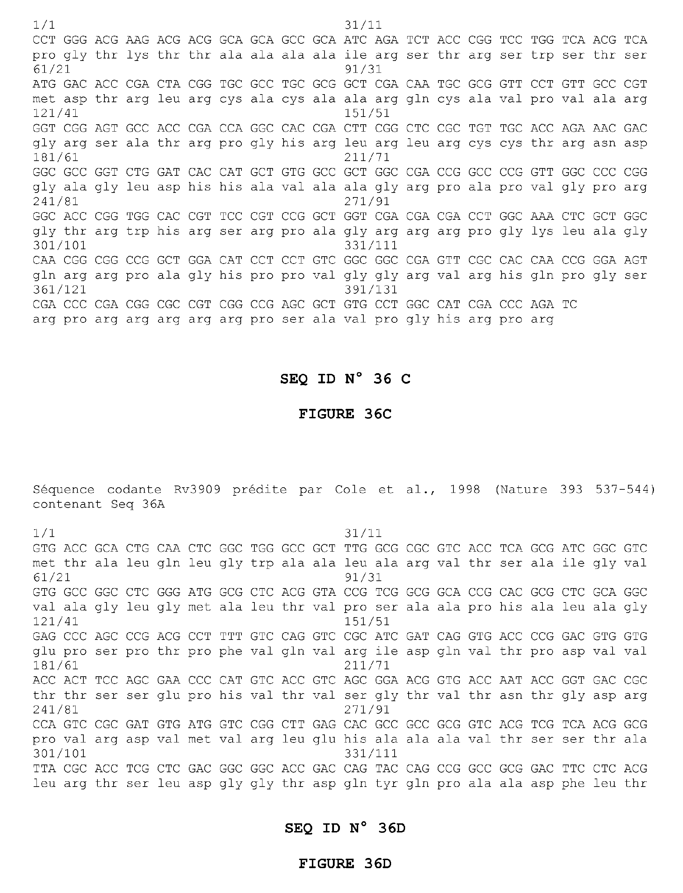

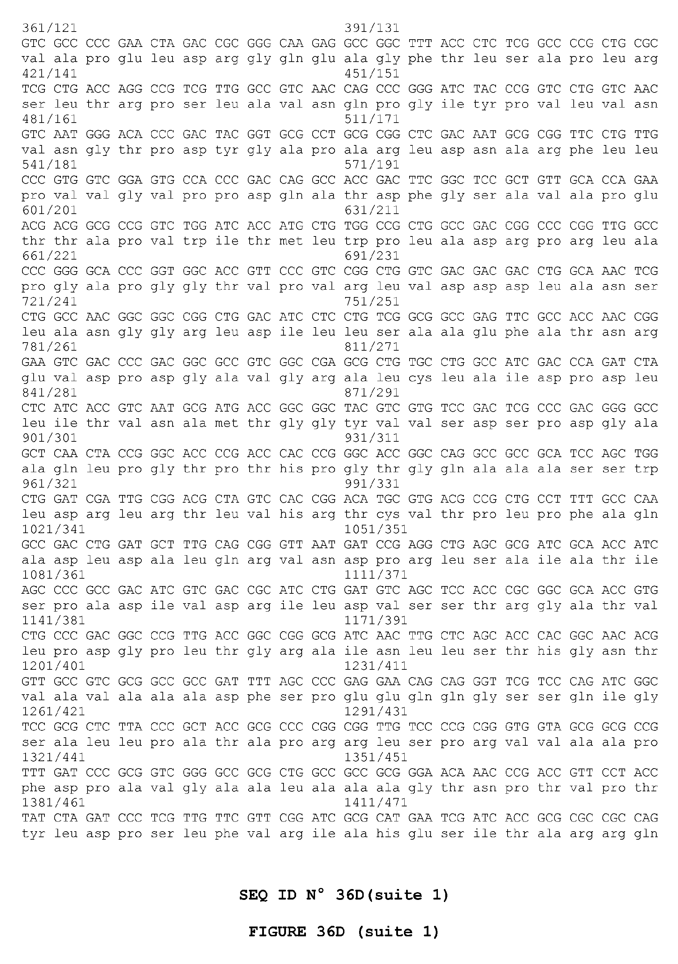

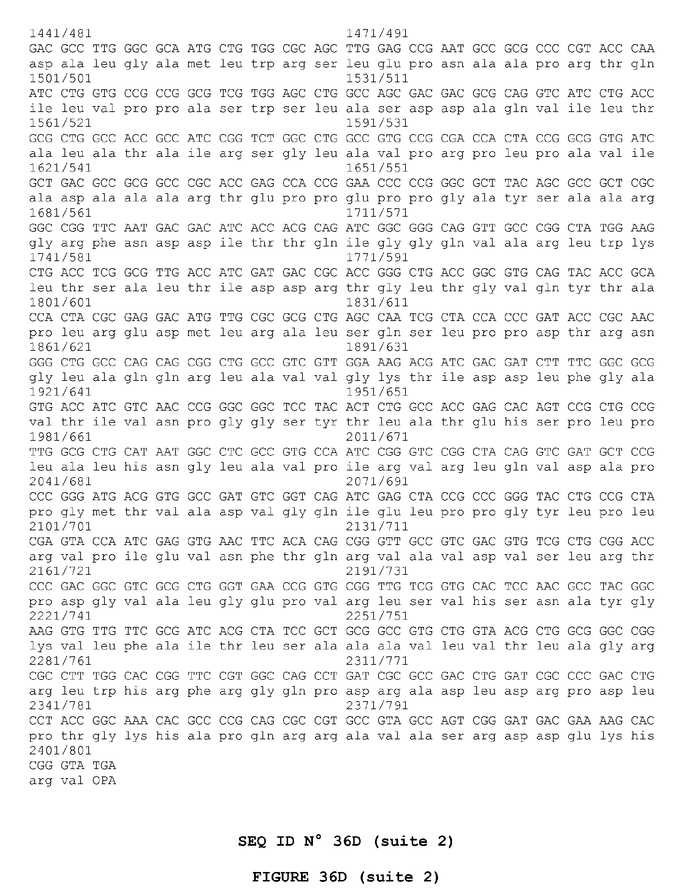

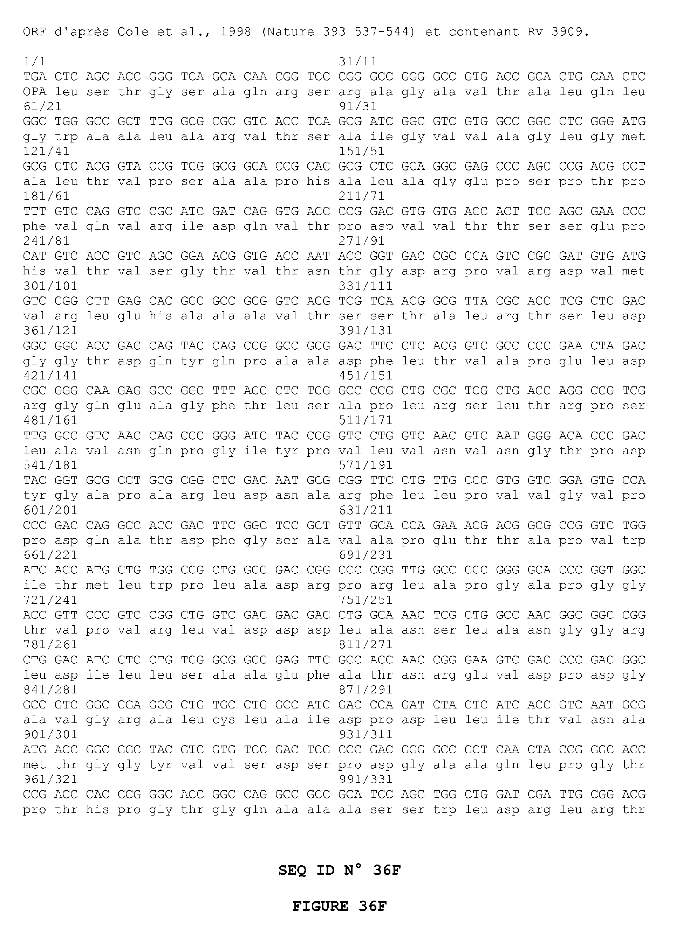

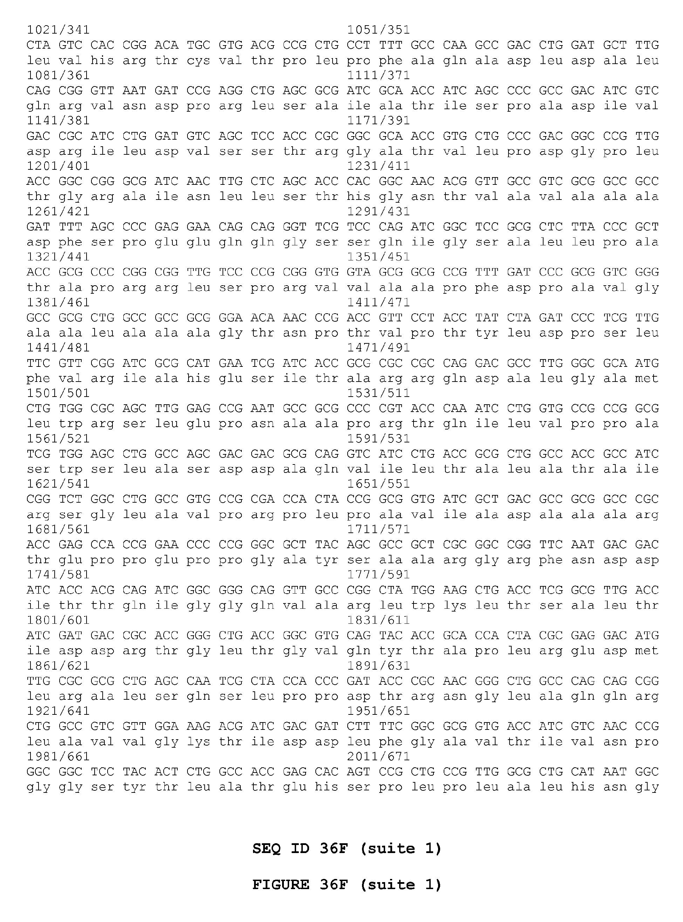

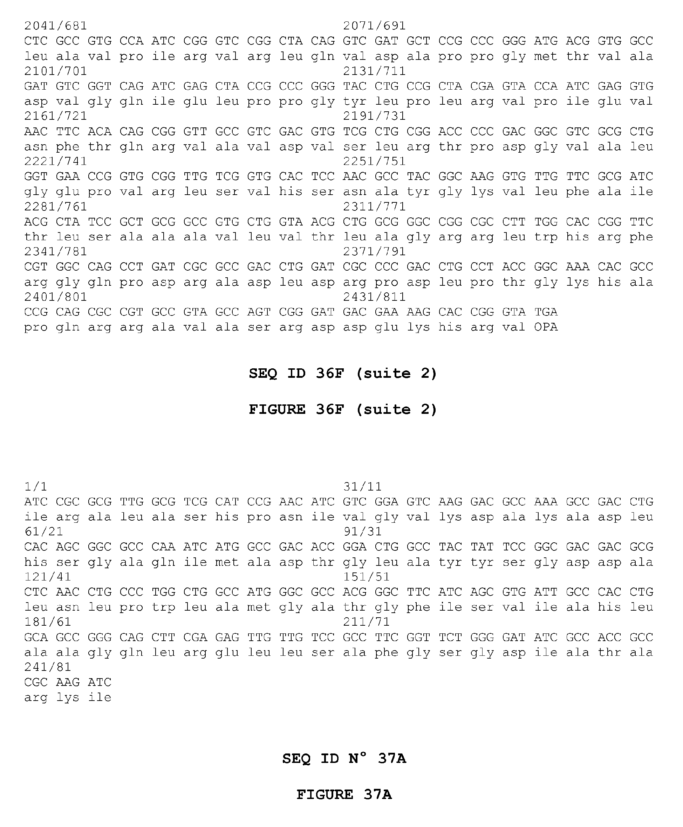

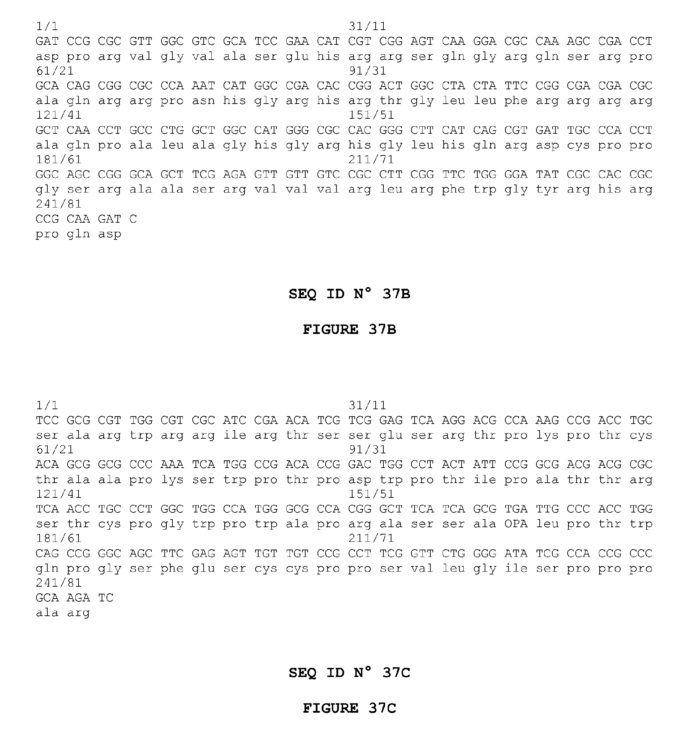

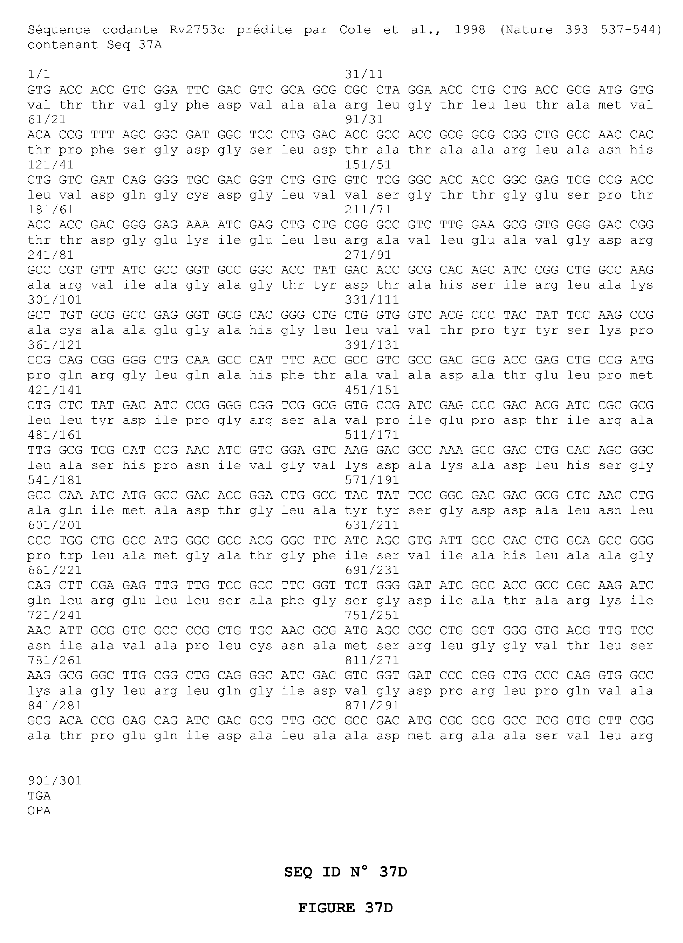

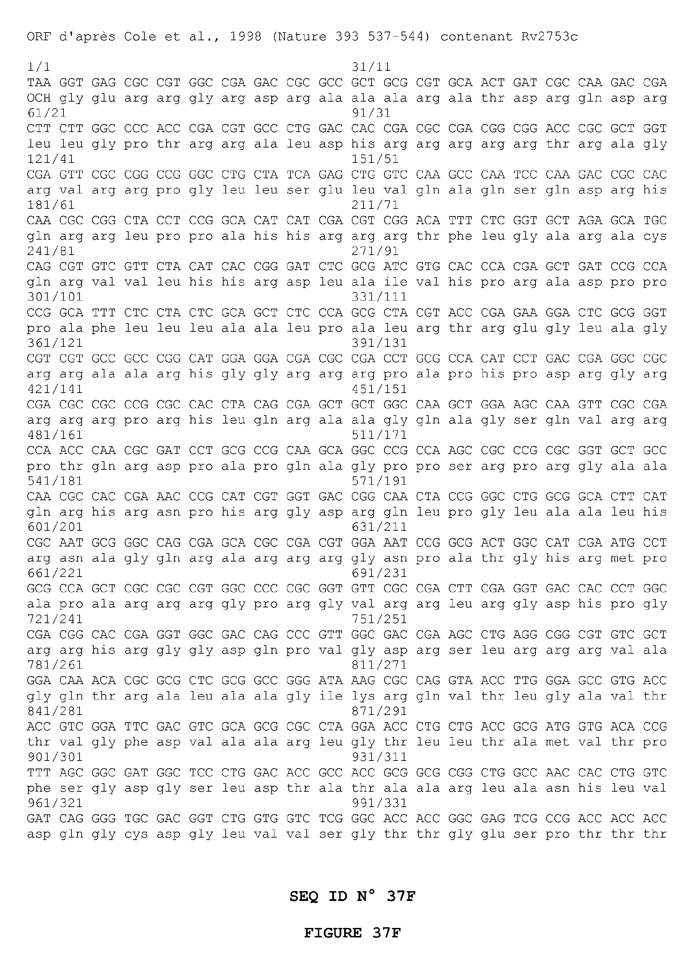

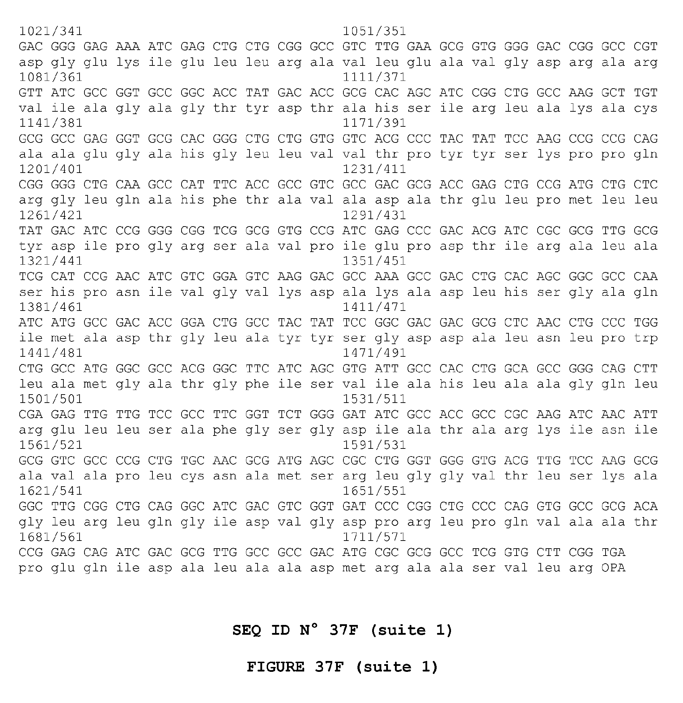

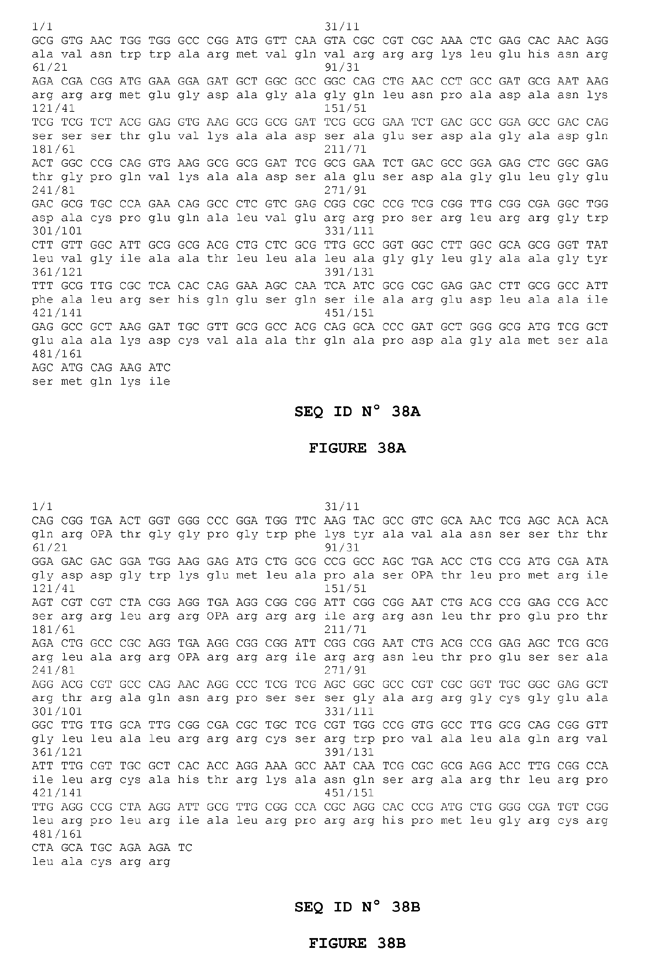

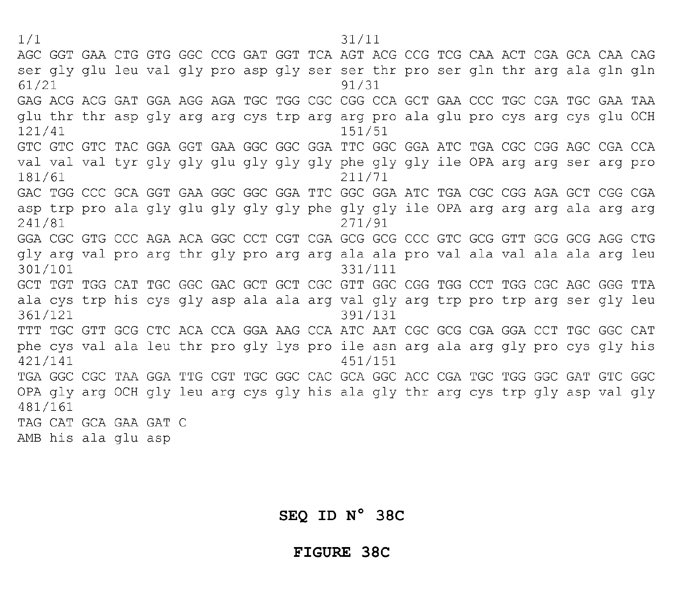

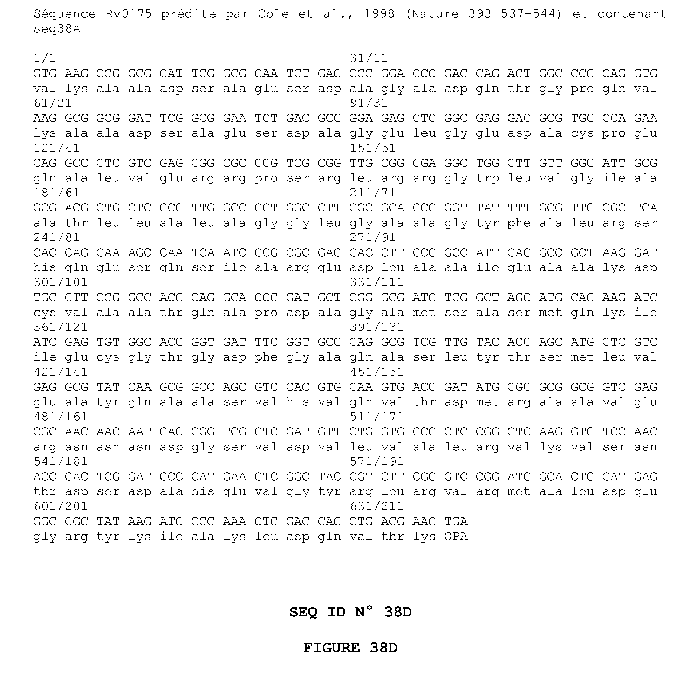

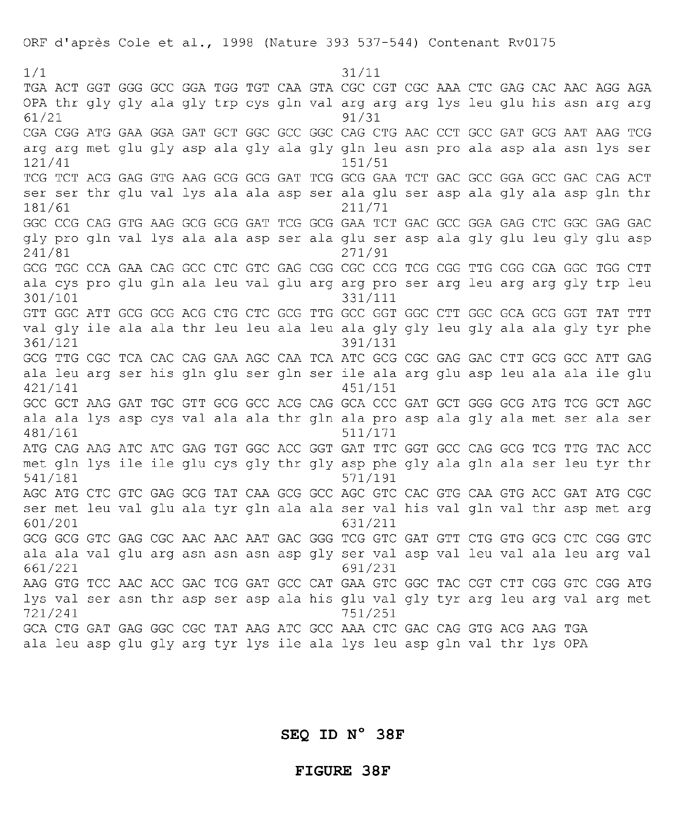

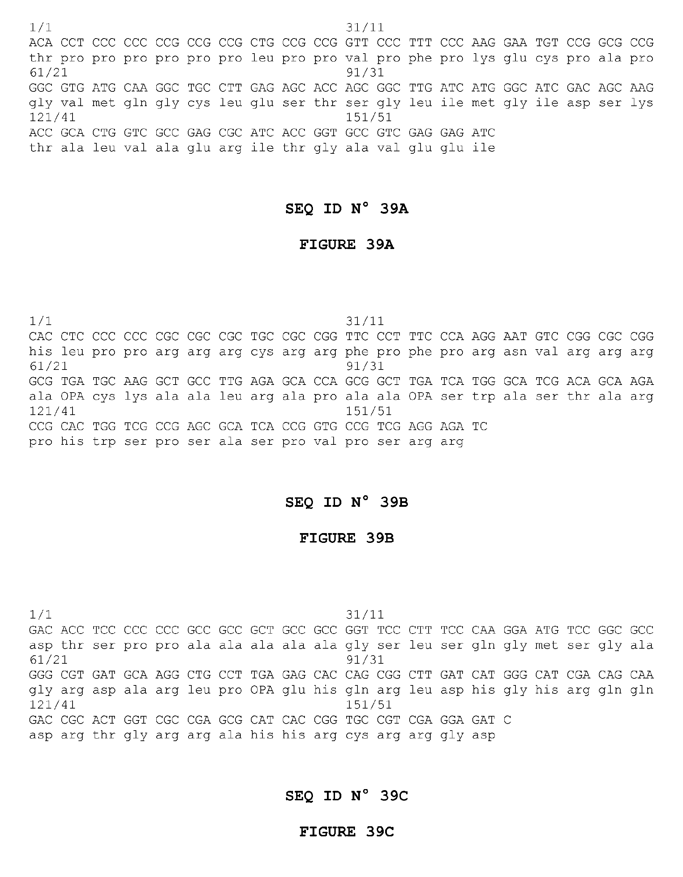

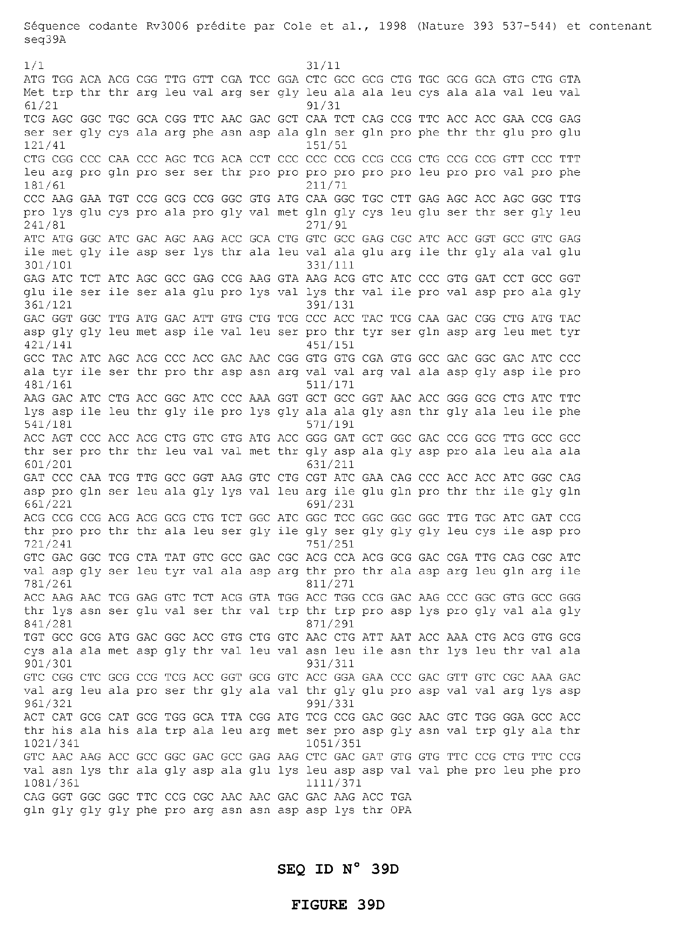

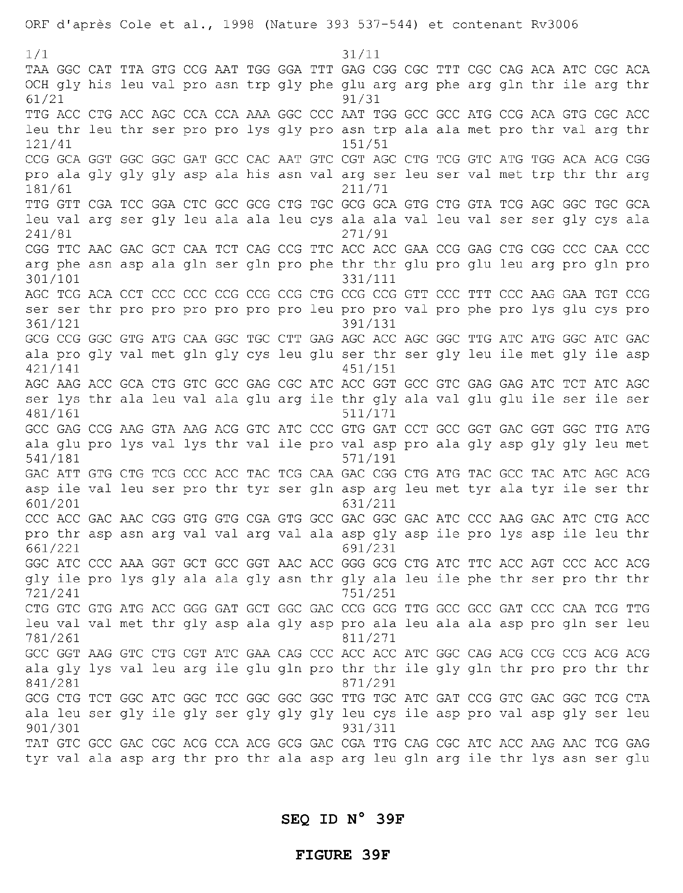

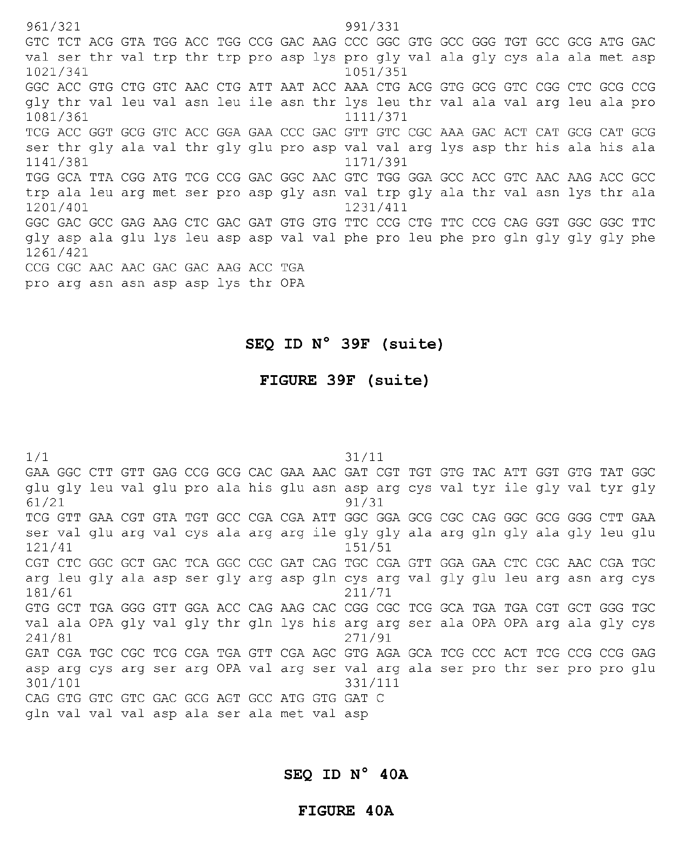

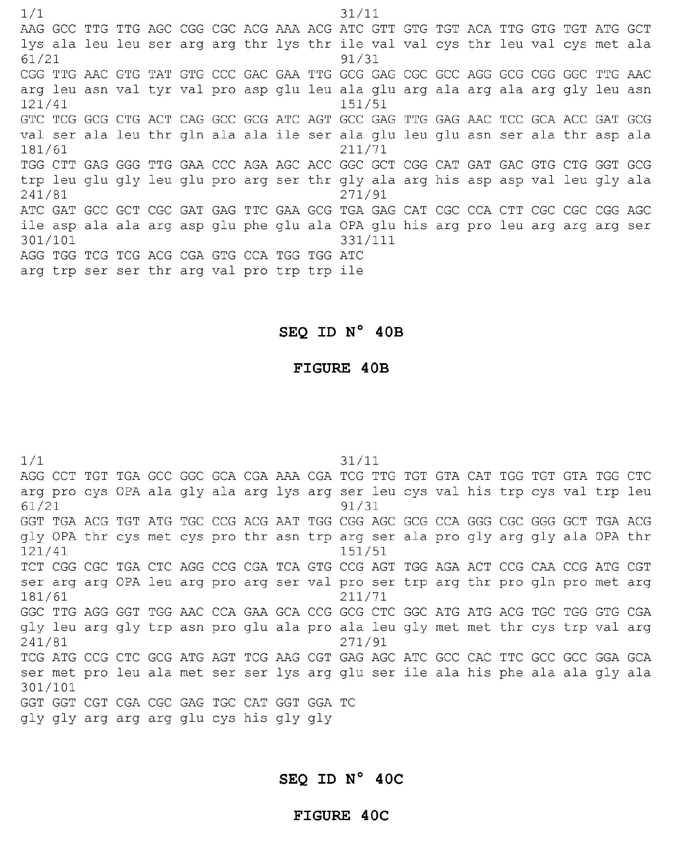

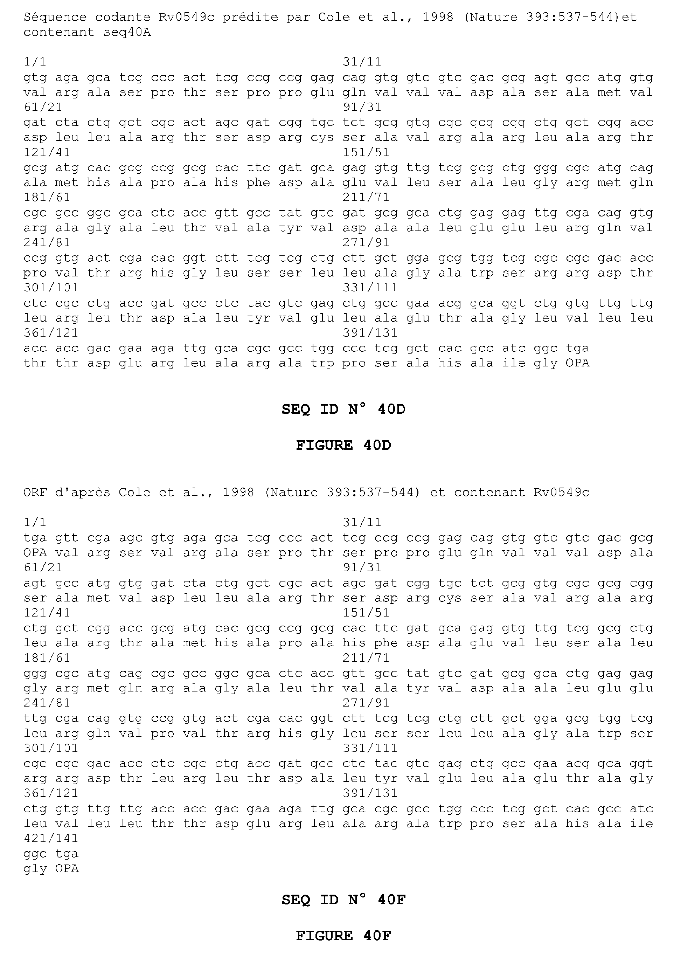

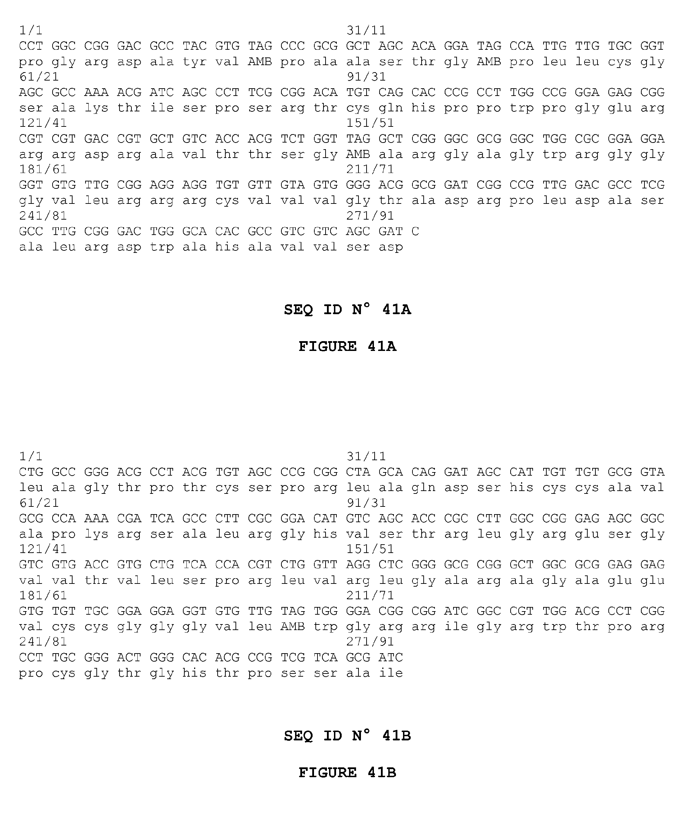

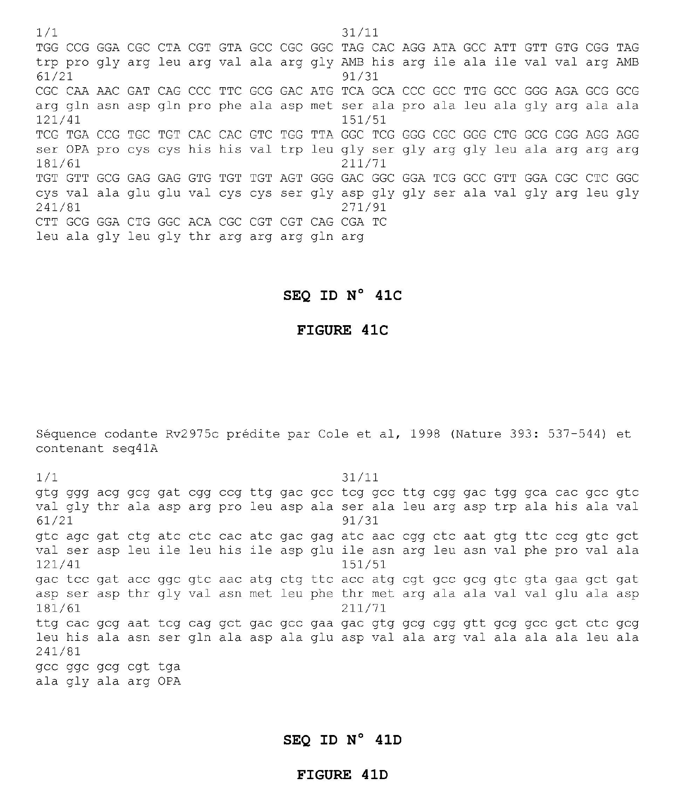

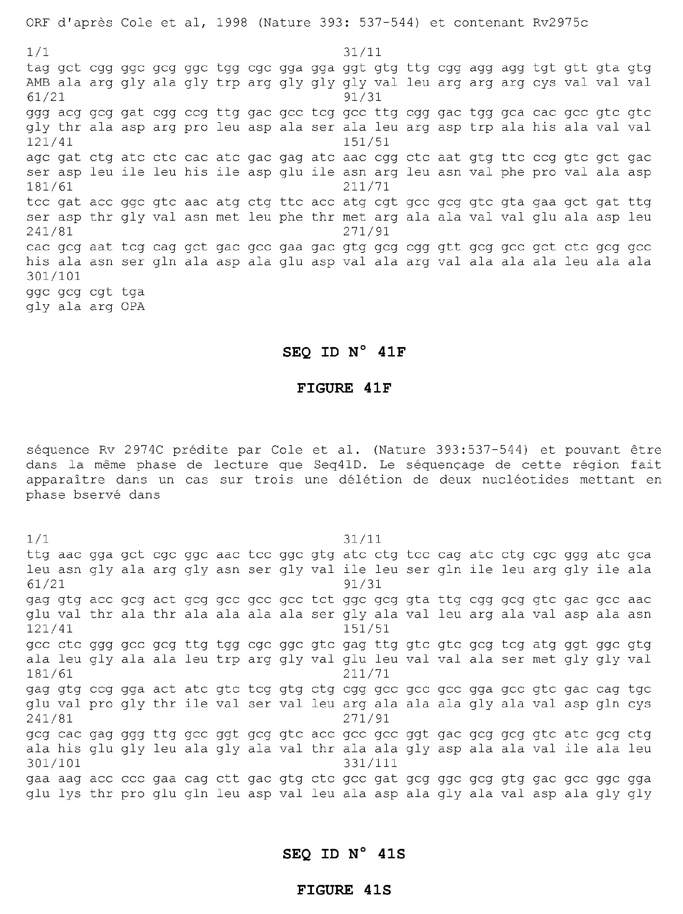

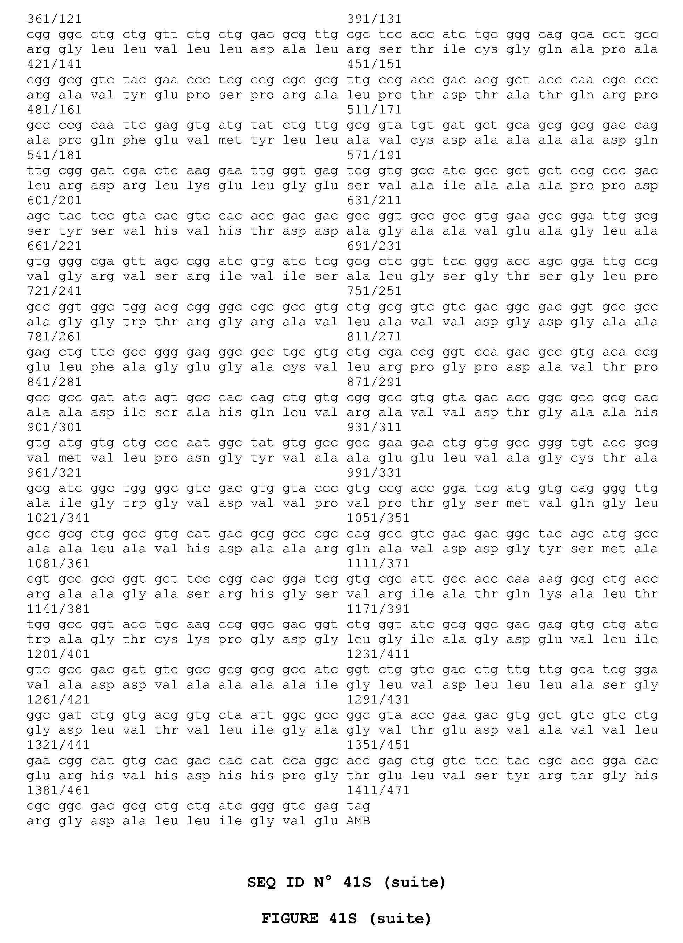

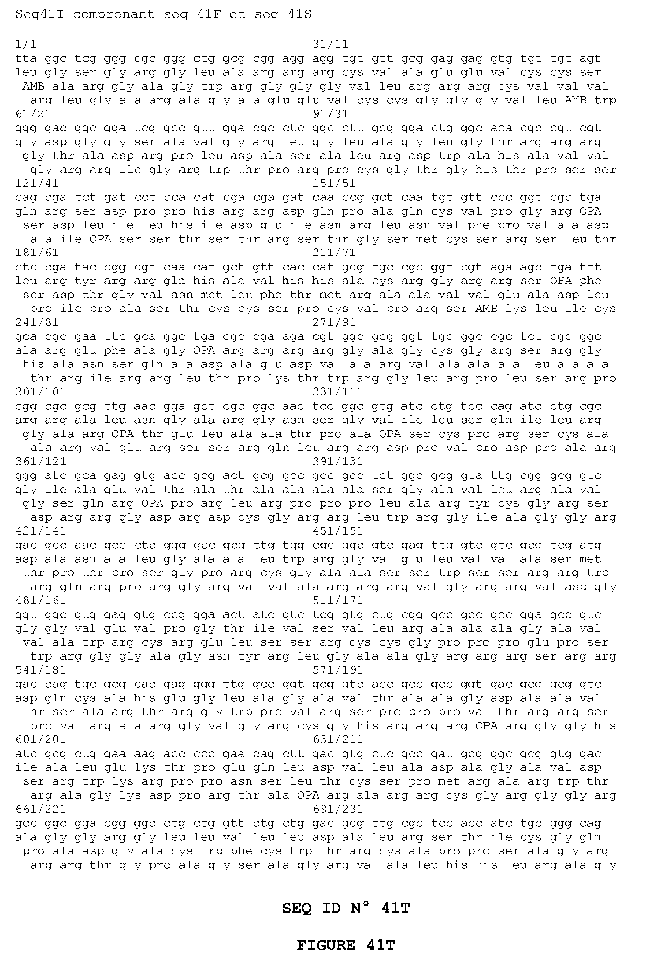

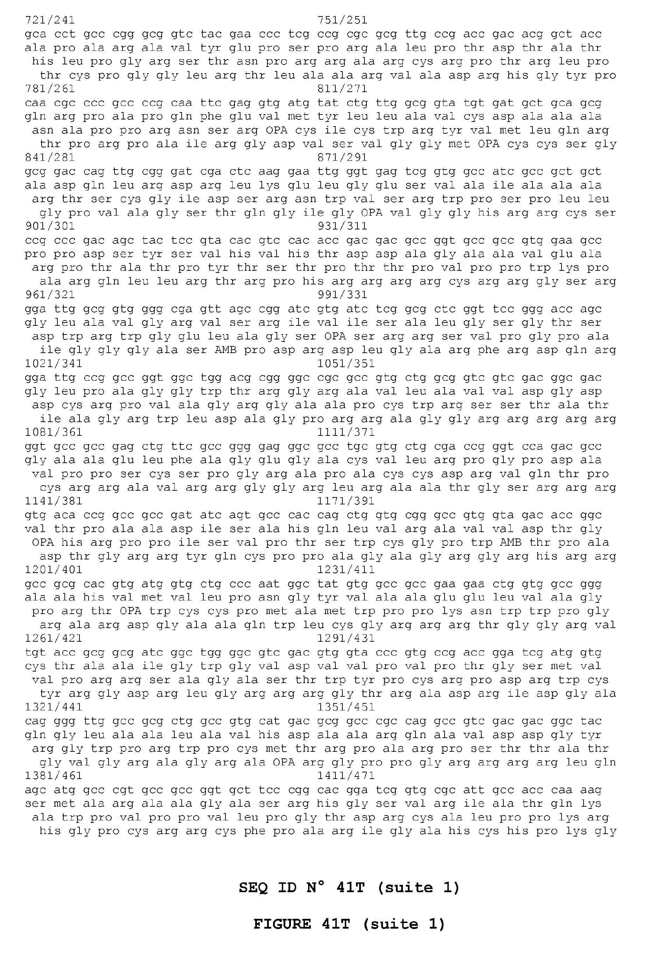

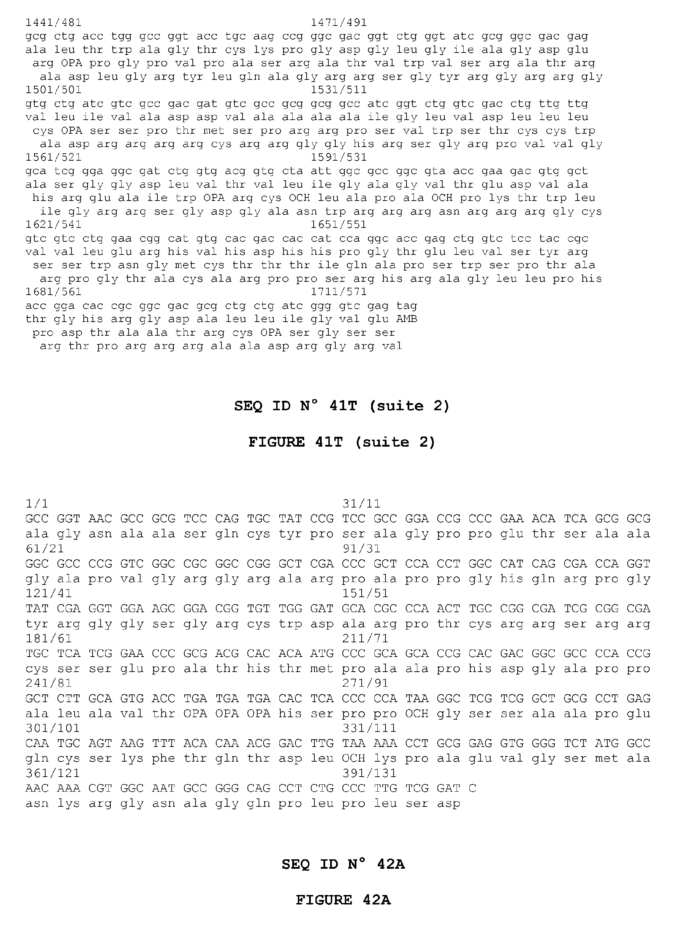

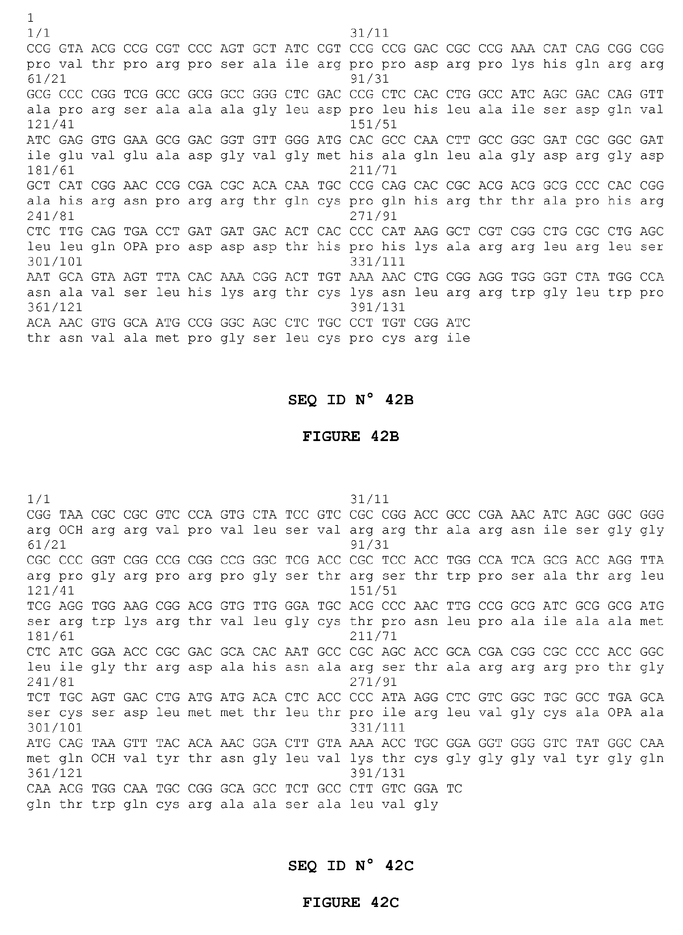

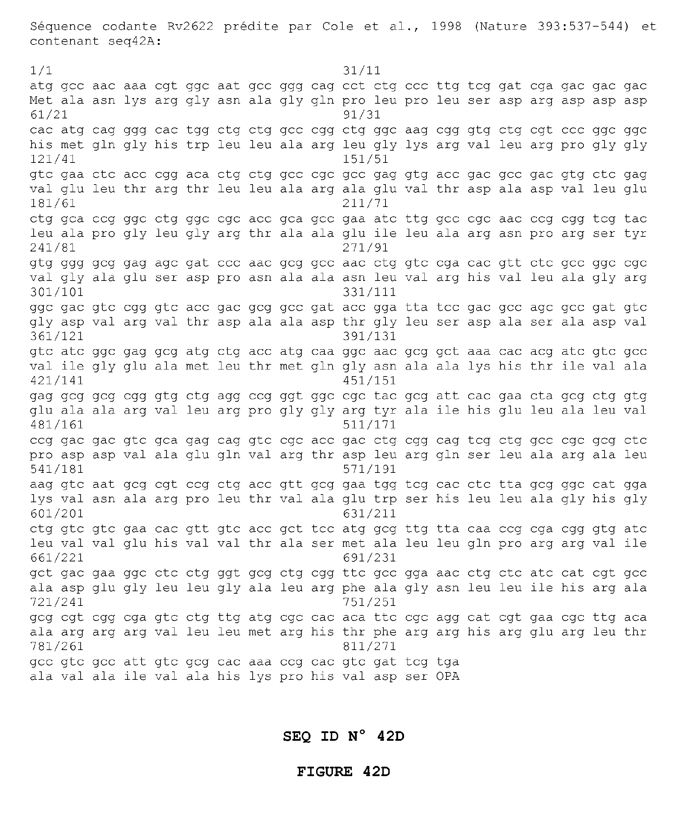

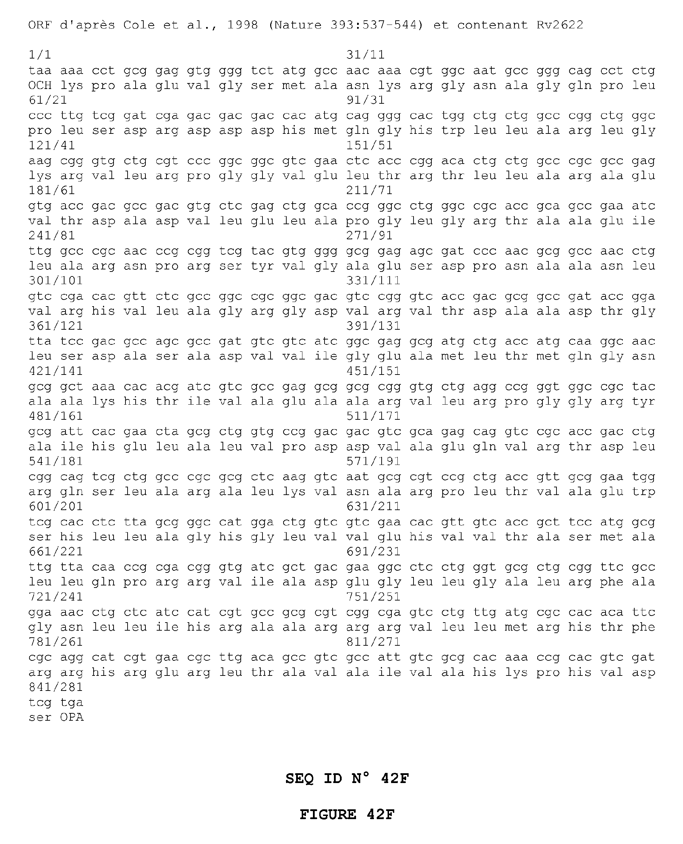

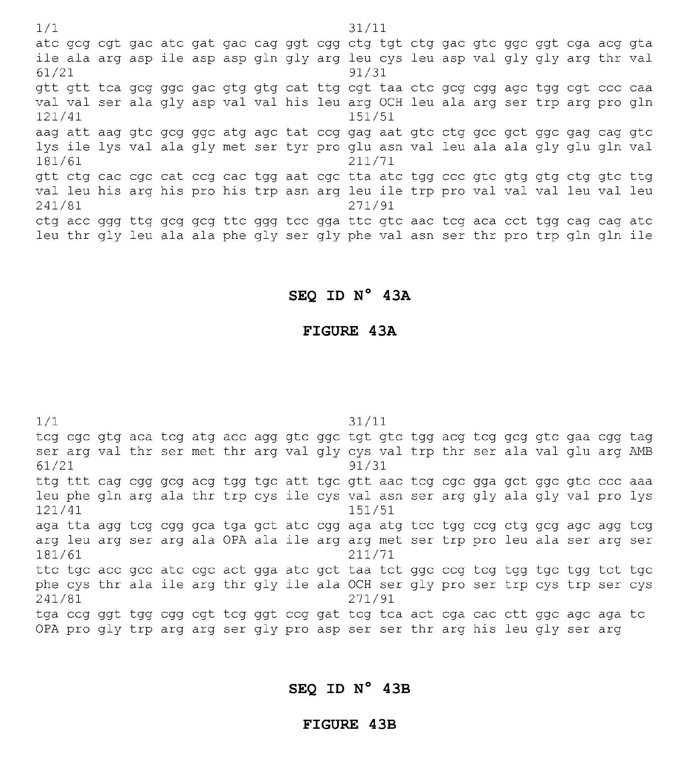

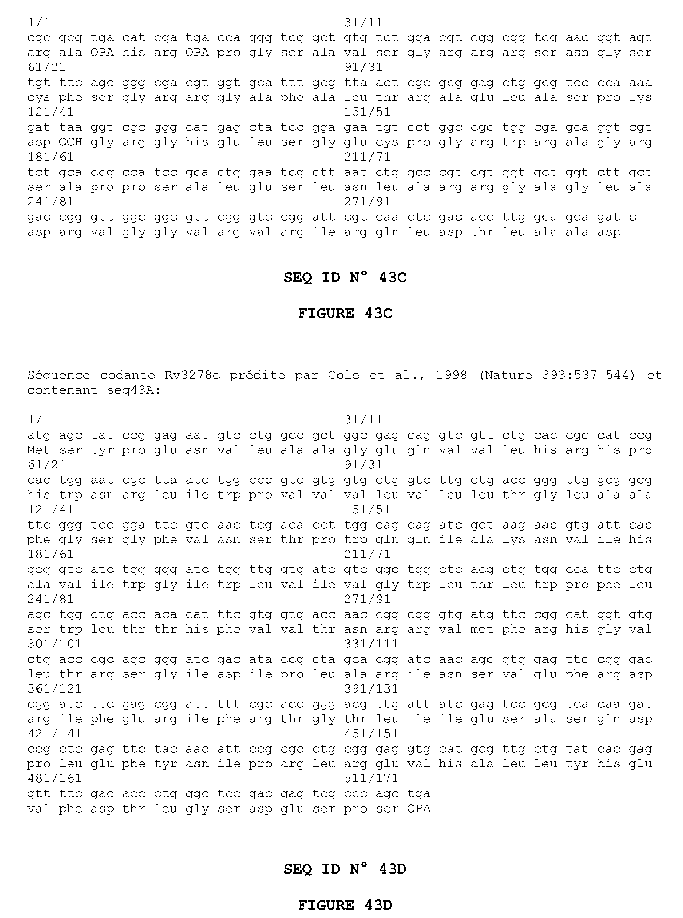

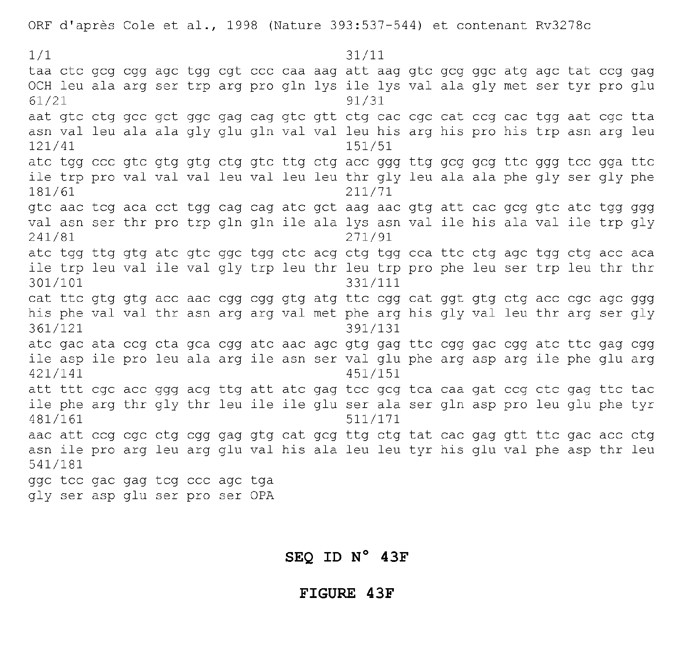

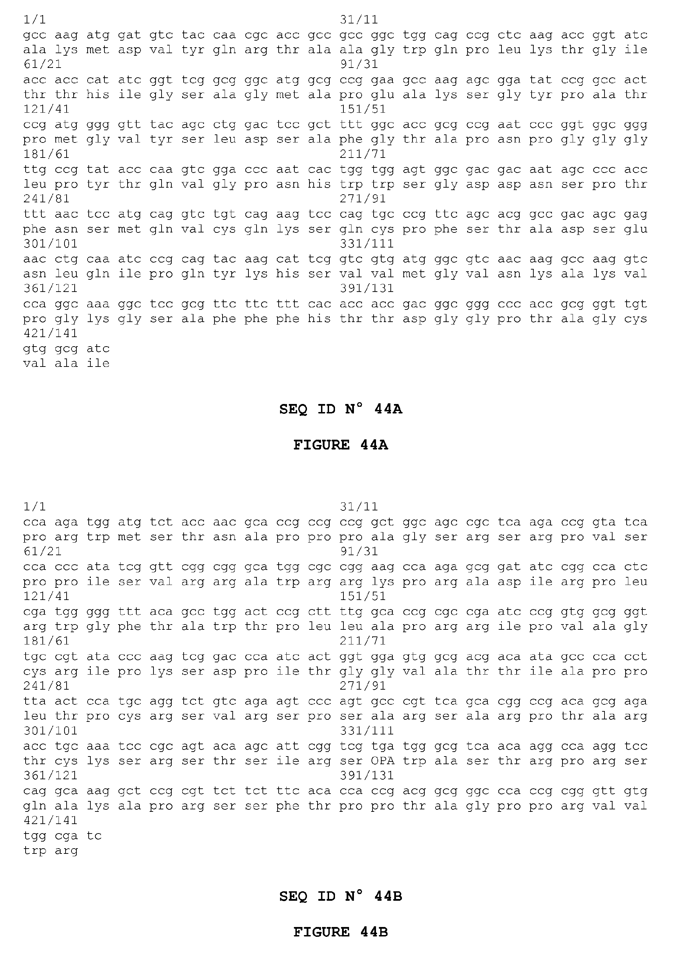

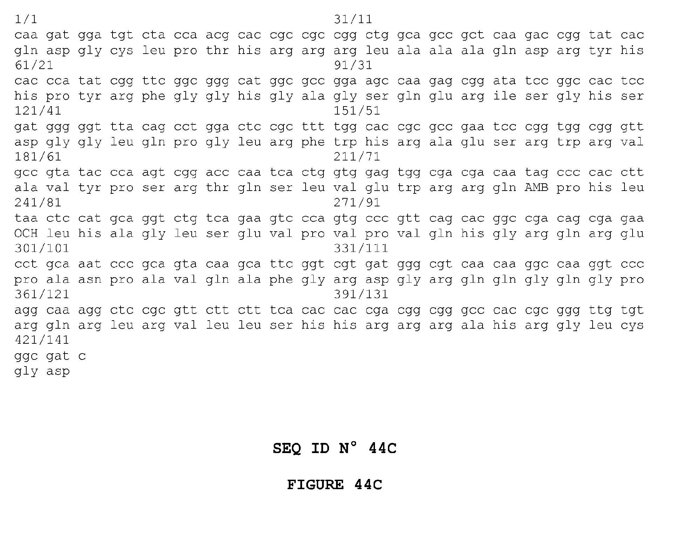

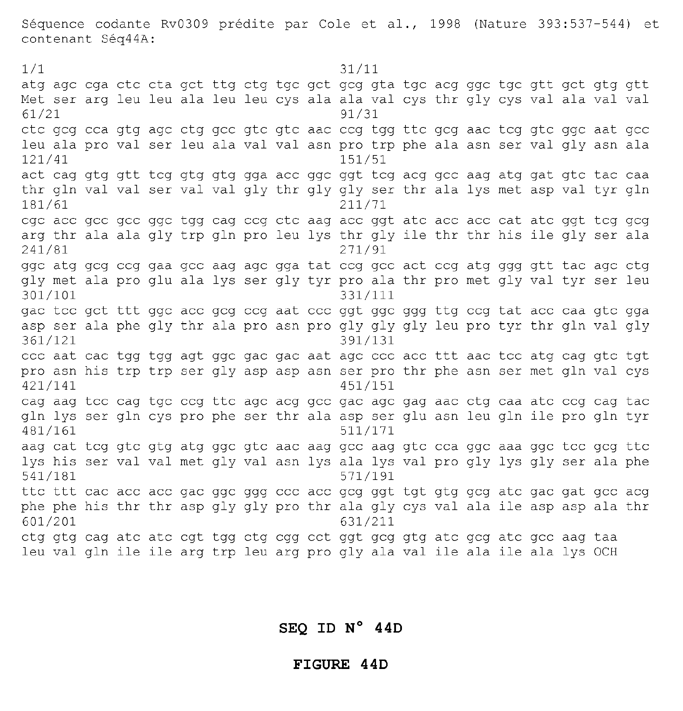

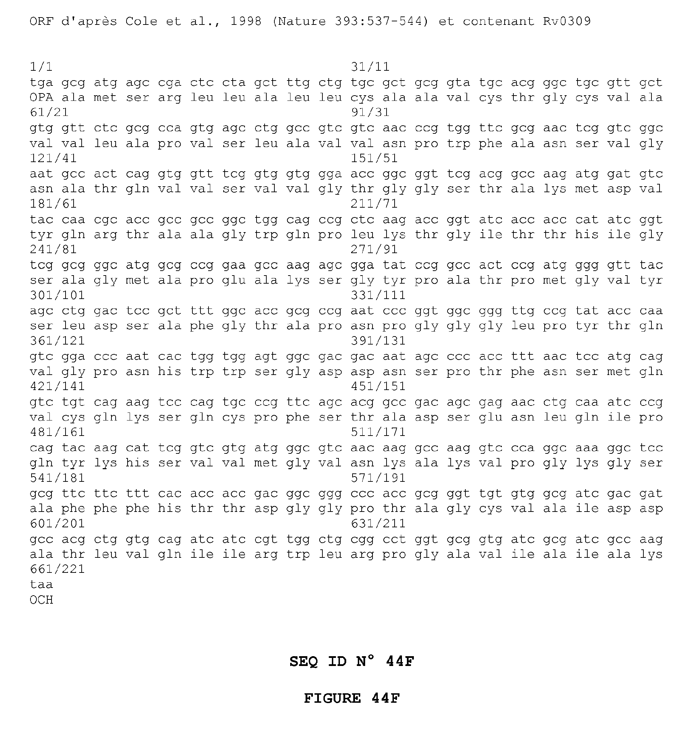

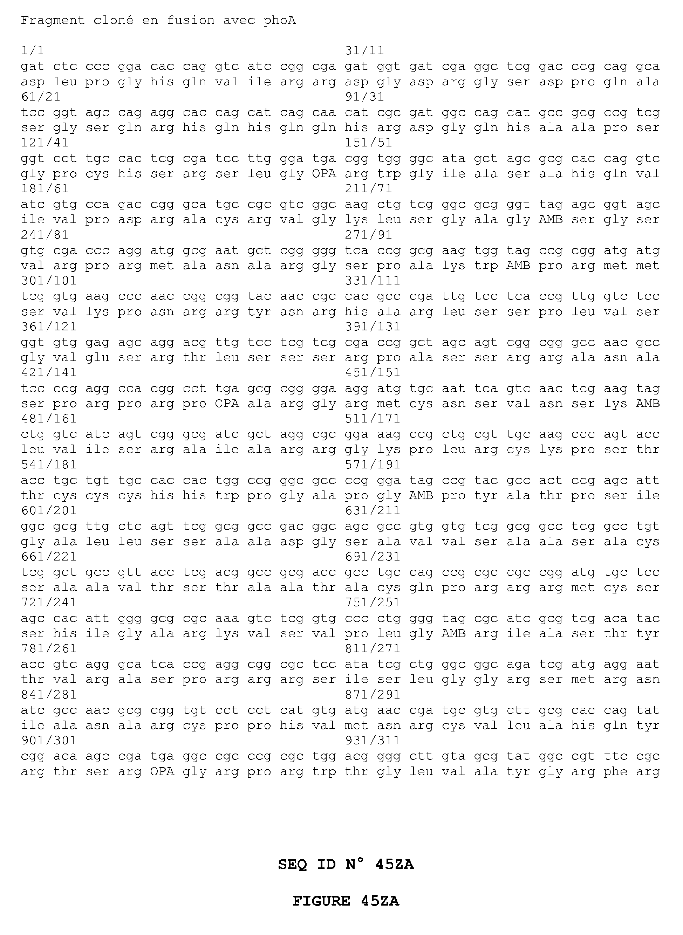

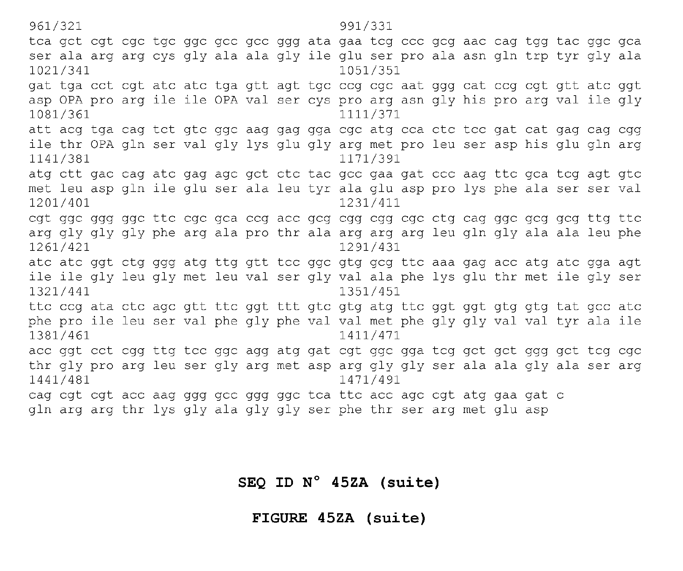

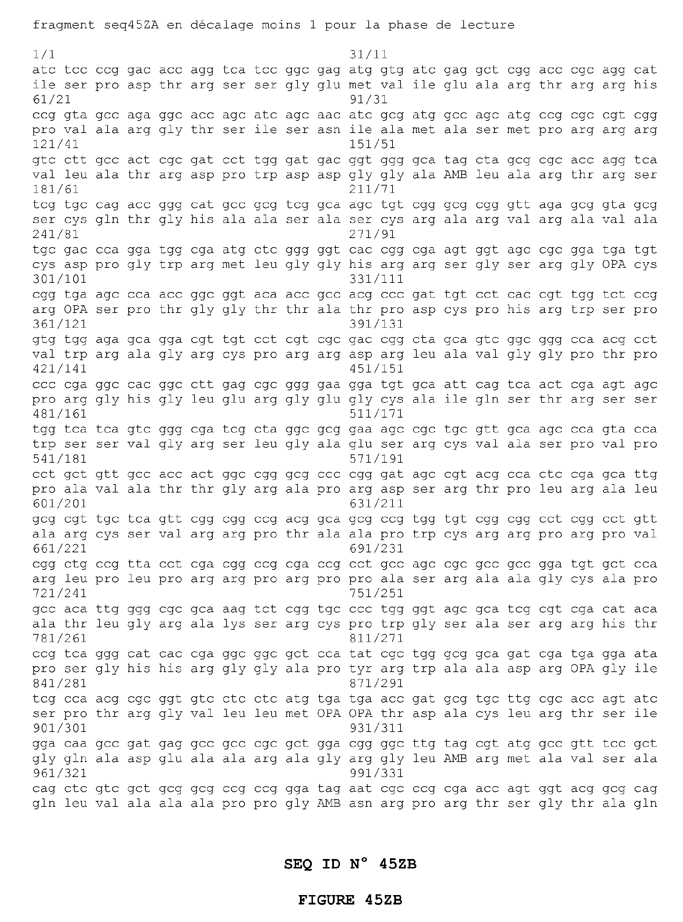

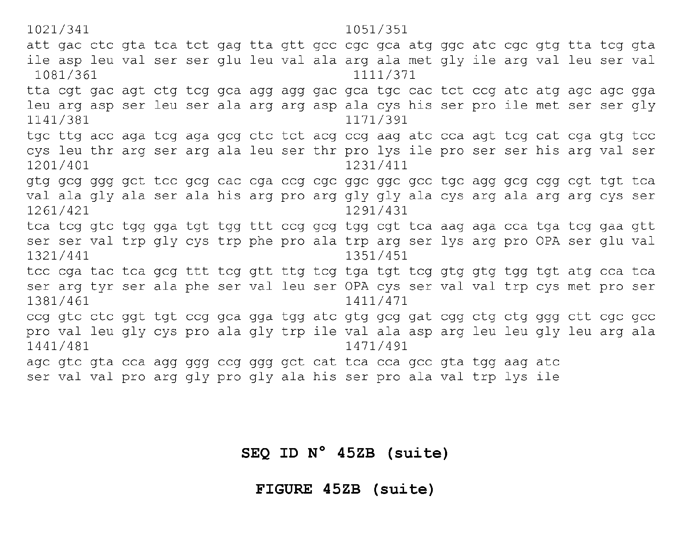

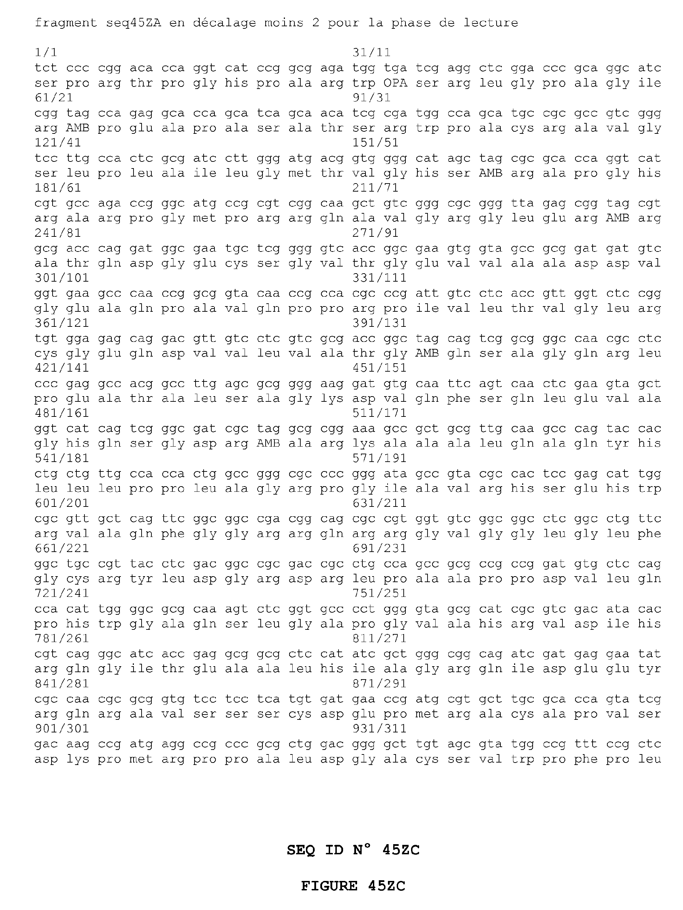

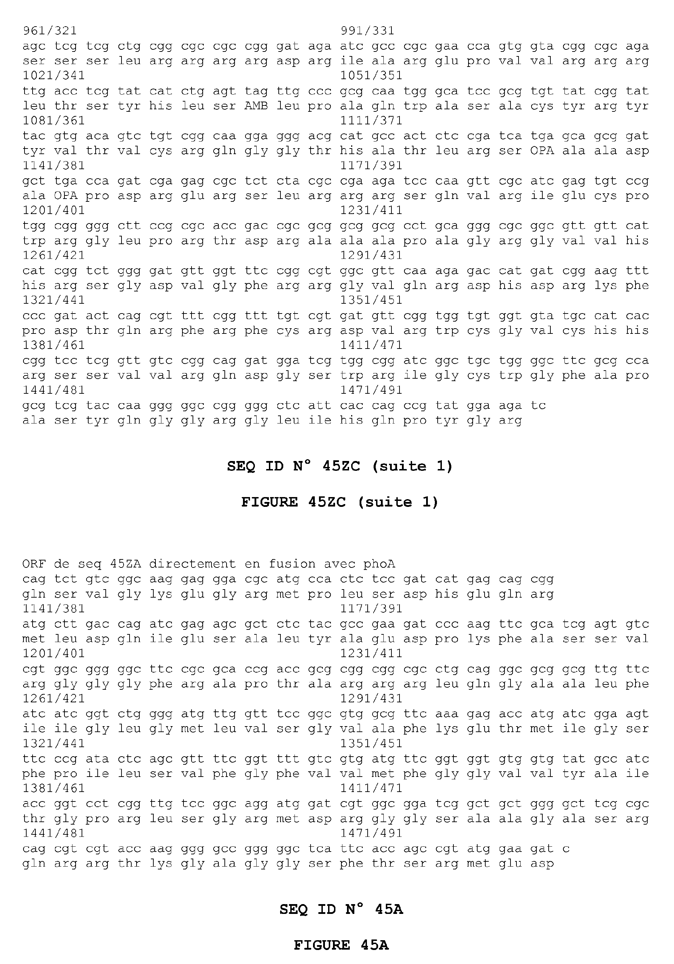

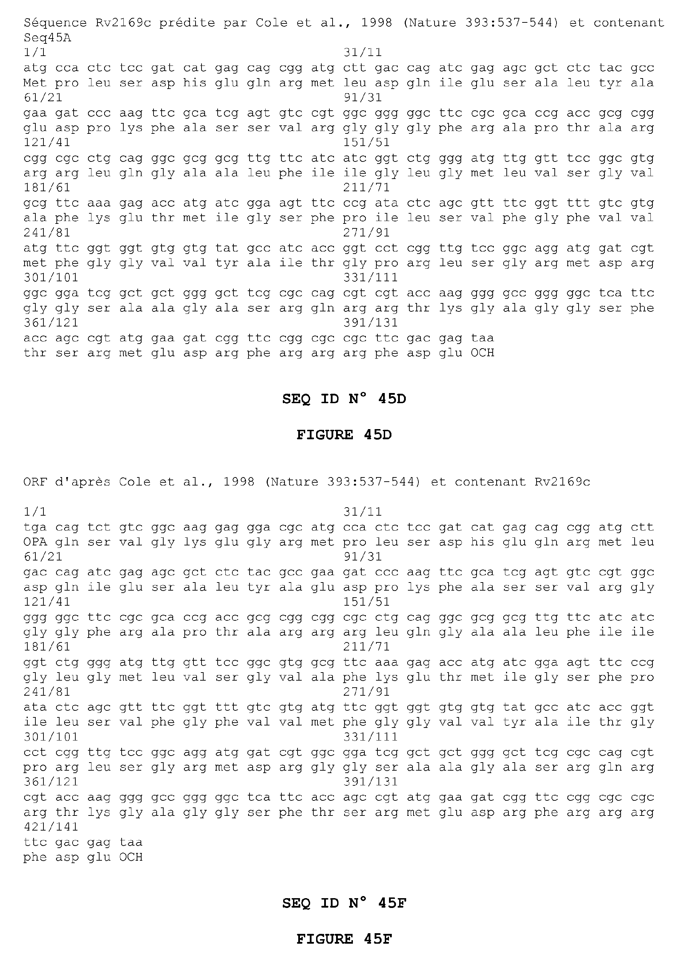

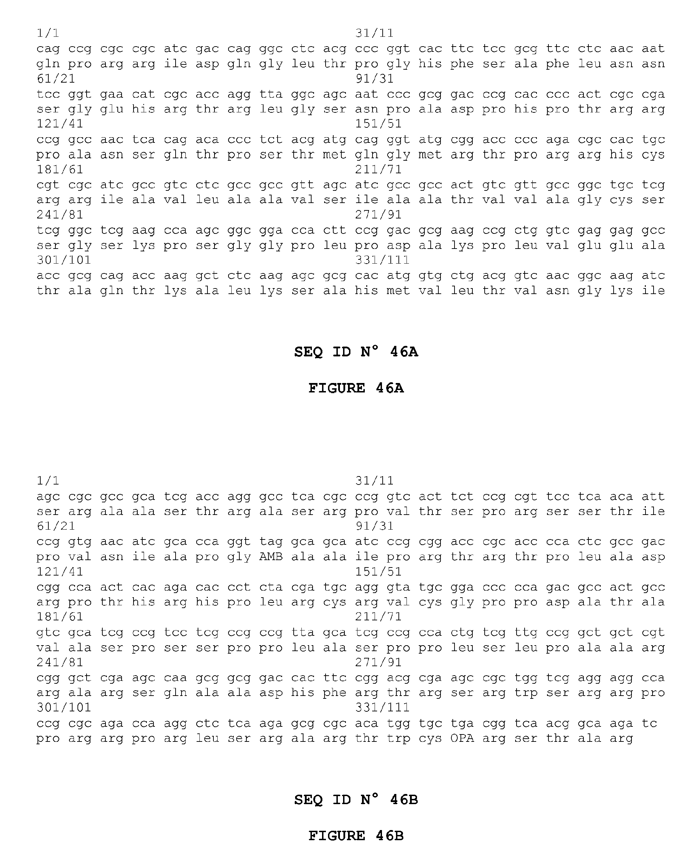

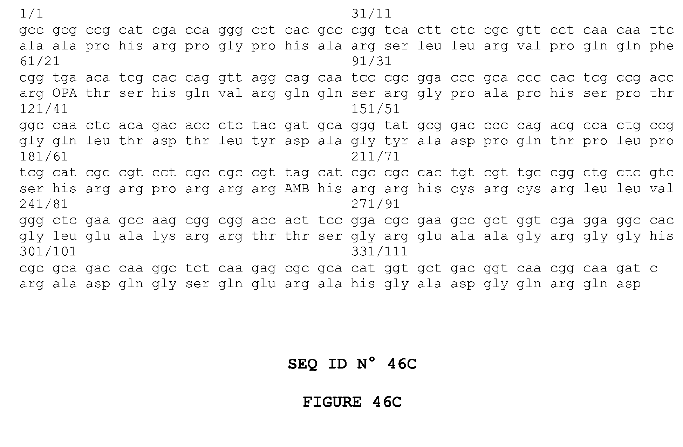

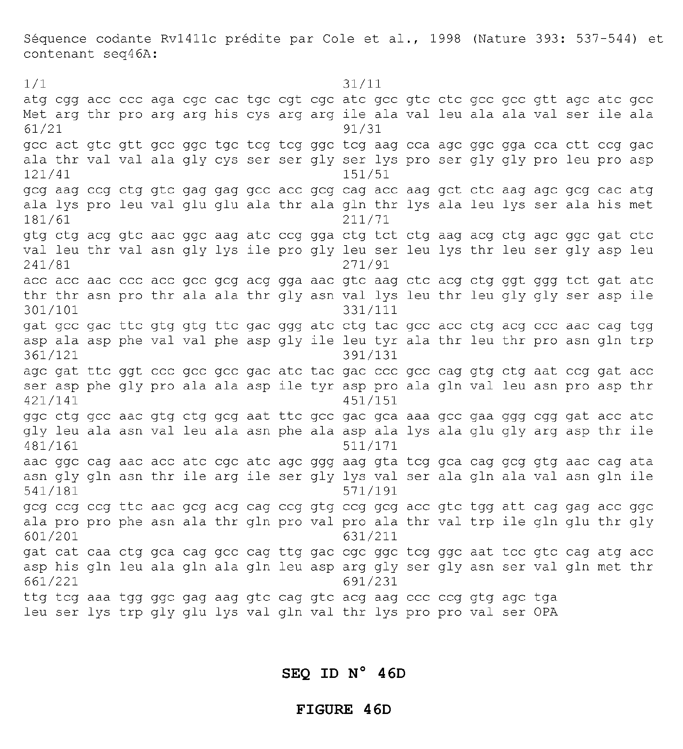

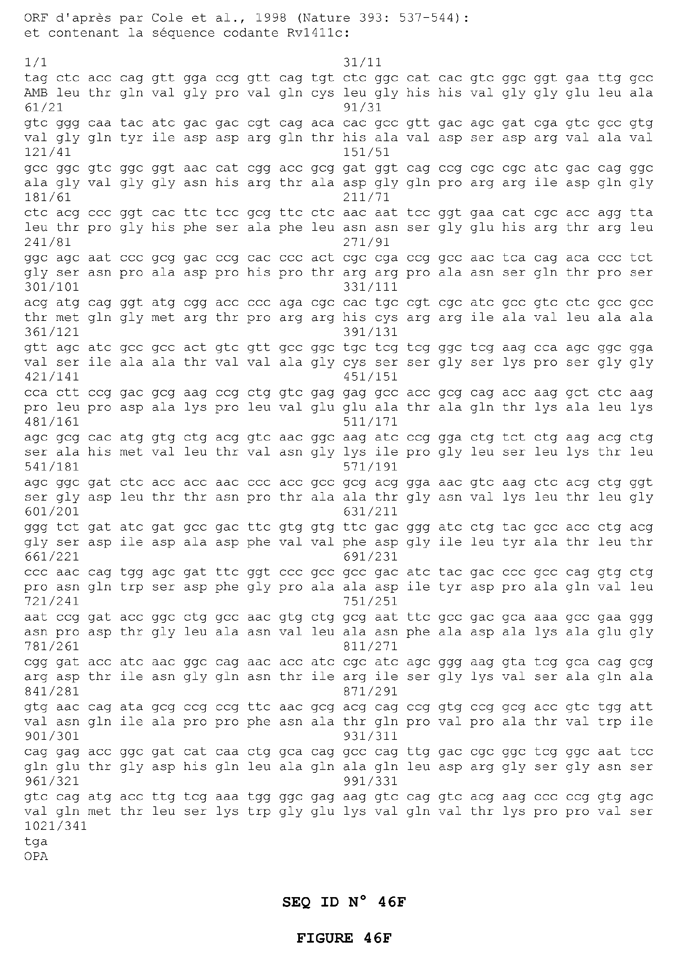

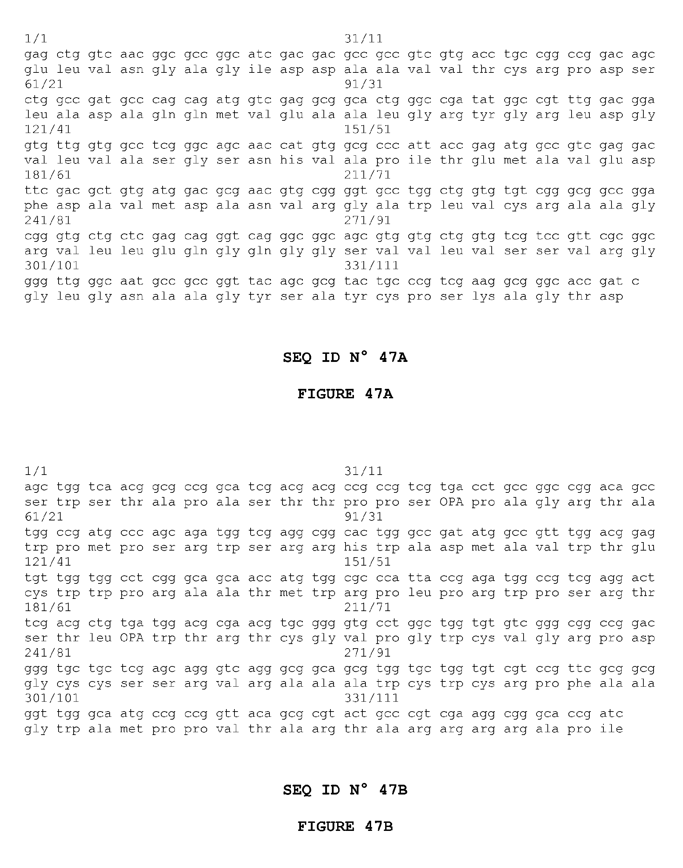

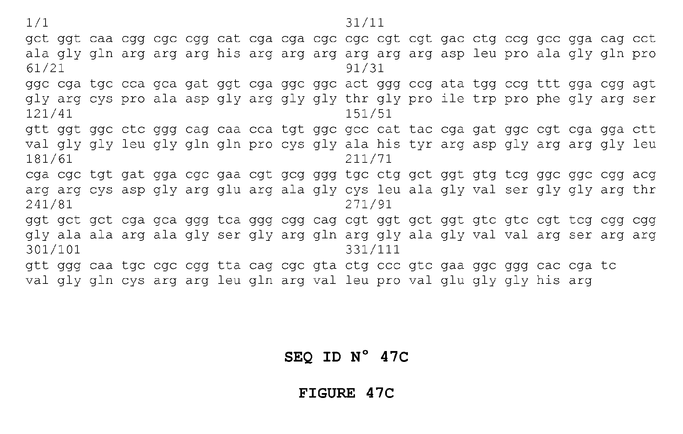

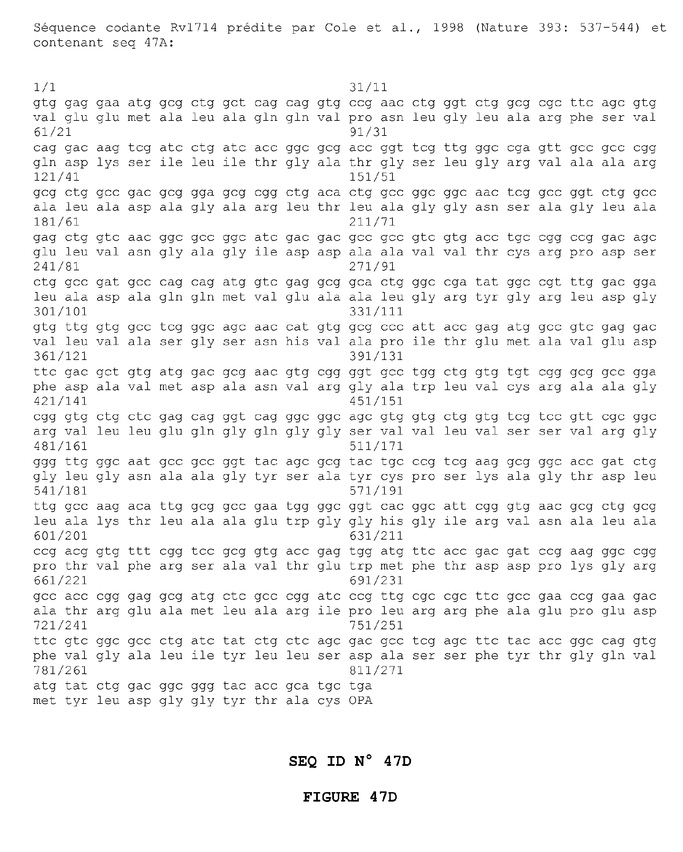

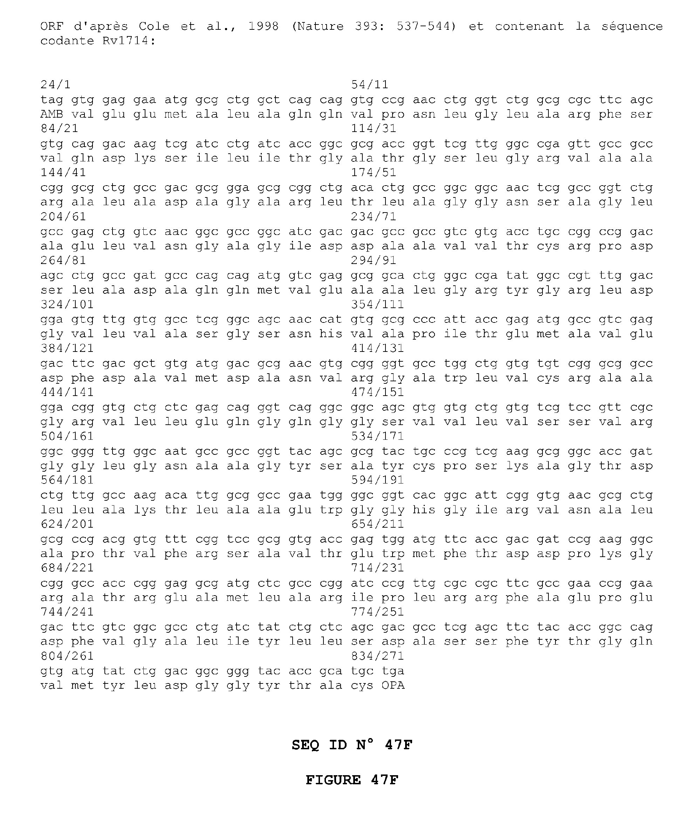

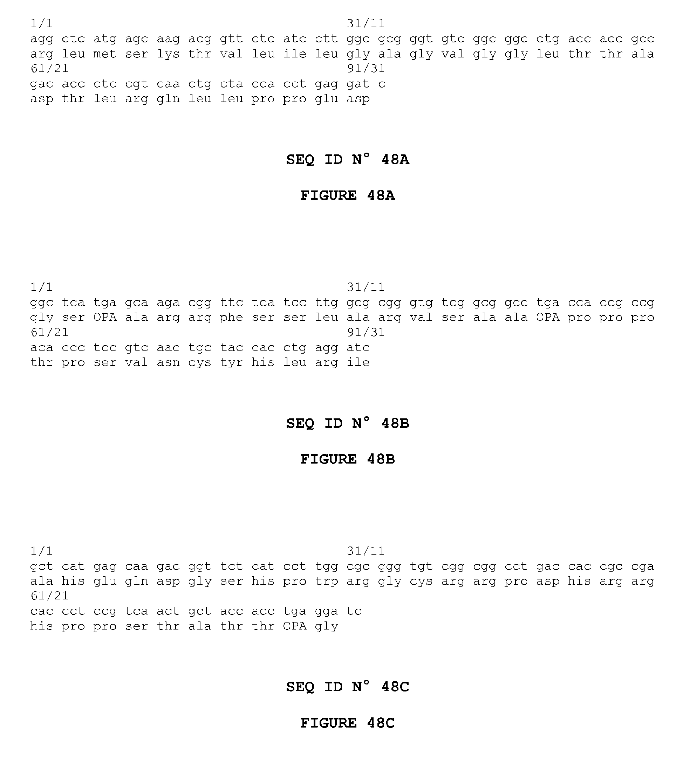

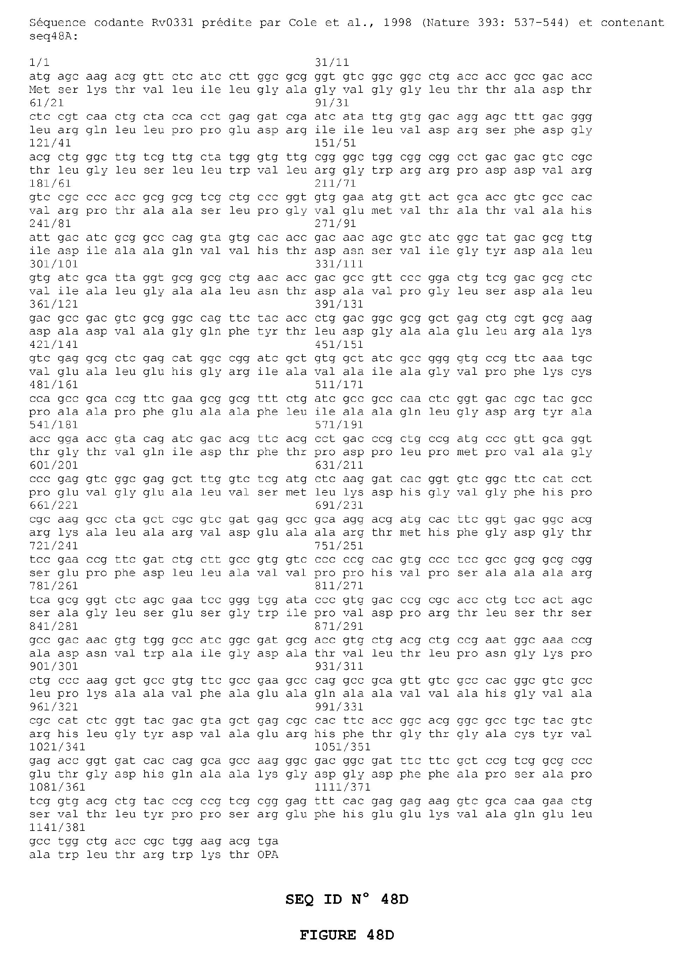

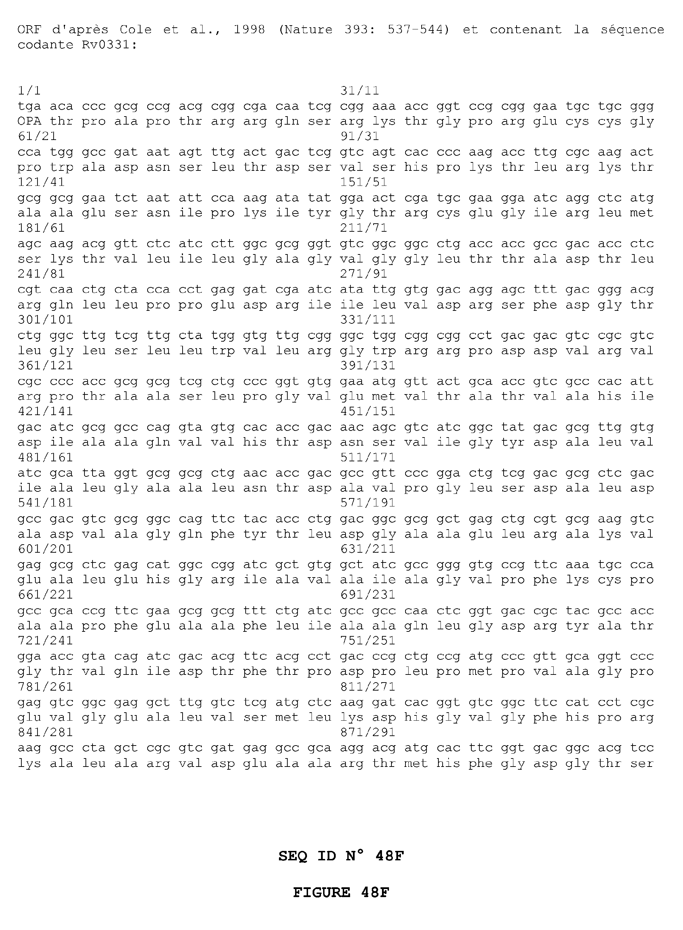

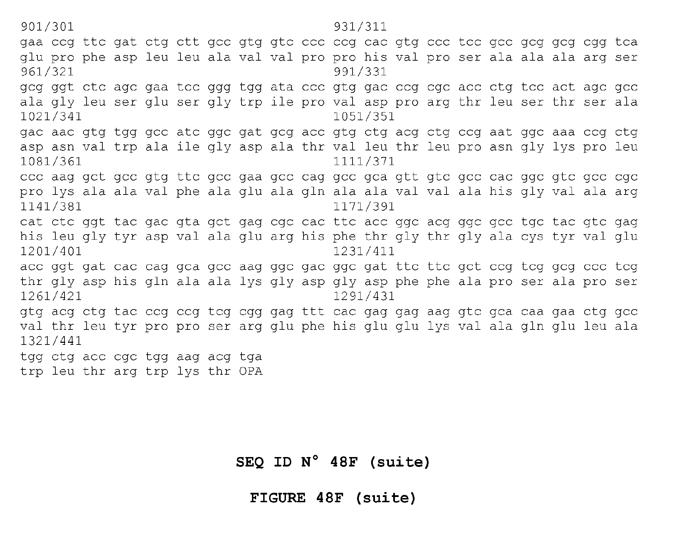

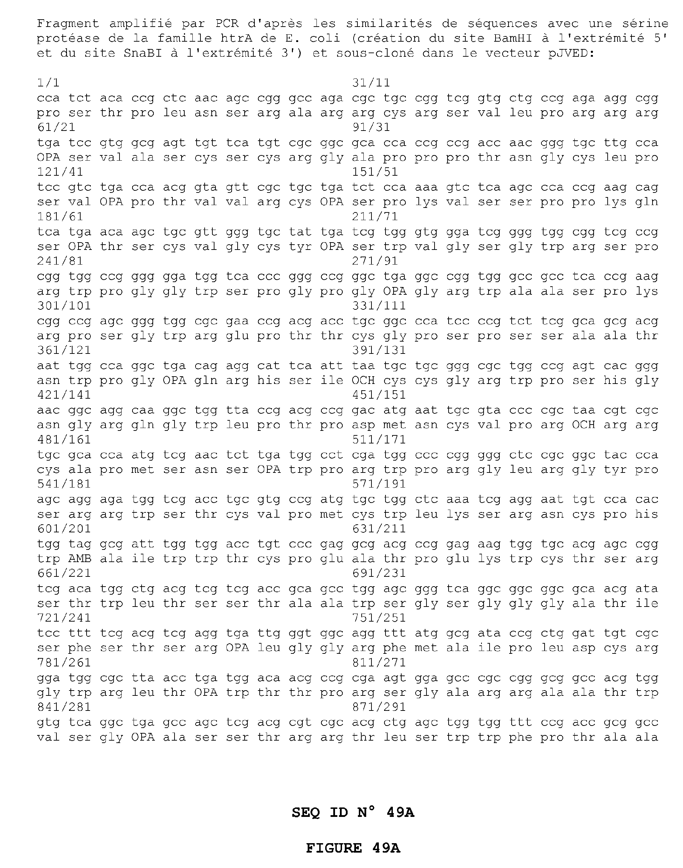

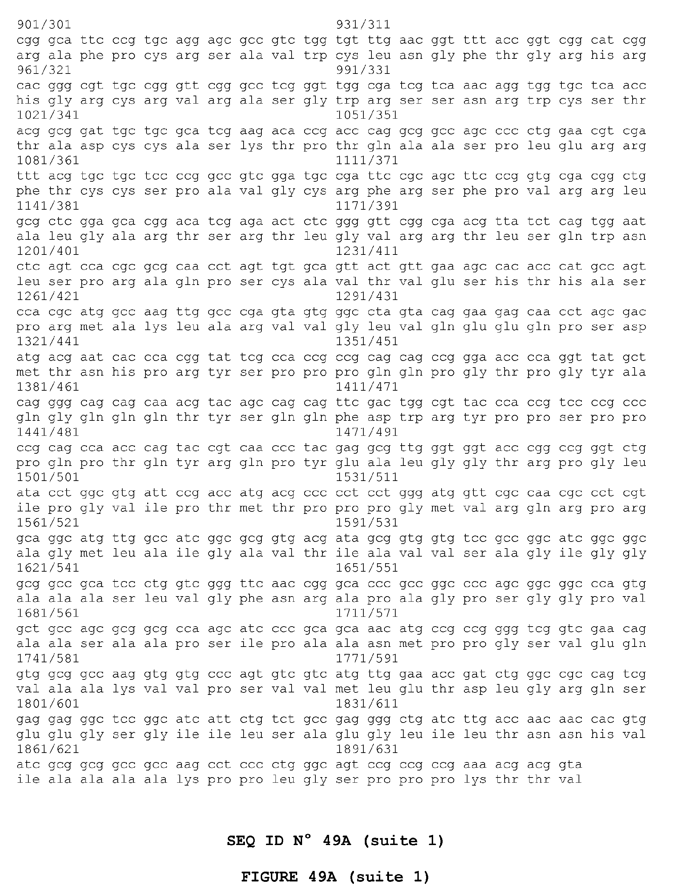

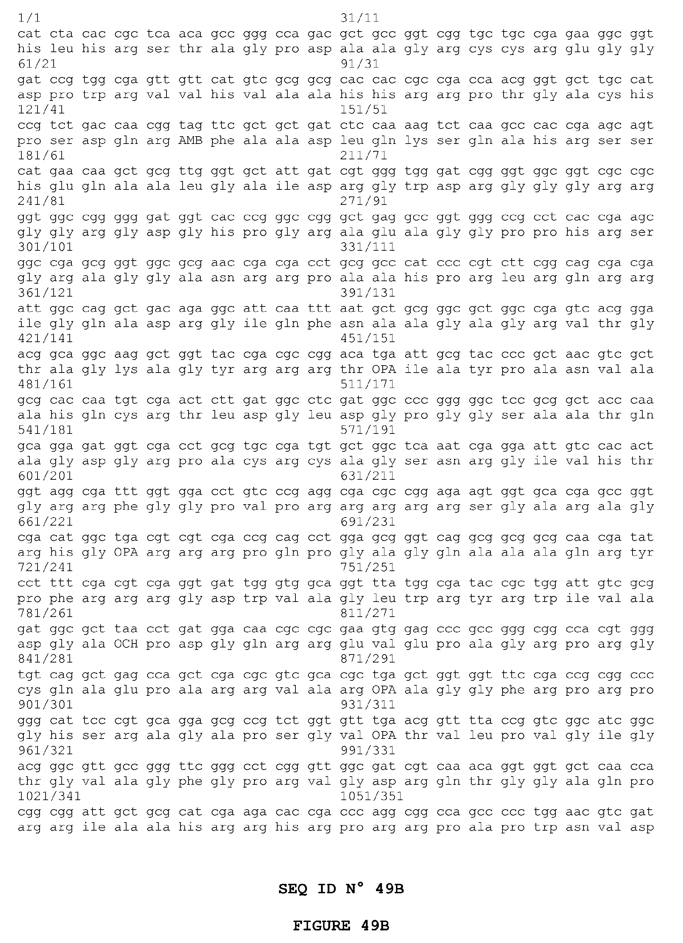

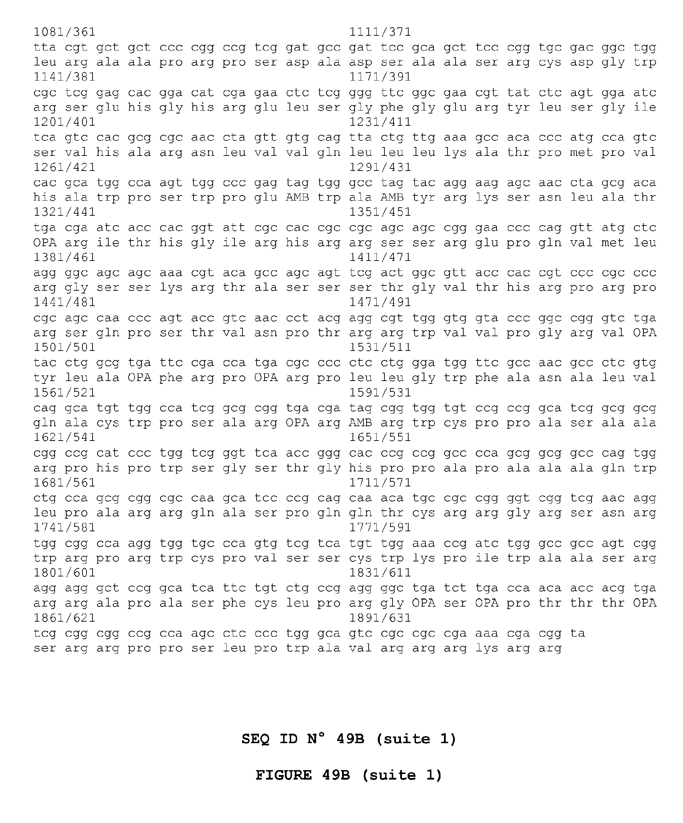

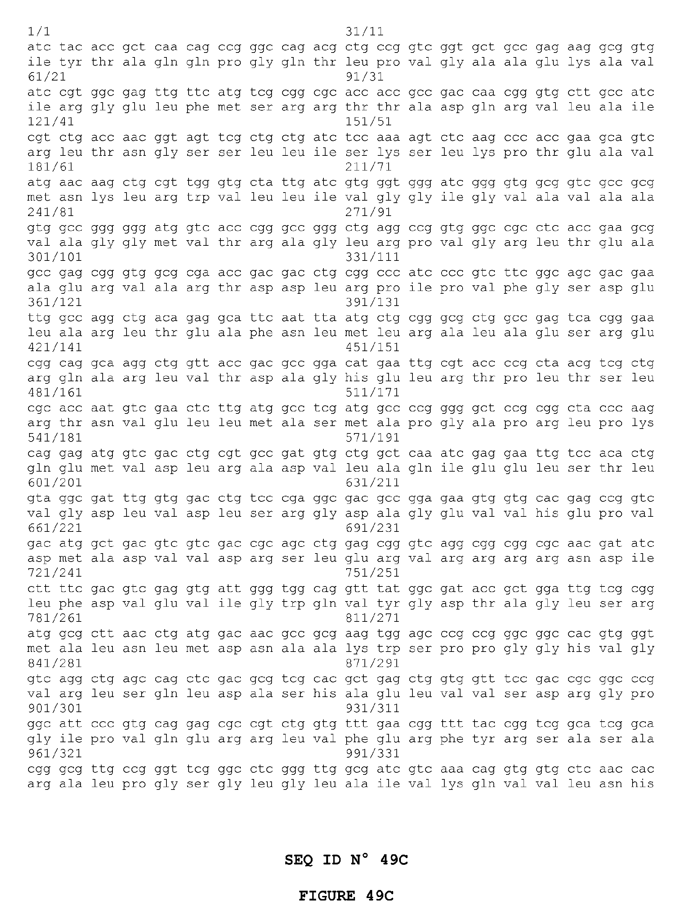

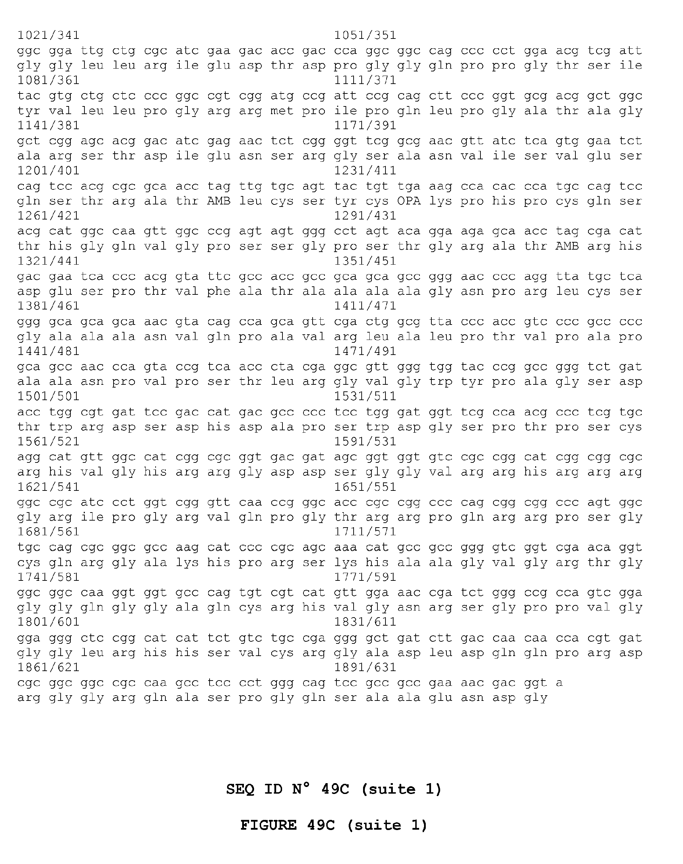

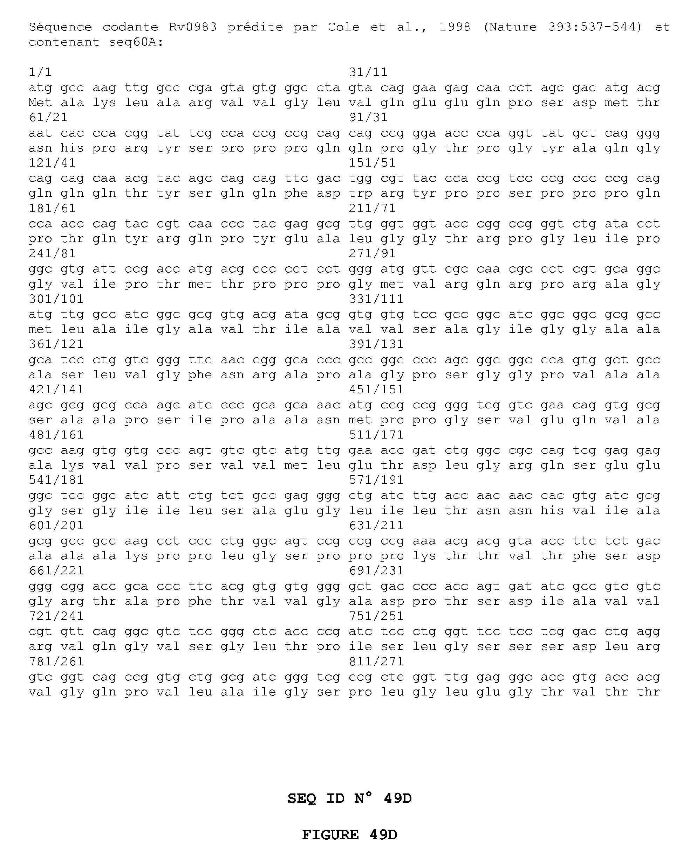

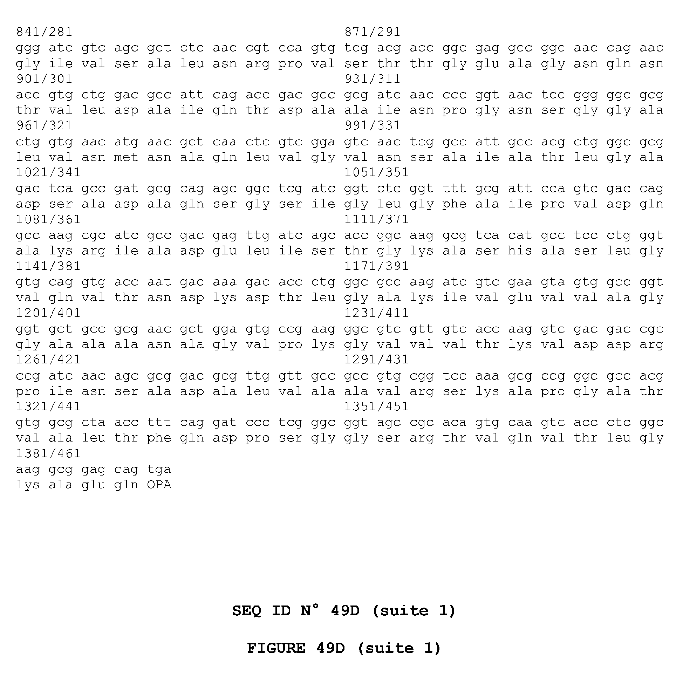

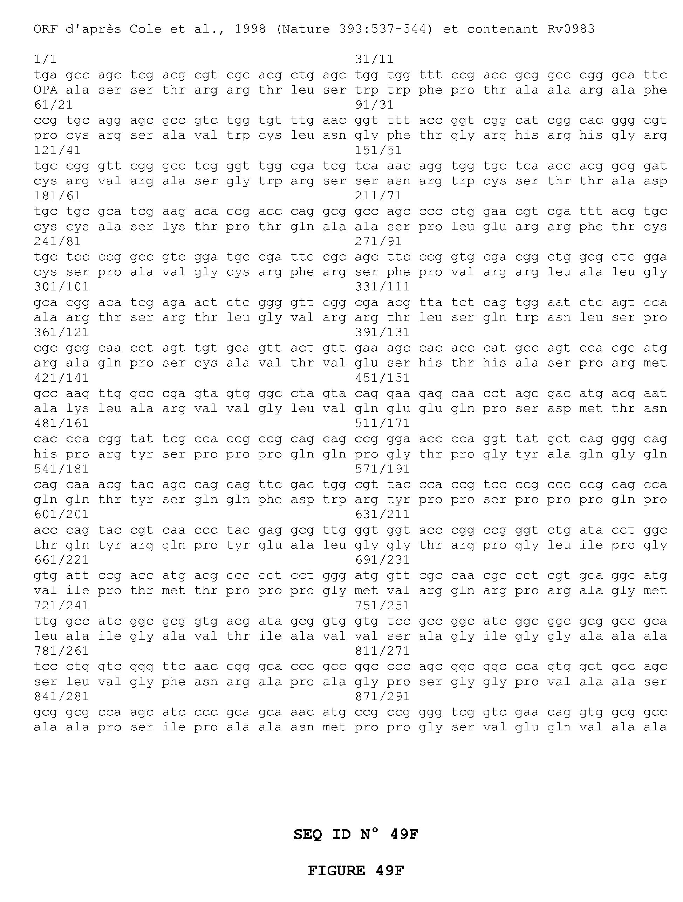

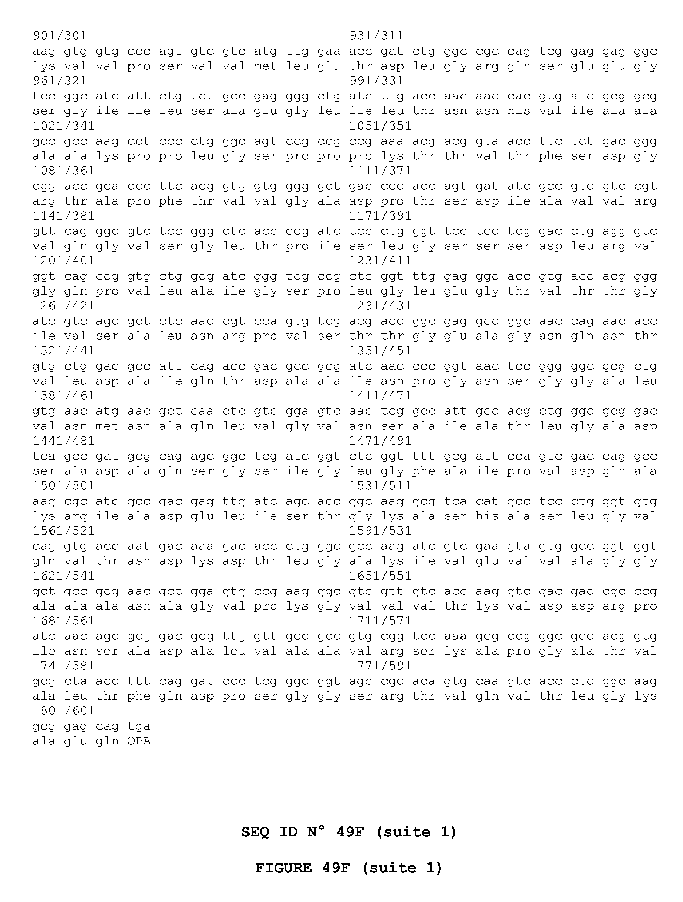

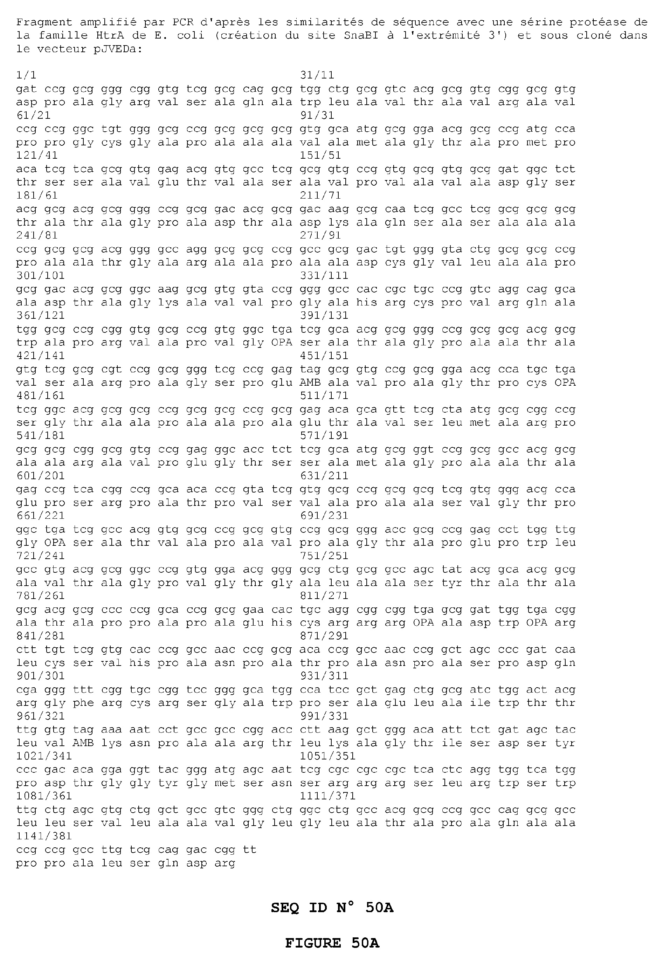

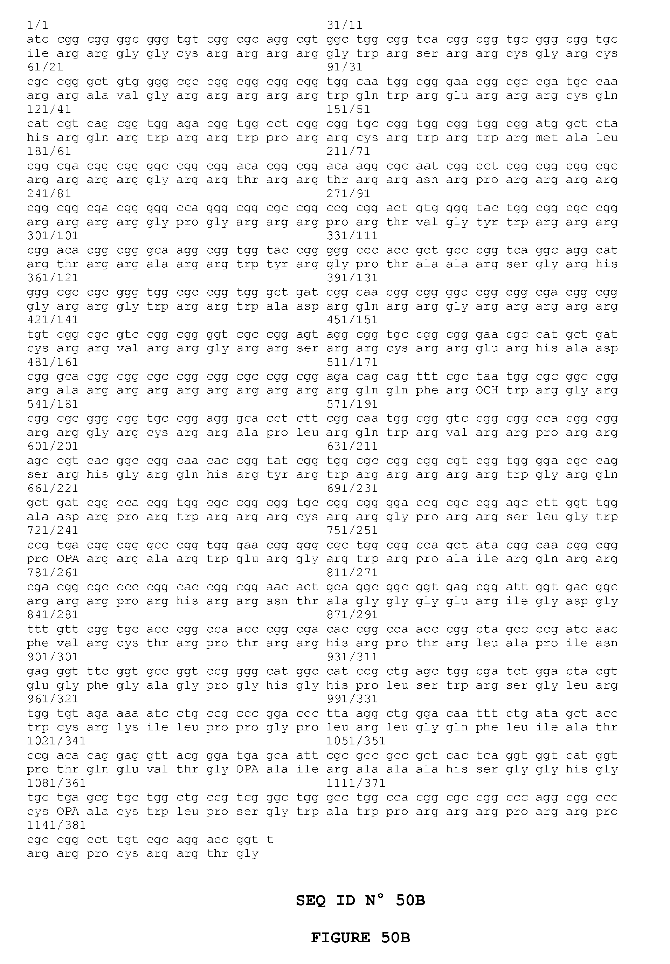

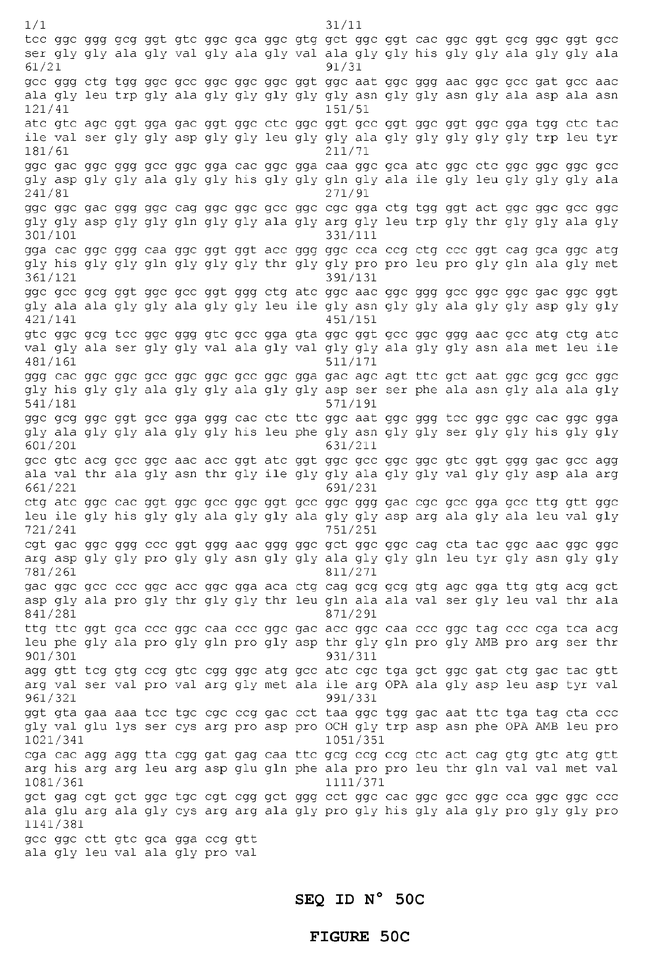

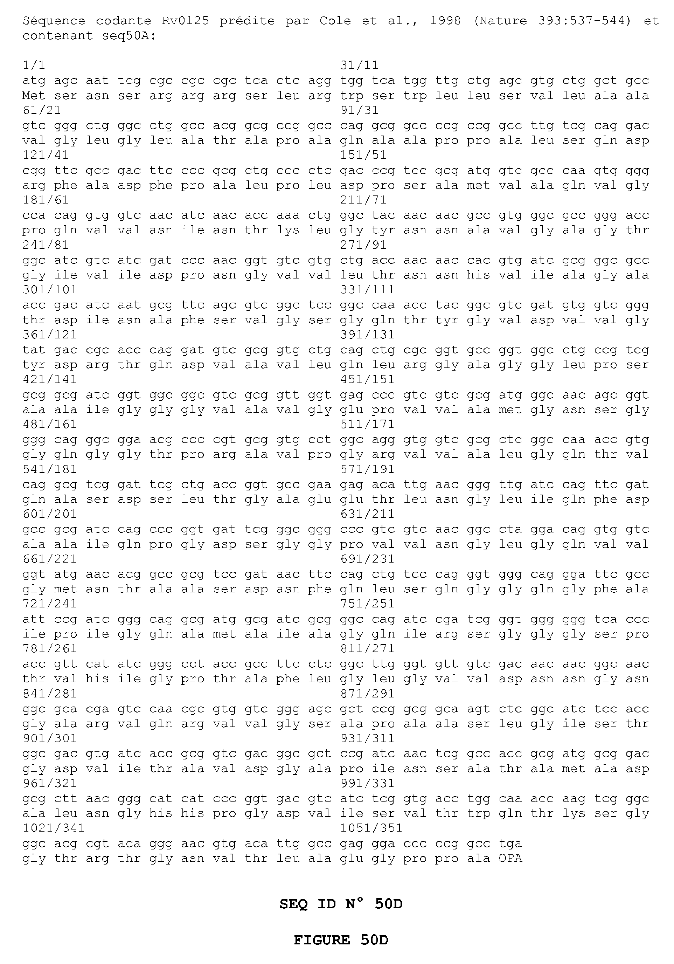

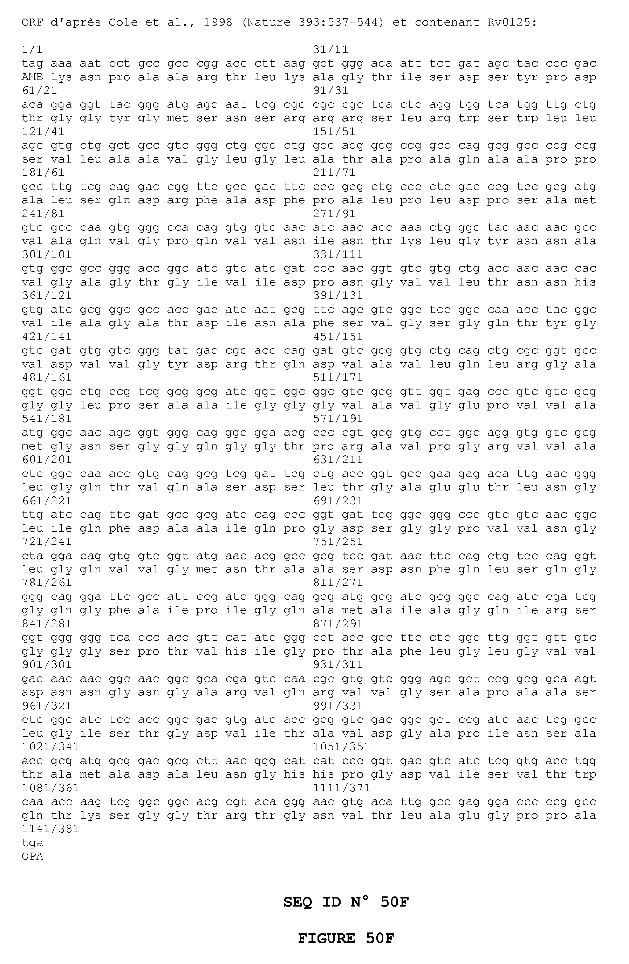

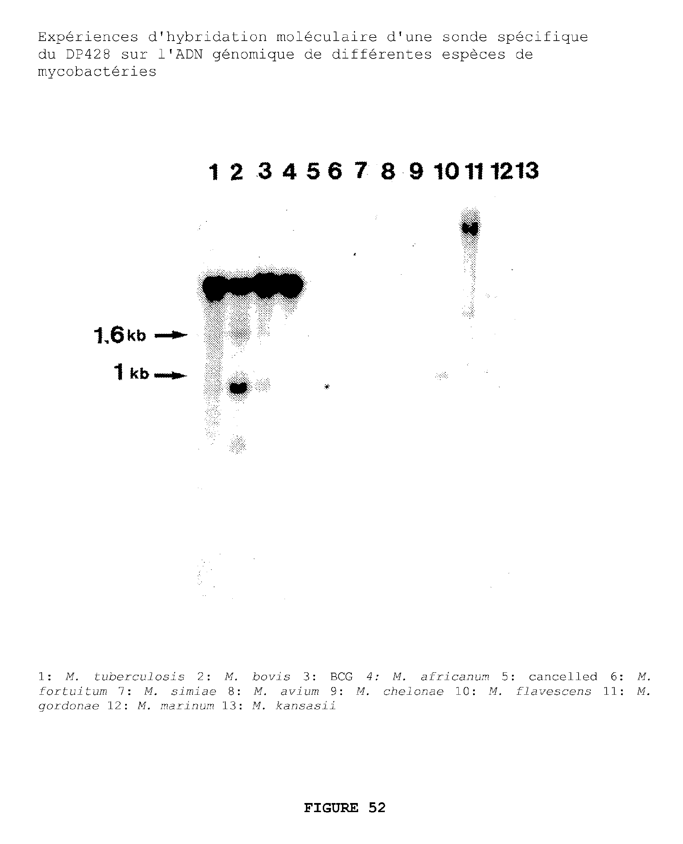

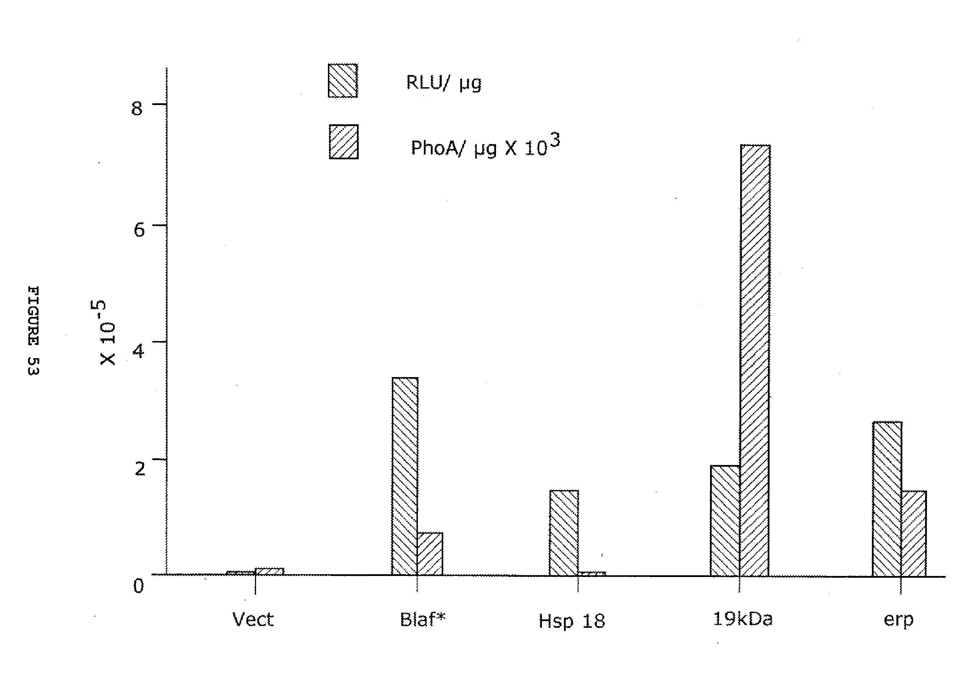

La série de Figures 1 :
   La série de Figures 1 illustre la série de séquences nucléotidiques SEQ ID N° 1 correspondant à l'insert du vecteur pDP428 (déposé à la CNCM sous le N° I-1818) et la série de séquences d'acides aminés SEQ ID N° 1 des polypeptides codés par la série des séquences nucléotidiques SEQ ID N° 1.
Figure 2 :
   Illustre la séquence nucléotidique SEQ ID N° 2 correspondant à la région incluant le gène codant pour le polypeptide DP428 (région soulignée). Sur cette figure ont été pris en compte à la fois les codons ATG et GTG d'initiation de la traduction. La figure fait apparaître que le polypeptide DP428 fait probablement partie d'un opéron comprenant au moins trois gènes. La région doublement encadrée inclut probablement les régions promotrices.
   La région simplement encadrée correspond au motif LPISG rapellant le motif LPXTG décrit chez les bactéries à Gram positifs comme permettant l'ancrage aux peptidoglycannes.
La série de Figures 3 :
   La série de Figures 3 représente la série de séquences nucléotidiques SEQ ID N° 3 correspondant à l'insert du vecteur p6D7 (déposé à la CNCM sous le N° I-1814).
La série de Figures 4 :
   La série de Figures 4 représente la série de séquences nucléotidiques SEQ ID N° 4 correspondant à l'insert du vecteur p5A3 (déposé à la CNCM sous le N° I-1815).
La série de Figures 5 :
   La série de Figures 5 représente la série de séquences nucléotidiques SEQ ID N° 5 correspondant à l'insert du vecteur p5F6 (déposé à la CNCM sous le N° I-1816).
La série de Figures 6 :
   La série de Figures 6 représente la série de séquences nucléotidiques SEQ ID N° 6 correspondant à l'insert du vecteur p2A29 (déposé à la CNCM sous le N° I-1817).
La série de Figures 7 :
   La série de Figures 7 représente la série de séquences nucléotidiques SEQ ID N° 7 correspondant à l'insert du vecteur p5B5 (déposé à la CNCM sous le N° I-1819).
La série de Figures 8 :
   La série de Figures 8 représente la série de séquences nucléotidiques SEQ ID N° 8 correspondant à l'insert du vecteur p1C7 (déposé à la CNCM sous le N° I-1820).
La série de Figures 9 :
   La série de Figures 9 représente la série de séquences nucléotidiques SEQ ID N° 9 correspondant à l'insert du vecteur p2D7 (déposé à la CNCM sous le N° I-1821).
La série de Figures 10 :
   La série de Figures 10 représente la série de séquences nucléotidiques SEQ ID N° 10 correspondant à l'insert du vecteur p1B7 (déposé à la CNCM sous le N° I-1843).
La série de Figures 11 :
   La série de Figures 11 représente la série de séquences nucléotidiques SEQ ID N° 11.
La série de Figures 12 :
   La série de Figures 12 représente la série de séquences nucléotidiques SEQ ID N° 12.
La série de Figures 13 :
   La série de Figures 13 représente la série de séquences nucléotidiques SEQ ID N° 13.
La série de Figures 14 :
   La série de Figures 14 représente la série de séquences nucléotidiques SEQ ID N° 14 correspondant à l'insert du vecteur p5B5 (déposé à la CNCM sous le N° I-1819).
La série de Figures 15 :
   La série de Figures 15 représente la série de séquences nucléotidiques SEQ ID N° 15.
La série de Figures 16 :
   La série de Figures 16 représente la série de séquences nucléotidiques SEQ ID N° 16.
La série de Figures 17 :
   La série de Figures 17 représente la série de séquences nucléotidiques SEQ ID N° 17.
La série de Figures 18 :
   La série de Figures 18 représente la série de séquences nucléotidiques SEQ ID N° 18.
La série de Figures 19 :
   La série de Figures 19 représente la série de séquences nucléotidiques SEQ ID N° 19.
La série de Figures 20 :
   La série de Figures 20 représente la série de séquences nucléotidiques SEQ ID N° 20 correspondant à l'insert du vecteur p2A29 (déposé à la CNCM sous le N° I-1817).
La série de Figures 21 :
   La série de Figures 21 représente la série de séquences nucléotidiques SEQ ID N° 21.
La série de Figures 22 :
   La série de Figures 22 représente la série de séquences nucléotidiques SEQ ID N° 22.
La série de Figures 23 :
   La série de Figures 23 représente la série de séquences nucléotidiques SEQ ID N° 23.
La série de Figures 24 :
   La série de Figures 24 représente la série de séquences nucléotidiques SEQ ID N° 24.
Figures 25 et 26 :
   Les figures 25 et 26 illustrent respectivement les séquences SEQ ID N° 25 et SEQ ID N° 26 représentant un couple d'amorces utilisées pour amplifier spécifiquement par PCR la région correspondant aux nucléotides 964 à 1234 inclus dans la séquence SEQ ID N° 1.
La série de Figures 27 :
   La série de Figures 27 représente la série de séquences nucléotidiques SEQ ID N° 27 correspondant à l'insert du vecteur p5A3.
Figure 28 :
   La séquence d'acides aminés telle que définie dans la figure 28 représente la séquence d'acides aminés SEQ ID N° 28 correspondant au polypeptide DP428.
Figure 29 :
   La figure 29 représente la séquence nucléotidique SEQ ID N° 29 du gène complet codant pour la protéine M1C25.
Figure 30 :
   La figure 30 représente la séquence d'acides aminés SEQ ID N° 30 de la protéine M1C25.
La série de Figures 31 :
   La série de Figures 31 représente la série de séquences nucléotidiques SEQ ID N° 31.
La série de Figures 32 :
   La série de Figures 32 représente la série de séquences nucléotidiques SEQ ID N° 32.
La série de Figures 33 :
   La série de Figures 33 représente la série de séquences nucléotidiques SEQ ID N° 33.
La série de Figures 34 :
   La série de Figures 32 représente la série de séquences nucléotidiques SEQ ID N° 34.
La série de Figures 35 :
   La série de Figures 35 représente la série de séquences nucléotidiques SEQ ID N° 35.
La série de Figures 36 :
   La série de Figures 36 représente la série de séquences nucléotidiques SEQ ID N° 36.
La série de Figures 37 :
   La série de Figures 37 représente la série de séquences nucléotidiques SEQ ID N° 37.
La série de Figures 38 :
   La série de Figures 38 représente la série de séquences nucléotidiques SEQ ID N° 38.
La série de Figures 39 :
   La série de Figures 39 représente la série de séquences nucléotidiques SEQ ID N° 39.
La série de Figures 40 :
   La série de Figures 40 représente la série de séquences nucléotidiques SEQ ID N° 40.
La série de Figures 41 :
   La série de Figures 41 représente la série de séquences nucléotidiques SEQ ID N° 41 correspondant à l'insert du vecteur p2D7 (déposé à la CNCM sous le N° I-1821).
La série de Figures 42 :
   La série de Figures 42 représente la série de séquences nucléotidiques SEQ ID N° 42.
La série de Figures 43 :
   La série de Figures 43 représente la série de séquences nucléotidiques SEQ ID N° 43.
La série de Figures 44 :
   La série de Figures 44 représente la série de séquences nucléotidiques SEQ ID N° 44.
La série de Figures 45 :
   La série de Figures 45 représente la série de séquences nucléotidiques SEQ ID N° 45.
La série de Figures 46 :
   La série de Figures 46 représente la série de séquences nucléotidiques SEQ ID N° 46.
La série de Figures 47 :
   La série de Figures 47 représente la série de séquences nucléotidiques SEQ ID N° 47.
La série de Figures 48 :
   La série de Figures 48 représente la série de séquences nucléotidiques SEQ ID N° 48.
La série de Figures 49 :
   La série de Figures 49 représente la série de séquences nucléotidiques SEQ ID N° 49.
La série de Figures 50 :
   La série de Figures 50 représente la série de séquences nucléotidiques SEQ ID N° 50.
Figure 51 :
   A. La construction Pjved : Plasmid navette (pouvant se multiplier chez les mycobactéries ainsi que chez E.*coli*) avec un gène de résistance à la kanamycine (issu de Tn903) comme marqueur de sélection. Le gène *phoA* tronqué (Δ *phoA)* et le gène *luc* forment un opéron synthetique.
   B. Séquence de la jonction entre *phoA* et *luc.*
Figure 52 :
   Hybridation génomique (Southern blot) de l'ADN génomique de différentes espèces mycobactériennes à l'aide d'une sonde oligonucléotidique dont la séquence est la séquence comprise entre le nucléotide en position nt 964 (extrémité 5' de la sonde) et le nucléotide en position nt 1234 (extrémité 3' de la sonde), extrémités inclues, de la séquence SEQ ID N° 1.
Figures 53 et 54 :
   Activités Luc et PhoA de *M. smegmatis* recombinant contenant le pJVED avec différents fragments nucléotidiques comme décrits en exemple. Les figures 52 et 53 représentent les résultats obtenus pour deux expériences distinctes réalisées dans les mêmes conditions.
Figure 55 :
   Représentation de l'hydrophobicité (Kyte et Doolitle) de la séquence codante du polypeptide DP428 avec sa représentation schématique. Le motif LPISG précède immédiatement la région C-terminale hydrophobe. La séquence se termine par deux arginines.
Figure 56 :
   Représentation de l'hydrophoicité (Kyte et Doolitle) de la séquence du polypeptide M1C25 de séquence d'acides aminés SEQ ID N° 30.
Figure 57 :
   A- Gel d'acrylamide (12 %) en condition dénaturante d'un extrait bactérien obtenu par sonication de bactéries *E. coli* M15 contenant le plasmide pM1C25 sans et après 4 heures d'induction par l'IPTG, coloré au bleu de Comassie.
      Ligne 1 : Marqueur de masse molaire (Prestained SDS-PAGE Standards High Range BIO-RAD@).
      Ligne 2 : Extrait bactérien obtenu par sonication de bactéries *E*. *coli* M15 contenant le plasmide pM1C25 sans induction par l'IPTG.
      Ligne 3 : Extrait bactérien obtenu par sonication de bactéries *E*. *coli* M15 contenant le plasmide pM1C25 après 4 heures d'induction par l'IPTG.
      Ligne 4 : Marqueur de masse molaire (Prestained SDS-PAGE Standards Low Range BIO-RAD@).
   B- Western blot d'un gel semblable gel (acrylamide 12 %) révélé grâce à l'anticorps penta-His commercialisé par la société Quiagen.
      Ligne 1 : Représentation du marqueur de masse molaire (Prestained SDS-PAGE Standards High Range BIO-RAD@).
      Ligne 2 : Extrait bactérien obtenu par sonication de bactéries *E. coli* M15 contenant le plasmide pM1C25 sans induction par l'IPTG.
      Ligne 3 : Extrait bactérien obtenu par sonication de bactéries *E. coli* M15 contenant le plasmide pM1C25 après 4 heures d'induction par l'IPTG.
      Ligne 4 : Représentation du marqueur de masse molaire (Prestained SDS-PAGE Standards Low Range BIO-RAD@).

La bande présente très majoritairement dans les lignes correspondant aux bactéries induites par l'IPTG par rapport à celles non induites par l'IPTG, comprise entre 34200 et 28400 daltons, correspond à l'expression de l'insert M1C25 cloné dans le vecteur pQE-60 (Qiagen@).

En ce qui concerne les légendes des autres figures qui sont numérotées par un caractère alphanumérique, chacune de ces autres figures représente la séquence nucléotidique et la séquence d'acides aminés de séquence SEQ ID dont la numérotation est identique au caractère alphanumérique de chacune desdites figures.

Les numérotations alphanumériques des figures représentant les SEQ ID comportant un nombre suivi d'une lettre ont les significations suivantes :
- les numérotations alphanumériques présentant le même nombre concernent une même famille de séquences rattachées à la séquence de référence SEQ ID dont la numérotation présente ce même nombre et la lettre A ;
- les lettres A, B et C pour une même famille de séquences distinguent les trois phases de lecture possibles de la séquence nucléotidique SEQ ID de référence (A) ;
- les lettres indexées par un prime (') signifient que la séquence correspond à un fragment de la séquence SEQ ID de référence (A) ;
- la lettre D signifie que la séquence correspond à la séquence du gène prédit par Cole et al., 1998 ;
- la lettre F signifie que la séquence correspond à la phase ouverte de lecture (ORF pour « Open Reading Frame ») contenant la séquence « D » correspondante d'après Cole et al., 1998 ;
- la lettre G signifie que la séquence est une séquence prédite par Cole et al., 1998, et présentant une homologie de plus de 70 % avec la séquence SEQ ID de référence (A) ;
- la lettre H signifie que la séquence correspond à la phase ouverte de lecture contenant la séquence « G » correspondante d'après Cole et al., 1998 ;
- la lettre R signifie que la séquence correspond à une séquence prédite par Cole et al., 1998, en amont de la séquence « D » correspondante et pouvant être en phase avec la séquence « D » en raison d'erreurs de séquençage possibles ;
- la lettre P signifie que la séquence correspond à la phase ouverte de lecture contenant la séquence « R » correspondante ;
- la lettre Q signifie que la séquence correspond à une séquence contenant les séquences « F » et « P » correspondantes.

En ce qui concerne la famille de séquences SEQ ID N° 4, l'insert précédent *phoA* contient deux fragments non contigus sur le génome, SEQ ID 4J et SEQ ID 4A, et donc issus d'un clonage multiple permettant l'expression et l'exportation de *phoA.* Ces deux fragments non contigus, les gènes et les phases ouvertes de lecture qui les contiennent d'après Cole et al., 1998, sont importants pour l'exportation d'un polypeptide antigène :
- les lettres J, K et L distinguent les trois phases de lecture possibles de la séquence nucléotidique « J » correspondante ;
- la lettre M signifie que la séquence correspond à la séquence prédite par Cole et al., 1998, et contenant la séquence SEQ ID N° 4J ;
- la lettre N signifie que la séquence correspond à la phase ouverte de lecture contenant la séquence SEQ ID N° 4M.

En ce qui concerne la famille de séquences SEQ ID N° 45, la lettre Z signifie que la séquence correspond à la séquence d'un fragment cloné fusionné avec *phoA.*

Enfin, en ce qui concerne la famille de séquence SEQ ID N° 41, la lettre S signifie que la séquence correspond à une séquence prédite par Cole et al., 1998 et pouvant être dans la même phase de lecture que la séquence « D » correspondante, la lettre T signifiant que la séquence correspondante contient les séquences « F » et « S » correspondantes.

### EXEMPLES

### Matériel et méthodes

### Cultures bactériennes, plasmides et milieux de cultures

*E. coli* a été cultivé sur milieu liquide ou solide Luria-Bertani (LB). *M. smegmatis* a été cultivé sur milieu liquide Middlebrook 7H9 (Difco) additionné de dextrose albumine (ADC), 0,2 % de glycérol et 0,05 % de Tween, ou sur milieu solide L. Si nécessaire, l'antibiotique kanamycine a été rajouté à une concentration de 20 µg/ml- 1. Les clones bactériens présentant une activité PhoA ont été détectés sur de l'agar LB contenant du 5-bromo-4-chloro-3-indolyle phosphate (X-P, à 40 µg/ml⁻¹).

### Manipulation d'ADN et séquençage

Les manipulations d'ADN et les analyses par Southern blot ont été effectuées en utilisant les techniques standard (Sambrook et al., 1989). Les séquences d'ADN double brun ont été déterminées avec un kit de séquençage Taq Dye Deoxy Terminator Cycle (Applied Biosystems), dans un Système 9600 GeneAmp PCR (Perkin-Elmer), et après migration sur un système d'analyse ADN modèle 373 (Applied Biosystems).

### Constructions des plasmides

Le plasmide pJVEDₐ a été construit à partir de pLA71, plasmide de transfert comportant le gène *phoA* tronqué et placé en phase avec *BlaF.* pLA71 a été coupé avec les enzymes de restriction *Kpn*I et *Not*I*,* retirant ainsi *phoA* sans toucher le promoteur de *BlaF.* Le gène *luc* codant pour la luciférase de luciole a été amplifié à partir de pGEM-*luc* et un site de liaison du ribosome a été rajouté. *phoA* a été amplifié à partir de pJEM11. Les fragments amplifiés ont été coupés avec *Pst*I et ligaturés ensemble. Les oligodéoxynucléotides utilisés sont les suivants :
pPV.luc.Fw : 5'GACTGCTGCAGAAGGAGAAGATCCAAATGG3'
luc.Bw : 5'GACTAGCGGCCGCGAATTCGTCGACCTCCGAGG3'
pJEM.phoA.Fw : 5'CCGCGGATCCGGATACGTAC3'
phoA.Bw : 5'GACTGCTGCAGTTTATTTCAGCCCCAGAGCG3'.

Le fragment ainsi obtenu a été réamplifié en utilisant les oligonucléotides complémentaires de ses extrémités, coupé avec *Kpn*I et *Not*I, et intégré dans pLA71 coupé avec les mêmes enzymes. La construction résultante a été électroporée dans *E*. *coli* DH5α et *M. smegmatis* mc² 155. Un clone *M. smegmatis* émettant de la lumière et présentant une activité *phoA* a été sélectionné et appelé pJVED/*blaF.* L'insert a été retiré en utilisant *Bam*HI et la construction refermée sur elle-même, reconstruisant ainsi le pJVEDₐ. Afin d'obtenir le pJVED_{b,c}, le multisite de clonage a été coupé avec *Sca*I et *KpnI* et refermé en enlevant un (pJVED_{b}) ou deux (pJVED_{c}) nucléotides du site *Sna*BI*.* Après fusion six cadres de lecture ont pu ainsi être obtenus. L'insert du pJVED/*hsp18* a été obtenu par amplification en chaîne par polymérase (ACP) de pPM1745 (Servant et al., 1995) en utilisant des oligonucléotides de la séquence :
18.Fw : 5'GTACCAGTACTGATCACCCGTCTCCCGCAC3'
18.Back : AGTCAGGTACCTCGCGGAAGGGGTCAGTGCG3'.

Le produit a été coupé avec *Kpn*I et *ScaI,* et ligaturé à pJVEDₐ, coupé avec les mêmes enzymes, quittant ainsi le pJVED/*hsp18.*

Le pJVED/*P19kDa* et le pJVED/*erp* furent construits en coupant avec *Bam*HI l'insert de pExp410 et pExp53 respectivement, et en les insérant dans le site *Bam*HI du multisite de clonage de pJVEDₐ.

### Mesure de l'activité phosphatase alkaline

La présence d'activité est détectée par la couleur bleue des colonies croissant sur un milieu de culture contenant le substrat 5-bromo 4-chloro 3-indolyl phosphate (XP), puis l'activité peut être mesurée quantitativement de manière plus préçise de la façon suivante :

*M. smegmatis* ont été cultivés dans un milieu LB additionnés de 0,05 % de Tween 80 (Aldrich) et de kanamycine (20 µg/ml⁻¹) à 37°C pendant 24 heures. L'activité de la phosphatase alkaline a été mesurée par la méthode de Brockman et Heppel (Brockman et al., 1968) dans un extrait soniqué, avec p-nitrophénylphosphate comme substrat de la réaction. La quantité de protéines a été mesurée par essai Bio-Rad. L'activité phosphatase alkaline est exprimée en unité arbitraire (densité optique à 420 nm x µg de protéines- ¹ x minutes- 1).

### Mesure de l'activité luciférase

*M. smegmatis* a été cultivé dans un milieu LB additionné de 0,05 % de Tween 80 (Aldrich) et de kanamycine (20 µg/ml- ¹) à 37°C pendant 24 heures et utilisé en pleine croissance exponentielle (DO à 600 nm comprise entre 0,3 et 0,8). Les aliquots de suspensions bactériennes ont été brièvement soniqués et l'extrait cellulaire a été utilisé pour mesurer l'activité de la luciférase. 25 µl de l'extrait soniqué ont été mélangés avec 100 µl de substrat (système d'essai luciférase Promega) automatiquement dans un luminomètre et la lumière émise exprimée en ULR ou RLU (Unités Lumineuses Relatives). Les bactéries ont été comptées par dilutions sérielles de la suspension d'origine sur milieu agar LB kanamycine et l'activité de la luciférase exprimée en ULR/µg de protéines bactériennes ou en ULR/10³ bactéries.

### Construction de banques génomiques de M. tuberculosis et de M. bovis-BCG

Les banques ont été obtenues en utilisant essentiellement pJVED_{a,b,c} précédemment décrits.

### Préparation de macrophages issus de la moelle osseuse et infection par M. smegmatis recombinants

Les macrophages issus de la moelle osseuse ont été préparés comme décrits par Lang et al., 1991. En résumé, les cellules de la moelle osseuse ont été prélevés du fémur de souris C57BL/6 agée de 6 à 12 semaines (Iffa-Credo, France). Les cellules en suspensions ont été lavées et resuspendues dans du DMEM enrichi avec 10 % de sérum foetal de veau, 10 % de milieu L-cell conditionné et 2 mM de glutamine, sans antibiotiques. 106 cellules ont été ensemencées sur des plaques 24 puits Costar à fond plat dans 1 ml. Après quatre jours à 37°C dans une atmosphère humide à 10 % de teneur en CO2, les macrophages ont été rincés et réincubés pendant deux à quatre jours supplémentaires. Les cellules d'un puits contrôle ont été lysées avec du triton x 100 à 0,1 % dans l'eau et les noyaux énumérés. Environ 5 x 105 cellules adhérentes ont été comptées. Pour l'infection, *M. smegmatis* portant les différents plasmides a été cultivé en pleine phase exponentielle (DO₆₀₀ₙₘ entre 0,4 et 0,8) et dilué jusqu'à une DO de 0,1 puis 10 fois dans un milieu pour macrophage. 1 ml a été ajouté à chaque puits et les plaques ont été centrifugées et incubées quatre heures à 37°C. Après trois lavages, les cellules ont été incubées dans un milieu contenant de l'amykacine pendant deux heures. Après trois nouveaux lavages, les cellules infectées adhérentes ont été incubées dans un milieu macrophage pendant une nuit. Les cellules ont ensuite été lysées dans 0,5 ml de tampon de lyse (Promega). 100 µl ont été soniqués et la lumière émise a été mesurée sur 25 µm. Simultanément, les bactéries ont été énumérées par étalement sur L-agar-kanamycine (20 µg/ml⁻¹). La lumière émise est exprimée en ULR/10³ bactéries.

### Analyses des banques de données

Les séquences nucléotidiques ont été comparées à EMBL et GenBank en utilisant l'algorithme FASTA et les séquences protéiques ont été analysées par similitude grâce aux banques de données PIR et Swiss Prot en utilisant l'algorithme BLAST.

### Exemple 1 : Les vecteurs pJVED

Les vecteurs pJVED (Figure 51) sont des plasmides portant un gène *phoA* tronqué de *E*. *coli* dépourvu de codon d'initiation, de séquence signal et de séquence régulatrice. Le site multiple de clonage (SMC) permet l'insertion de fragments des gènes codants pour d'éventuelles protéines exportées ainsi que leurs séquences de régulation. Dès lors, la protéine de fusion peut être produite et présenter une activité phosphatase alcaline si elle est exportée. Seules les fusions en phase pourront être productives. Ainsi, le SMC a été modifié de sorte que les fusions peuvent être obtenues dans six phases de lecture. En aval de *phoA,* le gène *luc* de la luciférase de luciole a été inséré. Le gène complet avec le codon d'initiation mais sans qu'aucun promoteur n'ait été utilisé devrait ainsi s'exprimer avec *phoA* comme dans un opéron synthétique. Un nouveau site de liaison des ribosomes a été inséré huit nucléotides en amont du codon d'initiation de *luc.* Deux terminateurs transcriptionnels sont présents dans les vecteurs pJVED, un en amont du SMC et un second en aval de *luc.* Ces vecteurs sont des plasmides de transfert *E. coli*-mycobacterium avec un gène de résistance à la kanamycine comme marqueur de sélection.

*phoA et luc fonctionnent comme dans un opéron, mais l'exportation est nécessaire pour l'activité phoA.*

Quatre plasmides ont été construits par insertion dans le SMC de fragments d'ADN d'origine diverse :
Dans la première construction nommée pJVED/*blaF*, le fragment de 1,4 kb provient du plasmide déjà décrit pLA71 (Lim et al., 1995). Ce fragment issu du gène β-lactamase (*blaF*) de *M. fortuitum* D216 (Timm et al., 1994) inclut le promoteur muté hyperactif, le segment codant pour 32 acides aminés de la séquence signal et les 5 premiers acides aminés de la protéine mature. Ainsi cette construction inclut le promoteur le plus fort connu chez mycobacterium et les éléments nécessaires à l'exportation de la protéine de la fusion *phoA.* Par conséquent, on peut attendre de cette construction une forte émission de lumière et une bonne activité *phoA* (cf. figures 53 et 54).
Dans une deuxième construction nommée pJVED/*hsp18*, un fragment de 1,5 kb a été cloné à partir du plasmide déjà décrit pPM1745 (Servant et al., 1995). Ce fragment inclut les nucléotides codants pour les dix premiers acides aminés de la protéine de choc thermique de 18 kb issue de *Streptomyces albus* (heat shock protein 18, HSP 18), le site de liaison du ribosome, le promoteur et, en amont, des sites régulateurs contrôlant son expression. Cette protéine appartient à la famille de alpha-crystalline de HSP à faible poids moléculaire (Verbon et al., 1992). Son homologue issu de *M. leprae,* l'antigène de 18 kDa, est déjà connu pour être induit durant la phagocytose par un macrophage murin de la lignée cellulaire J-774 (Dellagostinet al., 1995). Dans des conditions de culture standard, le pJVED/*hsp18*, montre une faible activité *luc* et aucune activité *phoA* (cf. figures 53 et 54).
Dans une troisième construction, nommée pJVED/*P19kDa,* l'insert issu de pExp410 (Lim et al., 1995) a été coupé et cloné dans le SMC de pJVEDₐ. Ce fragment inclut les nucléotides codants pour les 134 premiers acides aminés de la protéine connue de *M. tuberculosis* 19 kDa et de ses séquences régulatrices. Comme cela a pu être mis en évidence, cette protéine est une lipoprotéine glycosylée (Garbe et al., 1993 ; Herrmann et al., 1996). Sur les figures 53 et 54, on observe, pour cette construction, une bonne activité *luc* correspondant à un promoteur fort, mais l'activité *phoA* est la plus forte des quatre constructions. L'activité *phoA* élevée de cette protéine de fusion avec une lipoprotéine s'explique par le fait qu'elle reste attachée à la paroi cellulaire par son extrémité N-terminal.
Dans la quatrième et dernière construction nommée pJVED/*erp* l'insert provient de pExp53 (Lim et al., 1995) et a été cloné dans le SMC de pJVEDₐ. pExp53 est le plasmide initial sélectionné pour son activité *phoA* et contenant une partie du gène *erp* de *M. tuberculosis* qui code pour un antigène de 28 kDa. Ce dernier inclut la séquence signal, une partie de la protéine mature et, en amont du codon d'initiation, le site de liaison de ribosome. Le promoteur a été cartographié. Une boîte fer (iron box) putative du type *fur* est présente dans cette région et encadre la région -35 du promoteur (Berthet et al., 1995). Comme prévu (figures 53 et 54) cette construction présente une bonne émission lumineuse et une bonne activité *phoA.* Le fait que cette protéine de fusion, contrairement à la fusion avec la lipoprotéine de 19 kDa, ne semble pas attachée à la paroi cellulaire n'exclut pas que la protéine native y soit associée. De plus, l'extrémité C-terminal de *erp* est absente de la protéine de fusion.

### Exemple 2 : Construction d'une banque d'ADN génomique de M. tuberculosis dans les vecteurs pJVEDₛ et identification d'un des membres de ces banques, (DP428), induit au cours de la phagocytose par les macrophages murins dérivés de la moelle osseuse.

Les différentes constructions sont testées pour leur capacité à évaluer l'expression intracellulaire des gènes identifiés par l'expression de *phoA.* Dans cet objectif, l'activité *luc* est exprimée en URL pour 10³ bactéries en culture axénique et/ou dans des conditions intracellulaires. L'induction ou la répression suivant la phagocytose par les macrophages murins dérivés de la moelle osseuse peut être évaluée convenablement par la mesure des activités spécifiques. Les résultats de deux expériences distinctes sont présentés dans le tableau 2.

Le plasmide pJVED/*hsp18* a été utilisé comme contrôle positif pour l'induction durant la phase de croissance intracellulaire. Bien que l'induction du promoteur par le chauffage de la bactérie à 42°C n'ait pas été concluant la phagocytose de la bactérie conduit clairement à une augmentation de l'activité du promoteur. Dans toutes les expériences, l'activité *luc* intracellulaire a été fortement induite, augmentant de 20 à 100 fois l'activité basale initialement faible (Servant, 1995).

Le plasmide pJVED/*blaF* a été utilisé comme contrôle de la modulation non spécifique au cours de la phagocytose. De faibles variations ont pu être mises en évidence, probablement dues à des changements de conditions de cultures. Quoi qu'il en soit, ces faibles variations ne sont pas comparables à l'induction observée avec le plasmide pJVED/*hsp18.*

Tous les membres de la banque d'ADN ont été testés par mesure de l'activité du promoteur durant la croissance intracellulaire. Parmi eux, le DP428 est fortement induit au cours de la phagocytose (tableaux 1 et 2).

**TABLEAU 1**

| Construction | % Récupération | | URL/10³ bactéries extracellulaire | URL/10³ bactéries intracellulaire | | Induction | |
|---|---|---|---|---|---|---|---|
| pJVED/*blaF** | 0,5 | | 1460 | 1727 | | 1,2 | |
| pJVED/*hsp18* | 0,6 | | 8 | 57 | | 7,1 | |
| pJVED/*DP428* | 0,7 | | 0,06 | 18 | | 300 | |
| | | | | | | | |

| Construction | % Récupération | | URL/10³ bactéries extracellulaire | URL/10³ bactéries intracellulaire | | Induction | |
|---|---|---|---|---|---|---|---|
| | C57BL/6 | Balb/C | | C57BL/6 | Balb/C | C57BL/6 | Balb/C |
| pJVED/*blaF** | 7 | 1,1 | 662 | 250 | 911 | 0,4 | 1,4 |
| pJVED/*hsp18* | 6,7 | 1,7 | 164 | 261 | 325 | 1,6 | 2 |
| pJVED/*DP428* | 1,6 | 2,1 | 0,08 | 1,25 | 3,3 | 15,6 | 41 |

**TABLEAU 2**

| Construction | % Récupération | URL/10³ bactéries extracellulaire | URL/10³ bactéries intracellulaire | Induction |
|---|---|---|---|---|
| pJVED/*blaF** | 22 | 1477 | 367 | 0,25 |
| pJVED/*hsp18* | 7 | 0,26 | 6,8 | 26 |
| pJVED/*DP428* | 21 | 0,14 | 4 | 28 |

Le fragment nucléotidique codant pour la région N-terminale du polypeptide DP428 de séquence SEQ ID N° 28 est contenu dans le plasmide déposé à la CNCM sous le N° I-1818.

La totalité de la séquence codant pour le polypeptide DP428 a été obtenue comme détaillée ci-après.

Une sonde a été obtenue par PCR à l'aide des oligonucléotides de séquence SEQ ID N° 25 et SEQ ID N° 26. Cette sonde a été marquée par extension aléatoire en présence de ³²P dCTP. Une hybridation de l'ADN génomique de *M. tuberculosis* souche Mt103 préalablement digéré par l'endonucléase Sca1 a été réalisée à l'aide de ladite sonde. Les résultats de l'hybridation ont fait apparaître qu'un fragment d'ADN d'environ 1,7 kb était marqué. Du fait qu'il existe un site Sca1 s'étendant du nucléotide nt 984 au nucléotide nt 989 de la séquence SEQ ID N° 1, c'est-à-dire du côté 5' de la séquence utilisée comme sonde, la fin de la séquence codante est nécessairement présente dans le fragment détecté par hybridation.

L'ADN génomique de la souche Mt 103 de *M. tuberculosis,* après digestion par Sca1, a subi une migration sur un gel d'agarose. Les fragments de tailles comprises entre 1,6 et 1,8 kb ont été clonés dans le vecteur pSL1180 (Pharmacia) préalablement clivé par Sca1 et déphosphorylé. Après transformation de *E. coli* avec les vecteurs recombinants résultants, les colonies obtenues ont été criblées à l'aide de la sonde. Le criblage a permis d'isoler six colonies hybridant avec cette sonde.

Les inserts contenus dans les plasmides des clones recombinants précédemment sélectionnés ont été séquencés, puis les séquences alignées de manière à déterminer la totalité de la séquence codant pour DP428, plus spécifiquement la SEQ ID N° 2.

Un couple d'amorces a été synthétisé afin d'amplifier, à partir de l'ADN génomique de *M. tuberculosis,* souche Mt 103, la totalité de la séquence codant pour le polypeptide DP428. L'amplicon obtenu a été cloné dans un vecteur d'expression.

Des couples d'amorces appropriés pour l'amplification et le clonage de la séquence codant pour le polypeptide DP428 peuvent être aisément réalisés par l'homme du métier, sur la base des séquences nucléotidiques SEQ ID N° 1 et SEQ ID N° 2.

Un couple d'amorces particulier selon l'invention est le couple d'amorces suivant, capable d'amplifier l'ADN codant pour le polypeptide DP428 dépourvu de sa séquence signal :
- Amorce aller (SEQ ID N° 29), comprenant la séquence allant du nucléotide en position nt 1021 au nucléotide nt 1044 de la séquence SEQ ID N° 2 :
   5' -AGTGCAT**GCTGCTGGCCGAACCATCAGCGAC**- 3'
- Amorce retour (SEQ ID N° 30), comprenant la séquence complémentaire de la séquence allant du nucléotide en position nt 1345 au nucléotide en position nt 1325 de la séquence SEQ ID N° 2 :
   5'-CAGCCAGATCT**GCGGGCGCCCTGCACCGCCTG**- 3',
dans lesquelles la partie soulignée représente les séquences hybridant spécifiquement avec la séquence SEQ ID N° 2 et les extrémités 5' correspondent à des sites de restriction en vue du clonage de l'amplicon résultant dans un vecteur de clonage et/ou d'expression.

Un vecteur particulier utilisé pour l'expression du polypeptide DP428 est le vecteur pQE70 commercialisé par la société Qiagen.

### Exemple 3 : La séquence complète du gène DP428 et de ses régions flanquantes

Une sonde de la région codante de DP428 a été obtenue par ACP, et utilisée pour hybrider l'ADN génomique de différentes espèces de mycobactéries. D'après les résultats de la figure 3, le gène est présent uniquement dans les mycobactéries du complexe de *M. tuberculosis.*

L'analyse de la séquence suggère que DP428 pourrait faire partie d'un opéron. La séquence codante et les régions flanquantes ne présentent aucune homologie avec des séquences connues déposées dans les banques de données.

D'après la séquence codante, ce gène code pour une protéine de 10 kDa avec un peptide signal, une extrémité C-terminal hydrophobe terminée par deux arginines et précédée par un motif LPISG semblable au motif connu LPXTG. Ces deux arginines pourraient correspondre à un signal de rétention et la protéine DP428 pourrait être accrochée par ce motif à des peptidoglycanes comme cela a déjà été décrit chez d'autres bactéries Gram⁺ (Navarre et al., 1994 et 1996).

Le mécanisme de survie et de croissance intracellulaire des mycobactéries est complexe et les relations intimes entre la bactérie et la cellule hôte restent inexpliquées. Quel que soit le mécanisme, la croissance et la survie intracellulaire des mycrobactéries dépend de facteurs produits par la bactérie et capables de moduler la réponse de l'hôte. Ces facteurs peuvent être des molécules exposées à la surface cellulaire telle que LAM ou des protéines associées à la surface cellulaire, ou des molécules activement sécrétées.

D'un autre côté, intracellulairement, les bactéries elles-mêmes doivent faire face à un environnement hostile. Elles semblent y répondre par des moyens proches de ceux mis en oeuvre dans les conditions de stress, par l'induction de protéines de choc thermique (Dellagostin et al., 1995), mais aussi par induction ou la répression de différentes protéines (Lee et al., 1995). En utilisant une méthodologie dérivée de la PCR, Plum et Clark-curtiss (Plum et al., 1994) ont montré qu'un gène de *M. avium* inclus dans un fragment d'ADN de 3 kb, est induit après la phagocytose par des macrophages humains. Ce gène code pour une protéine exportée comprenant une séquence leader mais ne présentant pas d'homologie significative avec les séquences proposées par les banques de données. L'induction, pendant la phase de croissance intracellulaire, d'une protéine de choc thermique de faible poids moléculaire issue de *M*. *leprae* a également été mise en évidence (Dellagostin et al., 1995). Dans une autre étude, les protéines bactériennes de *M. tuberculosis* ont été métaboliquement marquées pendant la phase de croissance intracellulaire ou bien dans des conditions de stress et séparées par électrophorèse sur gel à deux dimensions : 16 protéines de *M. tuberculosis* ont été induites et 28 reprimées. Les mêmes protéines sont mises en jeu au cours de stress provoqué par un faible pH, un choc thermique, H₂O₂, ou au cours de la phagocytose par des monocytes humains de la lignée THP1. Quoi qu'il en soit, le comportement des protéines induites et réprimées était unique dans chaque condition (Lee et al., 1995). Pris ensemble, ces résultats indiquent qu'un dialogue moléculaire subtile est mis en place entre les bactéries et leurs hôtes cellulaires. De ce dialogue dépend probablement le sort de l'organisme intracellulaire.

Dans ce contexte, l'induction de l'expression de DP428 pourrait être d'une importance majeure, indiquant un rôle important de cette protéine dans la survie et la croissance intracellulaire.

La méthode utilisée dans ces expériences pour évaluer l'expression intracellulaire des gènes (cf. Jacobs et al., 1993, pour la méthode de détermination de l'expression de la luciférase de luciole, et Lim et al., 1995, pour la méthode de détermination de l'expression du gène *PhoA)* présente l'avantage d'être simple comparée aux autres techniques comme la technique décrite par Mahan et al. (Mahan et al., 1993) adaptée aux mycobactéries et proposée par Bange et al. (Bange et al., 1996), ou la méthode substractive basée sur l'ACP décrite par Plum et Clark-curtiss (Plum et al., 1994). Il existe indiscutablement une variabilité comme le montre la comparaison des différentes expériences. Bien que provoquer l'induction ou la répression soit suffisant, il est désormais possible de l'évaluer fournissant ainsi un outil supplémentaire d'études physiologiques des protéines exportées identifiées par fusion avec *phoA.*

### Exemple 4 :

### Recherche d'une modulation de l'activité des promoteurs lors des phases intramacrophagiques.

Des macrophages de moelle osseuse de souris sont préparés comme décrit par Lang et Antoine (Lang et al., 1991). Les bactéries de *M. segmentis* recombinantes, dont on a déterminé l'activité luciférase par 10³ bactéries comme précédemment, sont incubées à 37°C sous atmosphère humidifiée et enrichie en CO₂ à 5 %, pendant 4 heures en présence de ces macrophages de telle manière qu'elles soient phagocytées. Après rinçage pour éliminer les bactéries extracellulaires restantes, on ajoute au milieu de culture de l'amikacine (100 µg/ml) pendant deux heures. Après un nouveau rinçage, le milieu est remplacé par un milieu de culture (DMEM enrichi de 10 % de sérum de veau et 2 mM de glutamine) sans antibiotiques. Après une nuit d'incubation comme précédemment, les macrophages sont lysés à froid (4°C) à l'aide d'un tampon de lyse (cee lysis buffer, Promega), et l'activité luciférase par 10³ bactéries déterminée. Le rapport des activités à la mise en culture et après une nuit donne le coefficient d'induction.

### Exemple 5 :

### Isolement d'une série de séquences par séquençage directement à partir des colonies.

Une série de séquences permettant l'expression et l'exportation de *phoA* ont été isolées à partir de l'ADN de *M. Tuberculosis* ou de *M. Bovis* BCG. Parmi ce groupe de séquences, deux d'entre elles ont été davantage étudiées, les gènes entiers correspondant aux inserts ont été clonés, séquencés, et des anticorps contre le produit de ces gènes ont servi à montrer en microscopie électronique que ces gènes codaient pour des antigènes retrouvés à la surface des bacilles de la tuberculose. L'un de ces gènes *erp* codant pour une séquence signal d'exportation consensus, l'autre *des* ne possédait aucune caractéristique de gène codant pour une protéine exportée, d'après la séquence. Un autre gène DP428 a été séquencé avant que la séquence du génome de *M. Tuberculosis* ne soit disponible. Il contient une séquence ressemblant à la séquence consensus d'attachement au peptidoglycane, ce qui suggère qu'il s'agit aussi d'un antigène vraisemblablement retrouvé à la suface des bacilles de la tuberculose. L'étude des trois gènes *erp, des,* et celui codant pour DP428 montre que le système *phoA* que nous avons développé chez les mycobactéries permet de repérer des gènes codant pour des protéines exportées sans déterminant repérable par des études *in silico.* Ceci est particulièrement vrai pour les polypeptides qui ne possèdent pas de séquence signal consensus *(des)* ou non pas de similarité avec des protéines de fonction connue *(erp* et *DP428).*

Un certain nombre d'inserts ont été identifiés et séquencés avant la connaissance du génome de *M. Tuberculosis,* d'autres après. Ces séquences peuvent être considérées comme des amorces permettant de rechercher des gènes codant pour des protéines exportées. A ce jour, une série d'amorces ont été séquencées et les gènes entiers correspondants ont été soit séquencés, soit identifiés d'après la séquence publiée du génome. Pour tenir compte des erreurs de séquençage toujours possibles, les régions en amont ou en aval de certaines amorces ont été considérées comme pouvant faire partie de séquences codant pour des protéines exportées. Dans certains cas des similarités avec des gènes codant pour des protéines exportées ou des séquences caractéristiques de signaux d'exportation ou des caractéristiques topologiques de protéines membranaires ont été détectées.

Des séquences amorces s'avèrent correspondre à des gènes appartenant à des familles de gènes possédant plus de 50 % de similarité. On peut ainsi indiquer que les autres gènes détectés par similarité avec une amorce codent pour des protéines exportées. C'est le cas de la séquence SEQ ID N° 8G et SEQ ID N° 8H possédant plus de 77 % de similarité avec SEQ ID N° 8A'.

Les séquences pouvant coder pour des protéines exportées sont les suivantes : SEQ ID N° 1, 8, 9, 8G, 8H, 13, 3, 10, 19, 20, 6, 16, 22, 23, 24, 39, 44, 46, et 50.

Des gènes identifiés d'après les amorces à partir de la séquence du génome n'ont aucune caractéristique (d'après la séquence) de protéines exportées. Il s'agit des séquences suivantes : SEQ ID N° 4, 27, 11, 12, 14, 7, 15, 17, 18, 21, 31, 32, 33, 34, 35, 36, 37, 38, 40, 41, 42, 43, 45, 47, 48, et 49.

D'après la séquence d'autres organismes comme *E. coli,* on peut rechercher dans la séquence du génome de *M. tuberculosis,* des gènes possédant des similarités avec des protéines connues pour être exportées chez d'autres organismes bien que ne possédant pas de séquence signal d'exportation. Dans ce cas une fusion avec *phoA* est un protocole avantageux pour déterminer si ces séquences de *M. tuberculosis* codent pour des protéines exportées bien que ne présentant pas de séquence signal consensus. Il a été en effet possible de cloner SEQ ID N° 49, une séquence similaire à un gène de *E. coli* de la famille *htrA.* Une fusion de SEQ ID N° 49 avec *phoA* conduit à l'expression et à l'exportation de *phoA.* Des colonies *M. smegmatis* hébergeant une fusion SEQ ID N° 49 *phoA* sur un plasmide pJVED sont bleues.

SEQ ID N° 49 est donc considérée comme une protéine exportée.

La méthode *phoA* est donc utile pour détecter d'après la séquence de *M*. *Tuberculosis* des gènes codant pour des protéines exportées sans qu'ils ne codent pour des séquences caractéristiques des protéines exportées.

Même si une séquence possède des déterminants de protéines exportées, cela ne démontre pas une exportation fonctionnelle. Le système *phoA* permet de montrer que le gène suspecté code réellement pour une protéine exportée.

Ainsi, il a été vérifié que la séquence SEQ ID N° 50 possédait bien des signaux d'exportation.

**TABLEAU 3**

| SEQ ID N° | Référence de la séquence correspondante prédite par Cole et al. | | Annotation |
|---|---|---|---|
| SEQ ID N° 1 | Rv 0203 | * | Séquence hydrophobe en N-terminal |
| SEQ ID N° 4 SEQ ID N° 27 | Rv 2050 | | Pas de prédiction |
| SEQ ID N° 8 SEQ ID N° 9 | Rv 2563 | * | Protéine membranaire |
| SEQ ID N° 8G', H' | Rv 0072 | * | Possible protéine de transport transmembranaire de type ABC |
| SEQ ID N° 11 | Rv 0546c | ML | ProteinS-D Lactoyl Glutathione-méthyl glyoxal lyase |
| SEQ ID N° 12 | pas de prédiction | | non retrouvé dans *M.tuberculosis* H37rv |
| SEQ ID N° 13 | | | probable précurseur cutinase avec une séquence signal N-terminale |
| SEQ ID N° 3 | Rv 1984c | * | |
| SEQ ID N° 10 | | | |
| SEQ ID N° 14 SEQ ID N° 7 | pas de prédiction | | pas de prédiction |
| SEQ ID N° 15 | avec décalage de lecture, pourrait être en phase avec Rv 2530c | | pas de prédiction |
| SEQ ID N° 17 | Rv 1303 | ML | pas de prédiction |
| SEQ ID N° 18 | Rv 0199 | ML | pas de prédiction |
| SEQ ID N° 19 | Rv 0418 | * | site de fixation de lipo-protéine membranaire procaryote, similarité avec la N-acétyl puromycyne acétyl hydrolase |
| SEQ ID N° 20 | Rv 3576 | * | contient un site de fixation de lipoprotéine membranaire procaryote, similarité avec une sérine/thréonine protéine kinase |
| SEQ ID N° 6 | | | |
| SEQ ID N° 21 | Rv 3365c | ML | similarité avec une métallo peptidase à zinc |
| SEQ ID N° 31 | non prédite | | pas de prédiction |
| | | | |
| SEQ ID N° 32 | Rv 0822c | ML | Existence d'une région consensus avec la famille drac |
| SEQ ID N° 33 | Rv 1044 | | pas de prédiction |
| SEQ ID N° 34 | non prédite | | pas de prédiction |
| SEQ ID N° 35 | Rv 2169c | | pas de prédiction |
| SEQ ID N° 36 | Rv 3909 | ML | pas de prédiction |
| SEQ ID N° 37 | Rv 2753c | | similarité avec des dihydropricolinate synthases |
| SEQ ID N° 38 | Rv 0175 | | pas de prédiction |
| SEQ ID N° 39 | Rv 3006 | * ML | prédiction de séquence signal de lipoprotéine |
| SEQ ID N° 40 | Rv 0549c | | pas de prédiction |
| SEQ ID N° 41 | Rv 2975c pouvant être en phase avec Rv 2974c | | similarité avec protéine de substilis |
| SEQ ID N° 42 | Rv 2622 | | similarité avec une méthyl transférase |
| SEQ ID N° 43 | Rv 3278c | ML | pas de prédiction |
| SEQ ID N° 44 | Rv 0309 | * | pas de prédiction |
| SEQ ID N° 45 | Rv 2169c | ML | pas de prédiction |
| SEQ ID N° 46 | Rv 1411c | * | probable lipoprotéine avec une séquence signal N-terminale |
| SEQ ID N° 47 | Rv 1714 | | similarité avec une gluconate 3-déhydrogénase |
| SEQ ID N° 48 | Rv 0331 | | similarité avec une sulfide déhydrogénase et une sulfide quinone réductase |
| SEQ ID N° 49 | Rv 0983 | ML | Similarité avec une sérine protéase HtrA |
| SEQ ID N° 5 | | | |
| SEQ ID N° 16 | Rv 3810 | * ML | Protéine de surface Berthelet et al. 1995 |
| SEQ ID N° 22 | | * | Contient un site de fixation de lipoprotéine membranaire eucaryote |
| SEQ ID N° 23 | Rv 3763 | | |
| SEQ ID N° 24 | | | |
| SEQ ID N°50 | Rv 0125 | * | Site actif des sérines protéases Séquence signal N-terminale possible |

| | | | |
|---|---|---|---|
| Légende du tableau 3 : Correspondance des séquences selon l'invention avec les séquences prédites par Cole et al. 1998, Nature, 393, 537-544. * : Prédiction que la protéine codée par la séquence soit exportée. ML : Prédiction de similarité avec *M. leprae.* | | | |

### Exemple 6 :

### Caractéristiques et obtention de la protéine M1C25

L'extrémité N terminale de la protéine M1C25 a été détectée par le système *PhoA* comme permettant l'exportation de la protéine de fusion, nécessaire à l'obtention de son activité phosphatase.

La séquence d'ADN codant pour l'extrémité N terminale de la protéine M1C25 est contenue dans la séquence SEQ ID N° 20 de la présente demande de brevet.

A partir de cette séquence amorce, le gène complet codant pour la protéine M1C25 a été recherché dans le génome de *M. tuberculosis* (Fondation Welcome Trust, site Sanger).

Le centre Sanger a attribué à M1C25 les noms :
Rv3576,
MTCY06G11.23,
pknM.

### Séquence SEQ ID N° 29 du gène complet M1C25 (714 bases) : cf. Figure 29

Ce gène code pour une protéine de 237 AA, de 25 kDa de masse molaire. Cette protéine est référencée dans les banques sous les appellations :
PID:e306716,
SPTREMBL:P96858

### Séquence SEQ ID N° 30 de la protéine M1C25 (237 acides aminés) : cf. Figure 30

M1C25 contient un site de fixation à la partie lipidique des lipoprotéines de membrane des procaryotes (PS00013 Prokaryotic membrane lipoprotein lipid attachment site:
CTGGTCGGTG CGTGCATGCT CGCAGCCGGA TGC).

La fonction de M1C25 n'est pas certaine mais elle possède très probablement une activité « sérine/thréonine-protéine kinase ». Des ressemblances sont à noter avec la moitié C terminale de K08G_MYCTU Q11053 Rv1266c (MTCY50.16). Des similarités sont aussi retrouvées avec KY28_MYCTU.

En 5' du gène codant pour M1C25 se trouve un gène codant potentiellement pour une protéine régulatrice (PID:e306715, SPTREMBL:P96857, Rv3575c, (MTCY06G11.22c)).

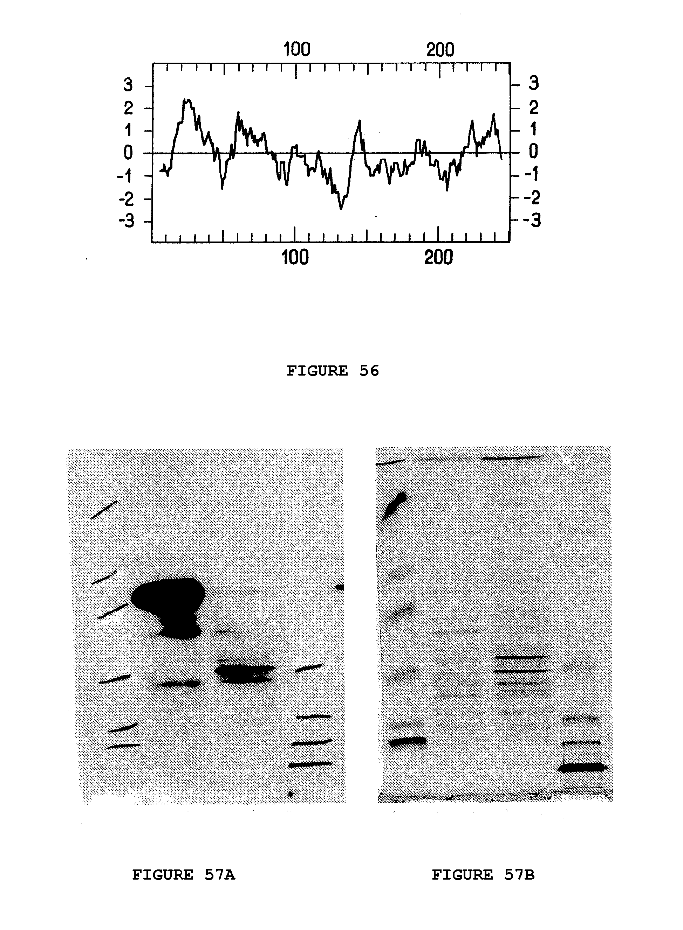

Le profil d'hydrophobicité (Kyte et Doolitle) de M1C25 est représenté à la figure 56.

Un site de clivage de la séquence signal est prédit (SignalP V1.1 ; World Wide Web Prediction Server, Center for Biological Sequence Analysis) entre les acides aminés 31 et 32: AVA-AD. Ce site de coupure est derrière un motif « AXA » classique. Cette prédiction est compatible avec le profil d'hydrophobicité. Dans cette séquence signal potentielle il est à remarquer la répétition trois fois de la séquence des trois acides aminés LAA.
Clonage du gène M1C25 en vue de la production de la protéine qu'il code :
Un couple d'amorces a été synthétisé afin d'amplifier, à partir de l'ADN génomique de M. tuberculosis, souche H37Rv, la totalité de la séquence codant pour le polypeptide M1C25. L'amplicon obtenu a été cloné dans un vecteur d'expression.
Des couples d'amorces appropriés pour l'amplification et le clonage de la séquence codant pour M1C25 ont été synthétisés :

- amorce aller :
   5' -ATAATACCATGGGCAAGCAGCTAGCCGCGC- 3'
- amorce retour :
   5' -ATTTATAGATCTCTGCTTAGCAACCTTGGCCGCG- 3'

La partie soulignée représente les séquences hybridant spécifiquement avec la séquence M1C25 et les extrémités 5' correspondent à des sites de restriction en vue du clonage de l'amplicon résultant dans un vecteur de clonage et/ou d'expression.

Un vecteur particulier utilisé pour l'expression du polypeptide M1C25 est le vecteur pQE60 commercialisé par la société Qiagen, en suivant le protocole et les recommandations proposés par cette marque.

Les cellules utilisées pour le clonage sont des bactéries : *E. coli* XL1-Blue (résistante à la tétracycline).

Les cellules utilisées pour l'expression sont des bactéries : *E. coli* M15 (résistante à la kanamycine) contenant le plasmide pRep4 (M15 pRep4).

La production de la protéine MC25 est illustrée par les figures 57 A et B (Extraits bactériens de la souche E. coli M15 contenant le plasmide pM1C25). Les cultures bactériennes et les extraits sont préparés selon Sambrook et al. (1989). L'analyse des extraits bacrériens est effectuée selon les instructions de Quiagen (1997).

### Références bibliographiques

AIDS thérapies, 1993, in Mycobacterial infections, ISBN 0-9631698-1-5, pp. 1-11.
Altschul, S.F. et al., 1990, J. Mol. Biol., 215 : 403-410.
Andersen, P. et al., 1991, Infect. Immun., 59 :1905-1910.
Andersen, P. et al., 1995, J. Immunol., 154, 3359-3372.
Bange, F.C., A.M. Brown, and W.R. Jacobs JR., 1996, Leucine auxotrophy restricts growth of Mycobacterium bovis BCG in macrophages. Infect. Immun., 64:1794-1799.
Barany, F., 1911, Proc. Natl. Acad. Sci. USA, 88:189-193.
Bates, J. et al., 1986, Am. Rev. Respir. Dis., 134 :415-417.
Bates, J. 1979. Chest. 76(Suppl.):757-763.
Bates, J. et al. 1986. Am. Rev. Respir. Dis. 134:415-417.
Berthet, F.X., J. Rauzier, E.M. Lim, W. Philipp, B. Gicquel, and D. Portnoï, 1995, Characterization of the M. tuberculosis erp gene encoding a potential cell surface protein with repetitive structures. Microbiology. In press
Borremans, M. et al., 1989, Biochemistry, 7:3123-3130.
Bouvet, E. 1994. Rev. Fr. Lab. 273:53-56.
Brockman, R.W. and Heppel L.A., 1968, On the localization of alkaline phosphatase and cyclic phosphodiesterase in Escherichia coli, Biochemistry, 7:2554-2561.
Burg, J.L. et al., 1996, Mol. and Cell. Probes, 10 :257-271.
Chevrier, D. et al., 1993, Mol. and Cell. Probes, 7:187-197.
Clemens, D.L., 1996, Characterization of the Mycobacterium tuberculosis phagosome, Trends Microbiol., 4:113-118.
Chu ,B.C.F. et al., 1986, Nucleic Acids Res., 14:5591-5603.
Clemens, D.L. and Horwitz M.A., 1995, Characterization of the Mycobacterium tuberculosis phagosome and evidence that phagosomal maturation is inhibited, J. Exp. Med., 181:257-270.
Colignon J.E., 1996. Immumologic studies in humans. Measurement of proliferative responses of culturered lymphocytes. Current Protocols in Immunology, NIH, 2, Section II.
Daniel, T.M. et al. 1987. Am. Rev. Respir. Dis., 135:1137-1151).
Dellagostin, O.A., Esposito G., Eales L.-J., Dale J.W. and. McFadden J.J., 1995, Activity of mycobacterial promoters during intracellular and extracellular growth. Microbiol., 141 : 2123-2130.
Drake, T.A. et al. 1987. J. Clin. Mocrobiol. 25:1442-1445.
Dramsi et al., 1997, Infection and Immunity, 65, 5:1615-1625.
Duck, P. et al., 1990, Biotechniques, 9:142-147.
Erlich, H.A. 1989. In PCR Technology. Principles and Applications for DNA Amplification. New York: Stockton Press.
Felgner et al., 1987, Proc. Natl. Acad. Sci., 84:7413.
Fraley et al., 1980, J. Biol. Chem., 255:10431.
Gaillard, J.L., Berche P., Frehel C., Gouin E. and Cossart P., 1991, Entry of L. monocytogenes into cells is mediated by internalin, a repeat protein reminiscent of surface antigens from Gram-positive cocci, Cell., 65:1127-1141.
Garbe, T., Harris D., Vordermeir M., Lathigra R., Ivanyi J. and Young D., 1993, Expression of the Mycobacterium tuberculosis 19-kilodalton antigen in Mycobacterium smegmatis: immunological analysis and evidence of glycosylation, Infect. Immun., 61:260-267.
Guateli, J.C. et al., 1990, Proc. Natl. Acad. Sci. USA, 87:1874-1878.
Harboe et al., 1996, Infect. Immun., 64, 16-22.
Herrmann, J.L., O'Gaora P., Gallagher A., Thole J.E.R. and Young D.B., 1996, Bacterial glycoproteins: a link between glycosylation and proteolytic cleavage of a 19 kDa antigen from Mycobacterium tuberculosis, EMBO J. 15:3547-3554.
Houbenweyl, 1974, in Meuthode der Organischen Chemie, E. Wunsch Ed., Volume 15-I et 15-II, Thieme, Stuttgart.
Huygen, K. et al., 1996, Nature Medicine, 2(8):893-898.
Innis, M.A. et al. 1990. in PCR Protocols. A guide to Methods and Apllications. San Diego: Academic Press.
Isberg, R.R., Voorhis D.L. and Falkow S., 1987, Identification of invasin: a protein that allows enteric bacteria to penetrate cultured mammalian cells, Cell, 50:769-778.
Jacobs, W.R. et al., 1991. Construction of mycobacterial genomic libraries in shuttle cosmids. Genetic Systems for Mycobacteria, Methods in Enzymology, 204:537-555.
Jacobs, W.R. et al., 1993, Science, 260:819-822.
Kaneda, et al., 1989, Science, 243:375.
Kiehn, T.E., et al. 1987. J. Clin. Microbiol. 25:1551-1552.
Kievitis ,T. et al., 1991, J. Virol. Methods, 35:273-286.
Kohler, G. et al., 1975, Nature, 256(5517):495-497.
Kwoh, D.Y. et al., 1989, Proc. Natl. Acad. Sci. USA, 86:1173-1177.
Landegren ,U. et al., 1988, Science, 241:1077-1080.
Lang, T. and Antoine J.-C., 1991, Localization of MHC classII molecules in murine bone marrow-derived macrophages. Immunology, 72:199-205.
Lee, B.Y. and Horwitz M.A., 1995, Identification of macrophage and stress-induced proteins of Mycobacterium tuberculosis, J. Clin. Invest., 96:245-249.
Lim, E.M., Rauzier J., Timm J., Torrea G., Murray A., Gicquel B. and Portnoï D., 1995, Identification of Mycobacterium tuberculosis DNA sequences encoding exported proteins, using phoA gene fusions, J. Bacteriol., 177:59-65.
Lizardi, P.M. et al., 1988, Bio/technology, 6:1197-1202.
Mahan, M.J. et al., 1993. Selection of bacterial virulence genes that are specifically induced in host tissues, Science, 259:686-688.
Manoil L., Mekolanos J.J. and Beckwith J., J. Bacteriol., 1990, 172, 515-518.
Matthews, J.A. et al., 1988, Anal. Biochem., 169:1-25.
Merrifield, R.D., 1966, J. Am. Chem. Soc., 88(21):5051-5052.
Midoux, 1993, Nucleic Acids Research, 21:871-878/
Miele, E.A. et al., 1983, J. Mol. Biol., 171:281-295.
Minton, N.P., 1984, Gene, 31, 269-273.
Montgomery et al., 1993, DNA Cell Biol., 12:777-783.
Navarre,W.W.et al., 1994, Molecular Microbiologie, 14(1): 115-121.
Navarre,W.W.et al., 1996, J. of Bacteriology, 178, 2:441-446.
Pagano et al., 1967, J. Virol., 1:891.
Pastore, 1994, Circulation, 90:I-517.
Patel, et al. 1990. J. Clin. Microbiol.:513-518.
Prentki, B. et Krish H.M., 1984, Gene, 29:303-313.
Pettersson R., Nordfelth J., Dubinina E., Bergman T., Gustafsson M., Magnusson K.E. and Wolf-Watz H., 1996, Modulation of virulence factor expression by pathogen target cell contact. Science., 273:1231-1233.
Plum, G. and Clark-Curtiss J.E., 1994, Induction of Mycobacterium avium gene expression following phagocytosis by human macrophages. Infect. Immun., 62:476-483.
Roberts, M.C., et al. 1987. J. Clin. Microbiol. 25:1239-1243.
Rolfs, A. et al. 1991. In PCR Topics. Usage of Polymerase Chain reaction in Genetic and Infectious Disease. Berlin: Springer-Verlag.
Sambrook, J. et al. 1989. In Molecular cloning:A Laboratory Manual. Cold Spring Harbor, NY: Cold Spring Harbor Laboratory Press.
Sanchez-Pescador, R., 1988, J. Clin. Microbiol., 26(10):1934-1938.
Schneewind, O. et al., 1995, Science, 268:103-106.
Segev D., 1992, in « Non-radioactive Labeling and Detection of Biomolecules ». Kessler C. Springer Verlag, Berlin, New-York, 197-205.
Servant, P. and Mazodier P., 1995, Characterization of Streptomyces albus 18-kilodalton heat shock-responsive protein. J. Bacteriol., 177:2998-3003.
Shiver, J.W., 1995, in Vaccines 1995, eds Chanock, R.M. Brown, F. Ginsberg, H.S. & Norrby, E.), pp.95-98, Cold Spring Harbor Laboratory Press, Cold Spring Harbor, New York.
Sorensen et al., 1995, Infect. Immun., 63, 1710-1717.
Stone, B.B. et al., 1996, Mol. and Cell. Probes, 10:359-370.
Stover, C.K., Bansal G.P., Hanson M.S., Burlein S.R., Palaszynski S.R., Young J.F., Koenig S., Young D.B., Sadziene A. and Barbour A.G., 1993, Protective immunity elecited by recombinant Bacille Calmette-Guerin (BCG) expressing outer surface protein A (OspA) lipoprotein: a candidate Lyme disease vaccine. J. Exp. Med., 178:197-209.
Sturgill-Koszycki, S., Schlesinger P.H., Chakroborty P., Haddix P.L., Collins H.L., Fok A.K., Allen R.D., Gluck S.L., Heuser J. and Russell D.G., 1994, Lack of acidification in Mycobacterium phagosomes by exclusion of the vesicular proton-ATPase. Science., 263:678-681.
Tascon, R.E et al.., 1996, Nature Medicine, 2(8):888-892.
Technique assemblage oligonucléotides, 1983, Proc. Natl. Acad. Sci. USA, 80:7461-7465.
Technnique des béta-cyanethylphosphoramidites, 1986, Bioorganic Chem., 4:274-325.
Thierry, D. et al., 1990, Nucl. Acid Res., 18:188.
Timm, J., Perilli M.G., Duez C., Trias J., Orefici G., Fattorini L., Amicosante G., Oratore A., Boris B., Frere J.M., Pugsley A.P. and Gicquel B., 1994, Transcription and expression analysis, using lacZ and phoA gene fusions, of Mycobacterium fortuitum B-lactamase genes cloned from a natural isolate and a high-level B-lactamase producer. Mol. Microbiol., 12:491-504.
Tuberculosis Prévention Trial, 1980, Mendis, « Trial of BCG vaccines in South India for Tuberculosis Infection », Indian Journal of Medical research, 1972 (Suppl.):1-74.
Urdea, M.S. et al., 1991, Nucleic Acids Symp. Ser., 24:197-200.
Urdea, M.S., 1988, Nucleic Acids Research, 11:4937-4957.
Verbon, A., Hartskeerl R.A., Schuitema A., Kolk A.H., Young D.B. and Lathigra R., 1992, The 14,000-molecular-weight antigen of Mycobacterium tuberculosis is related to the alpha-crystallin family of low-molecular-weight heat shock proteins. J Bacteriol.,174:1352-1359.
Walker, G.T. et al., 1992, Nucleic Acids Res., 20:1691-1696.
Walker, G.T. et al., 1992, Proc. Natl. Acad. Sci. USA, 89:392-396.
Wiker, H.G. et al., 1992, Microbiol. Rev., 56:648-661.
Yamaguchi, R. et al., 1989, Infect. Immun., 57:283-288.
Xu, S., Cooper A., Sturgill-Koszycki S., van Heyningen T., Chatterjee D., Orme I., Allen P. and Russel D.G., 1994, Intracellular trafficking in Mycobacterium tuberculosis and Mycobacterium avium-infected macrophages, J. Immuno., 153:2568-2578.
Young, D.B. et al., 1992, Mol. Microbiol., 6:133-145.
Yuen, L.K.W. et al., 1993, J. Clin. Microbiol., 31:1615-1618.

## Revendications

1. Polynucléotide **caractérisé en ce qu'**il comprend un polynucléotide choisi parmi :
a) un polynucléotide dont la séquence est choisie parmi les séquences nucléotidiques SEQ ID N° 1 et SEQ ID N° 2, telles que représentées à la figure 1 et à la figure 2,
b) un polynucléotide dont la séquence nucléique est la séquence comprise entre le nucléotide en position nt 964 et le nucléotide en position nt 1234, extrémités incluses, de la séquence SEQ ID N° 1, ou en position nt 941 et le nucléotide en position nt 1351, extrémités incluses de la séquence SEQ ID N° 2,
c) un polynucléotide dont la séquence est complémentaire de la séquence d'un polynucléotide défini en a) ou b),
d) un polynucléotide dont la séquence comporte au moins 50 % d'identité avec un polynucléotide défini en a), b) ou c),
e) un fragment d'au moins 12 nucléotides consécutifs d'un polynucléotide défini en a), b) ou c).

2. Polypeptide **caractérisé en ce qu'**il est codé par une séquence polynucléotidique selon la revendication 1.

3. Polypeptide **caractérisé en ce qu'**il comprend un polypeptide choisi parmi :
a) un polypeptide dont la séquence d'acides aminés est une séquence d'acides aminés choisie parmi les séquences d'acides aminés SEQ ID N° 1, SEQ ID N° 2 et SEQ ID N° 28,
b) un fragment biologiquement actif d'un polypeptide défini en a) ayant au moins 5 acides aminés et capable d'être exporté et/ou sécrété par une mycobactérie, et/ou d'être induit ou réprimé lors de l'infection par la mycobactérie ; et /ou
- capable d'induire, de réprimer ou de moduler directement ou indirectement, un facteur de virulence de mycobactérie ; et /ou
- capable d'induire une réaction d'immunogénicité dirigée contre les mycobactéries ; et /ou
- capable d'être reconu par un anticorps spécifique de mycobactérie.

4. Un polynucléotide purifié **caractérisé en ce qu'**il encode un polypeptide selon l'une quelconque des revendications 2 et 3.

5. Séquence d'acide nucléique utilisable comme amorce ou comme sonde ayant au moins 12 nucléotides, **caractérisée en ce que** ladite séquence est choisie parmi les séquences d'acide nucléique de polynucléotide selon l'une des revendications 1 et 4.

6. Séquence d'acide nucléique selon la revendication 5, **caractérisée en ce que** ladite séquence est choisie parmi les séquences SEQ ID N° 25 et SEQ ID N° 26.

7. Séquence d'acide nucléique selon la revendication 5 ou 6, **caractérisée en ce qu'**elle est marquée par un composé radioactif ou par un composé non radioactif

8. Vecteur recombinant de clonage, d'expression et/ou d'insertion, **caractérisé en ce qu'**il contient un polynucléotide selon l'une des revendications 1 et 4.

9. Cellule hôte, **caractérisée en ce qu'**elle est transformée par un vecteur recombinant selon la revendication 7.

10. Cellule hôte selon la revendication 9, **caractérisée en ce qu'**il s'agit de la souche de *E. coli* transformée par le plasmide pDP428 déposé le 28 janvier 1997 à la CNCM sous le N° I-1818 ou d'une souche de *M. tuberculosis, M. bovis* ou *M. africanum* possédant potentiellement tous les systèmes de régulation appropriés.

11. Procédé de préparation d'un polypeptide, **caractérisé en ce qu'**il met en oeuvre un vecteur selon la revendication 8.

12. Polypeptide hybride, **caractérisé en ce qu'**il comporte au moins la séquence d'un polypeptide selon l'une des revendications 2 et 3 et une séquence d'un polypeptide susceptible d'induire une réponse immunitaire chez l'homme ou l'animal.

13. Procédé pour la détection *in vitro* d'anticorps dirigés contre une mycobactérie et préférentiellement une bactérie du complexe *Mycobacterium tuberculosis* dans un échantillon biologique, **caractérisé en ce qu'**il comprend les étapes suivantes :
a) mise en contact de l'échantillon biologique avec un polypeptide selon l'une des revendications 2 et 3 ;
b) mise en évidence du complexe antigène-anticorps formé.

14. Procédé pour la détection d'une infection par une mycobactérie et préférentiellement une bactérie du complexe *Mycobacterium tuberculosis* dans un mammifère, **caractérisé en ce qu'**il comprend les étapes suivantes :
a) préparation d'un échantillon biologique contenant des cellules dudit mammifère plus particulièrement des cellules du système immunitaire dudit mammifère et plus particulièrement encore des cellules T ;
b) incubation de l'échantillon biologique de l'étape a) avec un polypeptide selon l'une des revendications 2 et 3 ;
c) détection d'une réaction cellulaire indiquant une sensibilisation préalable du mammifère audit polypeptide notamment la prolifération cellulaire et/ou la synthèse de protéines telles que l'interféron gamma ;
d) détection d'une réaction d'hypersensibilité retardée ou de sensibilisation du mammifère audit polypeptide.

15. Kit pour le diagnostic *in vitro* d'une infection par une mycobactérie appartenant au complexe *Mycobacterium tuberculosis,* comprenant :
a) un polypeptide selon l'une des revendications 2 et 3 ;
b) le cas échéant, les réactifs pour la constitution du milieu propice à la réaction immunologique ;
c) les réactifs permettant la détection des complexes antigène-anticorps produits par la réaction immunologique ;
d) le cas échéant, un échantillon biologique de référence (témoin négatif) dépourvu d'anticorps reconnus par ledit polypeptide ;
e) le cas échéant, un échantillon biologique de référence (témoin positif) contenant une quantité prédéterminée d'anticorps reconnus par ledit polypeptide.

16. Anticorps mono- ou polyclonaux, leurs fragments, ou anticorps chimériques, **caractérisés en ce qu'**ils sont capables de reconnaître spécifiquement un polypeptide selon l'une des revendications 2 et 3.

17. Procédé pour la détection spécifique de la présence d'un antigène d'une bactérie du complexe *Mycobacterium tuberculosis* dans un échantillon biologique, **caractérisé en ce qu'**il comprend les étapes suivantes :
a) mise en contact de l'échantillon biologique avec un anticorps selon la revendication 16 ;
b) mise en évidence du complexe antigène-anticorps formé.

18. Kit pour la détection spécifique de la présence d'un antigène d'une bactérie du complexe *Mycobacterium tuberculosis* dans un échantillon biologique, **caractérisé en ce qu'**il comprend les éléments suivants :
a) un anticorps polyclonal ou monoclonal selon la revendication 16 ;
b) les réactifs pour la constitution du milieu propice à la réaction immunologique ;
c) les réactifs permettant la détection des complexes antigène-anticorps produits par la réaction immunologique.

19. Procédé de détection et d'identification rapide d'une mycobactérie et préférentiellement de *M. tuberculosis* dans un échantillon biologique, **caractérisé en ce qu'**il comporte les étapes suivantes :
a) isolement de l'ADN à partir de l'échantillon biologique à analyser, ou obtention d'un ADNc à partir de l'ARN de l'échantillon biologique ;
b) amplification spécifique de l'ADN des mycobactéries appartenant au complexe *Mycobacterium tuberculosis* à l'aide d'amorces selon l'une des revendications 5 à 7 ;
c) analyse des produits d'amplification.

20. Procédé pour la détection de bactéries appartenant au complexe *Mycobacterium tuberculosis* dans un échantillon biologique, **caractérisé en ce qu'**il comprend les étapes suivantes :
a) mise en contact d'une sonde oligonucléotidique selon l'une des revendications 5 à 7 avec un échantillon biologique, l'ADN contenu dans l'échantillon biologique ayant, le cas échéant, préalablement été rendu accessible à l'hybridation, dans des conditions permettant l'hybridation de la sonde à l'ADN d'une bactérie du complexe *Mycobacterium tuberculosis* ;
b) détection de l'hybride formé entre la sonde oligonucléotidique et l'ADN de l'échantillon biologique.

21. Kit pour la détection de la présence d'une bactérie du complexe *Mycobacterium tuberculosis* dans un échantillon biologique, **caractérisé en ce qu'**il comprend les éléments suivants :
a) une sonde oligonucléotidique ou un couple d'amorces selon l'une des revendications 5 à 7 ;
b) les réactifs nécessaires à la mise en oeuvre d'une réaction d'hybridation ou d'amplification d'ADN.

22. Composition immunogène **caractérisée en ce qu'**elle comprend un ou plusieurs polypeptides selon l'une des revendications 2 et 3.

23. Vaccin **caractérisé en ce qu'**il contient un ou plusieurs polypeptides selon l'une des revendications 2 et 3, en association avec un véhicule pharmaceutiquement compatible et, le cas échéant un ou plusieurs adjuvants de l'immunité appropriés.
